# EUROPEAN PATENT APPLICATION

(11) **EP 3 020 828 A1**
(43) Date of publication of application: **18.05.2016**
(21) Application number: 14822572.5
(22) Date of filing: 11.07.2014
(51) Int. Cl.: C12Q 1/68, A61K 45/00, A61P 35/00, G01N 33/15, G01N 33/50

(54) **METHOD OF PREDICTING RESPONSE OF CANCER TO TREATMENT**

(30) Priority: 12.07.2013 JP 2013147061
(71) Applicant: National Cancer Center Research Institute, Tokyo 104-0045 (JP); Daiichi Sankyo Company, Limited, Tokyo 103-8426 (JP)
(72) Inventor: KOHNO Takashi, Tokyo 104-0045 (JP); TSUTA Koji, Tokyo 104-0045 (JP); TOMINAGA Yuichi, Tokyo 140-8710 (JP); ITO Kentaro, Tokyo 140-8710 (JP)
(74) Representative: Duckworth, Timothy John
(86) International application number: PCT/JP2014/068572
(87) International publication number: WO 2015/005473

(57) **Abstract**

It has been found that BRG1 and BRM have a synthetic-lethal relationship, and that a treatment for inhibiting BRM is a promising approach for treating a BRG1-deficient cancer having no mutation in known therapeutic target genes. Moreover, it has also been revealed that, in this therapeutic strategy, a BRM inhibitor may be administered to a cancer patient after the selection based on BRG1 function suppression, thus enabling an efficient treatment based on the companion diagnosis.

SEQUENCE LISTING

<110>  NATIONAL CANCER CENTER
       DAIICHI SANKYO COMPANY, LIMITED
<120>  METHOD FOR PREDICTING RESPONSE TO CANCER TREATMENT
<130>  IBPF14-519WO
<150>  JP 2013-147061
<151>  2013-07-12
<160>  8
<170>  PatentIn version 3.1
<210>  1
<211>  77665
<212>  DNA
<213>  Homo sapiens
<220>
<221>  CDS
<222>  (1)..(222)
<223>
<220>
<221>  CDS
<222>  (1122)..(1254)
<223>
<220>
<221>  CDS
<222>  (2038)..(2442)
<223>
<220>
<221>  CDS
<222>  (2754)..(2852)
<223>
<220>
<221>  CDS
<222>  (3515)..(3773)
<223>
<220>
<221>  CDS
<222>  (5166)..(5292)
<223>
<220>
<221>  CDS
<222>  (6999)..(7172)
<223>
<220>
<221>  CDS
<222>  (10677)..(10850)
<223>
<220>
<221>  CDS
<222>  (12062)..(12229)
<223>
<220>
<221>  CDS
<222>  (12343)..(12393)
<223>
<220>
<221>  CDS
<222>  (18878)..(19008)
<223>
<220>
<221>  CDS
<222>  (19189)..(19246)
<223>
<220>
<221>  CDS
<222>  (23751)..(23872)
<223>
<220>
<221>  CDS
<222>  (26230)..(26380)

<223>
<220>
<221>  CDS
<222>  (28798)..(28961)
<223>
<220>
<221>  CDS
<222>  (34806)..(34872)
<223>
<220>
<221>  CDS
<222>  (35440)..(35550)
<223>
<220>
<221>  CDS
<222>  (37574)..(37816)
<223>
<220>
<221>  CDS
<222>  (39367)..(39480)
<223>
<220>
<221>  CDS
<222>  (40180)..(40287)
<223>
<220>
<221>  CDS
<222>  (41271)..(41357)
<223>
<220>
<221>  CDS
<222>  (42149)..(42195)
<223>
<220>
<221>  CDS
<222>  (43633)..(43799)
<223>
<220>
<221>  CDS
<222>  (46579)..(46742)
<223>
<220>
<221>  CDS
<222>  (49139)..(49366)
<223>
<220>
<221>  CDS
<222>  (49616)..(49714)
<223>
<220>
<221>  CDS
<222>  (49972)..(50049)
<223>
<220>
<221>  CDS
<222>  (50763)..(50981)
<223>
<220>
<221>  CDS
<222>  (57156)..(57409)
<223>
<220>
<221>  CDS
<222>  (74104)..(74212)
<223>
<220>
<221>  CDS
<222>  (74637)..(74738)
<223>

<220>
<221>  CDS
<222>  (75602)..(75734)
<223>
<220>
<221>  CDS
<222>  (75894)..(76036)
<223>
<220>
<221>  CDS
<222>  (77633)..(77662)
<223>
<400>  1
atg tcc act cca gac cca ccc ctg ggc gga act cct cgg cca ggt cct      48
Met Ser Thr Pro Asp Pro Pro Leu Gly Gly Thr Pro Arg Pro Gly Pro
1               5                   10                  15
tcc ccg ggc cct ggc cct tcc cct gga gcc atg ctg ggc cct agc ccg      96
Ser Pro Gly Pro Gly Pro Ser Pro Gly Ala Met Leu Gly Pro Ser Pro
            20                  25                  30
ggt ccc tcg ccg ggc tcc gcc cac agc atg atg ggg ccc agc cca ggg      144
Gly Pro Ser Pro Gly Ser Ala His Ser Met Met Gly Pro Ser Pro Gly
        35                  40                  45
ccg ccc tca gca gga cac ccc atc ccc acc cag ggg cct gga ggg tac      192
Pro Pro Ser Ala Gly His Pro Ile Pro Thr Gln Gly Pro Gly Gly Tyr
    50                  55                  60
cct cag gac aac atg cac cag atg cac aag gtagggatcc ctgtgcccgc      242
Pro Gln Asp Asn Met His Gln Met His Lys
65                  70
ctcgcacctg cggcctctgc ccactagggc tgcaggcagc ctctggaccg agggccttac      302
ttggaggatg gggggaagcc ttcttgttgg aggtgtcctg ccttggctca gccccctacc      362
ccagggccca cggccatgaa cagaaggttc agctcgtcag accccagcct gtgctggcgc      422
atgatctggg ccccgcgggc acctgcccca ccgtttcccg ctcccttgct ttctgcatgt      482
gaaatttggg aatatcacta caaagttttc ttttggtaat gaagaaaaac atccaaattc      542
ctaaaaaggc ttttagccac gtggtttccc ccgttctggc tgtcaggctc acctgtgcag      602
ctcgcagagc cgagcagcgg gttccctttc ctccaaggcg tgcccctcag ccactgtctt      662
tacctcacct gcgctgagca gggccttcag tcagcaaggt ggagaaggct ctttatggtc      722
acaagtctct tttgcatgtc cctggatgtg gttagaggac atattccctt atacatacag      782
catctgaaag gaaaggtcat attagcatcc atgagaaaag gtgacatttc taagtgtcac      842
gggccacagg aggtcccggg tggtagaaga tgaggattgc ttgaccacag tctcccatat      902
gctcgtgctc cactgggcga catttatttt gtgtacctgt ctcgaggcct aagtaggtgt      962
cagaaccttg ccttggagtc atgctgggga ctggggagat gcgcgtcatc ttcgggtggc      1022
tgttctcggt gccctcgagc ttctctcggg cagcgcatag ctgcgctgcc acctcacgtt      1082
ccacatgctg accctgcctt gccatggtcc ctctcgcag ccc atg gag tcc atg      1136
                                          Pro Met Glu Ser Met
                                          75
cat gag aag ggc atg tcg gac gac ccg cgc tac aac cag atg aaa gga      1184
His Glu Lys Gly Met Ser Asp Asp Pro Arg Tyr Asn Gln Met Lys Gly
80                  85                  90                  95
atg ggg atg cgg tca ggg ggc cat gct ggg atg ggg ccc ccg ccc agc      1232
Met Gly Met Arg Ser Gly Gly His Ala Gly Met Gly Pro Pro Pro Ser
                100                 105                 110
ccc atg gac cag cac tcc caa g gtacagaact gcgttccttc ctgccttgtg      1284
Pro Met Asp Gln His Ser Gln
            115
tttgtcatac tccagagtcc tcagatcatt ttcctcccct gggttcccac aggatggatt      1344
cagggagtac ctaggatgat gtagccgggt gggtggcccg ccacagagag ctgtctggca      1404
tggcgtggct ggtgctctgt tctggccacc tcgtctcgga gcagccctgg aaggggaaat      1464
gctggttggg gggcagacca tgttcagcat gggtgataga ggagggctgt gcagggcagc      1524
agccccgtgc cacccaggaa cttactggaa atacagattc ctggggcctg ggactctgct      1584
ttaatgagcc cagcaggtgc cttttctgta cgaggacaat tgagaagtga tatggtgggt      1644
gactttccaa ggccttgctg tcatcagggg tgggaagctc aggggctgca ctgggaatgg      1704
aagtgttggg ggtgggcctg gcgagcccga ggatgtggac cccgcctgtg gacaggcagg      1764
cggaagccgg gagagagccg tgatttcatg cacaggttct cagcattaac cagaagcacc      1824
ttactgagac gtgggccaaa ccaaatccac caggtcggct gctgggctga ggtgccttca      1884
gcatgtgccc tccaggtgcc cctggttgac tcaagagaag gatgccattt ggctgtgccc      1944
tgtctcagag taggagctgg tgtaggggga agaggctgta aaaatcacag acatatgctg      2004
ccgagtgacc agtgggctga cctttctctg cag gt  tac ccc tcg ccc ctg ggt      2057
                                     Gly Tyr Pro Ser Pro Leu Gly
                                         120                 125

ggc tct gag cat gcc tct agt cca gtt cca gcc agt ggc ccg tct tcg     2105
Gly Ser Glu His Ala Ser Ser Pro Val Pro Ala Ser Gly Pro Ser Ser
                130                 135                 140
ggg ccc cag atg tct tcc ggg cca gga ggt gcc ccg ctg gat ggt gct     2153
Gly Pro Gln Met Ser Ser Gly Pro Gly Gly Ala Pro Leu Asp Gly Ala
            145                 150                 155
gac ccc cag gcc ttg ggg cag cag aac cgg ggc cca acc cca ttt aac     2201
Asp Pro Gln Ala Leu Gly Gln Gln Asn Arg Gly Pro Thr Pro Phe Asn
        160                 165                 170
cag aac cag ctg cac cag ctc aga gct cag atc atg gcc tac aag atg     2249
Gln Asn Gln Leu His Gln Leu Arg Ala Gln Ile Met Ala Tyr Lys Met
    175                 180                 185
ctg gcc agg ggg cag ccc ctc ccc gac cac ctg cag atg gcg gtg cag     2297
Leu Ala Arg Gly Gln Pro Leu Pro Asp His Leu Gln Met Ala Val Gln
190                 195                 200                 205
ggc aag cgg ccg atg ccc ggg atg cag cag cag atg cca acg cta cct     2345
Gly Lys Arg Pro Met Pro Gly Met Gln Gln Gln Met Pro Thr Leu Pro
                210                 215                 220
cca ccc tcg gtg tcc gca aca gga ccc ggc cct ggc cct ggc cct ggc     2393
Pro Pro Ser Val Ser Ala Thr Gly Pro Gly Pro Gly Pro Gly Pro Gly
            225                 230                 235
ccc ggc ccg ggt ccc ggc ccg gca cct cca aat tac agc agg cct cat g   2442
Pro Gly Pro Gly Pro Gly Pro Ala Pro Pro Asn Tyr Ser Arg Pro His
        240                 245                 250
gtaagactgg ctgccctggc cctcaggtgt ctcagagcga atggctgggg cgtgggtggc   2502
ggggtggaca gaccgtgctc tgttgccgac tgggttcccc ggtttgggat tgcacgggcc    2562
catactgcac ttctgggtgc tcgggtggtg ggggcagcta aatgctgctt ccccagctcc   2622
ccgcttcccc tggggccgct ggttaatagg tgtatctgct gtgtcccctg gagccagggc   2682
tgcccacggg gctgggcgca ggcataaacc tgggacgcac tgttttctct tttgtttctc   2742
cctacatgta g gt  atg gga ggg ccc aac atg cct ccc cca gga ccc tcg    2791
                Gly Met Gly Gly Pro Asn Met Pro Pro Pro Gly Pro Ser
                    255                 260                 265
ggc gtg ccc ccc ggg atg cca ggc cag cct cct gga ggg cct ccc aag     2839
Gly Val Pro Pro Gly Met Pro Gly Gln Pro Pro Gly Gly Pro Pro Lys
            270                 275                 280
ccc tgg cct gaa g gtgagctccc tcttctatgg tggtgcaccc gtgcccttac       2892
Pro Trp Pro Glu
        285
tccccatctc aagcttgggt ccttgagatg agctttgtca gggagaaagg gccgagggtg   2952
gtcaggctga actgcagcct gtacttttct tgtggtggtc cccgggtctc ccctgtagcc   3012
agtcagttcc caaaggctgt cgttcatccc tcctctgaca gcttgtggcc ttcacccagt   3072
ccctgagccc tgtatatgta attgctcagt aagtgctgca ggctcccatc tgttgggctc   3132
aaagaggcag tgggactcat tacccatcct cttggagcct gaggtttagc gatgggcatt   3192
gaggggcaaa ttgcttccac tggatttagt tggcactagg aggagatgac aggaaatgct   3252
gccatagagc cacagacaga acaaaaaggc cttgggagcc tggggctggc cccagtggag   3312
ggtgtgaagg acgagggtga ggctgagatc tggaggaggt tggagcccaa ggcaggtgtg   3372
aaggacaccc ctgggtgaaa ggtgggagat gggcggggtc tgcctgtccc cagtgcctca   3432
agcagctcag cagctttcca tttccagccc gggatgggcc ccagagctca acatgacgcc   3492
ctggcccctt gccttctccc ag ga  ccc atg gcg aat gct gct gcc ccc acg    3543
                         Gly Pro Met Ala Asn Ala Ala Ala Pro Thr
                                     290                 295
agc acc cct cag aag ctg att ccc ccg cag cca acg ggc cgc cct tcc     3591
Ser Thr Pro Gln Lys Leu Ile Pro Pro Gln Pro Thr Gly Arg Pro Ser
            300                 305                 310
ccc gcg ccc cct gcc gtc cca ccc gcc gcc tcg ccc gtg atg cca ccg     3639
Pro Ala Pro Pro Ala Val Pro Pro Ala Ala Ser Pro Val Met Pro Pro
        315                 320                 325
cag acc cag tcc ccc ggg cag ccg gcc cag ccc gcg ccc atg gtg cca     3687
Gln Thr Gln Ser Pro Gly Gln Pro Ala Gln Pro Ala Pro Met Val Pro
    330                 335                 340
ctg cac cag aag cag agc cgc atc acc ccc atc cag aag ccg cgg ggc     3735
Leu His Gln Lys Gln Ser Arg Ile Thr Pro Ile Gln Lys Pro Arg Gly
345                 350                 355                 360
ctc gac cct gtg gag atc ctg cag gag cgc gag tac ag  gtgagggcgg      3783
Leu Asp Pro Val Glu Ile Leu Gln Glu Arg Glu Tyr Arg
                365                 370
ggcccagttg ccaaggtcac tgccctgtgt cccccatgtc cccctgggga agccactcaa   3843
ctttctcccc cactctagca aacagtgccc tgtgcccacc actgccactt ggcctacacc   3903
tccttcctca cctccctgct cccaccctgg ctgcggtctg tttcccacac agcagtttga   3963

gttctctctc tttttttttt tttgagacgg agtttcactc ttgttgccca cgctggagtg 4023
caatggcaca atctcggttc actgcaacct gcatctcctg gcttcaagcg attcttctgc 4083
ctcagcctcc tgagtagctg ggattatagg catgcgccac cacacctggc taattttgta 4143
tttattttag tagaggcagg gtttctccat gttggtcagg ctggtctcga gctcctgacc 4203
tcagatgctc ccaaagtgct gggattacag gcatgagcca ccgcacctgg catgaattct 4263
ctttttatga ttgatatcag atctactccg tggattaaaa cctgcctgta gcttcaatta 4323
tctgcccaat aagctttgcc ttatgtccac tcctgcccag catccttcct cacccatccc 4383
agtctctctg ttcctctttt catttattta acaagttaga cttgttcctg ccccaggacc 4443
tttgcacagg ctgtgcatgg ctccttctac tcattcaggc ctgattgctg gggttccctc 4503
ctcatggagg cccttcctgt cacttccagc cactctctgg aattgcagtg gatattttat 4563
tttttttaaa tctcctttat ggcctgtgtt ttctaatgag actgagagtt ccttgctttg 4623
ttgttcttgt ttagtgctaa ggccctgcac ttaaaatggt ccctggcata cagttggtgc 4683
tcaatgagta tgtgcagaaa gactgattaa gagaattagc ccacataacc ttcgcatgta 4743
accttcgtaa ccttcagacc aagcctctag ggtcactccg aggggttgcc tcagttttga 4803
ggtgagaatg ctgaggtgtg cagttgaaat agattcccac cgccctgcgc ctcatccatg 4863
ccagagctgc atgaaaacct tggcccgcct gacgccagaa cctggctcac accactgaaa 4923
aggcatgacc tgggcgcgtc tatcaggaat cttgtttgct gtcccctgag gcccggtcct 4983
gaggagtgcc ccgggctatc tccctctctg ctgtgccctg cggccagcat cctcttctga 5043
ggcatctctt gcctcatgac tagtgcctgc ctctctcgag ggatgggtcc cctgcagctc 5103
cagctgtaac tgggtgccct ggcaaccctc tcaccgcctt tgctccttgt ctctgctccc 5163
ag g ctg cag gct cgc atc gca cac cga att cag gaa ctt gaa aac ctt 5211
Leu Gln Ala Arg Ile Ala His Arg Ile Gln Glu Leu Glu Asn Leu
    375                 380                 385
ccc ggg tcc ctg gcc ggg gat ttg cga acc aaa gcg acc att gag ctc 5259
Pro Gly Ser Leu Ala Gly Asp Leu Arg Thr Lys Ala Thr Ile Glu Leu
    390                 395                 400
aag gcc ctc agg ctg ctg aac ttc cag agg cag gtgggtgctg gcatggccgc 5312
Lys Ala Leu Arg Leu Leu Asn Phe Gln Arg Gln
405                 410                 415
agctttccga aaagggcctt tgtcaccaac actgctgcta aggctccaaa tacggcttgc 5372
ctggctagta ttacacctgc ctgggctgtg aaaagcagcc gtggccatcc atgggcactc 5432
aatcactgca gagccttcct cttacggcct gcgcagtgct ggggacgtgg acctcactac 5492
cctaaagcac ttgccgcctc aaagcagact tgtatgggct tctttcttcc tttccctttc 5552
cctttttttt tttttttgga aacagtcttg ctctatcacc taggctggag tgcagtggca 5612
taaactctgc tcactgcaac caacgcctcc tgggatcaag caattcttct gcttcagcct 5672
cccaagtagc tgggattaca agcgtgtgcc accatgccta atttttatat ttttagtaga 5732
gacagggttt caccatattg gccaggctgg tctcaaactc ttgacctcaa atgatcggcc 5792
tgccttggcc tcccaaagtg ctgggattac aggcatgagc caccacgtgc tgtcctgcat 5852
gggtctattt ctgagggtac tgtgcttaga ccctggagtg cagtggagag atcacagctc 5912
actgcaaact tgacctccca ggctcatgtc ctcctcccgc ttcagcctcc tgagtagctg 5972
ggactatagg cgtgcaccat tacacctggc tatatttttc ttttttttga aacagagtct 6032
cactctgtca cccaggctgg agtgcagtgg tgccatctca ccccactgtg gcctctgcct 6092
ctgtggttca agtgattttc ctgtctcagc cactgagtag ctgggattac aggtgtgcac 6152
caccatgccg ggctgatttt tttgaagtgc agtggcacaa tcttggctca ctgcaacctc 6212
tgccttccag gttcaagtga ttctcccacc tcagcctcct gagtagctgg gattacaggc 6272
atgtgcccca cgcctggcta atttttgtat ttttagtaga gacagtgttt caccatgttg 6332
gccaggctgg tcttaaactc ctgacctcag gtgatcctcc tgcctcagcc tcccaaagtg 6392
ctgggattac aggcatgagc caccacgcag gcctggtttt tgtattttta atagacacag 6452
ggtttcgcca tgtcgcccag gctagtctcg aactcctgag ctcaagccat ccacctgtct 6512
cagcctccca aagtgctggg attacaggca tgagccactg ctcctggccg cctagttaat 6572
ttttcttcta gtttttgtag aagtgaggtc tctccatgtt gcccaggttg gtctcaaact 6632
cctgggcttg agcaatcctc ccaccttggc ctcccaaagt gttgggatta ctggcgttaa 6692
gccaccacac ccagccaaga gtgttatact tctgaatgac tcctaagacg tgtgtgtggt 6752
agaggggagg caagctggag gtccgagagg accccctgga tagatgtcca gaagtgatca 6812
ccttctgtcc tgtgctcgcc ctgcccgggg gcacctgcta gacgtcccct gcacacacgg 6872
actgggtgtg taaggcactt acaatgctcc tttagcggtg aagcatgtga catcaccata 6932
cgtgtttgtc attgtggatg ccacagagct gtgcagtgcg cgggcttgtc ctcttccctc 6992
ctacag ctg cgc cag gag gtg gtg gtg tgc atg cgg agg gac aca gcg 7040
Leu Arg Gln Glu Val Val Val Cys Met Arg Arg Asp Thr Ala
                420                 425
ctg gag aca gcc ctc aat gct aag gcc tac aag cgc agc aag cgc cag 7088
Leu Glu Thr Ala Leu Asn Ala Lys Ala Tyr Lys Arg Ser Lys Arg Gln
430                 435                 440                 445
tcc ctg cgc gag gcc cgc atc act gag aag ctg gag aag cag cag aag 7136
Ser Leu Arg Glu Ala Arg Ile Thr Glu Lys Leu Glu Lys Gln Gln Lys
                450                 455                 460
atc gag cag gag cgc aag cgc cgg cag aag cac cag gtacgctccg 7182
Ile Glu Gln Glu Arg Lys Arg Arg Gln Lys His Gln
            465                 470

gtggccccaa ggccctgcag cccgcccacc tggctgcctg gcttgtccag cggttgccac 7242
ggggctgtgt ttgctttgtt cctaaacatg ctgcttgtct ctctcttttg ctcattgtaa 7302
cagtattcta ggtactgaga gtgtattggt gtaaaaaccc aatggtaggg agaagatagt 7362
gatgaaatcc acgcgtagca cgtctggaag gtgatagggc acaaagaaaa atcaagccag 7422
gatagagagg aaggaacggg ctgttgtaga actgatgttg ggggagttct ttcttttgga 7482
ggtgacatct gggcagaccc ccaaggaagg gaggcacaca gatctgtggg aaagagcttt 7542
ctgggaaaag gagaaggtgc aagggccctg gggctggaat gtgcctgtgt tttctctgga 7602
aagcaaggac ctgcatgagc cagagtagag tgagtggcgg ggagagctgg aggaagggag 7662
cgcagggagg tgacgagcag gtgatgcagg gccttgcgag ctgcatggag gatttgggct 7722
tttgctctga gtaaagtggg agccatagag ggttctgggc agtgcaggga ctgatctgac 7782
tcagggtttc ccaagatgaa tcagtaagac ggaaatcttc aaatctaaaa tcaagtaagg 7842
atttggggat gattgtgctg atttggtcat ttatagattt tctagagatt ttctataatt 7902
aatatcttat tggtgtagtg taaaataatc tagacagtca acacagcaat ctctgttaac 7962
tttttttttt ttttttgaga tggagtctcg ctgtgtcact gaggctggag tgcagtggtg 8022
caatctcggc tcactgcaac ctccatctcc tgggttcaag cagttttcct gcctcagcct 8082
acctagtagc tgggattaca ggcacctgcc accatgcctg gctaactttt tttttgtatt 8142
tttagtagag atggggtttc accatgttgg tcaggctggt ttcaaattcc tgacctcaag 8202
tgatctgtcc gtcttggcct ctcaaagtgc taagatggca ggcgtaatcc cccgtgcctg 8262
gccaacattt tttttttttt ttttttttga gatggagttt cactcttgtc acccggactg 8322
gagtgcaatg gcacgatctt ggctcactgc aacctctgcc tcctgggttc aagggattct 8382
cctgcctcag cctcccaagt agttgggatt acagatgtgc gccgccacac ctgtttgttt 8442
ttttcttttt ttgagacaga gtttcactct tgttgcccag gccggagtgc agtggtgcga 8502
tcttggttcg acacaacctc tgcctcctgg gttcaagcga ttttcctgcc tcagcctccc 8562
aagtagctgg gactacaggc acctgccacc atgcctggct aattttttat atttttagta 8622
gagatgaggt ttcactatgt tggccaggct gctggtctca gactgttgac ctcgtgatcc 8682
gcctgcctcg gcttcccaaa gtgctgggat tacagacgtg agccactgct cctgaccacc 8742
aacatttttt tatatatatt atttcagcct tttttcttgt atattaatac aaggttaaat 8802
agcccttttt aaattttttc taaaactttg cttttttttt tttttttttt gagtcagaat 8862
ctcactctgt tgcccaggct ggagtgcagt ggcacgatct ccactcactg cagcctctgt 8922
cttctgggtt caagcgattc tcctgcctta gccacctgag cagctggaat tacaggtgtg 8982
caccaccaca cctggctact ttttgtactt ttagtagaga tggggattca ccctgttggc 9042
caggctggtc ttgaactcct gacctcaagt gatccgcctg ccttggcctt ccaaagtgct 9102
ggaattacag gcgtgagccg ctgctcctgg ccacatcgct ttgtttttaa gtatcatgga 9162
aacatgactt ttgatgtcta cactgtattc catttctatg tgtccaggat ctaatcattt 9222
atgtcacctt tttcctgttt gaggacatgc agcatgtttc agcttttgaa aattaaaaat 9282
tatgctagga ccggaattct tggcttgaac attttggagc tggtctctga tatttcctta 9342
aaataaattt gtagtaatgg aataagttgg gtgaagggtt ggaagcattt ttgagacatt 9402
tcacatccgt gccatgctgc cctgccagtc gactgcactg tgtgcctacc tgctgtgttt 9462
gagggccttt cccacccaca gttgccggtg cttagtttgt tttttttttt gagacggagt 9522
ctcgctctgt cgcccacgct ggagtgcagt ggcgcgatct cggctcactg caagctctgc 9582
ctgctgggtt catgccattc ttctgcctcg gcctcctgag tagctgggac tacaggtgcc 9642
agccaccacg cccggctaat tttttgtatt tttagtagag acggggtttc accatgttag 9702
ccaggatggt atcgatctga ccttgtgatc tgcccacttc ggcctcccaa agtgctggga 9762
ttacaggcgt gagccaccgc gccctgccgc cagtgctgag ttttatcatc agagagagaa 9822
acccaaccgc ggcaggcaaa gggtgtccct ttattgtttt cataatagtc tcgtttttat 9882
tttatgtatt atatttattt ttttgagatg gagtctcact ctgtcaccca ggctggagta 9942
cagtggtgcg atctcagctc actgcaacct ctgtctcctg gattcaagcg attcccctgc 10002
tttggcctcc tgagtagctg ggattacagg catgtgccac cacacctggc taatattttt 10062
tgtatttgta gtagagatgg ggtttcacta tgttggctgg gctggtctcg aactcctgac 10122
ctcaggcatt ttgtcctcct cagcctccca aagcgatatt ctaggttttt tttttttttt 10182
ttttttttga gacagcgtct cactctgttg cccaggctgg agcacagtgg cacgatctcg 10242
gctcactgca agctccgcct cccgggttca cgccattctc ctgcctcagc ctcccgagta 10302
gctgggacta caggcggccc ctcaccacgc ccggctgatt ttttgaattt tttttgttag 10362
tagagatggg gttttaccat ggtctcgatc tcctgacctc atgatccgcc cgcctcagcc 10422
tcccaaagtg ctgggattac aggcatgagc cactgtgccc agccctaatt tttttatttt 10482
tatttttagt agagacgggg tttcaccgtg ttagccagga tgatctcgat ctcctgacct 10542
cgtgatctgc cctcctctgc ctcccaaagc gctgggatta caggccaata ttctaggttt 10602
taaacaagcc ttgcggggag atgtgtccac catgctgctg aagggtcagc cttctctttt 10662
gtgctttcct gcag gaa tac ctc aat agc att ctc cag cat gcc aag gat 10712
Glu Tyr Leu Asn Ser Ile Leu Gln His Ala Lys Asp
    475                 480                 485
ttc aag gaa tat cac aga tcc gtc aca ggc aaa atc cag aag ctg acc 10760
Phe Lys Glu Tyr His Arg Ser Val Thr Gly Lys Ile Gln Lys Leu Thr
                490                 495                 500
aag gca gtg gcc acg tac cat gcc aac acg gag cgg gag cag aag aaa 10808
Lys Ala Val Ala Thr Tyr His Ala Asn Thr Glu Arg Glu Gln Lys Lys
            505                 510                 515
gag aac gag cgg atc gag aag gag cgc atg cgg agg ctc atg 10850
Glu Asn Glu Arg Ile Glu Lys Glu Arg Met Arg Arg Leu Met

        520                 525                 530
gtatggtcct gccttcttga cgtgcgctct tctacatgtg tagctggtac tgcgggctcc 10910
aggggtcact ccccagggtt acgccaaggc atttcacagc tcggagttag aggtgggaca 10970
gagggaaaag agcaggcgcg ggcttgggag tcagtcctgg gctcggatat ttgctgatct 11030
ctctgatcat cagaatgact gtgtgacctc aggccactta gccggctcct ctgccttctc 11090
tggaaaatgg aggtggtgcc cccttccctc gttctgtgag agccaggtgg tgtgatccag 11150
acccccgacc cctagcataa acatctctga ccatttattt gtgactgagt cgctggttgg 11210
gcagatattg gcagcgtgtt ctgggccagg tgctgctctg tgtacgggga catggcccct 11270
gcctttgtgg gggctcctgg ttgtgcttga ggggcagata cgcgaatgaa gcaaacagag 11330
tagtggatgg tgaccgctac agtgaggagg gggaagtgga gtggaagggg agggacaggg 11390
tagtaagtaa aagccttgaa acagaggccc aaagggtggc cctgatgcac aaacgcagag 11450
acccagggaa ggagccccag gcagtgcgga caaaggttga ggtggcaggc agccatgtgc 11510
cagggcagcc ggtagccctg ggctcccaga acagcgttgt gtggggcagg aggtgaggtc 11570
agagcttaca ggggcaggtg ggaggtggga cctttcagac cattgggagg actctgggtt 11630
gagttctgat catgaagggg agcctcaccc gggaggtttt tggctgtggg gtgatgtgat 11690
ctgatgtatt cttagaaagg tggggggctg ctgtaggagg tggtgggagg gatgcacctt 11750
ggacttggtg gagagaaggc ttcgggtttg gggtgtattt cgtggacaga gtgggtagga 11810
ctggctcatg gcctctgtaa atggctgctg gcgggactgt ctgcctagcg ggtgcccttg 11870
gaacctagcc cttggtgggt tttgaggaaa tgattcctga atgaggagtc gattgccgtg 11930
tgaagggctg gtggcacggc acccgcgtga gctacgcgtg ccctcagtgc gcttctggat 11990
tgactggcca tgggtgctca cagacatgca cattgtgcca ccacattgca gtaaccccca 12050
tgcttttgta g gct gaa gat gag gag ggg tac cgc aag ctc atc gac cag 12100
Ala Glu Asp Glu Glu Gly Tyr Arg Lys Leu Ile Asp Gln
            535                 540
aag aag gac aag cgc ctg gcc tac ctc ttg cag cag aca gac gag tac 12148
Lys Lys Asp Lys Arg Leu Ala Tyr Leu Leu Gln Gln Thr Asp Glu Tyr
545                 550                 555                 560
gtg gct aac ctc acg gag ctg gtg cgg cag cac aag gct gcc cag gtc 12196
Val Ala Asn Leu Thr Glu Leu Val Arg Gln His Lys Ala Ala Gln Val
                565                 570                 575
gcc aag gag aaa aag aag aaa aag aaa aag aag gtgtgctggg cctggcatgg 12249
Ala Lys Glu Lys Lys Lys Lys Lys Lys Lys Lys
            580                 585
tgcccgccgc gggtgggatg ggagcagccg tcttcacgtg tgtggcctca gccttgtggg 12309
tcagggcctg accgtgtctc tctctatttc cag aag gca gaa aat gca gaa gga 12363
Lys Ala Glu Asn Ala Glu Gly
        590
cag acg cct gcc att ggg ccg gat ggc gag gtgaggaagc agggtttctt 12413
Gln Thr Pro Ala Ile Gly Pro Asp Gly Glu
595                 600
gtggaagtat caagctagcc ctaaggcgtt ggtctgtttc agactttaaa acctgtgttc 12473
ttttaaaatt acgaacaatg atatgtgatg atggtgagaa tcccagggtg tcctctgacg 12533
cccatcccac ccgaccccac atcccacatg ttgtgttttc ttccagacct ttttctatgc 12593
atgtgccaca ggattcatct ttcaggtttt actttgttgt aacgtccgac ttacaggaga 12653
ggtgcaagaa ccgtccagag atgtcctgtg tgttcttcac ccagagtctc cattttcacg 12713
ttttgtgttt tgttttgttt ctttgagaca gggtctctct gtcacccagg ctggattgca 12773
gtggcggatg gctcactgca gccttgacct cctgggctca agtgatccta cccttcgagt 12833
agctgggatt acggttatgc ccaccacacc ccagctaagt tgtttttttt tttgagatgg 12893
agttttgctc ttttgcccag gctagagtaa agtggtgcaa tcttggctca ttgcagcctc 12953
cgccccgcga gttcaatcaa ttgtcctgcc tcagcctcct gagtagctgg gattataggc 13013
atccaccacc acgcccattt aattttattt ttatttattt atttatttat tttagacgga 13073
gtctcgttct gtcacacagg ctggagtgca atggcacaat ctctgctcac tgcaagctcc 13133
gcctcctggg ttcacgccat tctcctgcct cagcctcccg agtagctggg actacaggca 13193
cctgctacca cgcccggcta attttttgtg tttttagtag agatgggatt tcaccgtgtt 13253
agccaggatg gtttcaatct cctgacctcg tgaacatcgg cctcccaaaa tgctgggatt 13313
acaggcttga tccaccatgg ccagccagtt tttgtatttt tagtagagac ggggtttcac 13373
catgttggcc aggctggtct tgaactcctg atctcaagtg atccacccgc cttggcctcc 13433
caaagtgcca ggattacagg cataagccac cacgcctggc ctaatttttt aaaatttatt 13493
ttctatagag acgggggttc catatgtttt tcaggctagt ctccaattcc tgggctcagg 13553
catcctcctg cctcagcctc ccaaagtgtt aggattatag gcatgagccc ccgcacctgg 13613
ccttcacctg tgttttttcc taaactcttt gagagtcaaa tgcagacgtg acagcgcctt 13673
acctccatgt aattcattat ctacttcata aagacaaggt tgtttctgca cactgtaccg 13733
aggtgacgga cacaggaggt gaacattggt acagcactgt tatccaatct ataggccttc 13793
gtatgaggtt tggccagttt ttccagttat cttcacagca gcagagaatc ctgggtcaca 13853
tttagtggag gtccctttag tcagctttag tcttaaagag ctccttgtgt gtgtgtttgt 13913
ttttttgcag agatggggtc tcatgttgcc caggctggtc ttgaactcct gggctcaagt 13973
gatcctccca cctcggcctt ccaaaggctg tgattatagg cgtgagccac cacgcttggc 14033
ttcaggccag ctatgtcatc cagtggcccc ggactttggt ttgtctgatg ttccttgtgg 14093
ttggagttgg gttatgtgtc tctggcaggc agacccaaca tggccctgag tccctctcag 14153

ggtgtcatat cgagagtgca tggcggaggt tagcaaataa ggtcactggt gatgctgact 14213
ttgaacccct gccaagggat ctccactgtc cagttactgt tttttctttt tgtaatcagt 14273
aagtaccctg tagggagata cttgagactc tgtaactatg ttgtaactcc tcagaattcc 14333
agctactcct tttagtatct tttttttttt ttttgcctgt atcagttttt gctctgatgg 14393
ctacaggatg gtgactttta tttccatcgt tctttctgca ttgatttgtt tgtttttcta 14453
tagtaggaaa agctttctct ttggtcctgt tagtcctgtg gccatggtct gccaacccct 14513
ggtgtagact tacggattct tgtttagttg aatgaatgga ttccgatcct ttaaaatgat 14573
ttcttttggt catttagctt tagccagcag gagccccttg ccattggctc ctgtttgctt 14633
ttggtacatg cccaccattc ctggagcact tccttcctca tcagactttt cttctgcttt 14693
ccctgcctga gctctctgga gccaactctt tctccaggga gtcctggttc cttttttttt 14753
tttttttgtc tgtcaagacg gggtctccct gtgtcaccca ggccggagtg tgtggtggtg 14813
cgatcatggc tccctgtagt ctcaacctcc tggactgaag cgatcctccc acctcagcct 14873
cctgagtacc tggggctaca ggcgcacgcc accatgcttg gttaagtgtt ctgtattttt 14933
ttgtagagac agggtttcac catgttgccc aggctggtct cgaactcctg agctcaagca 14993
atctacctgc cttggcctcc caaagtgcta ggattacaga catgagccac tatgcctggc 15053
tatatttcgt attattacag aagaatattt acatgccaag atctgacttt gggggtgctt 15113
cctgctagtg ggccatcact gcttctaggc cctctgagca gccggatctg ggaaatagat 15173
gtatgtacac atactcatct gcattgctgt ttgtatatat taaaacttgt ggctgggcat 15233
ggtggctcat gcctgtattc ccagtacttt gggaggccaa tatgggagga ctacttgagc 15293
ccaggagttc gagaccagtg tgggcaatat ggcaaaacct tgtctttata aaaaatttta 15353
aaaattagtt gggtgtagtg gcatgcacct gtggtcccag ctacttgaga ggctgaggtg 15413
ggaggatcac ttgagtccaa gagtttgagg ctatagtgag ctgtaatctc accattgcac 15473
tccagcctgg tcaagacagt gagaccctat ctcacacgca aaaagaatct tgaggttata 15533
ctgacacctc caattctaat ccaacagcac agggttaatt tttatccttt ccgcctttcc 15593
ataattctaa aatcagcaac ctgatttccc ttagcaataa taggcttact tacctgctcc 15653
attttagaat acacagaaag tacgttccaa agtgctaatg gacacctcta tgcagaagtc 15713
tcctagttag agttcagtat ttatagagtt cctctacctc aggatatgtg ttccaaatac 15773
tgtgtccaga agttactggg gccgggtgcg gtggctcatg cctgtaatcc cagcactttg 15833
ggaggccaag gtgggcggat cacctaaggt caagagttca agaccagcct gaccaacata 15893
gtgaaacctc gtctctgcta aaaatacaaa aaattagcca ggtgtggggg cacacgcctg 15953
tggtcccagc tacttgggag gctgaggcag gagaattggt tgaacctgag aggtggaggt 16013
tgcagtgagc caggttcatg gccattgcac tccagcctgg gtgacagagt gagattctgt 16073
ctcaaaaaaa aaaaaaaaga agttactttg gaccaggtgt ggtggctcat gcctataacc 16133
ccagcagttt gggttgctaa ggtgggtgga tcgcttgagc ccaggagttc gaaaccagcc 16193
tgagcaacat ggtgaaacct catctctaca aaaaattaac caggcttggt gcgcacgcct 16253
gtagtcccac ctactcgaga ggctgaggta gggggataac ttagcccagg aggtccaggc 16313
tgcagtgcac tgtgtttgta ccactgcatt ccagcctggg gagcttttta ttacttgaga 16373
cagggtctct ctctgtcagc cagttctgtc tcacagttct gaagccatct ggaggactca 16433
agaaaaaagt aatgggccag gtgcggtggc tcacatctgt aatcccagca ctttgggagg 16493
ccgaggcagg cagatcatga ggtcaggagt tcgagaccag tctggccagt atggtgaaac 16553
ccagtctcta ctaaaaatac aaacattagc caggcgtggt ggcaggcttc tgtagtccca 16613
gctactggga ggctgaggca ggagaatcac ttaaacccgg gaggcggatc acaccactgc 16673
actccagcct gggcgacaga gtgagactct gtctcaaaaa aaaaaaaaaa aagtcatgaa 16733
gtgggaaagt gcatgaagtg ggagcttgtg ctcagtcctg ctgtggctgc ctgtcctgtg 16793
gagtgttgag aacaggcacc ggcacttgct gctgcttgtg ggttttcttg ttctcggaca 16853
gtggtactca gcctctgttg tgtgttctag tcacctgggg agctctctat tattattact 16913
actattattt gagacagggt ctctctgttg tccaggctgg agtccagtga tgaggtcatg 16973
gctcaccgca gccttgacgt cctgggctca agtgatcctc cgaggacctt tttctaagta 17033
gtagctgagc atgaaggcat gtgcctgttg ttgcagctgc tcaggaggct gaggtgggag 17093
gattgcttta gtccaggagt tcaagaccaa cctgggcaac atagggagac tctgtctctc 17153
caaaaaataa aataattacc tgggcatggt ggtgtgcact tgcggtccca gctgattggg 17213
agccagaggt aggcggatca cttgagccca gggctttgag gctgtgatca cacctgtgaa 17273
taaccatggt actgagcccg tctccaaaaa ataaaaagta aattaaaaat cttaaaaagc 17333
gaggatgccc aagcctcact ccagaggagt tcaatcagaa ttttgggtgt aggggaggca 17393
tgcagcaatc ggcatctttg actgctcctc ccagtgatcc cagagaggat gtggagttga 17453
aagtccctga ctgaggcagc ctgcagagcc cattgttggg gtaagaggtt gctcttatgg 17513
tggccgctct gtgggtgagc ttttagcgcg tagagactga tatccctccg ctgtgtggac 17573
ggtattgaca ctgtgagcgc agcagttttg caggtagtta taaattcctg gaagatgaaa 17633
accaaaattt aaaccaataa ctttttctcc atcctggaag ttttttctag gaaatataca 17693
agaattgatg agggagaaaa tgtttatcca ttgaatctca acaggaggag tttcatattt 17753
gttgctaaga ggaattattt ttgaaatatt tcaattagct ttttaacaaa aaagaatgag 17813
gacccttgat gagatctaca aatagagtat aagtggggga aaaaatcaag gtccaaagaa 17873
tgtaaattat ataaccgtta gtgtaaaaaa gagggagaaa gtggaaacgt ctaagtcctt 17933
gtttcttatc catggaattg atctggaagg atggacagga aactgatacc agtggaaaac 17993
tgtgaagaga aaaacttagg ctggagcaca gaagaatggg gagagcccac atgatgctct 18053
tcagttttcg ttaaaaaaat aaaaatgggc cttgggaggc cgaggcgggc agatcacgag 18113
gtcaggagat cgagactgtc ttggctaaca cagtgaaacc ccgtctccac taaaaatact 18173
aaaaattagc tgggcatggt gatgggcacc tgtagtccca gctactctgg aggctgaggc 18233
aggagaatgg catgaacctg ggaggtggag cttgcagtga gccgataccg caccactgca 18293

ctccagcctg ggcaacagag tgagactcca tctcaaaaaa ataaataaat aaaaataaaa 18353
aataaaaatg ggccgagcat agtggctcac gcctggaatc ccagcacttt gggaggccaa 18413
ggcgggtgga tcacatgagg ccagaagttt gagaccagcc cggcctgaat tttcagctat 18473
gagtgtctgg actatttgaa aattacaatg tatatgccag ttaccttaaa gaaacaggtg 18533
ttgggagact gaggcagaga attgcttgaa cccaggaggc agaggttgta gtgagccgag 18593
agccgagatc acgccactgc actctagcct gggtgacaga gcgagactcc gtctcaaaaa 18653
aaaaaaaaaa aaaaaaagaa gaaacaagtg tcagccaagg ttgatgggtt atatattttt 18713
actggattac tgagtataca gtttacgcat tttcccacca ctggccatgt ttgcctgcca 18773
ttttctgtgc aaacagttga gtgggtgctt cccaccttgg cctctgtaag tgtttggtct 18833
ggaggccctg caacctcagt gtcacacgaa tgactctttt tcag cct ctg gac gag 18889
Pro Leu Asp Glu
605
acc agc cag atg agc gac ctc ccg gtg aag gtg atc cac gtg gag agt 18937
Thr Ser Gln Met Ser Asp Leu Pro Val Lys Val Ile His Val Glu Ser
    610                 615                 620
ggg aag atc ctc aca ggc aca gat gcc ccc aaa gcc ggg cag ctg gag 18985
Gly Lys Ile Leu Thr Gly Thr Asp Ala Pro Lys Ala Gly Gln Leu Glu
625                 630                 635                 640
gcc tgg ctc gag atg aac ccg gg gtgagttggg ccttgcattc cagatgcagt 19038
Ala Trp Leu Glu Met Asn Pro Gly
                645
ggggatccaa gtcctcggtg ggccttgttc cagggaggtg gcagccagga gcaattttac 19098
ttctgtttga attcccggca ggtttggtag ggaaagtgaa ttctgctggc tctgagcaga 19158
tttgtatgaa agcccttaca ttttttctag g tat gaa gta gct ccg agg tct 19210
Tyr Glu Val Ala Pro Arg Ser
    650                 655
gat agt gaa gaa agt ggc tca gaa gaa gag gaa gag gtaagagtgc 19256
Asp Ser Glu Glu Ser Gly Ser Glu Glu Glu Glu Glu
                660                 665
atttcctggc tttcaaggct ctcagtgccc actggcagtg acttccaccc tgtgtgttgt 19316
gaccgtctcc tgccctggct gggcatcttg tggggcaggg aacagcaggg cccaggcctg 19376
cccgaagtcg gtgctttaga gctgtcctat ccaatagggc agcttctggc gacatgtgac 19436
catttccatt ttaaattaaa tcagtcacat tacataaaat ttaagactca ttttctcttt 19496
cacactggct gcatttcagg tgcttggcat cccttatctg ggaatggtct gcctcatttg 19556
cccatttttt tttttttttt ttgaaacgga gtctcactct gtcacccagg ctggagtgca 19616
ctggtgcaat ctcagctcat tgcaacctcc acctcccggg ttcaagcaat tctcctgcct 19676
cagcctccca agtagctggg actacaggcg cctgccacca cgtccagcta gtttttttat 19736
gtttagtaga gatggagttt catcatattg gccaggctgg tctcaaactc ccgacctgaa 19796
gtgatccacc tgcctcagcc tcccaaagtg ctgggattac agacatgaac ctccacgtcc 19856
gacttatgat ttttttgttg ttgttttgaa atggagactt gctgtgttgc ccaggctgga 19916
gtgcagtggc gcgatgttgg ctcactgcag cctccacctc ccgtgttcaa gcgattctcc 19976
tgcctcagcc tccctagtag ctgggacttc aggcacctgc caccatgcct ggctaatttt 20036
tgtattttta gtagagacag ggttacacca tgttggccag tctggtcttg aactcttgac 20096
tggtgatttt tttttttttt gagacagagt ctcactctat agcccaggat ggagtgcagt 20156
ggcgcgatct cgctcactgc aacctccgcc tcccgggttc aagtgattct cctgcctcag 20216
tctcctgagt agctgggatt acaggcgccc gccaccatgc cagctaattt ttaaaatatt 20276
tttagtagag atggggttcc accatgttgg ccaggctggt ctcgaactgc tgaccttaag 20336
cgatccaccc acctgggcct cccaaagtat tgggattaca ggcacgaacc agtgcgtcaa 20396
agtctgttct gtgttagtca ctctaaccct cttatgatta ttgttcacat ggtacatctt 20456
tttcccttct tttactttca acctgaatct acaacagcat atgggttttg cattttcagc 20516
tagtatgaca gtgactgctt tttgactgag tttttaagac catttatatt tactgtaatt 20576
tctgatatga ttgggcttac atttgctatt tcttttctat gcctcctgtt tttgttcctc 20636
ctttactgcc ttttgtgttc aatgtttttt ttccctagat ttttattttt atagtgcctt 20696
tttttttttt gagacggagt ctcgctctgt cacccaggct ggagtgcagt ggagcaatct 20756
cggctcactg caatctctgc ctcccgggtt caagtgattc tcctgcctca gcctcccggg 20816
actacaggtg catgccacca aacccggcta attttttttt gtatttttag tagagacagg 20876
gtttcaccat gttggtcagg ctggtctcga actcctgacc tcaggtgatc tgcctgcctt 20936
ggcctcccaa agtgttggga ttacaggcgt gagccaccac gcctggcctc tcctattttt 20996
ttgcttacct cttcatgttt agttgaagcg tactttagct accctggagt ctgactttta 21056
aaactctggg aggttgtggt ggcgtctagt ctgtttagta atttcctggg gctcagtctg 21116
tgaaatctgg ttcctgcata gtgtgtatag cctctgctgt atttcctcaa ttcttttaaa 21176
cattttttgg ttttatctta ttcgtgattt ttttcctggt tcattagggg caggcagtgt 21236
ttctttgtca cttagtggtc atccaaagat tggccacagt ttgtgctcag acacgcgcct 21296
tctttctcct gctgatggtt cagttgtatg aggaggtgca ttccacattt gggccattgt 21356
cgggttctcc ctggcactta tgctttgctg agtcctttgg cgtctctttg catatgcaca 21416
gactcagcct cagccagtga tacgcttttc cctaccgtgg ctgatctcac ctccattagc 21476
agtgccactg gctgtgggca ttgcttcctt ctacaagtga ctgagcccct tccacctccc 21536
tgccaagcct catggcctgc agctctccag ctgtaagagg ttcatgtgtc cagagctgag 21596
gcagaagaag tgaaagtggc ccacagtaaa agtgctacaa actcccactc ttcccacctg 21656

aagttcagtt ttttaaggtt caatgattct agtagacctt tggtcaactc gctgagctct 21716
gaaatgctca ctctgtcaat ttgtccggct ttatagttgc ttttggggag gaaatttgcc 21776
cacctcctca tttgggttta gccagaagtc tcactcctat tttaattcca gcatgtctgt 21836
accagctctg attttcttgc ctaaaatgtc ggtgttatta ccacctgttt attccaagtg 21896
atctgtaaaa tcattttttg tgacctaagg gaaatcctac tggcttttca ctggaattat 21956
ttaacagatt aattgcagat catattttta aagctttgta gcagttccat tatagatgag 22016
ggaacaatca actgtgttgt atttgtagga tttaaatggc aatacaagaa tagtttaaga 22076
taaggaaatg cattcttgtc tatgttatca atatgtgtta tgcaaaatcg cagtaaaggc 22136
tggaaagcca ttaaatgatg caacatgaat gattaaacaa gttcgtagat aacttgcagt 22196
aaactgagat gcctcttgtc gccagtgtta tcagacatat tgtagatggt tgagacttgt 22256
cagtaaagct ggaagtaaat tacaagcaat aaatattgaa agttggttct aagatattta 22316
aagccaggcg cggtgtctca cgcctgtaat cccagcattt tgggagggca agttgggtgg 22376
atgatccaag gtcaggagtt cgagaccagc ctggccaaca tgatgaaacc ccgtctctac 22436
taaaaaatac aaaaattagc cggggtgatg gtgggctcta agatatttaa tacctaggaa 22496
atccaagaga atcaactgat aaactgcttt agtaggtcag cagagaatag acgtgtaatg 22556
ttcattgtat aacaggggta ggagagatat ggagtctact tctaattagt aatgaaagtc 22616
cacaataccc tgagatagcc ttaaaagaag cgtcggggcc gggtgcagtg gctcatacct 22676
gtaatcccag cactttggga ggctgaggca ggcagattct tgagctcagg agtttgagac 22736
cagcctgggc aacatagtga gtccctgtct ctaccaaaaa tgcaaaacaa ttagccaagt 22796
gtcatagtgc acgcctgtgg tcccagctac tcgagaggct gaggtgggag gatcgcttaa 22856
gtccagtggg tggaggtagc agcgagctga gatagcacca ctgccctcca gccagagtga 22916
cagagcaaga ccccatctca acaataacaa aaaaaaggaa gtgtcaggaa tctgtggtgg 22976
ggaaaccaaa aacggtttac tgggctgggt gcggtggctc acgcctgtaa tcccagcact 23036
ttgggaagcc aaggcaggcg gatcacgagg tcaggagttc aagaccagcc tggccaacat 23096
agtgaaaccc cgtctctact aaaaatacaa aaattagcca ggcatggtgg cgcatgcctg 23156
taatcccagc tacttgggag gttgaggcag gagaatcgct tgaacctggg agctggaggt 23216
tgcactgcgc cgagatcacg ccactgcact ccagcttggg caacagagtg acacttcgtc 23276
tcaaaaaaaa aaaaaaaaaa aaaaagatgt agccagtgtg gtggcatttg cctgtagtcc 23336
cagctacttg agaggctgag gcgagaggat tgcttgagcc caggagtttg agaccagccc 23396
ggggaatgtc acgagactac atatctacaa aaattcaaaa catttgagct gggtgtggtg 23456
gtgcatagct gtagttctag ctgctgggga ggctgaggca ggatgattgc ttgagctgag 23516
gagttcaaag ccatctgggc aacatcctga gactcccata tattaaaaaa aataataata 23576
aatacataat tttaaaacaa agatgtaaat aataaacagc acacattcca ggataggtag 23636
agggggtgag ggctgtaaga agatcacttg cttctgagac tgttctcccc atgggagtcc 23696
cgtccccccct ctctgggga tgaactgagg tgacatgggc ttgtctcttg gtag gag 23753
Glu

gag gag gaa gag cag ccg cag gca gca cag cct ccc acc ctg ccc gtg 23801
Glu Glu Glu Glu Gln Pro Gln Ala Ala Gln Pro Pro Thr Leu Pro Val
    670                 675                 680
gag gag aag aag aag att cca gat cca gac agc gat gac gtc tct gag 23849
Glu Glu Lys Lys Lys Ile Pro Asp Pro Asp Ser Asp Asp Val Ser Glu
685                 690                 695                 700
gtg gac gcg cgg cac atc att ga gtaaggggtc ccgacacagg ttgttctgtg 23902
Val Asp Ala Arg His Ile Ile Glu
                705
ccagcttcct gtgaggtggc gctggtggga gtggctagaa tcccagccat ccctagctga 23962
cattcagcac tgtccagcca gctgctactg tggaactaca gagaccaaat ttatttactt 24022
cacatttcat gtattttata gggatttaat agagcaattg cattcgcatg gttcaccata 24082
tgcttacgag gtacagtcag gtgtgcggga gaagccctcc tcaccccgtt ctctgctccc 24142
cggctccctc cctttccact accctccctg ggaaactttt ccagccttgt gtgtcatctt 24202
ccagagatgc tgtctgcatt agcaagcaaa atgtgcatat ttttgtcttc atttgcatac 24262
atatctccag tgtacattgg tgtgcacctt gttattttca ctgaaccttc cctcttggag 24322
gtcatcctgt gtccgtgcag agtgcttcca gtctggtcct cagcgtgcag tgtcatactg 24382
cgtcacgtta tattctcatt ttcgtagcct gtcctttggt gacaagcgtt taggttatat 24442
ttgtcatctt ttgctcctgt aacacatcac agttaacaca gtaatctctt cattgcttat 24502
ggtaaggaat taatagatcc tgttaaaaag caagtgaggg ctgggcgtgg tggctcaggc 24562
ctgtaatccc agcactttgg gaggccaagg caggtggatc acttgaggtc aggagttcaa 24622
gaccagcctg gccaacttgg tgaaaccgca tctctactaa aaatacaaaa attagctggg 24682
cgtggtggcg tgcacctgga gtcccagcta cttgggaggc tgagacagga gaatcgcttg 24742
aaatggggag gaggaggctg cagtgagctg agatagcgcc actgcactct agcctgggca 24802
acagagggag actccatctc aaaaaaaaaa aaaatgtagg tggataaaag tcactttttt 24862
tttttttttt tttttttttt ttttttttga gatagagtct tgctcttcac caggctggag 24922
tacagtggtg cgatcttggc tcactgcaac ctccacctcc tgggttcgag tgattctctt 24982
gcctcggcct cctgagtagc tggcacaaca ggcgcgggcc accacgtcca gctaattttt 25042
gtgtttttag tagagatggg gtttcaccat gttggccagg atggtcttga tctcttgacc 25102
tcatgatcta cccactttga gctcccaaag tgctgggatt acaggcgtga gccaccgcac 25162
ctggccagag ttacttttta aaagaaaaac attcagatta gattacaaat tgcctaagtt 25222
ttaacagctc tggttttgta tgaacctgtt cagtctgtgt ttcttttcac ctttaggctc 25282

atgaattgtt cttagaaagg gtgggttcta tgatgatcag catctggcta ggtgcagagt 25342
gtctttctgt gctgtggcaa gtgacagcat tctatttgtt gttttgagac agtcttgctc 25402
agtcgcccag gctggagtgc agtggctcga tctcttgact cactgcagcc tctgcctccg 25462
ggcttaagtg tttcttttac ctcagcctcc tgagtagctg ggttacaggc catgcaccac 25522
cacgcccggc taactttttt tttttttttt tttgagacag agtcttgctc tgtcgcccag 25582
gctagagtgc agtggcacga tctcggctct ctgcaagctc cgcctcccag gttcatgcca 25642
ttctcctgcc tcagcctccc aagtagctgg gactacaggc gcctgccacc acgcccggct 25702
aatttttgta tttttagtag agacggggtt tcaccatgtt ggccaggctg gtctagaatt 25762
cctgacctca ggtgatccac atgccttggc ctcccaaagt gctgggatta caggtgtaag 25822
ccactgtgcc cagcctaatt tttgtttttt gtagtagaga tggggtttca ccatgttggc 25882
caggctggtc ttgatctcct ggcctcaagt gatccacctg cttcagcctc ccaaagtgtt 25942
gaaattacag gcgtgagcca ccacgcccat cagagtgaca gaattcttgt actgtggtgg 26002
tgcaatcagg acccagagct cccaggagtc cagtctggac ccctcaggct tagtgcgggc 26062
tgccctgcct ggcctcacac tgtccggctg cacctctatg tgtcttacag tgctggccac 26122
aggtcattca gcagaaaggg ccactccgca gagcttgtgt caggagccag cacattgtca 26182
cagataggaa tgtgtgtcct tacccggcac ctccatctca ctcccag g aat gcc aag 26239
Asn Ala Lys
    710
caa gat gtc gat gat gaa tat ggc gtg tcc cag gcc ctt gca cgt ggc 26287
Gln Asp Val Asp Asp Glu Tyr Gly Val Ser Gln Ala Leu Ala Arg Gly
            715                 720                 725
ctg cag tcc tac tat gcc gtg gcc cat gct gtc act gag aga gtg gac 26335
Leu Gln Ser Tyr Tyr Ala Val Ala His Ala Val Thr Glu Arg Val Asp
        730                 735                 740
aag cag tca gcg ctt atg gtc aat ggt gtc ctc aaa cag tac cag 26380
Lys Gln Ser Ala Leu Met Val Asn Gly Val Leu Lys Gln Tyr Gln
    745                 750                 755
gtgaggtagg gggtggggag gccaccgcca cgtagctgcc tcggtgcagg tgttcccagg 26440
tggtgcgggc ttcagacctc aggcagacct gagttcacct tttgctcttt gtgggcaatg 26500
cactcttccc ctctgagcct cggtttcccc atgtgtaaag cagagatgtg gtctggtagg 26560
ttagctgcca ggaggacgtg caacagcaga aagtcagtgt ctactgggtg cccgatgtgc 26620
caggcagagt gggagcgatt tgcactccag tggcctcaca gaggagacgg ggccacctga 26680
ggagcagaga ggtgcagcac ctcctcagca tcgcaccgtg tgtaggactg aaccagggcc 26740
ggagctcaga tctgggcatc tgcatgctca ggacaggtta cctgagctct gtgtggtgtg 26800
aaacggcgag cgtcaggagc ttagatctag gagccggcag ccctggactc cgctctttct 26860
tctggggatg aggtagcagg tcctgtgtga gggtgaaaag ttggtggctc tgaagctctt 26920
cacctgggcc tgacttggga cagtcacgaa acagagctgc tcatgctctt aatgcggcct 26980
gccatccaga gctcattaca aggccccaca agacaaccag tacaactcca accaaagaac 27040
agctctctct ggttttcaat ttggttgttt cttttctttt cttttttttt tttgaaacgg 27100
agcctctctg ttgcccaggc tggagtgcaa tggcgcgatc tcagctcact gcaacctctg 27160
cctcccgggt tcaagtgatt ctcctgcctc agactcctga gtagctggga ctacaagtgc 27220
gtgccatcac gcccggctag tttttgtatt tttagtagag acagagtttc accatgtcgg 27280
ccaagctggt ctcaaactcc tgacctcagg tgatccgccc accttggcct cccaaagtgc 27340
tgggattaca gatgtaagcc agtgcaccca gccccaactt tctttaagac aactcttaaa 27400
ccttttgtgc tagtatcaac attcaaagct gcacatgcac ctatagtccc agctacttaa 27460
gaggctgaga tgggaggatc gcatgagccc aggagttgaa agtgggcctg ggcaacatag 27520
tgagacactg cctctacaaa aatattttaa aaattagctg ggggtggtag cacacatctg 27580
tagtcccaga tagttgggcg gcttaggtgg gaggagagcc cgagcccagg acttctgagc 27640
tgtagcgccc tatgtcgatc aggtgtctgt actaagttca gcatgaatat gatgaccttc 27700
caggagtggg gcaccaccag gtggcctaag gaggagcgaa tctgcccagg tcagaaatgg 27760
agcaggtcaa aactgatgtg ctgatccgta gtgagatagt ccctgtgaag agccactgca 27820
ctccagcctg ggtggcatag cgagacccca tctctataaa acaataatga aacaaaggca 27880
aagctcatgg ttagtggaat tttccaagag tcccatgcaa ttaaaataat tagaacttgg 27940
ctaggcgcag tggctcatgg ctgtgatgcc agcactttgg gagaccaagg cgggtggatc 28000
acctgaggtc aggagtttga gaccaggctg gccaacatga tgagaccctg tctctaccga 28060
aaatacaaaa aattagctgg gcatggtggt gcatgcctgt aatcccagct acttgggagg 28120
ctgaggcaga attgcttgaa cctgggaggc agaggttgca gtgagccaag atcgggccac 28180
tgcactccag cttgggtgag agtgagactc ggtctcaaaa aaaaaaaaat gaattagaac 28240
ttggccagaa cttacttcct caatccatta cgaacatttc caaattttcc ttcataacgt 28300
tatcttcagt ttgcaaatag tcttctgttt aaggcctgta tttcagaaag ctcccaggct 28360
ttggaaatcc ctctcttaat cttgaaatga tgttcaggca gtgaattcct cacgaagtcg 28420
ccctgaagcc ctcttggttt cctgagtgca agtgtcccgt ggtgtgggca ggtttttcct 28480
gtgtgctgtg tggggcatgg acaggcagac aatgtaacag gtgggagtga gctgttgaga 28540
tgtgtattag aaatgtcaca cgtgtccatc gttcgcacag tcatcctgca gcttcacaga 28600
cgttattgct aaggatgagg ctaagcgata aagaatcagt ttggttcaga ggaaaaatcc 28660
gtggtaaagg cagtagaggg tgggatgtgg cttaggcaga actcgtggct ggcggtgtct 28720
tgactctctg aagtgggagg accctctggt gtccgacccg gccttcagtc ctggcgtggc 28780
cgcatctgtc cttgcag atc aaa ggt ttg gag tgg ctg gtg tcc ctg tac 28830
Ile Lys Gly Leu Glu Trp Leu Val Ser Leu Tyr

    760                 765
aac aac aac ctg aac ggc atc ctg gcc gac gag atg ggc ctg ggg aag 28878
Asn Asn Asn Leu Asn Gly Ile Leu Ala Asp Glu Met Gly Leu Gly Lys
770                 775                 780                 785
acc atc cag acc atc gcg ctc atc acg tac ctc atg gag cac aaa cgc 28926
Thr Ile Gln Thr Ile Ala Leu Ile Thr Tyr Leu Met Glu His Lys Arg
                790                 795                 800
atc aat ggg ccc ttc ctc atc atc gtg cct ctc tc gtgagtaccc 28971
Ile Asn Gly Pro Phe Leu Ile Ile Val Pro Leu Ser
            805                 810
gctgccagca acatcccaca cgccgctcac acgctcctgt gtttgtttcc taagtttgcc 29031
gcagtagaat accacaaacg aggtggctta attacagaaa tttgttttcc tggaggccag 29091
aagtccaggg tcaaggtgta gcaggttgct tcctcctgag gcctctcttc ctgcttgcag 29151
ggatacgtgc ttacatgtat ctcactgtgt cttcacatgg tccttcctct gtgcgtgtct 29211
gtgtcctcat ctcttcttct tagaaggaca tgggtcaggt tggatgaggg tgcatcctaa 29271
tggcctgctt taacttcatt gcctctgtat tggccctgtc tccaggtaca gtcgcatcct 29331
gagctgctgg gggtcaggac tgctccacca gcctcggggc tgtgcacagc tccacccgtc 29391
acagcctctg cctctgggtc ccggatcaag tgggggcatg actgtgcatc ccctccaccc 29451
ctggggaggc ctggggtccc tctcccggga tgtccttggt gtccttttca agtcccacaa 29511
ccagcaaagt ccatgcaggg gagtcgcagg agacaagaac ctcccactag aaaagtaggg 29571
tgaggccatt gaggctccat tgaacagtct ctacttcctc actgtgcagt gccctgcaag 29631
ggccaccaga gccctcgggc acatcccagc atggcccagg tccttgggca tggtgggatc 29691
cggacaccct gagaggctct cacttgccac tgcctggctc caccggcctt tttctccctg 29751
caaacagagt ccgcccccct gcccacctct ctcgacatct ctctacccag ctcagcccct 29811
gcactaaagg gggacctgcc tagggttttc ccagaaagcc ccagggccgg ctcttactgg 29871
tccagttcaa gtcacatgtc cagcaagcag acagtcaccg gcctgggggt tagtctggtc 29931
tgattgggca aacttcagca tagcatttgg tctggcccta gagcacagcc cctaactcca 29991
accaggcact cattgggacc actacagcca cgagtcagca gcggaccccc aactcctggc 30051
aggctgggga gcggcatgcc catgctccaa ccccatggga aaatgccctt ctgcctataa 30111
gacagccagg ccccatgggt cacacaccca tagggtaggc ctgacgtcct gactgttgca 30171
gggaggactg gaggaaaggt gaggacccca gaaccctccc aggtttactg tggaactgtt 30231
tctttcttct atctccctgc ttcctacgtg gcgggtagaa cagactgctc tgaaggagca 30291
gggcgtcctt gtgcacagcg ctgcaggtag ctgcacctcc cctcgtggtc agacccagat 30351
tgctacagcg ttcctgctgc acctgcctga gctctgtccc ctgtctgtgt ctggaggctg 30411
ttccttccgg ctctgcccac ctcctggctc ccctctgccc ttcttcccct ggaggctgat 30471
gggtgcagtg gcagtcgccc tctgcagttc ccgattttcc ctcctttcac ttacagactt 30531
ctcagatcac catctctgct ggaacgccgc tctccagggt gctgttgttt tgcagccttg 30591
gttcttagtt gttaattttt tgctttaaaa aattttttta aacttttcat tttgaaatga 30651
tctcatttat ttttattttt ttttattttt tgaggagtct cgcactgttg accagcctgg 30711
agtgcagtgg tgccatcttg gttcactgca acctctgcct cctgggttca agcgattctc 30771
ctgcctcagc ctcccgagta gctgggatta gaggcacgcg ccaccatgtc cggctaactt 30831
ttttgcattt ttagtagaga cagggtttct ccatgttggc taggctggtc ttgaactcct 30891
gaccccatga tctgcccgcc ttggcctccc aaagtgctgg gattacagga gtgagccact 30951
gtgcctggcg acctcattct tagaaaacat tgtagagtta gtgtaaagaa ttgctatgca 31011
cccttcaccc atcttcccca ttctttcata accccaagac aattatgaaa accaggaaga 31071
taacatggac gtaggactaa tagcaaacca gaggccttat ttaccaatct tgtcagtttt 31131
ctcacaagtg gccttgctct ggtccaggat cccacactgt cacgtctgtg tggcatgcct 31191
gtggcagttc ctcatttcgt ctttgtcctg gtcgagcttg acgctgttga atcctgatcg 31251
gacctttggt agactctcct tcaatttggg tttgtccgat gtgatcgcgt ggagcaacac 31311
cacgccctgc ttgatgtgcc ttagcgggga aggcatgaga tgttgaggcc ttggtgtctc 31371
gggcaaggtg cctgctgggt ttcccactgt aaacctagga gttacacttt ccgttaataa 31431
ctgtctcgtg ggaacgtact tcgacacttg tgaatatctc atttctcttc ctactttcct 31491
caataatttc agcatctctt ctactccatt tcatgttgcc ataaaggaat acctgagact 31551
aggtgataaa gaaaagaggt ttagctgggc gcggtggctc actcctgtaa tcccagcact 31611
ttgggaggcc gaggcaggcg gatcacctga ggtcaggagt ttgagaccag cctgaccaac 31671
atggtgaaac cctgtcccta ccaaaaatac aaaaaaaaat tagccaggca tggtggcgca 31731
tgcctgtaat cccagccact caggaggctg agacaggaga atcacttgaa cctgggagac 31791
gggggttgca gtgagccaat atcgtgccat tgcattccag cctgggcaac aagaatgaaa 31851
cgccttctca aaaaaaaaaa gaggtttatt tggctcatga ttctgctggc tggaagactg 31911
ggcacctggt gagggcttca ggcggattcc actcctggtg gaaggcaaag gggagccagt 31971
gtgtagagat cccatgggga gggaggaggc gagggaggcg ggggtgccgg gctcctttta 32031
accatagctc ccacgagagt tcagagagca ggaactcatt catcccccat ccccagtaca 32091
gtacaggccg ttcacgaggg ttcacgaggc cgttcacgag ggaggccccc atgacacaga 32151
tgcctcccac caggccccac ctccaacatt ggaaatcaga cttcagcatg aggtttggag 32211
gtgccagata cccaaactat agcagcaaac gtcagataag tcttgcctga aaaaataatt 32271
acatgagagt tgccaaatac atggttacct gtttctgtcc gctcttctct acaaattaat 32331
tggaattcca ccgttaggaa gagctttccc gtctccccca tgtgcttgtt tgggtaatta 32391
ttatatcagt gtgggcgtga acaaatatta tttattttgt tgcttcagtt tttttcagat 32451
ttggcctggg agaattctcc ctggaagaat tgtgtcttct ctatgtattc agtcatttgt 32511

taatatgagt atggacccac ggatatttat ttagtactcc aggctacgca ccagtggctg 32571
ggtttttttt gttttgtggc tcaaatccat ccagctttgg ccactgggag tgctttcagt 32631
tggctcttct gtcctttgac acgcccccag catcgggtga attttgagga ctttcttgct 32691
ttttggcacc aagagatgct gcaggctcat gtagtctatt tcccgcccac tccctcaaat 32751
cagccatttc tccaaggagc cctggtttcc cttcttacag aatggttttg ggaaccaaga 32811
cctgggtcct ggatgggcct gttgcttctg gggtgtcatt gctcctgggg ccctctcagt 32871
ggtcagtgct aggagattca tgtgcgcgtg ctgatgcttt gtgcacgtat cgtctaaaag 32931
gccttctctg cctcggcatc tgtttctgtg ttaagctgaa cgtgagttca taccactgtc 32991
tgcggctctg agtgaggacc acaggttcct tctcacactt ccaccctgtg tgtgcgccca 33051
ttctcagctt ccgtggacaa cagtttcaga gtagtgagcc ggtacctccg ggaagcatct 33111
tgagccacta gagcgcaggc cttggggcca gttccttgtg tgttcagacc tccagattgc 33171
agtcagattc catttccagg gtgatagagg tcaccttctt cccctacttc agtgaggttg 33231
tgaggtgggt gctcctgcag tctttcccac tgcgccccac ctgggcaagc gtccatgctg 33291
tggaacgcca gctcctgcag gccagaagag ggcccgcgcg ctaggaaatg tttacggcgt 33351
atgggcatcc ggctgtgccg gccctctcac tgggaattgc tgcctcctgc catgagctgc 33411
ttgtcctgcc tccgtttgga cctctgtctc tgggacctgt cccctcagtc ccctcctgac 33471
ccttgctcct ggctcttctc cctcccagtg cagatgctcc gcacacctcc ggcttctccc 33531
ttggtgcgct gactgtcccc atcactccag ggaggtttca ctgttcctga gtcagcggaa 33591
gcacagggtc aggccctacg ggccaggtcc ggtggctcca agatgccatc agggacccag 33651
cctcccccag gtttctgccc agtcattctt gggtgactcc aaccctcagg ctccttacct 33711
catggttgca ggacggctgc actgcctccc tcgcctcatg caggagagtg ggcaggaaag 33771
agttctagga gagtgggcag ggcaaaaggc agcagaagcg caaggctgct gagcctctgc 33831
tccccacgct tcttcaggaa ctctccccag ccttcaccac agccatgcgt ccttctgtct 33891
attggctgtg ccgcctggtt ggctgtggct tccagcgggg ctggagatgg gttcatggtg 33951
gacacagcac ctccctcgtg gagctgcttg gggcaaaccc acgagtagtc cctgggactc 34011
cagcttcgag ccctgtcttt acggaccacc ccgtctagac tttctgagtg gcttccctcc 34071
cccactgaaa actccctgcc ccagagttcc tcccagagcc cgtccccttt gggactcagc 34131
tttattaccg cctccaccct ctcctcagtc tctaggctgt cggtctcagg tgactcaccc 34191
ctccccactg cattctgcct gcccccatgt cctccctcat tccagccctg gtcatcccac 34251
aggggaacag ccacgagttg ccgctccctc ccatccctgt cctcccgcct gccacctgag 34311
ccaggccccg cctcgcccct cactgtcctc gttctctctc acagtcagga accttccgtg 34371
gctggagggt ggcagaaaga ctgtttggtg tctcctatat cctcaggtct ccacagcact 34431
gttgctagcc tggctctggg cgtgaagcct gtgctctgaa gctgcatttt cccggcagca 34491
ggtccaggct tccccagccc tggcagcatt gtttctgtcc cccatcacat ctctgtgtcc 34551
ctacttctca ctctaaaggg ccttgccctc ttttcccagt ggcctccctt ctgtcctctg 34611
aggcagagcc cttcacgtcc tcgccagtct ctcttcctcc ctccctccct cagctgccct 34671
aaacacagtt tctctgccca ctcggagggc tgtggccatg ttggcctcgc ccctgacagc 34731
cagtggctat gggtttgcac agtgagccat tgatgagaga ccggcacttg actctcattt 34791
ccttgttcca tcag a acg ctg tcc aac tgg gcg tac gag ttt gac aag tgg 34842
Thr Leu Ser Asn Trp Ala Tyr Glu Phe Asp Lys Trp
    815                 820                 825
gcc ccc tcc gtg gtg aag gtg tct tac aag gtaggtcaca gccactgagg 34892
Ala Pro Ser Val Val Lys Val Ser Tyr Lys
                830                 835
tttcctctct tgctacggag gtgcaggcgg tggtgggcag gacgtccaca catacctctg 34952
gacagtgaac ctgagaatgc tgggtctcca gtcgcatgga gtctccagga cagcctggaa 35012
ctccagtcac atggatccgg gagtttggac tgggcaggga cagggcagat tagctcactg 35072
gggaggagac aggagtgagc atggtggcca gcactcagag gccagctcag gcgcctgaga 35132
tggggaccca ggaagagggg agcctgtcag ccaccaggaa tgtgcagatg gcggtgcagg 35192
ctgcgtggtt ccctcaggcc ccggccgccg ctggcctgca ctgcttcctc ttccccctgc 35252
agcgcgtgtt ctgcgtggtg aggtctgggg acgcgccagc ggccctgcca gcccctttcc 35312
ccactacccc tgtgaggacg agccctcccg ccgtgtcact gggcagttgc agggggtgcc 35372
tgtgcccctc ttgccacctg gccacccggc tccaaaagcc gagctgtgca tcctgcttcc 35432
cttgcag gga tcc cca gca gca aga cgg gcc ttt gtc ccc cag ctc cgg 35481
Gly Ser Pro Ala Ala Arg Arg Ala Phe Val Pro Gln Leu Arg
                840                 845
agt ggg aag ttc aac gtc ttg ctg acg acg tac gag tac atc atc aaa 35529
Ser Gly Lys Phe Asn Val Leu Leu Thr Thr Tyr Glu Tyr Ile Ile Lys
850                 855                 860                 865
gac aag cac atc ctc gcc aag gtaacgtgtc cctgtgggaa atgccaggcc 35580
Asp Lys His Ile Leu Ala Lys
                870
atgggccgag tgctcacacg tgggtcacgc tgcccgtctc ctccaaagcc cctacaagtt 35640
tcttcccgga gtcccacctg catgtgcgtg aagacagctg ccctgtgtag gggaaaggcc 35700
taggtggggg cgacctcagg tttacctccc taactgtctc caggcaggcc ctgagtcagg 35760
cccagaagct ggggccatct acacagcatg cgctctgcct cctctcgggc cttctgccca 35820
gagagcctca gcaccaaggc gtctcctgac ccgcctacag agtccccatg gggccttggc 35880
cagaccagtc ttgtcctcct gtctcattgc cttggccgtc cctccctggg gccctggctt 35940
ctcaccacag gccatgggct gagattcagc atgtggggga gagctcgagg gccaaatacc 36000

atgactgcct gctggccttg ggcagctggt tcgggctgtt ctgtgagggt cctactgtcc 36060
tggaagcctc ttctgggacg aaactgtgaa gggagtccca gcgtggccat gactgagaat 36120
acggcccaga tagaaaacag acatagccct ctgcctccaa aaactcatgt tcctcatttg 36180
aggaggggta aagatgtacc tggtctcacc cctaaacatc cagctgagtg agtgttgaac 36240
agtagagagt tttttgcgtt ttggtttttt ttttttgag accgagtctc gctctgttgc 36300
ccatgctgga gtgcagtgat gtgatcacag ctcactgcag gcctgacctc ctggcctcaa 36360
gcgatcctcc cacctcaccc tcctgagtag ctgggactac aggtgtgcgc caccactccc 36420
agctaacttt tttaaattta ttttttatgg agatggggtc ttgctgtact gcccaggctg 36480
gtctcaaact cctggcctca aggaatcctc tcgccttggc ctcacaaagc actgggcaac 36540
tgtgtctggc ctaacagtag agttcttgac ccaggtatgg ggggatggag gagctgttta 36600
tgggtggctc cgcctctcag tgcatggctg gagtgacagt actggaatac tggggctgtg 36660
aactgggtgt tttcttttct ttttctcttc tttttttttt ttattgagac agagtctcac 36720
agctgtcacc caggctggag tgcagtggag cgatctcagc tcactgcaac aacctctgcc 36780
tcctgggttc aagtgattct cctgcctcag ccttccgagg actgggtgct ttatcccgga 36840
gccaacaagg gaggaaactg aggcccaggg agctgggtat cccacgtgca ggctcttacc 36900
tcaggtccat gcttcaggtg cctcctttgt ggctcacccc tgctgactcc ttacaaagcc 36960
aggccgttgg caggcacagg aaccaggctc tgaaaccccg tgctcccctg ccagtggctc 37020
agttgatgta atcaccctgc agtgggagtg ctctttgagt gcggccactg cgccccctcc 37080
cgacccgcat ggccctgtat gtagtggcgc tggcccctac cccatggggc ccacattctg 37140
aaccacaggc tgctcattag aaatgtaccg tcaccaaccc agttcccctg agaccctcca 37200
caccagcagg acaagtcccc cagtcagcaa gatgtgtctt ctgccagaac gtgctcggca 37260
tttttctgtg ctgtagattc atatgtgtga ttttgcaggg aagccagatt gcttttttgc 37320
aatttgcatt gttcacctgc caatagtcat ggggtcacgg gcctgtctct gcccaaggct 37380
tgcaagaggc tacgttgtgt ggctccaact cggtgagtca gccccggggc aggacgtcag 37440
gcctgtgctc tccacccagc tgcgctcttc cacctggagc cttcctggct gctgggcgca 37500
gagtgggaga ttctccccat gtgccgggcc acctgctgcc ccctgccctg attgcccact 37560
ctggggcccg cag atc cgt tgg aag tac atg att gtg gac gaa ggt cac 37609
Ile Arg Trp Lys Tyr Met Ile Val Asp Glu Gly His
        875                 880
cgc atg aag aac cac cac tgc aag ctg acg cag gtg ctc aac acg cac 37657
Arg Met Lys Asn His His Cys Lys Leu Thr Gln Val Leu Asn Thr His
885                 890                 895                 900
tat gtg gca ccc cgc cgc ctg ctg ctg acg ggc aca ccg ctg cag aac 37705
Tyr Val Ala Pro Arg Arg Leu Leu Leu Thr Gly Thr Pro Leu Gln Asn
                905                 910                 915
aag ctt ccc gag ctc tgg gcg ctg ctc aac ttc ctg ctg ccc acc atc 37753
Lys Leu Pro Glu Leu Trp Ala Leu Leu Asn Phe Leu Leu Pro Thr Ile
            920                 925                 930
ttc aag agc tgc agc acc ttc gag cag tgg ttt aac gca ccc ttt gcc 37801
Phe Lys Ser Cys Ser Thr Phe Glu Gln Trp Phe Asn Ala Pro Phe Ala
        935                 940                 945
atg acc ggg gaa aag gtgggtttgc ccagctgtgc ccatgctgac ggttccaggt 37856
Met Thr Gly Glu Lys
    950
gcggctggct ttgctggttg gaacgtgttg agcaccagct acagctggct gggcctgtgc 37916
tgggtgcctg gtgagagtct gcatctgcat ggagcagggg agccctggaa cccaaggctg 37976
gggcagccac aagggcccca ggggagcagg gcagaggtca tggtggtgaa tctcggaatg 38036
ctcggggcgt ctctgaccct atgctccagg tgcagagtgg atggtgagga agatggccct 38096
gaggcagctg tgggaccgca agcgtgtgga gagcacagct agaggctgta gcgtgctgta 38156
ggccccttta ggtgccctca gaactcaccc tcagggcagt gggaaggaaa cttggaagat 38216
tccagttagc ggggccacat ggtcaggttc tgtcttacgt tgttttcaaa aagctcagtg 38276
gccagtgtgt ctcaatgtct gggtggcacc atcagccaga ctgtggcatg gcagcatcat 38336
cattgatgag tggtcccaca ccagcactga tccctgagcc tagagctcct tgctgttgga 38396
ggttgaaact gtgggacaga ctgtgtttcc tgctgagggg tttaagtaac cagatgctga 38456
agtgtgggca gaactacaga ggttacctcg aagtgccctg cccagcacgg ctgggcccac 38516
ccccttcttc ccaccttaga caggtccagg gcagctgggg ccttcctggc ctcatgcgtg 38576
agttccctgc cctctcggct gctggtcaag gcagagactc ttctgatggc gaaaccgagg 38636
cattccccaa agcctcgtga taaaggagtc cccaattttt agactcccgg gacccccaca 38696
ctaagggcca ggatctgatg ctatagtttt gtcactttct gagccgagca aaagctctgc 38756
caaaggattg ccagggaagc aatagaagaa aagttttggc gtcgtctcat tttcctttaa 38816
cttttttaat taaaacattt taaaagttaa ttattagtag aggagtaggc gccgtgggag 38876
gtcagggttt ctttcacctg gttccaccca ggctgaatga gagtcccttg gggctcccgg 38936
tggcagagcc ctccctgaga atcacccgct ataagcccca tgtggtcagc ctggcccctg 38996
cagcgctcct ggcatgagct tggcgtgggg tgggtctgtg gacactccag tttgtgttac 39056
tcgtggagaa gcagcatgcc cgctgccggg ggtctttctg gtgaggcaca gggagcagag 39116
tgctctgggt actgcggttc catgtcccag gaattacatg gagggaacca gatcctcaca 39176
gtttcctaag gaagagggtg ccctgcctgg ctcccagaaa gcataaagca ggctgaaaag 39236
ccacgtgcca agggcaagat caccccaggg gaccccgtcc accctgtccc cacatccgca 39296
ccttctagtg agacctctgt cgccctcctt tggaggtaac gcttgcttct cctgtcttgg 39356

gggcttccag gtg gac ctg aat gag gag gaa acc att ctc atc atc cgg     39405
           Val Asp Leu Asn Glu Glu Glu Thr Ile Leu Ile Ile Arg
               955                 960                 965
cgt ctc cac aaa gtg ctg cgg ccc ttc ttg ctc cga cga ctc aag aag     39453
Arg Leu His Lys Val Leu Arg Pro Phe Leu Leu Arg Arg Leu Lys Lys
            970                 975                 980
gaa gtc gag gcc cag ttg ccc gaa aag gtgatggagt tttgagggga           39500
Glu Val Glu Ala Gln Leu Pro Glu Lys
        985                 990
gccaccagtg aagcagcctc acgtgggggc tttctccagg gctgggcgtg ctcagggcct  39560
ctccccacag tcccaggcct gccctggtca atccagcttg ggggtggcga tgacgccact  39620
gggtctgtaa agccctgtgc tgctgttgta gggattgaaa agcactcata ggctcacata  39680
gctcacaata ggttagagca caaaatccca cttcctgtca gggggccagg tgcctgtgcg  39740
ggctctcctg gccacatcag gaggggccgt gggcttgggg ttgccccccg gccccccatc  39800
atggctctgg gttttcaagg cctgctgcag ccacacccct gggcccagtt cccacagcaa  39860
gacccatgga ctcggggtcc ccggctgcag ttgtccctct tggctctttg tttcctggct  39920
ggctgtagag aggcccccag aagatggctg gggggccctt tctggcccag taaccctctg  39980
gtatctctgc ctggtgcttc ctctcattgc ccaggtctcc cctcttctcc ccagctgtga  40040
gggtctcctc acccacaccc cagtgtgctc tggtcagagc tcatatgaca gggccgaggg  40100
ggtcagagct gggttcggat ggggggagtc aggcctcaag ccaccttggg ccctcgtgag  40160
cattatgtgt cccctgcag gtg gag tac gtc atc aag tgc gac atg  tct gcg  40212
                    Val Glu Tyr Val Ile Lys Cys Asp Met  Ser Ala
                                995                 1000
ctg cag cga  gtg ctc tac cgc cac  atg cag gcc aag ggc  gtg ctg     40257
Leu Gln Arg  Val Leu Tyr Arg His  Met Gln Ala Lys Gly  Val Leu
        1005                 1010                 1015
ctg act gat  ggc tcc gag aag gac  aag aag gtgggcccca gagtccccca     40307
Leu Thr Asp  Gly Ser Glu Lys Asp  Lys Lys
        1020                 1025
actgcattcc ccactgggtg tccaaggccg gcagcgtggc aggcagagca gagcgtgctc  40367
tgaccatcgg gtcatgatct ggtcatgatc cccagggcat ctggccagcc ctggttatga  40427
tggctggtgg cttgtgtcag gacacactga ctcagctgcc agtggcttct cctttgccta  40487
tgaaacactg gctccttctg caattggcag cctgggccca gcactaaccc cagcagggtt  40547
agagtgttct agaatgcccc ccttctctgt tattaagtga aaggaaggga gcgggctgtc  40607
tcctgcaggg gctgctctga gaatcccagg ccccaggccc cacctgcctg gctctcctca  40667
ccagcagtga aattatcctc ccaggaaatg caccagaccc cttgattctg cccccaaggc  40727
cttgacctcg gcctggcttc cccgagacgt tgtgtctgtg ccccttctcc cctgagctga  40787
gcagcagtgt gttccctagt ccattcccag cccagagcat gcaccccccg ccccgccccc  40847
acctccttcc tcatcctgga gcccagggag ccctgagtca tggctcaccc ggctgaggcc  40907
ctcttgccca gctgagaccc tctagggacc cccacagtaa cagtgcccag aacaaagctg  40967
gcatcacaaa gcatcgggga ctctgcctca gccctttgcc agtcttgttt ccgtcccgtc  41027
ctgctggccc tgtccctgcc atctgccctc tgctgtgcca ggcagtcatt tgtttcccag  41087
ggcttgttgg gggcctccgg gcctccagcc tgccatctcc tcactcccaa ttgctgtgcc  41147
aaaagccact cttccccact agagcgtccc catggccctt ctcccaacac ccacccatcc  41207
accaagccca ccccacccca ggagggcaag accccatttg ggtccctctc atctgccttc  41267
cag ggc aaa ggc  ggc acc aag acc ctg  atg aac acc atc atg  cag     41312
    Gly Lys Gly  Gly Thr Lys Thr Leu  Met Asn Thr Ile Met  Gln
            1030                 1035                 1040
ctg cgg aag atc  tgc aac cac ccc tac  atg ttc cag cac atc  gag     41357
Leu Arg Lys Ile  Cys Asn His Pro Tyr  Met Phe Gln His Ile  Glu
            1045                 1050                 1055
gtgagcccgc cgcggctggg acggctcagg ccctgctgtc tgctgagctc ctaggcagag  41417
ctggcttctc ctcgacagct ctttaaaaac agactcgaat attttcatta ggtaatgcct  41477
gtacatctgt ctctcatttg gtcttcgctt caccagtgct tacgggaaga atcccagcag  41537
ttagggcgca acgtgcgata ggaatatagg gccctgaaag cttgccctca gagagcagga  41597
gcgtttggag acttctctgg ggatgtgtcc ccccacagtg gggtcctgct gcgtctcttt  41657
ggctcactgg cttttctcac gttgcggggc gtcaagagtc accaccgtct tcagtgaagt  41717
gtgggggagt cagaaccctt tttggtcatg gtggctttga gaatctgggt tcttagaagc  41777
atgcatatgt tcgtgtttct tggtgtaaag tgtcagggtc gttggagacc gcctgctgcc  41837
ccgagagccc ctggggctgt ctcagcaccc tgagcgtggc tgtggcccca tgacgtcctc  41897
gtgcattcag ggtgtgaaga gtgctgtatg ggatgtcctt tccttcgttg gaggagtgtg  41957
agggaaagga tgtcagtgga ctaagaaaga tggttttggc atctgtgtct ggtctcagag  42017
ccgccgtggg cccgtgccga gcacacagtg tgggggcttt gtcctctgag cacgagcggt  42077
gtgtgcggac cgcagcgggg cccggtggcc tgctcctgcc tgtcactgac ccctctctcc  42137
ttgccttgca g gag tcc ttt tcc  gag cac ttg ggg ttc  act ggc ggc     42184
             Glu Ser Phe Ser  Glu His Leu Gly Phe  Thr Gly Gly
                         1060                 1065
att gtc  caa gg  gtgagaagct tcccaactgg atggggtggg caggtggtcc       42235
Ile Val  Gln Gly

    1070
acccagaggt tttctgtcgt tttgttggct ttattgctgc tgttatgttg tccaatttca 42295
aaggcagaaa attagaaaat acagaagaat ccaaagcaaa taatacaaat cttttttttt 42355
tttttttttt gaggtggagt tttgctcttg ttgcccaggc tggagtgcag tgacacaatc 42415
tcggctcact gcaacctctg cctcctgggt tcaaatgatt ctcctgcctc agcctcccga 42475
gtagctggga ttaccggcac ctgccaccac ccagctaatt ttttgtattt ttagtagaga 42535
tggggtttca ccatgttgac caggctgatc ttgaactcct gacctcaagt gatccacccg 42595
ccttggcctc ccaaagtgct ggaattacag gtgtgagcca ccgcgcccgg ccccattata 42655
caaatcttta gtgcttggag agctgaccac tattaacccc aagagacagg attcctgagg 42715
cagacctggc ttacagatcc cactctgccg cctccaagct ctgtgactct agacagcttt 42775
cttaacctct ctgagcctaa actctgctca ctagtacaga ggcactcttt acatcatgtg 42835
ggtgttttct atagcaccct ttgtaagggg cagcccagac tcctttaagc ccctaatgga 42895
tgtggatgct gtgtctagat tttcaccatt gcaaagaacc ttgctaaaag cattcttcca 42955
gctaaagcct ccattcaatt cccctggggt agtaagccat ttgcaagaag agaggcgcct 43015
aatgaaaaca tcacaaattc tttttgatgt gtttttttat actgagttgt ggaacagggt 43075
cctgtacacc tggcttcaag gctgggaggc ctggcttgga catgtggtca gccatatcac 43135
gtcgtccccc tgccagcctc agtgtccaca tctacaacac ttggggtttg gctggagaga 43195
acatgagaag ccgaggtggc ccttccttct gggtctagca gagcatgggt gtctccagtg 43255
tggcatttgc tcctggggct tctttatcgg gcaaggggac aaggtctggc ttcccggagc 43315
atccctgttc cagccccgtg ttgtgccggc catgtgactc acagctcctc tcattttcat 43375
gagtgtctgg gtttgacgag aaatacatgt gccaccctgg tgatacgcct ccccagcgag 43435
ggtggccggg cttatttcag ttgggggaaa tggctttgct gaagctttgg gtgggtgacg 43495
tctgcttagg atgcatgtct gtgcccttga acccggcgcc tggcctggag gcgggcgatg 43555
cacctcctgc cttacctgcc tgcagggttc caggtttaac atcctgcgcc ttctctcctg 43615
cctcctccac actccag g ctg gac ctg tac cga gcc tcg ggt aaa ttt 43663
Leu Asp Leu Tyr Arg Ala Ser Gly Lys Phe
        1075                1080
gag ctt ctt gat aga att ctt ccc aaa ctc cga gca acc aac cac 43708
Glu Leu Leu Asp Arg Ile Leu Pro Lys Leu Arg Ala Thr Asn His
        1085                1090                1095
aaa gtg ctg ctg ttc tgc caa atg acc tcc ctc atg acc atc atg 43753
Lys Val Leu Leu Phe Cys Gln Met Thr Ser Leu Met Thr Ile Met
        1100                1105                1110
gaa gat tac ttt gcg tat cgc ggc ttt aaa tac ctc agg ctt gat g 43799
Glu Asp Tyr Phe Ala Tyr Arg Gly Phe Lys Tyr Leu Arg Leu Asp
        1115                1120                1125
gtgagtatga gccagtgagg cgtttcttac agggttttgt tgttgtggct gccacagaag 43859
ctcctcactg gcattccctc ctgtgaggca gggtgaggcc caggcgctct gggtccagtg 43919
gccagagtgg gcctagccac aggctgagca tcttgggcac tcggcactgg gacaggccgt 43979
ggattggacg ccgggtgtcc tgactcctag gctgacagtc tttccacgac aatgctgggc 44039
gcactgggga aggggtgccc tgtgcacttt tagctggaag tgacagaatt ccataccatg 44099
tggacacagg aaagaaagca ccaataggct tacatagctc taaccccagg aggttaaaca 44159
tccttcaggc acagctggat ccaggcatgc aggtgatatt ggagagactt ttctccagct 44219
ctttgctctg ccttcttcca tcttggcttc ctttccacct gctcagtgtc cccaacaaag 44279
gcaggtccca ggctggctct catttagctg ctgtgagccg catgcccatc cctgggccat 44339
tcgtagcagc ccagtgactg ccaccttgcc acaggtcagg cctggacctg gaatcagact 44399
ctgtggtgag cacggaggga gggaagcaag cactgccagg caggagcagc agctgccgta 44459
gagaggctct cagcccaacc ccctgccctg cggtccctga tgggaaaggt caccgcctgt 44519
gtttctgcgc ctgcctgccc cagcacacgg gagctcaggg aggagaggct gtgagatgcc 44579
gacagggcac gtgagacatg tctgtcagtc ccagtgggtc agggagccct ggagtgagtt 44639
cttcgtggga gttactagaa ctgggcttac gagaaacacg agcatgagct gctgagacag 44699
acacggctca gccagtttgt tgttccctac acgacctccc agagcctttg ttgaagctcc 44759
tgtgcaaggt ctgtctcaca gcaggacacc ccagacctcc ataccccttg tcccgttgat 44819
cacttggacc tgtgtttgct gggccaacct gcccaggtgc cacccggagt gtgccctgtg 44879
tgtccctcca aactgcaggt cctccccggg gttctgtcca ggaccactct acagacacga 44939
gtctgttgca ggggtctccc cggcttgcct tcttccttgg gggcttccca ctttcagctt 44999
tgaggctgaa aaccaaaatc cttcagggtc ctcatggccg ggccaccccc caccctttcc 45059
tctcctacgg tctctccagc ggcttctccc gtcctcgaga gggccaaggc ttcctgaggg 45119
ccatcccgtc tgtgtcgtgt ccctgactcc cccaggagag agcctggttc ccacccatcc 45179
cagtgccctt ctgcctactc cataaggaca aggactttgg tcagttgaac tacagatgtg 45239
tcctctgcat gtgatgtggt catggccaca gggccagaga atccattgcg agtagaggaa 45299
gggtaggagc tcagtgaagt ctcctgagac tgcatctccc atgggtgggc tggcatctgg 45359
gagccgggtg gcccctcacc tcagcccctc ctccgagctg gagtagcagg ggcacatctg 45419
gtgcctgaga gagggtggga cggcaggtgg atggccaggc tactgtgggc ttctgcaccg 45479
tgcctgggca tgtgtaggct ggtggcagga aatggccagt ttttatttct tctttttttt 45539
gagacggagt ttcactcttg ttgcccaggc tggagtgcag tggcatgatc tcagctcact 45599
gcaacctccg ccgcctggat tcaagcgatt ctcctgcctc atcctccctg gtatctggga 45659
ttacaggcgc ccgccactac gcccagctaa ttttcttgta tttttagtag agacggggtt 45719
tcaccatgtt ggccaggctg gtctcgaact cctgtcctca ggcgatccac ccacctcagc 45779

ctcccaaagt gttgggatta caggcatgag tcaccgcgcc cggcctgcca gtttttattt 45839
catcaccaaa atgacttcag tgttcagggg cacctctgtc cgaactccca gcagcgcagg 45899
gagtgttgac attgccttaa tgcccacaac gctgagggca tcgtgggttg tcacccagga 45959
gacctgctct ggctccttga cttgggtttt ctggggcatg gggacactga tcctgccaga 46019
aaggacacga gcccccttta taaggtctcc atgcttttac cgtagaagat tctctttgtg 46079
aaccacaaaa ctttttgaga cagggctgaa ggcaggtgca cccacagtca ctgtccaccc 46139
acccccaggt cccttgtgtt gcgagcggcg gtgttgccgg cattggccgc tgtgtcttcc 46199
gctccccatg gaatggcaca gggtagagta gaggagagaa tccagggctg tggtggtggt 46259
ggctgtgctg acgctggacg ctgtggccta gctgtgggct gggtgctgga ctctgtgctc 46319
caggcccacc tcctctagtt ctcccagcgg ggcctgtttt catttctggg atgtgtggag 46379
agacgttttg tggtgaaaca ctggaaatcc agttattcgc cagtggccca caggcccgtg 46439
tggagagtgc ggagccaggg atctggggat gctggcaggt gctgatcctg ctcctgctct 46499
cagaagcagg tgttccttgg tgtccccact ctacccctga ggtcaccccg ctgaccctgt 46559
tctcctctgt gcccgtcag ga acc acg aag gcg gag gac cgg ggc atg 46607
Gly Thr Thr Lys Ala Glu Asp Arg Gly Met
        1130                1135
ctg ctg aaa acc ttc aac gag ccc ggc tct gag tac ttc atc ttc 46652
Leu Leu Lys Thr Phe Asn Glu Pro Gly Ser Glu Tyr Phe Ile Phe
        1140                1145                1150
ctg ctc agc acc cgg gct ggg ggg ctc ggc ctg aac ctc cag tcg 46697
Leu Leu Ser Thr Arg Ala Gly Gly Leu Gly Leu Asn Leu Gln Ser
        1155                1160                1165
gca gac act gtg atc att ttt gac agc gac tgg aat cct cac cag 46742
Ala Asp Thr Val Ile Ile Phe Asp Ser Asp Trp Asn Pro His Gln
        1170                1175                1180
gtaaaagcgg gccgggcccc aggtcgagga gaaggaaggg ggtgcctgca aaacctcgag 46802
gagacggccc tggcttgagg gtcctccagc ctcctccaca ttgtcttgga ccccaggagc 46862
cgggaggagc tgcacccata tctccatagg tcatcaggga gaaaagaggc ggggtcctcc 46922
tgtttcccaa aatacaaaca gcctttccct ctgattggga aagcagtgta taagccatcc 46982
gtcggtttgg tttttcttaa atcttggaat ggcacccatg aggaggtgga aagtatcctg 47042
atcaatctgc gccgtcactg tggggcggcc cctgcagtgt gccctgtgag cacacacact 47102
ggcgcttgtt cagacacaat tgggggtaaa ctccatgcca cttcgtttac ttcctcctct 47162
tgttccataa ccatgtaccc ctcatgggcc tcggcatcgg tggataccag gtcatgctgt 47222
tacatagcta caagaatttg aaaatgagtt aaacttttct ctgtgtttca ttataagact 47282
gataagttct tattgttttt aacatgtaaa caccagtgac agtcaggagt cccttttccc 47342
ttcctctcca accgtgctct cttctgagcc tctgccagcc catctgcctt cgcacatgta 47402
tgtactgaga ttaacacaga tgcggcgtgg cctagcatgt gcctcttcca ccaagcagtg 47462
ggtgtcaacc gccgagtgca catcggaagc tacccagcca tctaatcggc caccaggcgt 47522
cccggcagat ggccacccct ggacgtctgc tctcaaactc tggttggcag gcatccgtct 47582
tttctgttga tcagcctggt caccaaccca gcagggtccc tctttctctc agagtgcttg 47642
tgcaaacacc ctaggtctgc gcagccctca gacatggggt cgctccaccc cagacattgt 47702
cctcttcgtg ttctggttgc tactgccaaa gtggtttcac tcctacgctg gggtgtgaat 47762
gtaccctgcc cttccctgca gcgtgagggc agcagcgttc tcagtgtgta aattgtcgct 47822
tgtctgatgg gtgaatgccg tggtggtcac tggtggcccc ttttacgatc aggctggggt 47882
gtagcttctg cccagaccct ggtgaccagc cagtttctta ggaggtggtt gagttgtgtg 47942
tccccgttgg caggccggca gcaggcggaa gctgcacctc agggctgggg gcgcgttggg 48002
gagggctggc cctgccagga agtgtgctga cagggcagga gggctgagcg gcagacacaa 48062
ggcactcctg gtccccacca gctgtggcct ctgccacccc atgggtgcca gtcttaagtt 48122
tcagagcatc actggtactc accctggtcc tgcagctcct gggcccagag acaggatttc 48182
gtgttagcct cctcgtactg tgctggggtg atgataaaac taacaaccca ggctggacgc 48242
agtggctcac acccgaaatc ccaacatttt gggaggccga ggtgggtgga tcaactgagg 48302
tcaggagttc gagaccagcc tggccaacat ggcgaaaccc tgtctctact aaaaatacaa 48362
aaattagcca ggcatggtgg tgcatgcctg tagccccagc tactcaggaa gctgaggcag 48422
aaaaatcgct tgaacttcgg gaggcagagg ttgcagtgag ctgagatcgc accactgcac 48482
tccagcctgg gcgacagagc gagactttgt ctcaaaaaaa aaaaaaaaga aaaaaagccc 48542
atccacggca cctgcattgt tgggagctaa ctgcaccagt cactggccca cactcctggt 48602
gcttattcac tcattcgagg tatgcagtag ccacgggttg ggccccatgt aggctgaggg 48662
agttttgagg ctcacagagg tgagacctgg ttccggttct gtgtgcactg tgagagtggc 48722
agggcccaca gccaaaccaa gcactgccac aacacggccg agcttcggcc ccctgttgta 48782
aatggtgctc gactgtgtag gtccacgctg tggggagctg tgccgctgcc acgggagctg 48842
cttgagaata taatcccctg gggggttggt gctttcttcc cgaatatctg tggggtccca 48902
ataaggtaga aggtggcact tctggaggaa cgtgatgaga gtccccttcc cccgagggac 48962
acatggcggc ccaggctcca ccagctctgt tttcatgcgg cggcaggtca ggctgggcag 49022
aattgtcagg ccgagggtgg cacgcacagc acacctctcc agctagtgtc agaggccacc 49082
ttccctttta tgacctcctg ggctcctttg ggactgactg gcacctcttc cccag gac 49141
Asp
ctg caa gcg cag gac cga gcc cac cgc atc ggg cag cag aac gag 49186
Leu Gln Ala Gln Asp Arg Ala His Arg Ile Gly Gln Gln Asn Glu

    1185                    1190                    1195
gtg cgt  gtg ctc cgc ctc tgc  acc gtc aac agc gtg  gag gag aag      49231
Val Arg  Val Leu Arg Leu Cys  Thr Val Asn Ser Val  Glu Glu Lys
    1200                    1205                    1210
atc cta  gct gca gcc aag tac  aag ctc aac gtg gac  cag aag gtg      49276
Ile Leu  Ala Ala Ala Lys Tyr  Lys Leu Asn Val Asp  Gln Lys Val
    1215                    1220                    1225
atc cag  gcc ggc atg ttc gac  cag aag tcc tcc agc  cat gag cgg      49321
Ile Gln  Ala Gly Met Phe Asp  Gln Lys Ser Ser Ser  His Glu Arg
    1230                    1235                    1240
cgc gcc  ttc ctg cag gcc atc  ctg gag cac gag gag  cag gat gag      49366
Arg Ala  Phe Leu Gln Ala Ile  Leu Glu His Glu Glu  Gln Asp Glu
    1245                    1250                    1255
gtgagcccag caccggcccc gacccctccc cagcgtgaat ggtggacgcg tgagcggctt  49426
tcatttttgt ttttttacct tttttgcact cttatttttt ttgcatccct ttggagtaaa  49486
gggagtgtgg gctgaacgga aagaggatga gtacttgctt tttctttgaa gtggtttttt  49546
tttctaaact gctggtgaaa gacgccggat tgacagccct ggagactgaa gtcctctatt  49606
tatccacag agc aga  cac tgc agc acg ggc  agc ggc agt gcc agc  ttc    49654
          Ser Arg  His Cys Ser Thr Gly  Ser Gly Ser Ala Ser  Phe
              1260                    1265                    1270
gcc cac act gcc  cct ccg cca gcg ggc  gtc aac ccc gac ttg  gag      49699
Ala His Thr Ala  Pro Pro Pro Ala Gly  Val Asn Pro Asp Leu  Glu
            1275                    1280                    1285
gag cca cct cta  aag gtgagagggg tagttcagtc tccatgccca ttcaatcctc   49754
Glu Pro Pro Leu  Lys
            1290
ggcttctcgg ctgagacggc cagcaagggc cctggtccca cggagcgtgc gtgtgcgtgt  49814
gcgtgtgtgt gcctttcgct gccgtgtggg tccccatcca ccgcagccgt gccgggacca  49874
ccagctcatt cccacggacg ccgccgctcg cctctgagct cggccgccgc ccaccccggc  49934
ccctcctcag cggcactgac agtttgcaat cttatag gag gaa gac gag  gtg ccc  49989
                                         Glu Glu Asp Glu  Val Pro
                                                          1295
gac gac gag  acc gtc aac cag atg  atc gcc cgg cac gag  gag gag      50034
Asp Asp Glu  Thr Val Asn Gln Met  Ile Ala Arg His Glu  Glu Glu
        1300                    1305                    1310
ttt gat ctg  ttc atg gtaagcgctg caggctggat ggggcagttc aggcatccca   50089
Phe Asp Leu  Phe Met
        1315
ctctgctgcc accaggagca aagcagacgt cctagtgccc atggtggtat ccctagcagg  50149
tcagggagcc agggacagct cacagtgcag cccactccca cctccagact gacccgtctt  50209
ccacccccag tctcctgagg atggcatcgg agggcgagat gcacacccag ccttctgcat  50269
gtgacccgag acctgcccca ccagctctgt tttctaacgg gctctccagg gcttcatgca  50329
ctccctttca gagggagttc gccctatcca aggccaaggg actgaccagg ccttcagtcg  50389
cagagccccc ttgcccctgg gtgggaaaca ggaaataagc cacccaagca ggggcccctt  50449
ggcccgcagg cctcatgcct ccaccaacgc tgggccacgc agctgctgcc cccctgctgg  50509
ggtctgcagc cctcttgtgc aaccttccat cttttcgagt ttcctctgcc tcctgaggca  50569
gagcctctag tcagggtctg acggagccag gccaggtcag ccactgaaaa atcgagagct  50629
actgtttaac tctcgcagca gcgtggagcc ccacgggcag agaaaggccc ttctgaactc  50689
tcggtgttct ggctctagcg tgcccctggt gcctgcatgc tgatgcctct cccgttgcct  50749
ccctgcccac cag cgc atg gac  ctg gac cgc agg cgc  gag gag gcc cgc   50798
               Arg Met Asp  Leu Asp Arg Arg Arg  Glu Glu Ala Arg
                       1320                    1325
aac  ccc aag cgg aag ccg  cgc ctc atg gag gag  gac gag ctc ccc      50843
Asn  Pro Lys Arg Lys Pro  Arg Leu Met Glu Glu  Asp Glu Leu Pro
1330                    1335                    1340
tcg  tgg atc atc aag gac  gac gcg gag gtg gag  cgg ctg acc tgt      50888
Ser  Trp Ile Ile Lys Asp  Asp Ala Glu Val Glu  Arg Leu Thr Cys
1345                    1350                    1355
gag  gag gag gag gag aag  atg ttc ggc cgt ggc  tcc cgc cac cgc      50933
Glu  Glu Glu Glu Glu Lys  Met Phe Gly Arg Gly  Ser Arg His Arg
1360                    1365                    1370
aag  gag gtg gac tac agc  gac tca ctg acg gag  aag cag tgg ctc      50978
Lys  Glu Val Asp Tyr Ser  Asp Ser Leu Thr Glu  Lys Gln Trp Leu
1375                    1380                    1385
aag  gtacatgctg gagaggccca gcagctgccg caggccagcg ccaggcaggg        51031
Lys
1390
ctggggagac aaagggccca ccgccaggac tcaggcctgg gtccaaaatg ctttccttgg  51091
gccactcctg gccaggctcc gcaggcagcc gagagccttc cgatgtgggc cagggatggt  51151

caggtccttt tggctctgcc ttggaatgca agaaggaccc acggttcctg agcagctcaa 51211
aacctgctgc tggttatggt tggtctttca agtaaaaggg tttacctctt cccgaggtta 51271
aaaatcatgt tgctgatctc agtggactgc tggctttccc agtacctgcc aggagggagg 51331
cacaagggtg aatcatggac tattgaggcg ccctgcaaag cgttgcactg ctggctgcag 51391
gcagcctcac tttgggcaag tcccccccca tgagctgggg agactcagtc atggggaggg 51451
aggagagggc aagcccccaa gggcctctga acctggcagg tgctgcacgg ggctggcagt 51511
tgcttgtgga gctgacgtga gggcccgttc tcagcagatg gcagctggcc ctgcaacagc 51571
agctgcagat gtctttgagt cagtcgtgcg gtgctcctgg ggcttcaccc gacccaactt 51631
tcccagccca aggcacactg tgacagatgc ctggcactga tgtcctcact ggggttacgg 51691
tgacagatca gaccccttcc cagctcaggg gctctgagtt atagagacga tgattcagaa 51751
tccatccctg aagttgtgtc ataaaaatgt cctgaagcaa taattccgag aaggcaggtg 51811
gcagcttttg gctatgccat gcaaagtgcc acacccagga aagagccatg tggcctagcc 51871
catgtggcca aacagccccg gttccctcct gggacctcag cagcccagac tcatgagctt 51931
gcctgccaag ctgtgcacca gagggcgctg ttgaaacaca ccaggagccc aggtgacaga 51991
caggccatcc tgttccccag ccagcctccc aagcacagcg ctccaggacc cagaagagaa 52051
accctgaaaa gacagtgtga gccctgccac agacctgtgt gacagcagag ctgtttggct 52111
gctgtatgag tgtcaccggc cctgcatttt tttctttttt aataaagaca aagtcttgct 52171
gtgttaccca ggctggcctc cagttcctgg gggctcaagt gatcctcaca cctcagcctc 52231
ctgagtagtt cagactacag gtacacacca acacgcctgg ctaattttaa attttttgta 52291
aagtgggggt ctcactgtgt cactcaggct ggtctcaaac tcctgggctc aaacaatcca 52351
cccgcctcgg cctgcagagt agctgggatt acgagcatga gccacggtgc ccagctcagc 52411
cctgcatatt tgaggcagtg agtacctttt aaatttaaga tcagttcata cgtggctgct 52471
ctatgtacat ttttttctag gtgtaattta cataccatac agttcaccct tttggggtat 52531
atgatacaat catcttcagt gtatccaaga gttgtgcatc tctcaccaca gtccatttta 52591
gaacattctc atcgccccca aaagaagccc tgccccgatt tgccattcct ggtaattcat 52651
ggattctccc cagccccagg caaccactga tgtactttct gtccatatag ttttacctgt 52711
tctgggcgtt tcctgtcagt ggagtcctac agcgtgggat gctttggtgt caggcttttt 52771
tcacgcaatg taacatgctg acccatgatg cagcaggtgt gttgcattct ttgatggtga 52831
aataatcctg cctcgtacag atttgtcaca ttcggttttt ctgtccattc ttgatggaca 52891
cttgggttgt atccactttc gggctattac aaacaatact accgtgaaca ttcacgtaca 52951
cgtttttgcg tgcacgcgtt tctctttaat atgtgatttc tctttaatat ctgatttctc 53011
tttaatatgt gcctaggcgt agagttgccg gaacaatgtt agttctctat ttaacctttt 53071
gagggacccc agcctggttt ccttggtggc tacgccagtt cacagttcca ccagctgtgt 53131
atgagaattc ctctttctct acatcctcag caacccttgc tgttccctcg cccgctgatt 53191
ctagccatcc cagcacgtat gaagtgccat ctgagtgtgg ttggatttgc atttccctga 53251
tggctaatgg tgtggagtat ctgttcatat gcttcttggc cctttgtgta tcctctagaa 53311
agagatgtct agtcacatca tcctttaccc aaatttaaaa tatatttatc ttaatattga 53371
attgtaagtt atttacaagt gccttatcag atatcatttg taaatatttt gttccattct 53431
gcacattctc ttttcgcttc cgtgatatcc gttgaagcac agaagtgttt cattgtactt 53491
acagtcgatt tttctttttc acctgtgcct tggtgttgta tctgagaaac catcgcctat 53551
ccaaggtcac gaagacttgc ccctgtcttt tctcctagga gggttataat ttgatctctt 53611
ccatgtagat ccatgtaaga tccattttga gttaaatttt atacatgatg tgaggtaggg 53671
gtccatcttc atccatccaa ctggaaatcc agttgtccca gcagcatttg ttgaagagac 53731
tattttttcc cattgagccc atcttattac acagtatatt caaattcaca gttgtgaaaa 53791
atattataca aaacactgtc tccccactac tgttggctgt ggaattggca ccacttttct 53851
ggcagccatc tgttgccaag tgaactcaaa gccttcaaaa tgctcctatg gccgccatcg 53911
gacatggcca tgactgtggc agcctaaagc aacagaacta ttttctctgc attctgggcc 53971
agaagtctga acccacggtg tgggcagggc tgtgttcctt tcgaggccct aggagaggac 54031
ccttccttgg cttttccagc ttctgatggt tccagaagcc tcctggcttg gggcagcgtc 54091
accccatctc tgcctctgtc ttcatgggga cttctcctct gtgtcccctt ctgtgtcttt 54151
taagaacaat ttttaaaact taatcccaaa agtaaagacc tcatttgcaa agaaggtccc 54211
attccgaggt tccaggtaga catgagtctg gggggcaccc gccgctccgc cctgggtctg 54271
cagggggcct tctcctggtg cctcctgcct gctgtgccct ccatgctgct tctttgaccc 54331
ggttgccttc catcctcctg ggcccctcag gctctcagct tcccatcttc tctaagacat 54391
gtctgctcca tcattctggt gtctctctct tccctccttc ctggcacctg acacgtttgt 54451
taccccactt gtggggtgtt gctgtctgat caccatcttc cttccaaggg taggggaaag 54511
ggccctggct ggttcaagtc acagctctgt cctcagcgcc aggcacagtg cccagctcac 54571
tggcacagcc tcagattccc tggacacctg gctccccggc tgcatttttt accattggga 54631
taagttatta gtaccattga aaattaatac ctggggattc tcttaatatt gttcatattc 54691
atgttcttct cagcagtttt accatgagga atctgtctta aacaatgtca gttgtcaata 54751
gcgttattta aaatagcaac aacaataaaa aaaggtggtc agacctggca gcttgtgcct 54811
gtaatcccag cacttggaga agccaaagcg gggggatcat ttcagctcag gagtttgaga 54871
ccagtctggc caaaatagca agacctttgt ctctattaaa aaaaaaaatg ccaggcacag 54931
tgacacatgc ctgtaatccc aagcactttg ggaggccgag gtgggcagat cacctgaggt 54991
caggagctca agaccagcct ggccaacatg gcaaaaaccc gtttctacta aaaatacaaa 55051
aattagccag gcatggtggt gggcacctgt catcccagct acttgggagg ctgagtcagg 55111
agaatcactg gaacctagga gttggagttt gtagtgagcc aagatcgcac cactgcactc 55171
cagcctgggc gacagaatga gaccctgtct ctgaaaaaaa gggaaatgac ctaaatgccc 55231
aagaacaagg ggaggctaaa ttagggcacg ttgtgcactg aaacactaaa cagacattaa 55291

aaaattttgt tgtagaaaat tacaggaaaa gatatccatg acacagccag cagtgtggca 55351
cgtgggctac aattccagcg tggccttcag ttctgcacac gtgcgtcaaa ggtggggaga 55411
gttctggtgg tgggtggcgc tgagggctgc acaacactgg ggacgtgcct aatgcccctg 55471
aactgtgtac taaaacatgg ctgctttggt aaattttatg ttatatatga tttataatat 55531
atttttttaa atgcccagct actcaggagg ctaagatggg aagatcactt gagcccagga 55591
gttaaagtcc aaccagggca atgtagcaag accacatgtc taagaaaatt taaaaatcgg 55651
ctgggcgcgg tggctcacgc ctgtaatccc agcactttga gaggccgaca tgggtggatc 55711
acgaggttaa gagattaaga ccatcctggc caacatggta aaaccctgtc tctactaaaa 55771
ataccaaaat tagctggacg tggtggtggg cgcctgtagt cccagctact caggagggtg 55831
agggaggaga atcgcttgaa ccaggaaggt ggaggttgca gtgagctgag attgcaccac 55891
tgcactccag cctggtgaca gagcgagact gcgtctcaac aacaacaaaa aagaaaattt 55951
aaaaataatt tttcatacaa aaattagcca ggtgtggtgg cacgtacctg taatcccagg 56011
tacttgggag gctgaagcag gagaattgct tgaacccagg aggcggaggt tgcagtgagc 56071
cgagattgca ccactgcact ccaacctcgg tgacaagagc gaaactccgt ctcaaaaata 56131
taaaaataat ttttaaaatg tcttaaaaag gccgggtgtg gtggctcaca cctgtaatcc 56191
cagcactttg ggaggccgag gcgggcagat cacaaggtca agagtttgag accatcctga 56251
ccaacatggt gaaaccctgt ctctactaaa aatacaaaaa ttagctaggc gtggtggttt 56311
gcgcctgtaa tcccagctac tcaggaggct gaggcaggag agttgcttga acccaggagg 56371
cggaggttgc agtgagccaa gatcatgcca ctgcactcca gcctggtgac agagtgagac 56431
tccgtctcca aaaaaaaaaa aaaaaaaatc ttaaaaagaa gaaatagatg gaggccaggc 56491
ccagtggctt atatctgtaa tcctagcact ttgggaggct aagatgggag cactgcttga 56551
gaccaggagt tcaagaccag cctgggcaat attatgagac cccgtatcta caaaaaattt 56611
ttttaattag ctgagtgtgg tgacacgtgc ctgtagtccc agctacttag gaggctgagg 56671
tgggaggatc tcttgagccc tgggagttca agactgcagt gagctatgat ggtgccactg 56731
cactccagcc tgggcaacag gtcgagaccc tgtctctaaa aataaataag taaataaaag 56791
aaatagatgg ggagagtgtg gaacagaggt gtcaacatca cctccgggtg aagggatttc 56851
aggagcacgt tcttttctgt acagagaaga tagttctttt tttttggtca agaaattcaa 56911
ccattagttt tttaaagaca agcttgggtg gggtgcctgc tgggggcagt gctggtcttt 56971
cactgcagcc caggcacccc ttgagagtcc cagtgtgtgt tatgccccga gccagtcaag 57031
ctgaagggag agcgggtgcg ggggccctcc tccgtgtccc agcccggccc ctgggattgc 57091
gtcgcggcct ctgcttgtcg acctgggtgc tggctgtcct attttactac tattgaccct 57151
gaag gcc atc gag gag ggc acg ctg gag gag atc gaa gag gag gtc 57197
Ala Ile Glu Glu Gly Thr Leu Glu Glu Ile Glu Glu Glu Val
                1395                1400
cgg cag aag aaa tca tca cgg aag cgc aag cga gac agc gac gcc 57242
Arg Gln Lys Lys Ser Ser Arg Lys Arg Lys Arg Asp Ser Asp Ala
1405                1410                1415
ggc tcc tcc acc ccg acc acc agc acc cgc agc cgc gac aag gac 57287
Gly Ser Ser Thr Pro Thr Thr Ser Thr Arg Ser Arg Asp Lys Asp
1420                1425                1430
gac gag agc aag aag cag aag aag cgc ggg cgg ccg cct gcc gag 57332
Asp Glu Ser Lys Lys Gln Lys Lys Arg Gly Arg Pro Pro Ala Glu
1435                1440                1445
aaa ctc tcc cct aac cca ccc aac ctc acc aag aag atg aag aag 57377
Lys Leu Ser Pro Asn Pro Pro Asn Leu Thr Lys Lys Met Lys Lys
1450                1455                1460
att gtg gat gcc gtg atc aag tac aag gac ag gtaagcgagg 57419
Ile Val Asp Ala Val Ile Lys Tyr Lys Asp Ser
1465 1470
aggcggggag ggcgggggct gtaggggtcc ccgtgggagc aggcctggca tctgcactct 57479
gactctgcac actcaggctt gggccgctca ctctttcact catccacaaa cactgactga 57539
atctctgtgt ctttgagcct ggccctgagg aacatgcttt ctggaacagg gaagcacaca 57599
tgtatctgcg gtgatgagag ggaatgtcac atgtggtcga gaggagaggc aggggtgcgg 57659
gtctcggaga aggggacatt gcagcagagc cttgagagag gggaggttgg ggagagtgca 57719
ccagcagagg ccacagcagg cggagaggct ctgccaaagt tgtggcctgg tggcctcaag 57779
ggatgaggtg ggatgagggg ttatatggca gtaatgggtg ctgcccgtgt ggccaggagg 57839
ttggggacaa gaggctggca tgtctggagc agagaaagtg gcagccctct cctgtccttg 57899
caccctccca ggctacctgt gccatctctg tgttggtgag atggtggcca cactcctgct 57959
ctctgccagg ccctgctcca agcctggaga tcgctgtcct cacagacatg tcctatatac 58019
gaggcagcat gcggttccag ctgtggccac ggcccagcgg cggatcataa cgtgttggtg 58079
gatcatgagt gccatttata tgtttttcat ttactaatat ccttttccgt gagcgactct 58139
atatgggtac catttaaata agaagaaagg aatgttctca gcaactcccc agggaggcat 58199
gtgcaccttc tgccagccca gggcaggtca cagagctggg caggagccag caaaggtgta 58259
aggcccatgc ctggcccagg actcacccag tcttccactc tccaaggtct tatttcactt 58319
cctggtacac actggccagt acggctgtcc ccgtaatggg agggttgtac tgtttgattt 58379
taaataacag tttgctgtta gtcatgaagc agagcgtggg ggaattgtat ctgttggagg 58439
ggaaagtgaa gggagcccac atgcatggtt tggagagaat cttaccaagt gtgaagtttt 58499
agaggatggt aacaattgga gtgcctaaat taatgagtca aaattaccgc gcaaggccag 58559
ctgtggtggt ccacacctgt cagcccagca ctttgagagg ctgaggtggg aggatcactt 58619

gaggtcagga gtttgagacc agcatggaca acatggtgaa aacccatctt tactgaaaat 58679
aggaaaacaa ttacctgggc gtggtggcac gctttttggg aggctgagac aggagaatca 58739
cttgaaccca ggaagtggag gttgctgtgg accaagattg caccactgca ctccagcctt 58799
gggtgacaga gcaagacgcc cactccaaaa acaaaatact gcttaaaaag gttcttttag 58859
ccagatgcgg tggctcatgc ctgtaatccc agcactttag gaggccaagg cgggcggatc 58919
acttgaggtc aggagtttga aaccagcatg gccaatatgg cgaaaccccc tctctactaa 58979
aactacaaaa attcgccgag cgtagtggtg catgcctgta atcccagcta ctcgggaggc 59039
tgaggtagga gaatcttgaa gctgggaggc ggaggttgca gtgacccaag atcacatcag 59099
tgcactccag cctgggggac agagtgagac tccgtctcaa aaagagaaaa aaagaaaggt 59159
tcttttatta ttggaagaga aacagatgat gaaggaaagg agacatattc aagttcaatg 59219
ccagatctga aaatgtcatg tgttgtcagt attgagaaaa aaatatatag actctaattc 59279
acaaacctaa ctccaaggct aagtcctcaa agaggaaagc aagtggagga cttaagttac 59339
ttaaagtgcg ggatacagct gcgggcagta gcacatgcct ggggtcccca ctacttggga 59399
ggctgaggtg ggaggatcac ttgagcacag caggtccagg ctacagtgag ccatgatcac 59459
acagctgcac tccatcctgg gtgatggaat gagaccctgt atcttaaaaa aatgaaataa 59519
atacagaata cttatttaaa acatgggcca ggtatagtgg ctcgtgtaat cccagcactt 59579
tgggaggctg aggtgggcgg atcacttgaa gttaggagtt tgagaccagt ctggccaaca 59639
tggcaaaacc ccatctctac aaaaaataca aaaatagaaa ttaaactggc atggtgatgc 59699
gtgcttgagc tcccagctcg ggaggctggg cacgagaatc gcttgaaccc agaaggcaga 59759
gggtgcagtg agccaagatt gcaccactgc actccagcct gggtgacaga gtgagacaat 59819
acctgaaaaa caaaaaacaa aaacaaaaaa agggaattcg cagaaatcta atgacaatga 59879
cagaccctcc ccatgtttta tttctaatga taatcttgtg agggaaactt aacattacca 59939
acctaaagaa gacccttgaa gcgcagtggg gaaagcacgg gtgtctccat cacactggtc 59999
tcctgggtat ccacgtagag aggatgagcc tcgaccaccc ccacacacgc acacaccatg 60059
cacttggatc cacgtgacat ggactgtgga tgtaagaacg tgaaaggtaa aacaaaattc 60119
tagaagaaag cacgggagaa aatcttcatg agtaggcatc gatttcttcc acgggagcca 60179
aaagccacta atcacaaaca attgagaaat tagacctcac tgaaattaat aacatctgtt 60239
tattaatgga cacgctgatg gagacagtga aaactcctca tagatgagga aaagacaaca 60299
caatttaaag ctggacaaga gccgggaaca gacgcctcct tacacaggac atcccaggac 60359
taatcaatgt ggggaaaggt gctcacgtca ttgcattagc atcacaactt caagaaaatg 60419
ctcgttaaaa ccagagtgac ttgcaccagc acccgcccca gaacggccga atgcccatca 60479
gtgctggcaa gtctgaagca gtctggcctc acaccctgcg tgtaggagta tgcagctctt 60539
tggcaatctc ttctgttttg ctttgtttgt ttttaaccat cgaactgcag aagataatat 60599
tttctgaggc tcagcctgta cataccctct gacccagcag cctgcagccc tcgttctacc 60659
caggagagct gagtgcatct gtccacaccg acagacgtgt ccatagccac tttggtcata 60719
atctcccaaa acaagaatca gcccagtgtc catctacagg ggcatggatc agtggaggcc 60779
taatcgtaca gggccccaaa cagctgtgaa aacagacata gtactgctac ctggaagaag 60839
cttacatttt gttgagtgaa agaagccaga cactaaaaag ggcatactgg ccgggcacgg 60899
tggctcacgc ctgtaatccc agcactttgg gaggccgagg cgggcggatc acgaggtcag 60959
gagattgaga ccatcctggc taacatggtg aaacaccgtc tctactaaaa atacaaaaaa 61019
ttagccgggt gtggtggtgg gcgcctgtag tcccagctac tcgggaggct gaggcgggag 61079
aatggcatga acccaggagg cggagcttgc agtgagccga gaccgcgcca cggcactcca 61139
gcctgggcga cagagcggga ctccgtctaa aaaaaaaaag agggcctact gtgtgcctcc 61199
agttctgtga gggtcagaag ctgatccaca gtaatggctg ccaagcagtg cttaccttga 61259
gagagggaat agcaggaaag ccacacgacg agccttctag gatactgggg atattctctc 61319
tatagatctg gatgtgtata tgatgtgtac attcaccaag gtgtacacat gagatatgta 61379
cacttctctg tattccatta caccttggtt tttgaaaaga cagaaacagc atgctcagct 61439
tattaagaac ctgaatattt tcagacaaaa agtatgtcta gtgttagtcc actttttttt 61499
ttttttagtt ttagactcac agagccctct tagggtcata tttagttgta tatcatctga 61559
caaaagaggg aatggctcac ccagctgcta cagtcagtct tgtagccttt gtaagatttg 61619
atgataaatc atacaataaa ttaaaacttt cggctgggca cagtggctca cgcccataat 61679
cccagcactt tgggaggccg aggcggagga tcacttgagc ccaggagttc agatgagcct 61739
ggccaacata gtgagactct gtctctattt aaaaaaaaga agaaaaaaaa agtctgggtg 61799
tggtggctca cgcctgtaat cccagcactt tgggaggctg aggcgagcgg atcacaaggc 61859
caggagatca agaccatgct ggctaacaca gtgaaacccc gtctctacta aaaatacaaa 61919
aaattagcca ggcgtggtgg cgggcgcctg tagtcccaga tactcgagag gctgaggcag 61979
gagaatggcg tgaacccggg aggcagagct tgcagtgagc cgagattgtg ccactgcact 62039
ccagcctggg cgacagagcg agactccgtc tcaaaaaaaa aaaaaaaacc ccaaatctta 62099
actcttactg ctaagtgact gctttatcga aactgttctg tttcgggaca tttgattctg 62159
gtagagattt tgagacctgc tttgaagagg acaccaacat ttcagtcaca cagcaatgct 62219
cctgtcttac ctgggatcca tgtgggagga tgcctcagtg ggggatttga tctgtgtttt 62279
caatttaacc tcacatctta ctggaatgga tggattcaca gacctggggg tcgcccattg 62339
gactgggagt aactacgttg gaattacaag taggacaaca aagatgacta ttaaagaaag 62399
cctcctgagt gctggaagca cagcaggcgt gggtctgtcc actcactgta atgtgatttg 62459
cacaggaaaa catcacatag ggcacatcaa ctcaggaaag atagtcgttg aaaagctacc 62519
tcagctgggc gtagtggtca cgcctgtaat cccagaactt tgtaaggctg tgaggcttgt 62579
ggatcccttg agaccaagag tacgagacca acctgagcaa aacagggaga ccccgcctat 62639
gcaaaaaata caaaaattag ccaggcgtgg tggtgcactc ctgttgactc agctattcag 62699
gaagctgagg cagaaggacc acaagaagtc aaggccgcag tggggtataa tcacatcact 62759

gcactccacc atgggtgaca gaggtgagac cctgttgcaa aaaaaaaaaa aaaaaaagca 62819
aaagaaaagc tgcctccatt tagcaataat ttcaaacatg atgtttgggt acaaaattgg 62879
tgtacttaat ggatatttct gatattctta actaaatttg aagctgtgag ggaagacaaa 62939
ctcagggtcc acagggtggg aaaagtccac aagatcaccc tcaagtgccg tcattctcta 62999
ggaccccgag agccctagcg gttgttacgc ttgcagttat gatttattac agtgcaaggc 63059
tacagattaa aatcaaccaa ggcaagagat gtatggggcc tcttgggggc caaggtgggc 63119
ggatcacttg aggtgatctg gacggttcca gacacagggc tttcctttgt ctcctccctg 63179
tggagttgaa gacagcgctg atgttcccgg tgacgttgac acagagcact gccaaccaga 63239
gaagcctccg tgtccagagg tttttttttt tttttggaga tgcagtctca ctctgtcact 63299
caggctggag tgcagtggca ctatcttggc tcactgtaac ctctgcctcc tgggttgaag 63359
tgattcttct gcctcagcct cctgagcaac tgggattaca ggctcgcgcc accacgcctg 63419
gctaattttt gtatttttta gtagagatgg ggtttcacca tgttggccag gctggtctcg 63479
aactcccgac cttaagtgat ccgcccgcct cagcctcccg aagtgttggg attacaggca 63539
tgagccaccg tacccaccct gtgtccagca tttttactgg gacttggtca caaagacaca 63599
gctggctacc cacgtggctg acctccatct ccagcccctc tgcaggtgag cagaaactac 63659
atagcccaaa gccccaacct aggactggtc ccagacccca cgtaaacaaa gacagccatt 63719
aggtgtgaca ttctaaggac tcagagatca cctcccagga gccccaggca aaagccagac 63779
ctcatttggg gcgaagttac attctttttt attattatta ttatttgccc atcattgtct 63839
agaagttaca ttctgtatga cacatctgtc agatccctgg aagcttcctc gccaccgact 63899
gcatggcagg tggtgctcat gtcactcggc ccttccttgg ccccacacag ctgcaccagg 63959
ctgagaagag cttgaatgga ataaagtcag acacagctaa cagctcactt ttttgtctcc 64019
aactttgaat tgctcctttc caagaaaaga aaaaaaactt ctttttgttt aattggattt 64079
ttttttttat ttattttttg aggcagagtc tcactctgtc acccaggctg gagtgccatg 64139
gcacaatctc ggctccctgc aacctccacc ttccaggttg aagtgattct cctgcctcag 64199
cctcccaagt agctgggatt acaggtgcac accaccatca tgcctggccg gaatttttgt 64259
tttttagttg gctctccaga accctctaag tagttcagct ctgcaattga gttaaatgtg 64319
aagcctgaaa agagactggc atctgtgtag acctgagttc taacaattca aagagtgaac 64379
acgggacgtt gtcctctgga ttcggtgtca ggagcctgcc tcgccgagct ggagagtgtg 64439
cccatgactg caggtgggag ccacatggtc cgtctaggtg gcgacacctg aggacagaag 64499
tggtttagct gggcacccgc tgctgtgttg gggccagacc cttttggaga ggcagggacg 64559
cccttcctgt caggtggaat cttttcaaat attgcttttg atcgttggag atacttgcag 64619
ccccctagtt cgtgttttat tctggcagaa ttgtgtttga cgtgacctct gtgttgacct 64679
catcatacgt gtacagttgt cttgcctggg aatcttatac tctgttcttc tgcactcaga 64739
ttcacagcca tgaggccgct cctcccaagg ccctggagtg tggcactgcc agctgtcaca 64799
gccacggtcc tttacattgt atacacagag tgcagtgaca tgcccctcct gctcaggggc 64859
cacagtagga acaggctgga gtgttctgtg gtaccatgcc ggcagtgctg gcttctgctg 64919
tccaggggct tgtggtggct ccagtgggaa gggccacgta agcagaacag gaagtgtgag 64979
cagtgtccac tgggaggaga gggacaagtg tcccaggcag agaggacaca gtgcatgcag 65039
aggctggagg gaggctaggg gcttgagtgg ggctgcgcca gcaccagggg cctcacaggg 65099
gccctgggcc acacacctgc ccttagccaa gggcgtttct ccttggtggt ttgaaatctc 65159
aaaccacatt gaggaacctg ccagaaatcg aaaccagaat taagaaatta agttacatgt 65219
aggagatttt actttaaaat cagaacatgc agaaacgttc tctgtggctt cgggtcaggt 65279
tcgggcttgt ggggctgtac actgccagtt gataccttct tccctgggtt ctgtgttttt 65339
tttttttttt ttgagacaga gtcttgctct gtagcccagg ctggagtgca gtggcacagt 65399
cttggctcac tgcaacctct gcctccctcc ctgggttctt tatagcagtc agcagctggt 65459
ggctgaggca tttggggcac tgtgtccaag tgtcaaggcc gcatgtgtgt cagggggcac 65519
acccttggtg tgcttggtaa gggctcagag aagggcatct gctggtccat gtgggctcag 65579
ccagcagcct gctaaagacc accccgccag ccaccagcct gcctgcgaca gtgatagggc 65639
agagatgctg ctacaataaa gaccatgcgg gccaggcgca ggcgctcaca tctgtaatcc 65699
cagcactttg ggaggccaag ccgggctgat cacttgagct cagaagtttg agaacagctt 65759
ggccaacgtg gcaaaaaccc cgtctctatt aaaatacaaa aattagccag gcatggtggc 65819
actcgcctgt aatcccagct acttgggagg ctgaggcagg agaatcgctt gaacccagga 65879
ggaggtttcg gtgagccaag attgcaccac tgcactccag cctgggtgac agtgagacta 65939
cgtctcaaaa aaaaagactg tgctagacgg tggtgttgaa cttgaacttg atgcttgtgc 65999
tgaggtcagg agacacgtgc acagcttcag ggctagaggg gcacaatggc tgcagactat 66059
gctcgggcag ctcggaaaga cggtgacggg tccaggctgc cgcagctaag cctcacaaga 66119
gctctggccc caccaggcgg gccctctggg ttccattccc actccccact gcctggaggc 66179
agcagccctg tggttactgt aggagggacc tgaatgcagt gaagatctgc actccacacc 66239
tgtctctgtg cctcagtctc cacgtctgca gcacaggggc ctgactgaaa taacgtggcc 66299
aggcacacag cccagacgcc agcactgtgt gcactgtggg aggggacgat ggtccctagc 66359
ccagggcctg agctgctgcc ttgccaattc gtggtgacag ctttggcctg ggtctggcag 66419
gggctggctg ctactgctga aggcctgtct gcactgtttc cctgcctttc cacaagcact 66479
gtccctcctg gctcttcaga gttgagtggt tataccttgt gcaagaaaaa caatgaaaca 66539
gattgtctga atgtttcaga cctgcctgaa acattctgca ggagaggtca tcccaaggct 66599
tgaaagtgtt tgtcacactc cttcaagccc ccagggcagg gcccctgagg gaagggtgca 66659
ggtggagggt gggcaggagt gggagccatt ccttggggac aggggggctc ctcagggctg 66719
tctggccctt ggcctgtggt tccaggtcta tggtctgctt ggcacatagc ctggaccctg 66779
gtgctgtgca ggcactggct ggatgagggc accgagcaga tgagccccag gcatgcccac 66839
ttatgggcat aggctgggtg cagggcactg ggtccaacat aaccatggca gagggctcag 66899

ggggcctaaa agtcatggat ggagttgccc acgcagcctg cggtcaggtt ccctgtggag 66959
ccgtgctggg gttctgcgcc tgccccagga tttgctggaa gaagctcccc ctcctctgtt 67019
tgtgagtcgg gccctacccc caagtctccc cctcagtgat agagaccttt acataacaca 67079
aataaacaag gcaactggtt tagaagaatg agtctgaaaa ctagaatggc gtgtttaagg 67139
aaagcttagg acaggacaat tagagacaga ggtgcaggtg tgtggagttg gcagcaggag 67199
gcctggggga gccgctcgtg cctttccctc ctgccctggc ctcccttgga actcggagca 67259
gccccctgcc ttgaggaggc ccttcctttc agtgagcctg tgccgcagtc gtccgaggga 67319
acacagtggg gacatatttc cttttttttt tttttttttg agacagagtc tcactctgtc 67379
gccaggctgg agtgcagtag cctgatctca gctcactgca aattctgcct cctgggttca 67439
agcaattctc ctgtttcagc ctcccgagta gctgggacta caggcatgca ccaccacgcc 67499
cagctaattt ttgtattttt agtagagatg gggtttaacc acgttggcta ggatggtctc 67559
gatctcttga cctcatgatc cgccagcctc ggcctcccaa agtgctggga ttacaggagt 67619
gagccaccat gtccggccgg aaatgtttat taaaatagca gatgaggccg ggtgctgtga 67679
ctcaggcctg taatcccagc actttgggag gctgaggcgg gtggatcacg aggtcaggag 67739
ttcaagacca gcctgaccaa gatggtgaaa cccatctcta ctaaaagtac aaaaattagc 67799
cgagcgtagt gagacttgcc tataatccca gttacgaggg aggctgaggc aggagaatcg 67859
cttgaaccca ggaggcagag gttgcagtga gccgagattg tgccactgca ctccagcctg 67919
ggcaacagag cgagactccg tctcaaaaat aaataaataa aaatagatga tcatagacag 67979
gagggatgaa gtagaagatt caaagcagct taaaaaaagg aagtaaaaaa gaaataaggt 68039
caggagacaa gattgagtga ctgtgaaaca tcaacaatag gcagatttaa aataaagtca 68099
ggaaatagct agatcaaaga atagaaaggt gaaccaggtg tgatatccac ctgtagtccc 68159
agcttcttgg gaggccaacg cgggaaaatc acgtgagccc aggagttgga ggcaccgctg 68219
cactcaagcc tgggtgacac agtgagaccc tgtctcaaaa agcagcagga agagaggctg 68279
gcatgtgggg ctccaggggg tgtgaaggag ggtccacacc ctccttcctc cacctggcca 68339
tactgagcag cacattctct gagccaacgg cccctacaaa cacccaccct ttcctcctgg 68399
tagcccagag ggcgcccctg cctgctcact tcccaatgtc gggatcctgt gatttctgcc 68459
tcttcagaaa ggacttgagg cattttctga ctcagagcca ctcaggattc caggacctcc 68519
tgtggtgaca acagggacac cagtagtgat gaattcagaa aagagcgaac gcccctcaag 68579
ctgccctccc ccagccctgc agacagtggc catgggcctc cctgcactca ggaccgactg 68639
cacctgcctg cggcgtaggg gagctggggc tggcctttcc ttttcatctt cccctggcac 68699
tgggaaatgc tctgggtggt gccatgtata acaccctttg tcacagtgct ctcatttcgt 68759
cgtgtaaaag gccagtcccc gcctcgcagt gttggggcag ccagcacagg cacgtgagac 68819
aggagccaca cctgggttct catctggcat ctgcctcttg ccatgctcag gttcttgcct 68879
ccagatatag ggcatcaaac taagtggttt ccaaacttgg gttcttggtg gggagtgaga 68939
tcagggtttc ttaaaaactg cacttggagg ctgggcacgg tggctcacgc ctgtaattcc 68999
agcactttgg gaggccgagg cgggcggatc acctgaggtc aggagttaca aaaaacggaa 69059
cctaggctca tgcctataat cccagcactt cgggaggcca aggctggcga atcgctggaa 69119
tttaagagtt ccagaccagc ctggccaacg tggcaaaacc ctgtttctac tcaaaatacg 69179
taagttagcc gggcatggtg gtgggtgcct gtaatcccag ctactcggga ggcggaggtt 69239
tcagtaagcc aagattgcac cactgcactc cagcctgggt gacagagtga gactccgtct 69299
caaaaaaaaa aaaaaaaaga aagaaattca aatctcagtg tcgataaatg aagttttgtt 69359
ggaatgcagc caaagcccac cgatttctgc gttgtctgtg gttcccttca tggaacagtg 69419
gcagaactga gcagttgcaa cagaggcagg cccagcaagc ctcaaatatt tacactgagc 69479
tgagcgcagt gactcacacc tgtaattcca gtgctttggg aggctgatgt gggaggatcc 69539
cttgagccca ggagtttgag accagcctgg gcaacctagt gagaccccat ctctacaaaa 69599
tattttttaa attagccagg tgttgtggcg tgtgcctgta gtcctagcta cttgggaggc 69659
tgaagcagta ggatctcatg agcccaggag ttggaggctt cagtgagcta ggatggcacc 69719
actgcactct agcctgggca acagaacaag atcgtgtctg taaaaagaga gaaaagaaac 69779
agacaattcc tagactgcag caggtgtttg cagtagtgca tccagcaaag gactccccag 69839
ggaaggaaat gggccaaaga tgggaacaag cgctgcttgg aagagcctgt gaaacagtgc 69899
ttggcttctc tgcacaacaa ggaaacgtgt ttagggccac attgtagaat ggctacaagg 69959
acaagcacca ctcacgctgg ctcagtatct cccaagtaga gcatttcccc accctgtgac 70019
ctagagtatc ttctcccaga taaattccta ggagaactgc aaactaaaca cttgttcctg 70079
agtgtccaca gacacttgct tcctggtgtc caaccctgag cacagcccag atgcccggca 70139
gcgggaactg ggcatggctg gtgtgtgccc acctcgggac ctgctgctgc actcggcaac 70199
agggacagca aggaggcagg aggccaggcc aggtttgggg gctcccctgt gtgtttctgc 70259
atcatcaact tgaagaagca gccaacctcg gcccaggatc atccctgggg gaaggactga 70319
caggagcgca tctttagcct catgcttaag atctgtgcat tgggccaggt gcggtggctc 70379
agcctgtgat cccagcactt ttgggaggcc aaggtgggtg gatcaggagg tcaggagttc 70439
gagaccagcc tgaccaacat ggtgaaaccc gtctctacta acaatacaaa aattagctag 70499
gtgtggtggt gcacctgtaa tgccagctac ttggaggctg aggcaggaga atcgcttgaa 70559
ccctggaggc agaggttgca gtgagccgag atcacgccac tgcatgatgt gtgcattggt 70619
gttaagtcag ctattaagag gtgtgggctg ggtgtgtgtg ggggtcagag gaggtgatgt 70679
ttgccctgag atgaggtcag agcccacatt cccatggggg tggactcccc tttgtaggcc 70739
ttgacacgag catgggagcg gagctagggg cactgacatc aaaggtggtg gcgcctatcc 70799
agaggactcc gggtttcatt ccaagtgcgg ggaggctgca gcatgtccct ctttaatata 70859
gaggtgaggg aggctctcag caccctgcct gttgcaggga agcctgagga caggacctcc 70919
agggaactct gcaccagagc ggaatcctca gaccggcaac tgggtggtct tgattcatcc 70979
agaacgtaat tctcctagag gtctgggaag gggtgctctt gtaccttggt tttgtgtttt 71039

gtttttgttt gtttttgag acagagtctc actctgtcgc ccaggctaga atgcagtggt 71099
gcgatctcgg ctcactgcaa cctctgccac ccaggttcag gtgattctcc tgcctcagcc 71159
tcccgagtag ctgggattgc aggtgcctgt caccacgcct ggctcatttt tgtattttta 71219
gtagagacgg ggtatcacta tcttggccag gctggtcttg aactccggac ttcgtgatcc 71279
acccacctca gcctcccaaa gttctggggt tacaggcgtg agccactgcg cccagccgca 71339
ccttgttttt tttttgctgc ctgtagctgt ttctcattag cctgatggag gtggctgttg 71399
gggtggtggg gtgcttatta taaagccttc tgctgtcaca gccctggggt ctcacttcct 71459
gggctctctg catagggagc cgccctccag ggcaagggcc acagtgtgca gtaagccccc 71519
catggccaca gggcagggcg tttcaagcca agagtctgca ctgtttgctg gccactaagg 71579
gcattgaatg gatcacaaaa ctggcttttt tttcccccag tcaaatagaa atggggtttt 71639
gccatgttgt ccaggctggt ctcaaactcc tgggctcaag caatcctccc acgttggcct 71699
cccaaagtgc tgggattaca ggcacgagcc accatatctt taaaaatgta atagaatgga 71759
aaatggcaaa gcgcgctgct tttcatagaa gagactatcg cattggggtt cttctgttgc 71819
ccctgcctgt gctgcacgtc ctgcttcaca gtgtaaagcg caactcttgc tgtgccttat 71879
tgtaacaggt atagggtgga tgctgccatc atgaagagag aggagagctc tttgcttata 71939
aaattccgta gagtggagaa caggatgtgc cggcttagga gagggaagag gtacaggccc 71999
gctctgcccg agctgcagat gggacacaca tttcccgact cagaggggag tcgtgtcttt 72059
ctccagggtc accgagcagc aagcgcatcc actgtgtgtt cttattttct gagcctgtgg 72119
actgggttta ccaacagttg cctgtggcag agccggccac cagcccgctc ataggaaaaa 72179
ggaagcctgt gcctccaggg agggcccacg atgggaatgc agtctgagaa gggtatgtgc 72239
tcccatgctg gaccggctgg tcccacaccc aggaccaaaa acacctcctg gcaggagatg 72299
gggagatgta cttcatgaac acacgttggg gaaggacact cctggaaggc agaggctcaa 72359
ggcgggctgt gtccaggcca ggagattcac tcccacctca atttcctcct cagcacggct 72419
ggcctggcac tggtgcctgc ttcagagttt agtgtgtgta aagtgtggct ggcccagcaa 72479
gcaggggatg atggcggttg tcaaggccag gggaggtgca gggacaggat tctggctccc 72539
gtgtgtaacg tctgcatctc aggaggctct catgttagaa gctgggtcag gggcttcgtg 72599
atgcacacaa acaccaaaca agcaggaggc cttccgggat cctgctcacg agcagcttcc 72659
tggaggcaag cctggcccgt ctgccccttg gtgacatcag gaccccagca acagaaccag 72719
ggaagccacc atgttgtcgc tgccattttc cagcgtggta aacagaagca agacaaggcg 72779
cactcaggac acccaaacag acggcggggc cagccagtgg catgggcggg ctgccctgcc 72839
ctctggaatt ttctgggaaa gaacataagg aaagaagcgg tgctgatgag gcagctggag 72899
ttatcaggac ccaagctcag ctcctccttc ccatcaggcc ccgttggcca cttttaggct 72959
tcgtccgcct ggtggctccc tgcttggggc cgttccctgg ctaggccctt cctctgggcc 73019
aaagcagaga ggagagcgcg tttgccatgc gttctcattt tcccagcctg cgggctgggt 73079
tctgtcagag caaggtcagc tctcagagct tctgtcccca cccaccctgt cctgctgagc 73139
caggctcagg tatgggtaag agctgggagc gctgtgcacg aggcctggca gcatctcatc 73199
actgccgctg taccgagtgc acaagggcgc ttcctgtggt gttcaggggg ttagatcagt 73259
ccccagtgag gacagagcag agatccagat gcatcccagg ccggctgcag tggcttctgc 73319
ctctaatccc agcacttcgg gaggccaagg caggcagatc acgaggtcaa gagatcgaga 73379
ccatcctgac caacatggtg aaaccctgtc tctactaaaa atacaaaaat tagccgggtg 73439
tggtggctgg catctgtaat cccagctact cgggaggctg aggcaggaga attgcttgag 73499
cccaggaggc agaggttgca gtgagccgag atcattccat tgcgctccag tctggcgaca 73559
gaacaagact ctgtctcaaa aaaaacaaaa aacaaaaaaa tgtatcacag gcctgtttgt 73619
gtggttgtct ctgggaaatg ttccagaagc tacaagaaag cacatgtgca ggccaggcat 73679
ggtggctcac acctgtaatc ccagcacttt gggatgctga ggcgggctga tcacctgagg 73739
tcgggagttt gagaccagtc tgaccaacat ggagaaaccc tgtctctact aaaaatacaa 73799
aattagcctg gcatggtggt gcacgactgt aaaaaatccc agtgactcca aaggctgagg 73859
caggagaatc tcctgaacct gggaggtgga ggttgcagtg agccgagatc gcaccattgc 73919
actccagcct gggcagcaag agcgaaactc cgtctcaaaa aaaaaaaaag cgcatgtgca 73979
agaaatagct cctgagctga gttggaattt ctcatcctta aacaatgtgg gaacctgctg 74039
aggtggggtg ggggctcccg ggtgggcgga ctcgggggtg atagccgccg gttctgcctt 74099
gcag c agc agt gga cgt cag ctc agc gag gtc ttc atc cag ctg ccc 74146
Ser Ser Gly Arg Gln Leu Ser Glu Val Phe Ile Gln Leu Pro
                1480                1485
tcg cga aag gag ctg ccc gag tac tac gag ctc atc cgc aag ccc 74191
Ser Arg Lys Glu Leu Pro Glu Tyr Tyr Glu Leu Ile Arg Lys Pro
1490                1495                1500
gtg gac ttc aag aag ata aag gtaaccctga cgttgtacct gcgccccgca 74242
Val Asp Phe Lys Lys Ile Lys
1505                1510
tgtgcccgga ggggagtctg acccaggggc acccccatct gagagctgtg gtgtgtgggc 74302
agaatgacca gaaaccacct aggcggtgcc ttgggctacc tggttaggga cctggtcgtg 74362
ggcttttggg gttccttgta agggttgggg gtgtcctgag ggatgtcagt gggcagtcgg 74422
tgttgggtgt tccttcaagg tcccactcac ttagtgctgg gcctcagtca tgcagttccc 74482
atgcctagtg agccaggtta ccgaggctgg cgcttcggcc acatcctcat aggcacgagg 74542
aatcctagcc cgtggggtct ccagcacaca gccaggcctg cgggcaggcg aggcggggtc 74602
ctgaggtaag acctgctcct cccgtccact gcag gag cgc att cgc aac cac 74654
Glu Arg Ile Arg Asn His
            1515

aag tac cgc  agc ctc aac gac cta  gag aag gac gtc atg  ctc ctg     74699
Lys Tyr Arg  Ser Leu Asn Asp Leu  Glu Lys Asp Val Met  Leu Leu
        1520                 1525                 1530
tgc cag aac  gca cag acc ttc aac  ctg gag ggc tcc ctg  gtgagggcac  74748
Cys Gln Asn  Ala Gln Thr Phe Asn  Leu Glu Gly Ser Leu
        1535                 1540                 1545
cgctgggggt tggggatggg ccactcccac agctgggctt tgacccaacc cgcccctcct  74808
tcccttctga attgatgggt taaaaacaag tcccgctagc tgtggtggtt cgtgcctgta  74868
atcccagcac tttgggaggc caaggcggga ggatcacttg agaccaggag ttcacgacca  74928
gcctgggcaa cataacaaga ccctgtcttt aaaaaaaaaa aagaaaaaat taaaaatttc  74988
caacttggga tattttagca attacaaaag cagtatgtat ttaccgaaga aaataaagat  75048
aggcacaaca gaagaaatag actcagagtc ctctgtctgt tggaatttta ttttaaattc  75108
cctggggcac attctcttca aaggaatcct gtgggtttgt tctttcctgt gatagaattg  75168
acacatacac agcctcaact tggcaaactt tcttaagtgt ctttgggatt gattcctctc  75228
agaaaaaaga cttgactttg atgagtttgg gaaagatgct ggcttcaaca aggggcatgt  75288
atttccaatg ttattgcaga cctcagagat tgtgcagaag gagggaggca cctgcagcta  75348
ttcccaaacc agtgtgcctg acctttcacc ctggagcatt tcagaagtct gatgctcggg  75408
cccctatgga ggaagattat ggagagggcc ttccaccctg ggtgggctga agccccgacc  75468
cgctgaggct cgcattggcc actgatcagc tgtccagggg caacacaggg ctggggctgg  75528
ggctggggcc agggccgggc aggcagccct ccagtcgggc ccatccactc aagcccctgg  75588
tgtctctgcc cag atc tat gaa gac tcc  atc gtc ttg cag tcg  gtc ttc   75637
               Ile Tyr Glu Asp Ser  Ile Val Leu Gln Ser  Val Phe
                              1550                 1555
acc agc gtg  cgg cag aaa atc gag  aag gag gat gac agt  gaa ggc     75682
Thr Ser Val  Arg Gln Lys Ile Glu  Lys Glu Asp Asp Ser  Glu Gly
        1560                 1565                 1570
gag gag agt  gag gag gag gaa gag  ggc gag gag gaa ggc  tcc gaa     75727
Glu Glu Ser  Glu Glu Glu Glu Glu  Gly Glu Glu Glu Gly  Ser Glu
        1575                 1580                 1585
tcc gaa t gtgagtcccg ggggggttca ggacgccggg gttcacgctg gcccgagagc   75784
Ser Glu

ccccaaggcc ccagcttttc acagccctcc cggctcccag acgccccttg ctgtgggggt  75844
gctgcattcc cagagctcaa ggctgtcttt ccctcccggt cccctccag ct  cgg tcc  75901
                                                      Ser Arg Ser

gtc aaa gtg  aag atc aag ctt ggc  cgg aag gag aag gca  cag gac     75946
Val Lys Val  Lys Ile Lys Leu Gly  Arg Lys Glu Lys Ala  Gln Asp
        1595                 1600                 1605
cgg ctg aag  ggc ggc cgg cgg cgg  ccg agc cga ggg tcc  cga gcc     75991
Arg Leu Lys  Gly Gly Arg Arg Arg  Pro Ser Arg Gly Ser  Arg Ala
        1610                 1615                 1620
aag ccg gtc  gtg agt gac gat gac  agt gag gag gaa caa  gag gag     76036
Lys Pro Val  Val Ser Asp Asp Asp  Ser Glu Glu Glu Gln  Glu Glu
        1625                 1630                 1635
gtgaggccgg gcccccgagc aggcagagct ggcatgtggc aggaggcatc ccggggccct  76096
gatgggacag ccctgtgggg cggtgctccc acgcccccac gctgcaggtg ggaaagctga  76156
ggcttgacct gccatggctg accccaggtc acacagccag tatgtggcag ggctgccact  76216
caaagccaag cctgtgtgac cccggaacca gctctcagta cccgtgggtg tctggggact  76276
gggggacacg tgagtcctca ggacaggcag caggcggtcc cagggcacca gggctcctgg  76336
gctgaggccg ggcaggccag ggcttgatta gggttgggta ggaggtgcta ggattgggga  76396
cccaccagac agtgccccgt ctcctcagtg gcatagagaa tgggcagaaa cgccaagacc  76456
acagtggata gtttttgggg tgcagtgggg agccggagaa gatccacttg ctggctttaa  76516
ataatctcac ctctgtctct ggcccttcct ggctgtggga cctggcaggt cactttgctt  76576
ctcaagcttc agtttcctct tatgcaaagc aaggaggctg catggattag ggggctgccc   76636
ccaccgaagg gcctggctcc cgctgaagcc cagcgaatgc cattttgtca tcacagaagc  76696
agcaaatttc agtgcctggg aattagtctg acgaaagacg cacgtgacgt ttgaggacat  76756
tcatgaagtc ctcatgtgga ggggaggccc ctgtgtcagg caggagggtc tgggctgtga  76816
tgctgtctct cttgcctaat ccagagcagg gtgttttatg gcccatgcca ggctgtttaa  76876
aaaggtttta tgaggccagg tccagtggct cacacctgta atcccagcac tttgggaaac  76936
tgaggcagga ggctcacttg agcctgggag gtcgaggctg cagtgagccg tgatagcacc  76996
actgcactcc agcctgggca acagagcaag accctgtctt taaaaaaaaa aaaaaaaata  77056
tatatatata tatatatata tatatatata tatatgaaag agcaaagatc tttcacagtc  77116
aggaagaacc aagatggttt ggaagctgta ggtcaggggc caagggccag gcaggtgaca  77176
tgggacagag cagggaaggc acgtggagcc tcgaaggtcc cgagagggcc gagtgtggtc  77236
acagggactc gtcctgtaga gaaacatgaa gtttgggcat ggccatggcg ccatctcggc  77296
tcactgcaag ctccaccccc cgggatcgcg ccattctcct gcctcagcct cccaagtagc  77356
tgggactaca ggcgcccgcc accacgcccg gctaattttt tgtatttttt agtagagacg  77416
gggtttcacc gtgttagcca ggatggtctc gatctcctga cctcgtgatc cgcccgcctt  77476

ggccttccaa agtgctggga ttacgggcgt gagccaccgt gcccggccca catcagtgtt 77536
ttcttgagca agtttattta aagtttggca ggtccctggc aaggtgccct ggcaggggtg 77596
gccaacgcac actctctcct cctgtcccct ctccag gac cgc tca gga agt ggc 77650
Asp Arg Ser Gly Ser Gly
        1640
agc gaa gaa gac tga 77665
Ser Glu Glu Asp
    1645

<210> 2
<211> 4944
<212> DNA
<213> Homo sapiens
<220>
<221> CDS
<222> (1)..(4941)
<223>
<400> 2
atg tcc act cca gac cca ccc ctg ggc gga act cct cgg cca ggt cct 48
Met Ser Thr Pro Asp Pro Pro Leu Gly Gly Thr Pro Arg Pro Gly Pro
1               5                   10                  15
tcc ccg ggc cct ggc cct tcc cct gga gcc atg ctg ggc cct agc ccg 96
Ser Pro Gly Pro Gly Pro Ser Pro Gly Ala Met Leu Gly Pro Ser Pro
            20                  25                  30
ggt ccc tcg ccg ggc tcc gcc cac agc atg atg ggg ccc agc cca ggg 144
Gly Pro Ser Pro Gly Ser Ala His Ser Met Met Gly Pro Ser Pro Gly
        35                  40                  45
ccg ccc tca gca gga cac ccc atc ccc acc cag ggg cct gga ggg tac 192
Pro Pro Ser Ala Gly His Pro Ile Pro Thr Gln Gly Pro Gly Gly Tyr
    50                  55                  60
cct cag gac aac atg cac cag atg cac aag ccc atg gag tcc atg cat 240
Pro Gln Asp Asn Met His Gln Met His Lys Pro Met Glu Ser Met His
65                  70                  75                  80
gag aag ggc atg tcg gac gac ccg cgc tac aac cag atg aaa gga atg 288
Glu Lys Gly Met Ser Asp Asp Pro Arg Tyr Asn Gln Met Lys Gly Met
                85                  90                  95
ggg atg cgg tca ggg ggc cat gct ggg atg ggg ccc ccg ccc agc ccc 336
Gly Met Arg Ser Gly Gly His Ala Gly Met Gly Pro Pro Pro Ser Pro
            100                 105                 110
atg gac cag cac tcc caa ggt tac ccc tcg ccc ctg ggt ggc tct gag 384
Met Asp Gln His Ser Gln Gly Tyr Pro Ser Pro Leu Gly Gly Ser Glu
        115                 120                 125
cat gcc tct agt cca gtt cca gcc agt ggc ccg tct tcg ggg ccc cag 432
His Ala Ser Ser Pro Val Pro Ala Ser Gly Pro Ser Ser Gly Pro Gln
    130                 135                 140
atg tct tcc ggg cca gga ggt gcc ccg ctg gat ggt gct gac ccc cag 480
Met Ser Ser Gly Pro Gly Gly Ala Pro Leu Asp Gly Ala Asp Pro Gln
145                 150                 155                 160
gcc ttg ggg cag cag aac cgg ggc cca acc cca ttt aac cag aac cag 528
Ala Leu Gly Gln Gln Asn Arg Gly Pro Thr Pro Phe Asn Gln Asn Gln
                165                 170                 175
ctg cac cag ctc aga gct cag atc atg gcc tac aag atg ctg gcc agg 576
Leu His Gln Leu Arg Ala Gln Ile Met Ala Tyr Lys Met Leu Ala Arg
            180                 185                 190
ggg cag ccc ctc ccc gac cac ctg cag atg gcg gtg cag ggc aag cgg 624
Gly Gln Pro Leu Pro Asp His Leu Gln Met Ala Val Gln Gly Lys Arg
        195                 200                 205
ccg atg ccc ggg atg cag cag cag atg cca acg cta cct cca ccc tcg 672
Pro Met Pro Gly Met Gln Gln Gln Met Pro Thr Leu Pro Pro Pro Ser
    210                 215                 220
gtg tcc gca aca gga ccc ggc cct ggc cct ggc cct ggc ccc ggc ccg 720
Val Ser Ala Thr Gly Pro Gly Pro Gly Pro Gly Pro Gly Pro Gly Pro
225                 230                 235                 240
ggt ccc ggc ccg gca cct cca aat tac agc agg cct cat ggt atg gga 768
Gly Pro Gly Pro Ala Pro Pro Asn Tyr Ser Arg Pro His Gly Met Gly
                245                 250                 255
ggg ccc aac atg cct ccc cca gga ccc tcg ggc gtg ccc ccc ggg atg 816
Gly Pro Asn Met Pro Pro Pro Gly Pro Ser Gly Val Pro Pro Gly Met

260                 265                 270
cca ggc cag cct cct gga ggg cct ccc aag ccc tgg cct gaa gga ccc      864
Pro Gly Gln Pro Pro Gly Gly Pro Pro Lys Pro Trp Pro Glu Gly Pro
        275                 280                 285
atg gcg aat gct gct gcc ccc acg agc acc cct cag aag ctg att ccc      912
Met Ala Asn Ala Ala Ala Pro Thr Ser Thr Pro Gln Lys Leu Ile Pro
    290                 295                 300
ccg cag cca acg ggc cgc cct tcc ccc gcg ccc cct gcc gtc cca ccc      960
Pro Gln Pro Thr Gly Arg Pro Ser Pro Ala Pro Pro Ala Val Pro Pro
305                 310                 315                 320
gcc gcc tcg ccc gtg atg cca ccg cag acc cag tcc ccc ggg cag ccg     1008
Ala Ala Ser Pro Val Met Pro Pro Gln Thr Gln Ser Pro Gly Gln Pro
                325                 330                 335
gcc cag ccc gcg ccc atg gtg cca ctg cac cag aag cag agc cgc atc     1056
Ala Gln Pro Ala Pro Met Val Pro Leu His Gln Lys Gln Ser Arg Ile
            340                 345                 350
acc ccc atc cag aag ccg cgg ggc ctc gac cct gtg gag atc ctg cag     1104
Thr Pro Ile Gln Lys Pro Arg Gly Leu Asp Pro Val Glu Ile Leu Gln
        355                 360                 365
gag cgc gag tac agg ctg cag gct cgc atc gca cac cga att cag gaa     1152
Glu Arg Glu Tyr Arg Leu Gln Ala Arg Ile Ala His Arg Ile Gln Glu
    370                 375                 380
ctt gaa aac ctt ccc ggg tcc ctg gcc ggg gat ttg cga acc aaa gcg     1200
Leu Glu Asn Leu Pro Gly Ser Leu Ala Gly Asp Leu Arg Thr Lys Ala
385                 390                 395                 400
acc att gag ctc aag gcc ctc agg ctg ctg aac ttc cag agg cag ctg     1248
Thr Ile Glu Leu Lys Ala Leu Arg Leu Leu Asn Phe Gln Arg Gln Leu
                405                 410                 415
cgc cag gag gtg gtg gtg tgc atg cgg agg gac aca gcg ctg gag aca     1296
Arg Gln Glu Val Val Val Cys Met Arg Arg Asp Thr Ala Leu Glu Thr
            420                 425                 430
gcc ctc aat gct aag gcc tac aag cgc agc aag cgc cag tcc ctg cgc     1344
Ala Leu Asn Ala Lys Ala Tyr Lys Arg Ser Lys Arg Gln Ser Leu Arg
        435                 440                 445
gag gcc cgc atc act gag aag ctg gag aag cag cag aag atc gag cag     1392
Glu Ala Arg Ile Thr Glu Lys Leu Glu Lys Gln Gln Lys Ile Glu Gln
    450                 455                 460
gag cgc aag cgc cgg cag aag cac cag gaa tac ctc aat agc att ctc     1440
Glu Arg Lys Arg Arg Gln Lys His Gln Glu Tyr Leu Asn Ser Ile Leu
465                 470                 475                 480
cag cat gcc aag gat ttc aag gaa tat cac aga tcc gtc aca ggc aaa     1488
Gln His Ala Lys Asp Phe Lys Glu Tyr His Arg Ser Val Thr Gly Lys
                485                 490                 495
atc cag aag ctg acc aag gca gtg gcc acg tac cat gcc aac acg gag     1536
Ile Gln Lys Leu Thr Lys Ala Val Ala Thr Tyr His Ala Asn Thr Glu
            500                 505                 510
cgg gag cag aag aaa gag aac gag cgg atc gag aag gag cgc atg cgg     1584
Arg Glu Gln Lys Lys Glu Asn Glu Arg Ile Glu Lys Glu Arg Met Arg
        515                 520                 525
agg ctc atg gct gaa gat gag gag ggg tac cgc aag ctc atc gac cag     1632
Arg Leu Met Ala Glu Asp Glu Glu Gly Tyr Arg Lys Leu Ile Asp Gln
    530                 535                 540
aag aag gac aag cgc ctg gcc tac ctc ttg cag cag aca gac gag tac     1680
Lys Lys Asp Lys Arg Leu Ala Tyr Leu Leu Gln Gln Thr Asp Glu Tyr
545                 550                 555                 560
gtg gct aac ctc acg gag ctg gtg cgg cag cac aag gct gcc cag gtc     1728
Val Ala Asn Leu Thr Glu Leu Val Arg Gln His Lys Ala Ala Gln Val
                565                 570                 575
gcc aag gag aaa aag aag aaa aag aaa aag aag aag gca gaa aat gca     1776
Ala Lys Glu Lys Lys Lys Lys Lys Lys Lys Lys Lys Ala Glu Asn Ala
            580                 585                 590
gaa gga cag acg cct gcc att ggg ccg gat ggc gag cct ctg gac gag     1824
Glu Gly Gln Thr Pro Ala Ile Gly Pro Asp Gly Glu Pro Leu Asp Glu
        595                 600                 605
acc agc cag atg agc gac ctc ccg gtg aag gtg atc cac gtg gag agt     1872
Thr Ser Gln Met Ser Asp Leu Pro Val Lys Val Ile His Val Glu Ser
    610                 615                 620
ggg aag atc ctc aca ggc aca gat gcc ccc aaa gcc ggg cag ctg gag     1920
Gly Lys Ile Leu Thr Gly Thr Asp Ala Pro Lys Ala Gly Gln Leu Glu

625                 630                 635                 640
gcc tgg ctc gag atg aac ccg ggg tat gaa gta gct ccg agg tct gat      1968
Ala Trp Leu Glu Met Asn Pro Gly Tyr Glu Val Ala Pro Arg Ser Asp
                645                 650                 655
agt gaa gaa agt ggc tca gaa gaa gag gaa gag gag gag gag gaa gag      2016
Ser Glu Glu Ser Gly Ser Glu Glu Glu Glu Glu Glu Glu Glu Glu Glu
            660                 665                 670
cag ccg cag gca gca cag cct ccc acc ctg ccc gtg gag gag aag aag      2064
Gln Pro Gln Ala Ala Gln Pro Pro Thr Leu Pro Val Glu Glu Lys Lys
        675                 680                 685
aag att cca gat cca gac agc gat gac gtc tct gag gtg gac gcg cgg      2112
Lys Ile Pro Asp Pro Asp Ser Asp Asp Val Ser Glu Val Asp Ala Arg
    690                 695                 700
cac atc att gag aat gcc aag caa gat gtc gat gat gaa tat ggc gtg      2160
His Ile Ile Glu Asn Ala Lys Gln Asp Val Asp Asp Glu Tyr Gly Val
705                 710                 715                 720
tcc cag gcc ctt gca cgt ggc ctg cag tcc tac tat gcc gtg gcc cat      2208
Ser Gln Ala Leu Ala Arg Gly Leu Gln Ser Tyr Tyr Ala Val Ala His
                725                 730                 735
gct gtc act gag aga gtg gac aag cag tca gcg ctt atg gtc aat ggt      2256
Ala Val Thr Glu Arg Val Asp Lys Gln Ser Ala Leu Met Val Asn Gly
            740                 745                 750
gtc ctc aaa cag tac cag atc aaa ggt ttg gag tgg ctg gtg tcc ctg      2304
Val Leu Lys Gln Tyr Gln Ile Lys Gly Leu Glu Trp Leu Val Ser Leu
        755                 760                 765
tac aac aac aac ctg aac ggc atc ctg gcc gac gag atg ggc ctg ggg      2352
Tyr Asn Asn Asn Leu Asn Gly Ile Leu Ala Asp Glu Met Gly Leu Gly
    770                 775                 780
aag acc atc cag acc atc gcg ctc atc acg tac ctc atg gag cac aaa      2400
Lys Thr Ile Gln Thr Ile Ala Leu Ile Thr Tyr Leu Met Glu His Lys
785                 790                 795                 800
cgc atc aat ggg ccc ttc ctc atc atc gtg cct ctc tca acg ctg tcc      2448
Arg Ile Asn Gly Pro Phe Leu Ile Ile Val Pro Leu Ser Thr Leu Ser
                805                 810                 815
aac tgg gcg tac gag ttt gac aag tgg gcc ccc tcc gtg gtg aag gtg      2496
Asn Trp Ala Tyr Glu Phe Asp Lys Trp Ala Pro Ser Val Val Lys Val
            820                 825                 830
tct tac aag gga tcc cca gca gca aga cgg gcc ttt gtc ccc cag ctc      2544
Ser Tyr Lys Gly Ser Pro Ala Ala Arg Arg Ala Phe Val Pro Gln Leu
        835                 840                 845
cgg agt ggg aag ttc aac gtc ttg ctg acg acg tac gag tac atc atc      2592
Arg Ser Gly Lys Phe Asn Val Leu Leu Thr Thr Tyr Glu Tyr Ile Ile
    850                 855                 860
aaa gac aag cac atc ctc gcc aag atc cgt tgg aag tac atg att gtg      2640
Lys Asp Lys His Ile Leu Ala Lys Ile Arg Trp Lys Tyr Met Ile Val
865                 870                 875                 880
gac gaa ggt cac cgc atg aag aac cac cac tgc aag ctg acg cag gtg      2688
Asp Glu Gly His Arg Met Lys Asn His His Cys Lys Leu Thr Gln Val
                885                 890                 895
ctc aac acg cac tat gtg gca ccc cgc cgc ctg ctg ctg acg ggc aca      2736
Leu Asn Thr His Tyr Val Ala Pro Arg Arg Leu Leu Leu Thr Gly Thr
            900                 905                 910
ccg ctg cag aac aag ctt ccc gag ctc tgg gcg ctg ctc aac ttc ctg      2784
Pro Leu Gln Asn Lys Leu Pro Glu Leu Trp Ala Leu Leu Asn Phe Leu
        915                 920                 925
ctg ccc acc atc ttc aag agc tgc agc acc ttc gag cag tgg ttt aac      2832
Leu Pro Thr Ile Phe Lys Ser Cys Ser Thr Phe Glu Gln Trp Phe Asn
    930                 935                 940
gca ccc ttt gcc atg acc ggg gaa aag gtg gac ctg aat gag gag gaa      2880
Ala Pro Phe Ala Met Thr Gly Glu Lys Val Asp Leu Asn Glu Glu Glu
945                 950                 955                 960
acc att ctc atc atc cgg cgt ctc cac aaa gtg ctg cgg ccc ttc ttg      2928
Thr Ile Leu Ile Ile Arg Arg Leu His Lys Val Leu Arg Pro Phe Leu
                965                 970                 975
ctc cga cga ctc aag aag gaa gtc gag gcc cag ttg ccc gaa aag gtg      2976
Leu Arg Arg Leu Lys Lys Glu Val Glu Ala Gln Leu Pro Glu Lys Val
            980                 985                 990
gag tac gtc atc aag tgc gac atg  tct gcg ctg cag cga  gtg ctc tac      3024
Glu Tyr Val Ile Lys Cys Asp Met  Ser Ala Leu Gln Arg  Val Leu Tyr

995                    1000                    1005
cgc cac atg cag gcc aag ggc gtg ctg ctg act gat ggc tcc gag    3069
Arg His Met Gln Ala Lys Gly Val Leu Leu Thr Asp Gly Ser Glu
    1010                    1015                    1020
aag gac aag aag ggc aaa ggc ggc acc aag acc ctg atg aac acc    3114
Lys Asp Lys Lys Gly Lys Gly Gly Thr Lys Thr Leu Met Asn Thr
    1025                    1030                    1035
atc atg cag ctg cgg aag atc tgc aac cac ccc tac atg ttc cag    3159
Ile Met Gln Leu Arg Lys Ile Cys Asn His Pro Tyr Met Phe Gln
    1040                    1045                    1050
cac atc gag gag tcc ttt tcc gag cac ttg ggg ttc act ggc ggc    3204
His Ile Glu Glu Ser Phe Ser Glu His Leu Gly Phe Thr Gly Gly
    1055                    1060                    1065
att gtc caa ggg ctg gac ctg tac cga gcc tcg ggt aaa ttt gag    3249
Ile Val Gln Gly Leu Asp Leu Tyr Arg Ala Ser Gly Lys Phe Glu
    1070                    1075                    1080
ctt ctt gat aga att ctt ccc aaa ctc cga gca acc aac cac aaa    3294
Leu Leu Asp Arg Ile Leu Pro Lys Leu Arg Ala Thr Asn His Lys
    1085                    1090                    1095
gtg ctg ctg ttc tgc caa atg acc tcc ctc atg acc atc atg gaa    3339
Val Leu Leu Phe Cys Gln Met Thr Ser Leu Met Thr Ile Met Glu
    1100                    1105                    1110
gat tac ttt gcg tat cgc ggc ttt aaa tac ctc agg ctt gat gga    3384
Asp Tyr Phe Ala Tyr Arg Gly Phe Lys Tyr Leu Arg Leu Asp Gly
    1115                    1120                    1125
acc acg aag gcg gag gac cgg ggc atg ctg ctg aaa acc ttc aac    3429
Thr Thr Lys Ala Glu Asp Arg Gly Met Leu Leu Lys Thr Phe Asn
    1130                    1135                    1140
gag ccc ggc tct gag tac ttc atc ttc ctg ctc agc acc cgg gct    3474
Glu Pro Gly Ser Glu Tyr Phe Ile Phe Leu Leu Ser Thr Arg Ala
    1145                    1150                    1155
ggg ggg ctc ggc ctg aac ctc cag tcg gca gac act gtg atc att    3519
Gly Gly Leu Gly Leu Asn Leu Gln Ser Ala Asp Thr Val Ile Ile
    1160                    1165                    1170
ttt gac agc gac tgg aat cct cac cag gac ctg caa gcg cag gac    3564
Phe Asp Ser Asp Trp Asn Pro His Gln Asp Leu Gln Ala Gln Asp
    1175                    1180                    1185
cga gcc cac cgc atc ggg cag cag aac gag gtg cgt gtg ctc cgc    3609
Arg Ala His Arg Ile Gly Gln Gln Asn Glu Val Arg Val Leu Arg
    1190                    1195                    1200
ctc tgc acc gtc aac agc gtg gag gag aag atc cta gct gca gcc    3654
Leu Cys Thr Val Asn Ser Val Glu Glu Lys Ile Leu Ala Ala Ala
    1205                    1210                    1215
aag tac aag ctc aac gtg gac cag aag gtg atc cag gcc ggc atg    3699
Lys Tyr Lys Leu Asn Val Asp Gln Lys Val Ile Gln Ala Gly Met
    1220                    1225                    1230
ttc gac cag aag tcc tcc agc cat gag cgg cgc gcc ttc ctg cag    3744
Phe Asp Gln Lys Ser Ser Ser His Glu Arg Arg Ala Phe Leu Gln
    1235                    1240                    1245
gcc atc ctg gag cac gag gag cag gat gag agc aga cac tgc agc    3789
Ala Ile Leu Glu His Glu Glu Gln Asp Glu Ser Arg His Cys Ser
    1250                    1255                    1260
acg ggc agc ggc agt gcc agc ttc gcc cac act gcc cct ccg cca    3834
Thr Gly Ser Gly Ser Ala Ser Phe Ala His Thr Ala Pro Pro Pro
    1265                    1270                    1275
gcg ggc gtc aac ccc gac ttg gag gag cca cct cta aag gag gaa    3879
Ala Gly Val Asn Pro Asp Leu Glu Glu Pro Pro Leu Lys Glu Glu
    1280                    1285                    1290
gac gag gtg ccc gac gac gag acc gtc aac cag atg atc gcc cgg    3924
Asp Glu Val Pro Asp Asp Glu Thr Val Asn Gln Met Ile Ala Arg
    1295                    1300                    1305
cac gag gag gag ttt gat ctg ttc atg cgc atg gac ctg gac cgc    3969
His Glu Glu Glu Phe Asp Leu Phe Met Arg Met Asp Leu Asp Arg
    1310                    1315                    1320
agg cgc gag gag gcc cgc aac ccc aag cgg aag ccg cgc ctc atg    4014
Arg Arg Glu Glu Ala Arg Asn Pro Lys Arg Lys Pro Arg Leu Met
    1325                    1330                    1335
gag gag gac gag ctc ccc tcg tgg atc atc aag gac gac gcg gag    4059
Glu Glu Asp Glu Leu Pro Ser Trp Ile Ile Lys Asp Asp Ala Glu

1340                    1345                    1350
gtg gag cgg ctg acc tgt gag gag gag gag gag aag atg ttc ggc      4104
Val Glu Arg Leu Thr Cys Glu Glu Glu Glu Glu Lys Met Phe Gly
    1355                    1360                    1365
cgt ggc tcc cgc cac cgc aag gag gtg gac tac agc gac tca ctg      4149
Arg Gly Ser Arg His Arg Lys Glu Val Asp Tyr Ser Asp Ser Leu
    1370                    1375                    1380
acg gag aag cag tgg ctc aag gcc atc gag gag ggc acg ctg gag      4194
Thr Glu Lys Gln Trp Leu Lys Ala Ile Glu Glu Gly Thr Leu Glu
    1385                    1390                    1395
gag atc gaa gag gag gtc cgg cag aag aaa tca tca cgg aag cgc      4239
Glu Ile Glu Glu Glu Val Arg Gln Lys Lys Ser Ser Arg Lys Arg
    1400                    1405                    1410
aag cga gac agc gac gcc ggc tcc tcc acc ccg acc acc agc acc      4284
Lys Arg Asp Ser Asp Ala Gly Ser Ser Thr Pro Thr Thr Ser Thr
    1415                    1420                    1425
cgc agc cgc gac aag gac gac gag agc aag aag cag aag aag cgc      4329
Arg Ser Arg Asp Lys Asp Asp Glu Ser Lys Lys Gln Lys Lys Arg
    1430                    1435                    1440
ggg cgg ccg cct gcc gag aaa ctc tcc cct aac cca ccc aac ctc      4374
Gly Arg Pro Pro Ala Glu Lys Leu Ser Pro Asn Pro Pro Asn Leu
    1445                    1450                    1455
acc aag aag atg aag aag att gtg gat gcc gtg atc aag tac aag      4419
Thr Lys Lys Met Lys Lys Ile Val Asp Ala Val Ile Lys Tyr Lys
    1460                    1465                    1470
gac agc agc agt gga cgt cag ctc agc gag gtc ttc atc cag ctg      4464
Asp Ser Ser Ser Gly Arg Gln Leu Ser Glu Val Phe Ile Gln Leu
    1475                    1480                    1485
ccc tcg cga aag gag ctg ccc gag tac tac gag ctc atc cgc aag      4509
Pro Ser Arg Lys Glu Leu Pro Glu Tyr Tyr Glu Leu Ile Arg Lys
    1490                    1495                    1500
ccc gtg gac ttc aag aag ata aag gag cgc att cgc aac cac aag      4554
Pro Val Asp Phe Lys Lys Ile Lys Glu Arg Ile Arg Asn His Lys
    1505                    1510                    1515
tac cgc agc ctc aac gac cta gag aag gac gtc atg ctc ctg tgc      4599
Tyr Arg Ser Leu Asn Asp Leu Glu Lys Asp Val Met Leu Leu Cys
    1520                    1525                    1530
cag aac gca cag acc ttc aac ctg gag ggc tcc ctg atc tat gaa      4644
Gln Asn Ala Gln Thr Phe Asn Leu Glu Gly Ser Leu Ile Tyr Glu
    1535                    1540                    1545
gac tcc atc gtc ttg cag tcg gtc ttc acc agc gtg cgg cag aaa      4689
Asp Ser Ile Val Leu Gln Ser Val Phe Thr Ser Val Arg Gln Lys
    1550                    1555                    1560
atc gag aag gag gat gac agt gaa ggc gag gag agt gag gag gag      4734
Ile Glu Lys Glu Asp Asp Ser Glu Gly Glu Glu Ser Glu Glu Glu
    1565                    1570                    1575
gaa gag ggc gag gag gaa ggc tcc gaa tcc gaa tct cgg tcc gtc      4779
Glu Glu Gly Glu Glu Glu Gly Ser Glu Ser Glu Ser Arg Ser Val
    1580                    1585                    1590
aaa gtg aag atc aag ctt ggc cgg aag gag aag gca cag gac cgg      4824
Lys Val Lys Ile Lys Leu Gly Arg Lys Glu Lys Ala Gln Asp Arg
    1595                    1600                    1605
ctg aag ggc ggc cgg cgg cgg ccg agc cga ggg tcc cga gcc aag      4869
Leu Lys Gly Gly Arg Arg Arg Pro Ser Arg Gly Ser Arg Ala Lys
    1610                    1615                    1620
ccg gtc gtg agt gac gat gac agt gag gag gaa caa gag gag gac      4914
Pro Val Val Ser Asp Asp Asp Ser Glu Glu Glu Gln Glu Glu Asp
    1625                    1630                    1635
cgc tca gga agt ggc agc gaa gaa gac tga                          4944
Arg Ser Gly Ser Gly Ser Glu Glu Asp
    1640                    1645


<210>  34
<211>  1647
<212>  PRT
<213>  Homo sapiens
<400>  3
Met Ser Thr Pro Asp Pro Pro Leu Gly Gly Thr Pro Arg Pro Gly Pro

1               5                   10                  15
Ser Pro Gly Pro Gly Pro Ser Pro Gly Ala Met Leu Gly Pro Ser Pro
            20                  25                  30
Gly Pro Ser Pro Gly Ser Ala His Ser Met Met Gly Pro Ser Pro Gly
        35                  40                  45
Pro Pro Ser Ala Gly His Pro Ile Pro Thr Gln Gly Pro Gly Gly Tyr
    50                  55                  60
Pro Gln Asp Asn Met His Gln Met His Lys Pro Met Glu Ser Met His
65                  70                  75                  80
Glu Lys Gly Met Ser Asp Asp Pro Arg Tyr Asn Gln Met Lys Gly Met
                85                  90                  95
Gly Met Arg Ser Gly Gly His Ala Gly Met Gly Pro Pro Pro Ser Pro
            100                 105                 110
Met Asp Gln His Ser Gln Gly Tyr Pro Ser Pro Leu Gly Gly Ser Glu
        115                 120                 125
His Ala Ser Ser Pro Val Pro Ala Ser Gly Pro Ser Ser Gly Pro Gln
    130                 135                 140
Met Ser Ser Gly Pro Gly Gly Ala Pro Leu Asp Gly Ala Asp Pro Gln
145                 150                 155                 160
Ala Leu Gly Gln Gln Asn Arg Gly Pro Thr Pro Phe Asn Gln Asn Gln
                165                 170                 175
Leu His Gln Leu Arg Ala Gln Ile Met Ala Tyr Lys Met Leu Ala Arg
            180                 185                 190
Gly Gln Pro Leu Pro Asp His Leu Gln Met Ala Val Gln Gly Lys Arg
        195                 200                 205
Pro Met Pro Gly Met Gln Gln Gln Met Pro Thr Leu Pro Pro Pro Ser
    210                 215                 220
Val Ser Ala Thr Gly Pro Gly Pro Gly Pro Gly Pro Gly Pro Gly Pro
225                 230                 235                 240
Gly Pro Gly Pro Ala Pro Pro Asn Tyr Ser Arg Pro His Gly Met Gly
                245                 250                 255
Gly Pro Asn Met Pro Pro Pro Gly Pro Ser Gly Val Pro Pro Gly Met
            260                 265                 270
Pro Gly Gln Pro Pro Gly Gly Pro Pro Lys Pro Trp Pro Glu Gly Pro
        275                 280                 285
Met Ala Asn Ala Ala Ala Pro Thr Ser Thr Pro Gln Lys Leu Ile Pro
    290                 295                 300
Pro Gln Pro Thr Gly Arg Pro Ser Pro Ala Pro Pro Ala Val Pro Pro
305                 310                 315                 320
Ala Ala Ser Pro Val Met Pro Pro Gln Thr Gln Ser Pro Gly Gln Pro
                325                 330                 335
Ala Gln Pro Ala Pro Met Val Pro Leu His Gln Lys Gln Ser Arg Ile
            340                 345                 350
Thr Pro Ile Gln Lys Pro Arg Gly Leu Asp Pro Val Glu Ile Leu Gln
        355                 360                 365
Glu Arg Glu Tyr Arg Leu Gln Ala Arg Ile Ala His Arg Ile Gln Glu
    370                 375                 380
Leu Glu Asn Leu Pro Gly Ser Leu Ala Gly Asp Leu Arg Thr Lys Ala
385                 390                 395                 400
Thr Ile Glu Leu Lys Ala Leu Arg Leu Leu Asn Phe Gln Arg Gln Leu
                405                 410                 415
Arg Gln Glu Val Val Val Cys Met Arg Arg Asp Thr Ala Leu Glu Thr
            420                 425                 430
Ala Leu Asn Ala Lys Ala Tyr Lys Arg Ser Lys Arg Gln Ser Leu Arg
        435                 440                 445
Glu Ala Arg Ile Thr Glu Lys Leu Glu Lys Gln Gln Lys Ile Glu Gln
    450                 455                 460
Glu Arg Lys Arg Arg Gln Lys His Gln Glu Tyr Leu Asn Ser Ile Leu
465                 470                 475                 480
Gln His Ala Lys Asp Phe Lys Glu Tyr His Arg Ser Val Thr Gly Lys
                485                 490                 495
Ile Gln Lys Leu Thr Lys Ala Val Ala Thr Tyr His Ala Asn Thr Glu
            500                 505                 510
Arg Glu Gln Lys Lys Glu Asn Glu Arg Ile Glu Lys Glu Arg Met Arg
        515                 520                 525
Arg Leu Met Ala Glu Asp Glu Glu Gly Tyr Arg Lys Leu Ile Asp Gln
    530                 535                 540
Lys Lys Asp Lys Arg Leu Ala Tyr Leu Leu Gln Gln Thr Asp Glu Tyr
545                 550                 555                 560

Val Ala Asn Leu Thr Glu Leu Val Arg Gln His Lys Ala Ala Gln Val
                565                 570                 575
Ala Lys Glu Lys Lys Lys Lys Lys Lys Lys Lys Lys Ala Glu Asn Ala
            580                 585                 590
Glu Gly Gln Thr Pro Ala Ile Gly Pro Asp Gly Glu Pro Leu Asp Glu
        595                 600                 605
Thr Ser Gln Met Ser Asp Leu Pro Val Lys Val Ile His Val Glu Ser
    610                 615                 620
Gly Lys Ile Leu Thr Gly Thr Asp Ala Pro Lys Ala Gly Gln Leu Glu
625                 630                 635                 640
Ala Trp Leu Glu Met Asn Pro Gly Tyr Glu Val Ala Pro Arg Ser Asp
                645                 650                 655
Ser Glu Glu Ser Gly Ser Glu Glu Glu Glu Glu Glu Glu Glu Glu Glu
            660                 665                 670
Gln Pro Gln Ala Ala Gln Pro Pro Thr Leu Pro Val Glu Glu Lys Lys
        675                 680                 685
Lys Ile Pro Asp Pro Asp Ser Asp Asp Val Ser Glu Val Asp Ala Arg
    690                 695                 700
His Ile Ile Glu Asn Ala Lys Gln Asp Val Asp Asp Glu Tyr Gly Val
705                 710                 715                 720
Ser Gln Ala Leu Ala Arg Gly Leu Gln Ser Tyr Tyr Ala Val Ala His
                725                 730                 735
Ala Val Thr Glu Arg Val Asp Lys Gln Ser Ala Leu Met Val Asn Gly
            740                 745                 750
Val Leu Lys Gln Tyr Gln Ile Lys Gly Leu Glu Trp Leu Val Ser Leu
        755                 760                 765
Tyr Asn Asn Asn Leu Asn Gly Ile Leu Ala Asp Glu Met Gly Leu Gly
    770                 775                 780
Lys Thr Ile Gln Thr Ile Ala Leu Ile Thr Tyr Leu Met Glu His Lys
785                 790                 795                 800
Arg Ile Asn Gly Pro Phe Leu Ile Ile Val Pro Leu Ser Thr Leu Ser
                805                 810                 815
Asn Trp Ala Tyr Glu Phe Asp Lys Trp Ala Pro Ser Val Val Lys Val
            820                 825                 830
Ser Tyr Lys Gly Ser Pro Ala Ala Arg Arg Ala Phe Val Pro Gln Leu
        835                 840                 845
Arg Ser Gly Lys Phe Asn Val Leu Leu Thr Thr Tyr Glu Tyr Ile Ile
    850                 855                 860
Lys Asp Lys His Ile Leu Ala Lys Ile Arg Trp Lys Tyr Met Ile Val
865                 870                 875                 880
Asp Glu Gly His Arg Met Lys Asn His His Cys Lys Leu Thr Gln Val
                885                 890                 895
Leu Asn Thr His Tyr Val Ala Pro Arg Arg Leu Leu Leu Thr Gly Thr
            900                 905                 910
Pro Leu Gln Asn Lys Leu Pro Glu Leu Trp Ala Leu Leu Asn Phe Leu
        915                 920                 925
Leu Pro Thr Ile Phe Lys Ser Cys Ser Thr Phe Glu Gln Trp Phe Asn
    930                 935                 940
Ala Pro Phe Ala Met Thr Gly Glu Lys Val Asp Leu Asn Glu Glu Glu
945                 950                 955                 960
Thr Ile Leu Ile Ile Arg Arg Leu His Lys Val Leu Arg Pro Phe Leu
                965                 970                 975
Leu Arg Arg Leu Lys Lys Glu Val Glu Ala Gln Leu Pro Glu Lys Val
            980                 985                 990
Glu Tyr Val Ile Lys Cys Asp Met  Ser Ala Leu Gln Arg  Val Leu Tyr
        995                 1000                 1005
Arg His  Met Gln Ala Lys Gly  Val Leu Leu Thr Asp  Gly Ser Glu
    1010                 1015                 1020
Lys Asp  Lys Lys Gly Lys Gly  Gly Thr Lys Thr Leu  Met Asn Thr
    1025                 1030                 1035
Ile Met  Gln Leu Arg Lys Ile  Cys Asn His Pro Tyr  Met Phe Gln
    1040                 1045                 1050
His Ile  Glu Glu Ser Phe Ser  Glu His Leu Gly Phe  Thr Gly Gly
    1055                 1060                 1065
Ile Val  Gln Gly Leu Asp Leu  Tyr Arg Ala Ser Gly  Lys Phe Glu
    1070                 1075                 1080
Leu Leu  Asp Arg Ile Leu Pro  Lys Leu Arg Ala Thr  Asn His Lys
    1085                 1090                 1095
Val Leu  Leu Phe Cys Gln Met  Thr Ser Leu Met Thr  Ile Met Glu

1100                    1105                    1110
Asp Tyr Phe Ala Tyr Arg Gly Phe Lys Tyr Leu Arg Leu Asp Gly
        1115                    1120                    1125
Thr Thr Lys Ala Glu Asp Arg Gly Met Leu Leu Lys Thr Phe Asn
        1130                    1135                    1140
Glu Pro Gly Ser Glu Tyr Phe Ile Phe Leu Leu Ser Thr Arg Ala
        1145                    1150                    1155
Gly Gly Leu Gly Leu Asn Leu Gln Ser Ala Asp Thr Val Ile Ile
        1160                    1165                    1170
Phe Asp Ser Asp Trp Asn Pro His Gln Asp Leu Gln Ala Gln Asp
        1175                    1180                    1185
Arg Ala His Arg Ile Gly Gln Gln Asn Glu Val Arg Val Leu Arg
        1190                    1195                    1200
Leu Cys Thr Val Asn Ser Val Glu Glu Lys Ile Leu Ala Ala Ala
        1205                    1210                    1215
Lys Tyr Lys Leu Asn Val Asp Gln Lys Val Ile Gln Ala Gly Met
        1220                    1225                    1230
Phe Asp Gln Lys Ser Ser Ser His Glu Arg Arg Ala Phe Leu Gln
        1235                    1240                    1245
Ala Ile Leu Glu His Glu Glu Gln Asp Glu Ser Arg His Cys Ser
        1250                    1255                    1260
Thr Gly Ser Gly Ser Ala Ser Phe Ala His Thr Ala Pro Pro Pro
        1265                    1270                    1275
Ala Gly Val Asn Pro Asp Leu Glu Glu Pro Pro Leu Lys Glu Glu
        1280                    1285                    1290
Asp Glu Val Pro Asp Asp Glu Thr Val Asn Gln Met Ile Ala Arg
        1295                    1300                    1305
His Glu Glu Glu Phe Asp Leu Phe Met Arg Met Asp Leu Asp Arg
        1310                    1315                    1320
Arg Arg Glu Glu Ala Arg Asn Pro Lys Arg Lys Pro Arg Leu Met
        1325                    1330                    1335
Glu Glu Asp Glu Leu Pro Ser Trp Ile Ile Lys Asp Asp Ala Glu
        1340                    1345                    1350
Val Glu Arg Leu Thr Cys Glu Glu Glu Glu Lys Met Phe Gly
        1355                    1360                    1365
Arg Gly Ser Arg His Arg Lys Glu Val Asp Tyr Ser Asp Ser Leu
        1370                    1375                    1380
Thr Glu Lys Gln Trp Leu Lys Ala Ile Glu Glu Gly Thr Leu Glu
        1385                    1390                    1395
Glu Ile Glu Glu Glu Val Arg Gln Lys Lys Ser Ser Arg Lys Arg
        1400                    1405                    1410
Lys Arg Asp Ser Asp Ala Gly Ser Ser Thr Pro Thr Thr Ser Thr
        1415                    1420                    1425
Arg Ser Arg Asp Lys Asp Asp Glu Ser Lys Lys Gln Lys Lys Arg
        1430                    1435                    1440
Gly Arg Pro Pro Ala Glu Lys Leu Ser Pro Asn Pro Pro Asn Leu
        1445                    1450                    1455
Thr Lys Lys Met Lys Lys Ile Val Asp Ala Val Ile Lys Tyr Lys
        1460                    1465                    1470
Asp Ser Ser Ser Gly Arg Gln Leu Ser Glu Val Phe Ile Gln Leu
        1475                    1480                    1485
Pro Ser Arg Lys Glu Leu Pro Glu Tyr Tyr Glu Leu Ile Arg Lys
        1490                    1495                    1500
Pro Val Asp Phe Lys Lys Ile Lys Glu Arg Ile Arg Asn His Lys
        1505                    1510                    1515
Tyr Arg Ser Leu Asn Asp Leu Glu Lys Asp Val Met Leu Leu Cys
        1520                    1525                    1530
Gln Asn Ala Gln Thr Phe Asn Leu Glu Gly Ser Leu Ile Tyr Glu
        1535                    1540                    1545
Asp Ser Ile Val Leu Gln Ser Val Phe Thr Ser Val Arg Gln Lys
        1550                    1555                    1560
Ile Glu Lys Glu Asp Asp Ser Glu Gly Glu Glu Ser Glu Glu Glu
        1565                    1570                    1575
Glu Glu Gly Glu Glu Glu Gly Ser Glu Ser Glu Ser Arg Ser Val
        1580                    1585                    1590
Lys Val Lys Ile Lys Leu Gly Arg Lys Glu Lys Ala Gln Asp Arg
        1595                    1600                    1605
Leu Lys Gly Gly Arg Arg Arg Pro Ser Arg Gly Ser Arg Ala Lys
        1610                    1615                    1620

Pro Val Val Ser Asp Asp Asp Ser Glu Glu Glu Gln Glu Glu Asp
    1625                1630                1635
Arg Ser Gly Ser Gly Ser Glu Glu Asp
    1640                1645

<210> 4
<211> 163717
<212> DNA
<213> Homo sapiens
<220>
<221> CDS
<222> (1)..(225)
<223>
<220>
<221> CDS
<222> (3930)..(4059)
<223>
<220>
<221> CDS
<222> (10444)..(10878)
<223>
<220>
<221> CDS
<222> (18207)..(18462)
<223>
<220>
<221> CDS
<222> (25575)..(25701)
<223>
<220>
<221> CDS
<222> (27650)..(27823)
<223>
<220>
<221> CDS
<222> (29269)..(29442)
<223>
<220>
<221> CDS
<222> (31794)..(31964)
<223>
<220>
<221> CDS
<222> (41396)..(41449)
<223>
<220>
<221> CDS
<222> (44190)..(44320)
<223>
<220>
<221> CDS
<222> (44544)..(44601)
<223>
<220>
<221> CDS
<222> (47207)..(47307)
<223>
<220>
<221> CDS
<222> (48607)..(48754)
<223>
<220>
<221> CDS
<222> (52810)..(52973)
<223>
<220>
<221> CDS
<222> (54325)..(54391)

<223>
<220>
<221>  CDS
<222>  (55064)..(55174)
<223>
<220>
<221>  CDS
<222>  (57807)..(58049)
<223>
<220>
<221>  CDS
<222>  (59478)..(59591)
<223>
<220>
<221>  CDS
<222>  (67635)..(67742)
<223>
<220>
<221>  CDS
<222>  (68363)..(68449)
<223>
<220>
<221>  CDS
<222>  (72548)..(72594)
<223>
<220>
<221>  CDS
<222>  (74981)..(75147)
<223>
<220>
<221>  CDS
<222>  (81232)..(81395)
<223>
<220>
<221>  CDS
<222>  (86800)..(87027)
<223>
<220>
<221>  CDS
<222>  (90436)..(90513)
<223>
<220>
<221>  CDS
<222>  (94697)..(94915)
<223>
<220>
<221>  CDS
<222>  (132664)..(132881)
<223>
<220>
<221>  CDS
<222>  (152549)..(152654)
<223>
<220>
<221>  CDS
<222>  (153119)..(153220)
<223>
<220>
<221>  CDS
<222>  (157074)..(157206)
<223>
<220>
<221>  CDS
<222>  (162244)..(162386)
<223>
<220>
<221>  CDS
<222>  (163682)..(163714)
<223>

<400> 4
atg tcc acg ccc aca gac cct ggt gcg atg ccc cac cca ggg cct tcg 48
Met Ser Thr Pro Thr Asp Pro Gly Ala Met Pro His Pro Gly Pro Ser
1               5                   10                  15
ccg ggg cct ggg cct tcc cct ggg cca att ctt ggg cct agt cca gga 96
Pro Gly Pro Gly Pro Ser Pro Gly Pro Ile Leu Gly Pro Ser Pro Gly
            20                  25                  30
cca gga cca tcc cca ggt tcc gtc cac agc atg atg ggg cca agt cct 144
Pro Gly Pro Ser Pro Gly Ser Val His Ser Met Met Gly Pro Ser Pro
        35                  40                  45
gga cct cca agt gtc tcc cat cct atg ccg acg atg ggg tcc aca gac 192
Gly Pro Pro Ser Val Ser His Pro Met Pro Thr Met Gly Ser Thr Asp
    50                  55                  60
ttc cca cag gaa ggc atg cat caa atg cat aag gtaagagttt gttctcccat 245
Phe Pro Gln Glu Gly Met His Gln Met His Lys
65                  70                  75
tcaaactcaa cttctgataa gtggatggct aatatttctg ataaccccat ccccctttct 305
actgttgtta acaagaaaat catgcaagat ctttgtcttg tatatctcat atattattaa 365
aaacgcatat tgtgaggtac ttttattgct acagatgttg catcagttat gaagatcaga 425
tgttggcttt ttaaaatact gacatagact atgcagttct gataagtaga tcctgtgatt 485
tgtgggttga aggtattttt gtcttggtga attagtacat tagtctataa gtcttatttt 545
ttcctatctg tgttaagtaa cttatccact aatgaacata ctgaaaatta ttctttccta 605
aaaaggctga catttattaa caaaaattag atcccaaaga gtagaaggaa aaagtcctta 665
tctaaaggat tttttggaaa gggctttatt gaaattaaat agtttctagg gaatccttaa 725
aatgcacatt gctgtctctc tgtgaagcac ttttattctg gttttggaag aaggttctga 785
tttttgcaga gctcacgtcc agctgtttat ttctggacta aggttgtaat tatcctttat 845
ttatgaggct tggattgttg ctgtggggat gatcatatct gtacaacttg gcattatgcc 905
ttcacaggag atgcaaagaa gagagacagc cttttgagaa agtaaacaag atcctctgtg 965
acagtttgct ttgaaagtaa gcaatagcca agaaaaatct atgaggacat tacatactac 1025
ctgaaggttt tgcctaatga gacacgtaga ggttttacac tccttgggat gatatcacag 1085
gtggataggg tggcagggct gcttcttgac attaagtagg tgtattcatt aggattctcc 1145
agagaaacgg aaccaatagg agatagagat atatgtaaaa agagacttat tatgaggaat 1205
tggctcacac agttatggaa gctgagagtt ctattatctg cagtcaccaa tctggatacc 1265
cagcagaacc tgtggtatag ttccggtctg agtctacagg ctcagacctg agcctgagaa 1325
ccaggagttc caggagaact gatgatgtga gttccagtga tgtgagaacc aggagaacca 1385
ggagaactga tgatgtgagt tccagtctga gtccaaagag ccagaagagt cagtggagta 1445
agttcaagtc tgagggcagg agaagaccca gcttaagcag tcaggcagag agaaagcttc 1505
tcctttcctc tagctttttt tttttttttt tttcttattc aggccctcca tggattttgg 1565
gtgaggccca ccgacattgc tggaggcaat ctgatttatt cagtctaaca attcaaatga 1625
taatctcatc caggaatacc ctgacagaca tacccagaaa taatgtttaa tcaaatatct 1685
aggcacctga ggcccagtca agttgacaca taaaatgaac cattacagta gacaagccgt 1745
ggaagatcct ggcttaaaac aaaaagatta ccacagaaaa gagcaagtct ttcctccagt 1805
tacgtaggac tttaggtcag ttttagggta gggcatcaga gcccaaaagg gaagatcctt 1865
ttgttaaaag tgttgtaatt tgatgatttt tcaaattagg aaagtaatat atggttattt 1925
tgaaaaggaa aacaatactg agataaacag ataatcttac cttcacccca agtaacagct 1985
tgttgtacat gctttttcta tcttgctttt ctgtatgttc attacaaata ctgtgtaagc 2045
acagatatgt atatattggt gttttttttt ggttactttt cttttgctat tatagaatca 2105
tgttgtctct gttaattact ctgtaacttt cttttttcat ttaacaatgt attatggata 2165
tccctatgac attctctagt tttctgtctc ctctttttaa cagatacatt tgttagcttg 2225
tcagagatct agagaaataa agctgctcgt tttccatgtc atatttttca ggggacctat 2285
tgaacatatt gacatccaag tcctgaaatt gttttctgtt tagattaaat gaatttggga 2345
gctatattct ggatctgatc aatgacttgt aaaccttcct ctaagtggct tacataattt 2405
cagaataatt tgaaggtgtc tcaccttcaa aatgaaagat aaaatgtgag ggaaatactt 2465
agctaaactt tattgttctt tagcagttaa aaattcaatg tgtgagtgtt gggtcagtgt 2525
tatgtttttc tcctggattg tttttattaa ttgttatctc ttctgatccc ttcattttat 2585
gtcctagcat ttttattaca ctttctcata ttctctctac cctcttaaat actccctctt 2645
tttgttttgc tttgctcgca ttctgtgagt tttgcatttt ttgccatcct gcttctcctt 2705
ggccccagcc cctcagatag ttccctgcgt gaggacacgg gtgagggttt agagctcaca 2765
cctgctgacc agatgccttc acttttttct atgcttttta taggcagttt tatttcttct 2825
cttattcttc cttcttcctc tgaggcttca gctttttgct tttcaacatt actggaaata 2885
aataaacaag tggcctaaat acatctgctg tgttgctttg atttcctctc tattattact 2945
atttaacaga ctcagtgaca ctctttatac cagattcttc ccatttttct tccaaacatc 3005
ctctttatga tacatttttt cctcttttca agtgcatact tttgactctg cagaaattaa 3065
ctttcttttc tccaaaactg caaacactca tactttcttg cttgaagtca tttggtttct 3125
ataaactcct ggaaatattt ctttcaggct gttttgaaaa ttgctattac agtttgggga 3185
ggaatagact tttccttttt caaacacatg cttcattgcc caattctgga ttggattaat 3245
gtcgattcaa tagctgttta ttgcatgtct gtcacgtatt atgcctcaag ttttgggtcc 3305
agcccctgtg ttcgtttatt tccaaactgt gcctttaacc ttttccgttc ctcctccctc 3365
tgtagtacag ctttatctat tgcacacact ttccttttca gtattcaccc cccattcacg 3425

gctctgtcct gtctgttcct ctctttccaa ctgccatgga accctgtggt ttcaactttc 3485
aaactttcca tggttccttc ccttccaatg aagaaaaatt accaaaaact ggatcatcac 3545
agatgtgtca aacagccttc cttcagactt cagactaact tcaacatata acaggtttca 3605
agattactta ccagggtatg gaaaaatagc ctatatttgt ctaccttgtg accaggactt 3665
cattttcatt gcctgttctg atattacaaa aaacaaacaa acaaacaaac aaacaaaaaa 3725
acaaccctcc actgtttaaa gacttaacat ttgacttaca gatagtttgc tgatcctttt 3785
ggtctagtct ctttttttaa ctctagaatg ggtagtagat gatagtgcca cacttttaaa 3845
gaacttagga aggggtctga aaactccaaa tagaaatatt ttaccttata gaggtgtcta 3905
actaatgtcc tttaaatgtt tcag ccc atc gat ggt ata cat gac aag ggg 3956
Pro Ile Asp Gly Ile His Asp Lys Gly
                80
att gta gaa gac atc cat tgt gga tcc atg aag ggc act ggt atg cga 4004
Ile Val Glu Asp Ile His Cys Gly Ser Met Lys Gly Thr Gly Met Arg
85                  90                  95                  100
cca cct cac cca ggc atg ggc cct ccc cag agt cca atg gat caa cac 4052
Pro Pro His Pro Gly Met Gly Pro Pro Gln Ser Pro Met Asp Gln His
                105                 110                 115
agc caa g gtttgtgtcc gctgcacacc tgatactggt tccccagcat agtctttcag 4109
Ser Gln

tctttcctgt tggatatttt aattatgaat tccaaagaat gtgtctaagg aaggttgaac 4169
ctagtaggtt tcttttcctt atggaaatct acctccgtcc tcaccaatac aacctgatga 4229
tttatatggg aaatttattt tccgaagtcc tagtaatttt actagccacc atgtgtccaa 4289
gcagtctgca aagagctcta tgtactttat ctagaatctt tatcgtgatc ttttagggta 4349
aattaggtta tcccagtttt atagatagag aagataaagc tggacaattt actttgtttt 4409
gctaagttta catgtccaat cggtgacagg gccagaaaga gaatctaggc ctgcttccca 4469
gcaacagtgt agatgtgaag gtaaggtgaa ccacctgtgt cccttgaatt gcatgcttat 4529
ccattgtcta taaggcagct ttatctggca gtgaaattat gtgtcatttc tggtgggtgg 4589
ctgggcctgc catagcaaat taccacaaac agaaatgtat tctcttatat aattctggag 4649
gcaaagtctg aaatcaaagc atcagcaggg ccatgctctc tctgaaggct ttagagaagt 4709
atccttcctt gctgcttcct agtttctggt ggttgccagc aatccttggc attcctggac 4769
ttgcagatgc atcactgcaa tctctgcctt agtatccaca tgaaattttt cctgtgtgtc 4829
tgtctgtgtc cagattgtct tctttttatg ggggccattc attggattag ggtcttccct 4889
aatgcagtat ggcctcatct taatgtgatt acatctacaa aaatactgtt tcaggccggg 4949
cctggtggct gaagcctgta atcccagcgc tttgggaggc tgagacgggc ggatcacgag 5009
atcaagagat cgagaccatc ctggtcaaca tggtgaaacc ccgtctctag taaacataca 5069
aaaatcagct gggcgtggtg gcacatgcct gtagtctcag ctacttggga ggctgaggca 5129
tgagaatcgc ttgaacccgg gggcagaggt tgcagtgagc cgagatcgca ccattgctct 5189
ctagcctggc gacagagcaa gactgcgtct caaaaaaaaa aaaaaaaaaa aaaaactatt 5249
tcaaagtaag gcacattccc aggtactggg ggttaggact tcaacatatt tggaggaggg 5309
ggaacacaat tcaactcaca acaatggcta aatcagtata tggcatattt cttttcttct 5369
tcatacgtaa caagtcctct ttcttttctt cactggtgaa aactattcac gtacctcaaa 5429
gccttttaat acaatgtagc ctgatttcag agtcattatg tggtcatttc aacatatgac 5489
ggacatgagt taatctagca ctaattaaaa ttaatatgta gttattccaa ttcaaggttt 5549
atggtttatg cgttgctata tataatcctg tgtaattaat gtgtgtgttt cctctatcca 5609
tgatcagcta tccatccact cgagggaaga cttttggctg tgaggattta gagaagtcta 5669
tgcacagttg ttttcttttt gaaatataaa taggcaagtc atggaggcat aggagtgcct 5729
ccccactagt atttgaacag gcttttgaac atcccagact cttatctgaa ttttggtggc 5789
aggcaggcat aattgttgca gataactcat agaatcttga ccatgtcaga agagcagcct 5849
aaaagcactg ggaatttttg cagtacaaga agaacagatt tgactcattc acggttatga 5909
atgaagtaag acaatatttc caggcttatc atttacattt gaaggggcca agtatgttca 5969
tggagctaat ataagccttt atttatttat ttatttattt atttatttat ttatttattt 6029
atatttttga ggcacagtct ccctctgttg cccaggctgg agtgcagtgg catgatctca 6089
gctcactgca acctctacct cccgggttca agcgattctc ctgcctcagc ctcctgagta 6149
gctgggacta caggcgtgtg ccaccacacc cagctaattt ttgtattttt aatagagaca 6209
gggtttcacc atgttgccca ggctggtctt tgaactcctg acctcaggtg atccaccctc 6269
ctcagcctcc cacagtgctg ggattacagg catgagccac catgcctggc cttaaacctt 6329
tatttttaat acctgggagg tatagatgag taacgtttag tgattcttga agccaggtgc 6389
ttactttttc tgctcctgtt gttgcagtag ctctaatagt ggagtcctag gaaaacctga 6449
aaactaaaac cactttaaat gcacaaatca taattgagag catttttact tttatttctg 6509
tgatcacttg gctcccctct tcaacttaag aatgatgtct tatttatctt ggaatccaca 6569
acacttagga tgatatctag ctagcacatg gctagataga tattagtaaa ttttgttcaa 6629
ttaggtgtgg aagtttgtgt attaaagagt caacagttta aaatgatctc aacttttata 6689
taatttcaat gaagtataat aactttaaat gcaaaggtct gcattaaagg gaatcagaat 6749
taacatgtaa tattctctaa aatagtgaag tgcattgtag gctgaggaat gagttattta 6809
agggtatatt tttgaataaa gcaatattag tataatgcag tcctatttta tataatgatc 6869
taaaaagaat ttagacttgg taatagcacg tgtctttaaa aaactcactg tgaaaagcca 6929
tttattaata taaaaacaca ggaacagaga gatgtggtat tgattggaag gctgcgagct 6989
tttaactacc tagcaaccta ttaggttatt ttagggtatt ggtcaagaag gaaagccatt 7049

tccttcaata aatactgaat tgtgtacctt atggtcccaa tgataacatt gtttattttt 7109
agtccattgg atagaaaccc ttcaaaagca ggtatatatt taaatagtgt gtgtgtgtgt 7169
gtgtgtgtgt gtttgtatgt gtgcgtgtag agaaaattca tgtagaattg gcacttagaa 7229
gttcaagatt attaatagga gagaggttat tgcatctctc tgaggcaagg tgaaactggg 7289
gagataatag cccttgaaga ctagggaaag aaaactgcca ctttttgact actttcgaat 7349
gagcaaatca aagaagattc caaaaagagc agaatctgca tcaacactgt catcctacag 7409
accaatgtat attcactctc aggcaatttc ttcactctgt tttctcaact aaagataatt 7469
accaaaatat gtgatgttgt atcacttaga ttctgttttt cctcaagttt tatgggatgc 7529
tattcatgta gaaataaaaa tctctatttt taataccata cggtgacaga atgattctat 7589
tcacatcccc tacaaattct gggaaatgat tcttctcact ttcattttct tctgatcaaa 7649
tttgaggtaa aattttgttg tattgactaa ctcagaaatg atttttattt ccctctcaat 7709
tttctgatca aatttgaggt aggaattttt ttgtattgat tttccagtga ccaggttctt 7769
tttttcagtt tgaattctta tgtttaatgt aactgtctca ccttcataat gttgccttgt 7829
tcatagttta aaagagtgat ttaagaaata gtgttataca gttgaattta gactaggttg 7889
cctaatattt tggttcattg tattaggcat cttatgattt gccatgagtg agattgtaat 7949
tgatgacctt aggaaatagg aaagaccgga aatcaggaaa catatgacta attttaagcc 8009
ctggtcgtat ctcttctact gtgaaaatgt tagtagcttt ggtggaaaga tttttcctct 8069
tatggtagac tcagtgctgg ccagaaagac gtcataatga acagactgcc attttgtact 8129
agcgttacaa ctagggggtt aatgtaagcc ttcttaaaga agaatgtatg ctgaagttaa 8189
gtgtctgtta ttgatattct ctaagtgttt attgttataa taacaacatg tcaggattta 8249
aatttctgct agtcttgcaa gctttcaagg gaattttctt ggctaattta tataaccaac 8309
taaaactgta atcactgcat ggtgtttgtg cttcctcacg gagtagggat ttgtggaatg 8369
aaatttagca attcttctat ttacctttag ggcaggattc tcctgaatag agttctactt 8429
ctcatcagtt ggtggagcaa aacccattct tggtttaggg gatcacgcta aatctgcctc 8489
cctctccaaa tgacaattgg agataaaagt aactgaatct tttattttct gtggtcttgt 8549
gaattatgat ggtgaatccc cagttggtgg cggtcatata ttctccggcc tctgtgattc 8609
tgcagatgca cagagctctt tcagttcaaa tttttcttag gttcgggcat gcagcaggaa 8669
tttaataaac acttatgtga tgacctttgt gagaactgtg acacgattac atttattaaa 8729
gggcagagac ttttcatcta tgtatgtaat gccatattct gggacacctt tgacttgatt 8789
aattggcact ttgtttcagc tcaaggacac cttttggatt gctttaggcc actttgttct 8849
ttccttcatc ttggagctcc ctccaacaat gttcagtcat atgtctttct aggaattcta 8909
tggccaagct atgcaggtca tctcctgaaa taagtccctt gaggtttttt gtgcattttg 8969
atttttcttc cccagaaaag tgttcttcaa tttcccattt agatctctta taaaccaaac 9029
agtttactct catttttatg ttatatcaca agtggttcta gaaggttttt gaaactttca 9089
agtccctaac ttattttcat ataaccaaat ttgtattaga tcatggaaag ggcatatgca 9149
ttagaatcag acagaccttt gtttaaatat taaagctccc aaaagttaat tgtgtgactt 9209
tgggtaactg gtttactctc tgagcctcat tttcctcatt ttaaaaaatg tggtaacagt 9269
atatatcttt taggttgttg taaggattga agaaataatg tatgttctac ccatatcttc 9329
ttgattcccc ttaccatttc tataccccca ccagcctgat ttcctgtgcc cagaaccact 9389
gtcttggttc acttccgtct actgacacct acctttccca cataatggca aagtccctgg 9449
agaataataa tctttagggc attagtcata accagtggct taaccagtga ctgctgattg 9509
caggagtata agtaccccag cttctttacc cctttcctga aataatttgg aggcaagtta 9569
tttactatat attctagagt ttctgcactg aattaatctt caggtaccca ctgcagcagc 9629
tggcttgaaa ataccccccc tttactggct gccttccctt ccttatatta ccttttcata 9689
cccctattag tgtcccctta ccttcccagt aaattactag tactcaatcc ttaaataaga 9749
ctctgctact agggagaacc caactgtgac agttgagtgc aagtcaactc cttgcatagt 9809
acctacaaca tgtaggcact caataattgc aagttattca taacatgcat aataataaac 9869
caagtgttcg ctcttcacct aagaaattct ttattcccca ttgagatata tcaggttatg 9929
ttctcgcagt tggagctact tttaccattc agtactgtcc cttggttagt ggtgaacaac 9989
aaccactaac ggtttacctt ctaggacaca tgtagctatt gatcacttga agtgtggtta 10049
gttcgaattg agatgtcctt taagtgtaaa atatttagtg gatttcaaag acttaatatg 10109
aaaaaaagaa tgtagaatgt ctcatcaaca gtttttacat tttaaaatga tgttttgggt 10169
atgttggttt aagtaaaata catttaaatt aactttacct gctttttttt tttttaacac 10229
tttaaagtgg ctaccagaaa attttaaatt ttacatatat ataaagcaca tattgatatg 10289
taaagatctt aaactccata taattaataa atgatatgtc attcaaattt ctgtcagaca 10349
gtgttgctgt ggacaattat tagagtattc agggatatct ctctttcagg gttgtcaggg 10409
gcagcctgtg atttcctttt gtgttttatt ttag gt tat atg tca cca cac cca 10463
Gly Tyr Met Ser Pro His Pro
    120                 125
tct cca tta gga gcc cca gag cac gtc tcc agc cct atg tct gga gga 10511
Ser Pro Leu Gly Ala Pro Glu His Val Ser Ser Pro Met Ser Gly Gly
                130                 135                 140
ggc cca act cca cct cag atg cca cca agc cag ccg ggg gcc ctc atc 10559
Gly Pro Thr Pro Pro Gln Met Pro Pro Ser Gln Pro Gly Ala Leu Ile
            145                 150                 155
cca ggt gat ccg cag gcc atg agc cag ccc aac aga ggt ccc tca cct 10607
Pro Gly Asp Pro Gln Ala Met Ser Gln Pro Asn Arg Gly Pro Ser Pro
        160                 165                 170
ttc agt cct gtc cag ctg cat cag ctt cga gct cag att tta gct tat 10655

Phe Ser Pro Val Gln Leu His Gln Leu Arg Ala Gln Ile Leu Ala Tyr
    175                 180                 185
aaa atg ctg gcc cga ggc cag ccc ctc ccc gaa acg ctg cag ctt gca    10703
Lys Met Leu Ala Arg Gly Gln Pro Leu Pro Glu Thr Leu Gln Leu Ala
190                 195                 200                 205
gtc cag ggg aaa agg acg ttg cct ggc ttg cag caa caa cag cag cag    10751
Val Gln Gly Lys Arg Thr Leu Pro Gly Leu Gln Gln Gln Gln Gln Gln
                210                 215                 220
caa cag cag cag cag cag cag cag cag cag cag cag cag cag caa cag    10799
Gln Gln Gln Gln Gln Gln Gln Gln Gln Gln Gln Gln Gln Gln Gln Gln
            225                 230                 235
cag ccg cag cag cag ccg ccg caa cca cag acg cag caa caa cag cag    10847
Gln Pro Gln Gln Gln Pro Pro Gln Pro Gln Thr Gln Gln Gln Gln Gln
        240                 245                 250
ccg gcc ctt gtt aac tac aac aga cca tct g gtaggttaat acgcaaccaa    10898
Pro Ala Leu Val Asn Tyr Asn Arg Pro Ser
    255                 260
atgaataatg ccatggtcca actcggataa caaagactgc tcaccaaaac accgggttgt    10958
taaaagcccg gggctgacgt agccttttgt tatacctcac tggctctcta tccttgctcc    11018
acttagatgg ccaagattct tggtcttccc ctgctgttga gacctagggc agtgtttctt    11078
aaccttaaaa ggtggttgtg caagctggag acccagggaa aaagcacctg tgattttgcg    11138
ttctctttga agcggttcaa ggtttcttgg aagcccatcc acacacacaa ggttaagaac    11198
ctctggactt agtgcatttc atccccagaa ctcactgctc agatcagccc cactagagcc    11258
attttataat ttgttgattt gtttatttct atcccttttt gttatgaaga acatctggag    11318
aggcttacaa gaagcatgcc tgttgtaaaa taattatgag tagcaaaatc agcaacaagg    11378
gaaaatagaa aaaaaagtaa gaaattgagg caagaaagta gggctgcaaa agttctcata    11438
gttgctgtat ttgcttcaaa gatgacttga gtttcttgac aatcagagcc aaaaggagac    11498
aaagtcctgt atctaagtct cgccctttgg aaggaagttc cttaccagtt tctcagaaaa    11558
agccatttct agcatgagag tatagagttg atttcccagg tgagattatt ttttaaatta    11618
ttatcatcat cattattgta agaggcactg catgacagaa acatcaatag catttgttca    11678
taatagacta acagattcaa catggcctat ttcttataca ccttgggatg aagtctgaag    11738
ctatgacatt atacaatggg tagaaatgat acaatccttt gcatggcctc ttggtcttct    11798
gtcttgatcc cattgataga cttcagaggt gcaggggact gtgaagtgat cttccatgaa    11858
ggttgttttc tctaggaagc gttttgttaa agaactggaa aagcagagat tgtatgggtc    11918
tactctaatg aaaactttgg attctaggga atgaggtact tccttgacgt tgataccatt    11978
tcctctgagt ggtttccatt catttaggca agttctgaca tgcaggttgt tgcaaacagc    12038
tagtttggga tattggtact gttcctgggt tttggccctt ataccatggg agagagttca    12098
gattagtatt ttttcattaa tattctgcct attgagtttg tatacgttta ttttctggta    12158
gaggcagctc caagagtagg cctacctaag agatatcctc atcttattaa ttgttatagc    12218
cttaagaatg gatgtcttag taagtctgag ctgctgtaat gaaaggtcat tgactgggtg    12278
gcttaaaaac agcaaattta tttcttacag ctctgaatgc tgggaattcc aacctcaaaa    12338
caccagcaga ttcagtgtct ggtgagggcc tgcttcctga ttcatagatg gcttccttct    12398
cactgtgtcc tcacatggca gaagagacaa ggcagctctc tggagcctct tttacaaggg    12458
catgaatccc aattatgagg actctactct catgaaatag tcacctccca aagaccccac    12518
cttctaatac catctccctg ggaattagga ttttaacata tgaattttga ggggaccata    12578
aactttcaga ccatggcagt gggttttaaa agggcaagaa agtatcattt catctccaga    12638
atttctgtgt ggctaccatg gaggcatttg gtaggtggag gatcatagag aactgagatt    12698
tgggaacagg aaaggttata caagaaacta tgcagaaaat ggcagtttga atcctaaaag    12758
tatttagagg ttttaaaact gcaaaactcc agctcttgag aatgcagcca gatagaagaa    12818
aaaagcacgt atgtatatgt gtgtgtggct tatcctccct gagtgcctgc tggcctactc    12878
atgtgccatt ctcagtgata agtcctttgc atgtgtcaca caactaatga tggagagtcg    12938
ggattcagac cccaggaatt tgacttaaga tctcatgttc ttaaccactt tcccatactg    12998
gctccttcat ggcaaaaagg ctcatgacaa ggaaaaaaac ctatcacaaa gtctgtgtac    13058
caagatgcaa gcattgtact tctgtgtgta aagagacagt atctgcacac cttacttaga    13118
tccttttccc tcttactctg agacacaata tcaacaaaat tggtaatctt tatatcccat    13178
gtctcagtat gtgaagagta ttaactagga gaatttcaag tagaggccag ctggtaacta    13238
ccagctacat gaaaggctga tggagaggag ggatcagaac ttgaactccc aaaaggtagt    13298
agtctgggat gatgtgttct gggtatgatc tgctatacct cacagttggt cctgggatat    13358
catcttgtca cagacatcac caagcattgg tatttttaag aacgagctag actcagtaat    13418
aaaagattcg aagatatcag ttaagaatgt aggaagcaga atgatttgtt tgagaaacct    13478
ttttcatcca aggttaaaat aatgtaactg aacacagtag taaaatttaa aggatgaagt    13538
tccatttatc tggatacctg tacacaataa atgcttcatg tcctaatcaa cccaatcctg    13598
agtgcctttt ctggatcatt ttcaggagag ggctctattt tttttttttt tttaacctgg    13658
ctccggtacc tacatctttc gtcatcaggt gcctgtcatt aaagggaatt cactttatta    13718
ctcactgctc atgtcttttt aaattttcaa tagtcggagc ttcattagaa atgacaatat    13778
ttgggcaggt agggttttat ggaagagtgg aaaggagacg tgaatgaagg aggaaagaag    13838
atatctcctt caaatagaat gagcccattt ctgtcactga gagggcactg ccatttgaca    13898
gcattgctga acttgtcccc acatctcagg tttcattaga gaaacagata actttacttg    13958
ccaaaaataa tttttttttt agattggctt ttagaaaaga taaaataagg catgattttc    14018

ttgttaaagt tcatattagt ctatttattg tttatctcct ttttataaaa taaattactt 14078
ttaaacaccc ctgctaatat ttcaggatat tattcactta cgtagaggta gttgtgttcc 14138
cattttgctt ctgttccatt tcctggtgtt attcatgtgt gaagagaatc tttttggctg 14198
ggttggcctg cctcaaagct gctccaaact tataattcac caaaagtgac atcatcccag 14258
ccaccaccaa agaattatga ttggagtcga ggcattgccc atggaacaca taactctgca 14318
gtggtttccc ctacaaaatg tcagccactc ttacgatgga atgtgccctt gcctgctgtg 14378
ggcaccgctc aaatctaacc catcattttt cttttcattt tgattcactt ttcctttcag 14438
agattcaaac aaaaagaaaa atgggaactg aaaaaatttg gggtttttga ggggtgaggg 14498
agagacctta tggtagaaaa taaagtgttt tttttgatat ctgaaatttt atatgctcaa 14558
tttaaatgtt ttattgcgtt tgttttttcc aacctgtgga aaccccttaa attcttactg 14618
aatgcataga tcttttattt gcatgaatgt cctgtatgcc ggaagactaa gtgggagcac 14678
tgaatgcttt gaagttctgt gcctgtgaga cttgggtatt ttaatacatt gttaccctat 14738
tgcatcaaat cacgcctagg gatgcattca ataaagactg ccccctcccc gcttagctgc 14798
gctgggactc ccttctggag ggccagagtt tctactttgt ttctactttg actcctaaaa 14858
tgaggactga agggaaagca gatggcattt tgtacctctg acaatgaagc ctaaattgtt 14918
tacgttggag agggagtggg agataagcta aagaatgtga tttgtcttcc atttgaagat 14978
accagggaaa agtcttgtca agtagcaggc caccggtgtc tagtgtagag gagacgattt 15038
ctgtcgatag agagcaaagc cagccaggca aacgaacccg taagccgcct gagggacaga 15098
caggcataaa acctaatgaa tgggtaggta gactttgggc aaggctaatt gcagcaagct 15158
gtgttcagtt ccccggcctc tgttaagtag tcatttttat acaatctgtc ggagggaaga 15218
ggaaatagac ccacacggta aatgaacttg tggtgtttag ctaggaagtg gagtttgctc 15278
taagtagctc tggtagcaga ctccagggag cacatggcag gcggactcat ctgagtatct 15338
gtgctgagga gttgtgttaa actgttgaaa cacttagaaa ttgctataga aaaagagtac 15398
tcccaaagtt gccagctaat atggtaccga atttcctgaa agacctttct tttgccatgt 15458
ggtttttcta taaagctgag tcaattaggc ttctttatct ccctgtcctt ggcaataaaa 15518
cgacaaaagc cgagggttga acaggtagac actgttatct cactgaatct tcaccgctgc 15578
cctatgaggt ggctattgtt actctcccca ttctatagat gaggacactg agccacagat 15638
tggttgagta acttgctcag gctcacacaa ctagcaggtg ataacagctg aggtgaaaga 15698
acattttttc tgggaagata cagggtgttt aaaggtacct gtttagtgca cagcaagttg 15758
cagtactttt aaattggaga ctggaatatg taagtagatg tttgaatctc tctctgtctt 15818
tcagtgttac aggtgaatca acaaaattag aatatctttc tatttagtga ctgaagaagg 15878
cggtccatgc atatatcaga tctagtttgg agtcacagac tagattctgg accagagatg 15938
aaagatatgc tttgggtacc tctttttgat taaatgtagt ttttaaaata gtgacttgac 15998
tatacctcaa ggagtcttca taatcttctg catgggtatt tataggcaat aatgctaaag 16058
aagacagtgc cagtacaagt gaaatgataa catgattgca gtgttacggc ttttgttgat 16118
gaattatttg aactgggagg aaaaacactt tgagaactaa atttaaagta attcttccga 16178
tattatcagt aagacctaaa gatggttgct tttagaggac ctaaatgtat taaattcctg 16238
atttagcatg cttatagcaa ggcttcaaat atgtttaatt ctaccagctt cttgagcttc 16298
ttaaggagag tcatagaatc atagggaatt aaaatgaatg ttagaaatct gtatctcctc 16358
tgacttgact ggatcacacc tagagcaacc caaatgaaaa atgtaccttg ttttaaagct 16418
ctccagggaa aaatattcta aaacttcccc tgataactta ctccaatgca tagtaaccct 16478
gagacttaat atttacaaat tggtttctta tatatggatg aaaggaactc taaatgtatc 16538
gtattataaa aggtatgcat tacataaagg accttttata caggaaaaaa agtatttaga 16598
tgaaaaaact tcttaattat ggaggtacat ctacaatacc taaaattaaa aataaaatga 16658
agtgcatttt cctcctaaga ttttctcatc aaccccgaga accactgctg gaaattacct 16718
gaagaagaaa tcttaccttg ctctgtatct ttaggaaatg ttttacctaa ttttagaagg 16778
aggggaaaag aacgataaca tcacagacac acacacacac acacacacac acacacacga 16838
cacaaacaca ggcacacact gtagtttcct aagcagtttg ttctaattct tcacatcccc 16898
ctgtcaaaat gtttgcttat agctttcttt gtattgtcat ttaagcccat ttctaattgg 16958
tgattgttgg aaattgaata aatgtgttca gtttttcata atgtatgtcg ttgtatggtt 17018
aattattaaa tcgctctgaa gtatgtagac tattattttt gttaccattt aatagctatt 17078
ttttcttaga aaggatttag aaaaaattgg aaaaatgctc tggactttaa catctgctta 17138
ctgagattct tttaaaaaaa tttaactcat ctggtagcat cactaaaatt ataattttaa 17198
ggcaactgaa aattcatcta ggagaagaaa tggaacactt aaaaatttaa gtctgtacct 17258
agtaattaac ttaaactcct gaagttcaat gcggaaaatt attttaaatt aacttaatca 17318
atgcactttg acaagctttc agattacata ttgttttatt ctggtaacat gggataatgc 17378
tcccttgaat cctaatattt gccaattttt attaggtgga atgagcagat tttttttggc 17438
taacaatgaa tgttttaaga agtgttttct aatatgtagt aagaaaaaat ttaagttccc 17498
agacttcact gattaaacct gaaatatgct ttataggcct taaaagaaaa agtgtgtgct 17558
ccttgtagtt cacatcagaa gactgataaa ctcgttgctc tgtttgtaaa gcatgggttc 17618
tgacagcagg gctcttccag attgcgatag aggtctgtcc taaaagggct tcagattggg 17678
atagaggtct gtcctaaaag ggcttgaggt gcaatttgca agatgtgatt ttaggaccat 17738
acccaaattg catctgactg ttgggtgact ccctttcttt tctgaactct caatgcacct 17798
gtaattaaaa aaagaaaaag aaagaaaccc attgcagagc tacattaaaa gctggcagta 17858
gtttccttaa tcaattgtat taagtcaccg tcattttggg atcatttgca cccgtgttcg 17918
ttagtgaatg ccatttcccg atggggatag atgccagatt cttttttctt tctgtgtctt 17978
tttccttctc cgttttcttt ttccttttac cccgttccct gtcttgccct cctctccctc 18038
cctcctcttc cttcttgccc tccttttttt tttttccttt ctcttccctc aggtgtttaa 18098
gacacttaat ggtccccagc actgggcccc ggggggcggc gggcaggccg ggtgtggccc 18158

gcggggcgcc gtcccgcccg agctgcccat gtcgctcttg tcccgcag gc ccg ggg   18214
                                                        Gly Pro Gly
                                                            265
ccg gag ctg agc ggc ccg agc acc ccg cag aag ctg ccg gtg ccc gcg   18262
Pro Glu Leu Ser Gly Pro Ser Thr Pro Gln Lys Leu Pro Val Pro Ala
            270                 275                 280
ccc ggc ggc cgg ccc tcg ccc gcg ccc ccc gca gcc gcg cag ccg ccc   18310
Pro Gly Gly Arg Pro Ser Pro Ala Pro Pro Ala Ala Ala Gln Pro Pro
        285                 290                 295
gcg gcc gca gtg ccc ggg ccc tca gtg ccg cag ccg gcc ccg ggg cag   18358
Ala Ala Ala Val Pro Gly Pro Ser Val Pro Gln Pro Ala Pro Gly Gln
    300                 305                 310
ccc tcg ccc gtc ctc cag ctg cag cag aag cag agc cgc atc agc ccc   18406
Pro Ser Pro Val Leu Gln Leu Gln Gln Lys Gln Ser Arg Ile Ser Pro
315                 320                 325                 330
atc cag aaa ccg caa ggc ctg gac ccc gtg gaa att ctg caa gag cgg   18454
Ile Gln Lys Pro Gln Gly Leu Asp Pro Val Glu Ile Leu Gln Glu Arg
                335                 340                 345
gaa tac ag  gtaacgcacc ccgccagcaa ggggccccct gcggtgtgct           18502
Glu Tyr Arg

agcacctgcc gcccaagccg aggggggtga ggcgcctgcc tccttggttg gccaaactgt  18562
gattttcacc cgtgcggtcg gaaaactttc atccactcct ggggccttcc cggtgctgag  18622
gggaggcacc ggggttttga agatcaaaag ccgacgggga cttcctgtgg acccgagaga  18682
gtgatcatct cacactgcga ctgtggaata cactgtccgt gtttgtgctt gtgcggttcg  18742
gtgatgacgg aggggctggg acgctgagtg tgttgcgctg gcctgatttt catgttaact  18802
gtacgtttct actaaagcat ccttcggtag cttggtataa tctttgtttt aattcacgtg  18862
tgtctatgca gtgatccagc cttatgggta taccctgtgc gagtgtcttt atacaaggtc  18922
tatacttaaa tcattagtcc agaagcttaa tttttacctg attcagccat ttagcagaat  18982
caggggtttt ggtaaactct tcctgtaaag agccagatag tcaatgtttt atgcactctg  19042
gggccatacc gtctctgtca caactcacct ctacccttgc agtgtgaaaa cagctgcaga  19102
caataatggg ctggctgcct ttcggtaaac tttacaaaaa caggctggag gcagaatttg  19162
gcctgcaggc tgtgtttacg agacccctga tgtaaatggt taatgtcaac ccaaaaggga  19222
gtccggcaag tattaagtca agataaaatg aagctaaggt tgtggattct ggagtctgcc  19282
agaaagcata ctgtgtcatc agtaacttct taccgtggcg aagttatgtc tcttttctgt  19342
gactcagttt cctcctaggc taaaagaaga tacctctcat agtgttggga agataaaatg  19402
atactgaaac attcaataaa taatacctat tattatttcc caaagacttt aattttaagg  19462
aaaattagag tttaaaaatt atagagaatg ttggtaaaca gtgtctgggc ttccttttaa  19522
aaggttacct taatctttat tgaaaagcac atcactcatt tcttatcaga ctaaaatact  19582
aagttaccag cctagagagg taatctgaaa gaaatcagaa taacttgcca ttagatgtgt  19642
ttattttttt ggatgaagaa tataacttta ttctcatcac tattaataat catctgttaa  19702
gagattttat attgctattt ccaccatttc atgtattccc agagagctag atactcattg  19762
tcaattgata tttcagtgct gataggcaga ttctgctggg tgaataattg ctgtgtgtta  19822
taaagtcaaa tactagcata ttcctctatt catgtgcaat agactaatgt gcagtacatt  19882
ctgtatgtta ctgttgagtt tttaaaaagc taaaagtatt ggaaaaagtc agtaatagaa  19942
tagagaaata caatgtggat gctctaaaat tctgacttcc taagagctga tatttttttt  20002
cctaccctag gaaaggaaaa agctctgtac aagctcccta gttaaaacat tttttttttc  20062
catttgaatt accagtacct ttgttaaact atgtgaacta cccttagaag agagatgctt  20122
tctatagcta gaaattttta catatgtcta gacttctttc ccttattcag cctggtatgt  20182
gtgtgtgcac aagtgtgttt taacttttta ttttgctaac tcctaaatgt tcttaatcat  20242
tttttctgga attttcattg caggactttc ttgtaataag tttattttag gaactttttt  20302
ggagattacc ttagtggatg gtcttccaaa gtgcacagta ttaagtagta ctatttggcc  20362
tattccaatg tgtaaaaagc ttttgttctg ccctcctatg tcaataaaaa atgctgtcag  20422
tatcttttaa attacatgca ttacaaaatg tggagaatgg caatttttca tagtattatt  20482
attgctttat aaatgcctac tcttttgtaa agccatgtgg cttttgaaac gactgatctg  20542
gacaaccctg aatccagaaa ggtcatgggt cctttggtct gaattttctc atggccacct  20602
ggagaagaag cacagtttgg tggtgacagc attatggggc atgttttgta ttctttggtt  20662
acagagcaat agctctcttc tgcctatttg aaggtaagtt gttcaactgc agagattaaa  20722
tttatccctt aaaagggtta aatgaagtcc cttaggcact tgcaaaagac aacaaatgaa  20782
ccattttttt tcttcttttg accctttgtg acagaatggc ttaccagcct ttagcattga  20842
taactgttag gatccagttc atctttatca tccgatcaaa gattagagag gggtctaaaa  20902
aacctctttt acaatttgat aaacttagtt tcagcaaaaa tcatctgtca gaaattcttg  20962
aacagatgtt aatttctttg ggtcttgtaa tatcatttac cttgtctatg aaatttgaca  21022
ggaagttttc agcatcttaa atttttttaa cgtttaagtc agttggctgc ttttccttgt  21082
tcatcataaa atcagaacgt tgacaaattg accttctttt tcattcatag cataaataaa  21142
tgtctgccta tgagcacttt gctgcagctc tgtacaagct ctctagttca aacatttttc  21202
ccacttaaat tggatccttt ctttttgacc atctttctct tataatggaa ctttattact  21262
tccatagtaa gtgtcagaga gatgtttccc tgtactgctt tttttttttc ttttttttct  21322
ttttggcaca cttttgatgc cttctcattg tgcctaactt cgctttcttc tatttagagt  21382

tagatgtctg atcaaaagta cctcaggaat catcacattg gcatgtaaac taattccaca 21442
catgtttgcc tggaagatgt atttcacact tgaaggggtt tatttgggaa gaacatgtct 21502
gaattatatc agccctcttc ggaataaagt cagactttag tatgaaaaga gcggtatatc 21562
ctctttgtgt caggaagcag gcataggctg cttacctgtg attttttttt ttttttgacc 21622
tgcccttgtc gccattgcaa tcctgaaaat tccttctcaa aaattaccag tagctgcttt 21682
accagcagag ttacaaaaag tcacacacgc tctcttttta aagtgtcact cagctccttg 21742
aaggaacaaa atgtaccagt gtcattttct gttcagataa ctctcctaca ggaatcataa 21802
acttctcctc tggacctgaa caagtatctg ttgcatttaa tgcaactctt tttcttgcca 21862
gaacagtaac ccctcgtgat ttactgttca atctgtatga aaaacctgtg tgacctaatt 21922
ctgcatcaat tttctgtact cgtgaggacg ctaaatgagt cccctgcagt tatgcagtat 21982
tacatcttgt tgttgtttgg ggaagtttag aatcctgaat ctctttatcg gactacctgc 22042
tgaatctcac tgatggggag atgaaattgt ctggagagga acattattta aaatgaagat 22102
ttctattaac aacaacaaca tcaacaaaga tataatcttc tgtgttaaag aagcccctcc 22162
tgtacctttg cttcttcccc accatgtgct gaatcaaatg catataattt tactatggag 22222
tctacctttc ctcttctcct agagtttgcg ctttgtactt cttgcctccc ttccagaaaa 22282
gggattcttt tgtgccaggg aatcagattc actgacaaat aactgggctc cttggagaag 22342
gagtcatttc agggagaggt gcacccccct cgctcccact ctctctctcc agtgcctaag 22402
tcacacagtg ccccatctcc ccggggttcc caccccccat ctcctcattt ccgctccact 22462
tccctgcttc ccacctccca gttcaatgtg atgtaatctc taccctatca ccccagttat 22522
ctgacgatta cttatttagt cctagttcca tggttgtttg gaagattaag taggaattcc 22582
tctaatttgg catctgaccc actttgtcaa agagagtaaa tcagaatacc aagggatagt 22642
ttgagttcta ggatattcaa ataagaaaga tctgcaagga atctagaacc aaggatgcgg 22702
aataaccaaa tcattggcca tgggctcacg ccactataat acttaatttt cctttggtat 22762
ggccttaacc tgtgaaagta aagttgtctc agttttattt tttcccttta aattatatat 22822
ttcaaaaaaa atcaatagtg gatttttttc tttacatctt aattgttaag atgcatcatc 22882
tgttgctgta tccagagatt tggggctaca tagatgttgt agaaaatgag taaatgaagt 22942
agatgtctgt ttcatatagt gaaaattaaa aaccacggta ataatctcac gagtatcact 23002
agtacaagga atgtggaaga aagttgggag aatttgaaca attttgccaa aagagtaaaa 23062
tgatgagttt attattgttg catgaaatac aaaactttca gtcttttgaa tgattaaacc 23122
acttaaaaat gctattgttt gtgttggtat gcatagttaa tagcacgggc caggcatggt 23182
ggctcatgcc tgtaatccca gcacttcggg aggccgatgc ctgcggatca cctgaggtca 23242
ggagtttgag accagcctgg ccaacatggc aaaaccccgt ctctactaaa aatacaaaaa 23302
ttagctgggc gtggtggcac gtgcctatag ccccagctac atgggaggct gagaggcagg 23362
agaatcgctt gaacctggga ggcggaggtt gcagtgagca gagatagtgc cactgcactc 23422
cagcctggcc gacagagcga gactccatct caaacaaaca aagaaaaaac aataacgaca 23482
gcacggtagc agtgataata ttaacacatt atgatgacaa atcatttaat aaattagaac 23542
gaataacgtg cgtatagcaa ataaaacaag aatcagtctt agatactatt tttagtgaag 23602
tccctgaaaa atagttttcc ttaccaccat ttttaaattt ttaaaaatga tgacatttta 23662
tttcccttcc aaactaagca actgtaggtt ttttaaaaaa tcactcattt tgatatttgg 23722
gctatatgaa gtattagatc ttctacagca tttaggtcca ggaggtttca ctttttcacg 23782
ttaatgaaaa tgatggctgt cgtcatcagc atcttggcaa ggaagacaaa tggtctggtt 23842
gccttaggac agaaccagaa gttagaaaga aaaaagtctg tcctcccagc tctgtactct 23902
ggcaacatca tcatgtttgg aaaaaactgt aatattcatt ttttaagaca atttcgactt 23962
ttattttaga ttgaggggat tcatgaatag gtttcttaac atgggtatgt tgcatgatgc 24022
tgaggtttgg ggtgcagttg atcccatcat tcagctaatg agcatagtac ctaaaagttt 24082
ttcatccctt cctgcgctcc ctccctcccc cgtctagtag tccccagtgt gtattgttgc 24142
catctttatg tccatgaata cccagtgttt agctgccact taaaagtgag aacatgcggt 24202
atttggtttt ctgttccggc attaatttgc ttaggataat ggcctccagc tgcatccatg 24262
ttgctgcaaa ggacatgatt tggttctttt ttatggctgt gcataatttt taacagaatt 24322
cttaattttc agcttttata gttgaggtcc aggaaggttc tcggaaagtg tatcttaatt 24382
atctatcatt ttattgcagt ggttttggta tcaggcaaat tgaggttcca atcttggccc 24442
ccttctttac tagctctgtg gtatctccag gtgtcttcag ttttcctcat cagcaaaatg 24502
ggaccattat aacttttacc ttacagaggc gtgtctgtgt gtgtaagaga gagacaatta 24562
aataacattg tacattaaag gcacttagtg ctgttaatat tagcctactt taacttatta 24622
cttattatta ttctttcttt gttgaaatct agttcagtac agtttgcctc agatttaggg 24682
gatgtagttc taaaaatagt cttaaatggc atataattat tttgtcagaa taggattcag 24742
gtgaactcta aaaccagcag tttatatatc taacagtaat agtatcccaa gtgaatggct 24802
aaagtcagag gtcttttatt ttctactctg cctttattat gaagaagtac atgcagttat 24862
atttttgatg gtgtgtaaaa cgagaatcct caacatcctt ttctagaaac tcttggtttt 24922
tgagtactat ttactgctgg ccctgatttt tttaaaacag cctcatttgt attttctttt 24982
ctcaagcaag ctctgctcac agaggaagtg gttgtggctg gcagagcgct ttctgaatgt 25042
ttagggttgg ctgatttctg tggcatcagc acctaaaact gttcttgctc actgcgcttt 25102
aatggctgtc gtcatcagca tcttggcaaa gaagacaaat ggtctggttg ccttaggaca 25162
gaactagaag ttcggaagaa aaaagtctgt gctctcagct ctgcattctg tctggctggg 25222
agttagaaag cttgtcaatt ggttctgtct cttatattct ctctctgcta agagtgagca 25282
aggacatttg acttccccac tagatgcagg gtgaaccttc tttccctagt gttagataga 25342
ggttatggag gaagccacat tgttaagtaa catatacctg tctgcattaa tgcaaaggta 25402
gcttttctct gcttaacatc aaaaattcta gttgggtgtt agagatcaac tatcagtatc 25462
tggaatatgt tttgccccgg acttaaacct aatgtcattg tttatcattt aagattaaat 25522

tgtaatgtgt ttttcccctg cccccttttc cccattttat tgtttccttt ag a ctt 25578
Leu
350
cag gcc cgc ata gct cat agg ata caa gaa ctg gaa aat ctg cct ggc 25626
Gln Ala Arg Ile Ala His Arg Ile Gln Glu Leu Glu Asn Leu Pro Gly
                355                 360                 365
tct ttg cca cca gat tta aga acc aaa gca acc gtg gaa cta aaa gca 25674
Ser Leu Pro Pro Asp Leu Arg Thr Lys Ala Thr Val Glu Leu Lys Ala
            370                 375                 380
ctt cgg tta ctc aat ttc cag cgt cag gtaatacatt ttccccagtg 25721
Leu Arg Leu Leu Asn Phe Gln Arg Gln
        385                 390
aatctgagat gtaggaaata aatgtaattg ttcctaaagt gttatctgtg ttgcctttag 25781
tctattcaat attgttacaa agatataaag atataaatat agtaaaatag tgaatctttt 25841
agaccaacag cttacaacta aaaatgatga agatgaactg cttattattc tggttttctc 25901
ttctgtatat gggaaagagt ttttcctcct gtactatggg ttacaaagtc agagttatgg 25961
cgttttgtta ccatggatga aaaaccttgg gaacaagtgg ggtagctcat gtttgctttt 26021
atgactccaa ggaaaaacca gaaggtagtg gattggagct tctttgtaag cacagatgta 26081
aagtgcttgc cacgactgtg aaaataattt taacaaatat ggtagccacg ctactgaatt 26141
agtggttcat catttataga aatacataaa gcaatgtctt atagcttatt tattcaccga 26201
tgtctaagtt tggagtgttt gatcacatca ggctcatgta ttgactaatc agtaaattat 26261
ctgctcttgt agtttacagc agttattaaa agccagtata atgacctatt ttctgatttc 26321
taaattagta agagacaagt ggaatgagtt agagctaatt ttcactttgg cgagttttgc 26381
cttgtggact gaaaaggttc aagtttgcta gtccgcagat tgcctcacta acccaaaggg 26441
gataaggaac gtttcattgt tagtcagatg ggtttgtttt gttaaagtct ggaacatgcg 26501
aaatgaataa ttaatgagtc aaagtagttt ggaagtattg acttggtttg ttggaggatg 26561
tgactaaaat cacaggccac tccagttgct attgtggtag gaatttgggt gtatatttgc 26621
cttaccggct ataatctcac agatggacat tagtggtggg taggtgctac ttggctcttg 26681
gtttgtgaga gtagatttca gcttaaattt taaaaagaaa agggaaaatt ctctgagttc 26741
tgtttacttc tttcttattg gtgacctcat aagatgaatt aataaagtgc tggataaaat 26801
cttactaaat gaatattaac caagtatatt ttccggaata tgtttatgtt caaattagcg 26861
ttgccatgac ggactcttat tgatttctga tttttgtttg atattatttt aaaaatctgt 26921
tatttatgaa gcttcctaat taaattaact gtaactaatg atatttcaga tgcagtggaa 26981
tgattattta atgtgtattc tccagtccct accccctacc accacataac atgcaataca 27041
gataaaagta ttacatttgt gatctgaaat aagttcatga tataatataa aatgtagatc 27101
tcttggaaag aagatcatta ttggagtggg aagaagccaa aactcatatt tcccatcctt 27161
tgtatgagac tatgaattaa cgttgtttga gtacctgctg ctaccacacg tgaaagtaat 27221
tcttttattc tttttttgga gcatcattaa aattaatctt taaacacaaa gagaatgtta 27281
acatgacact taaaacaaag cggaaaattt aaaaagctta tttagctaat tggtatcatt 27341
agaataatta gagaaaatga agttatttaa aaggcaagac aacatctttt ctttctttta 27401
ctgttacagt actataattg cttttgattt gaattatttg gggagaaaaa tcttgtaccg 27461
ttcttgaaaa tattgcatac atttggcatg attttagttc cttcatttaa tacaaaccaa 27521
aggtgattga gaagcttgtg gagattcccc gccccactct attccattaa atgcaaccgc 27581
gagaaggcca gagttcagga acctagcttc tgttagggaa ggctgtctaa ctgctctctt 27641
cttgacag ctg aga cag gag gtg gtg gcc tgc atg cgc agg gac acg acc 27691
Leu Arg Gln Glu Val Val Ala Cys Met Arg Arg Asp Thr Thr
            395                 400                 405
ctg gag acg gct ctc aac tcc aaa gca tac aaa cgg agc aag cgc cag 27739
Leu Glu Thr Ala Leu Asn Ser Lys Ala Tyr Lys Arg Ser Lys Arg Gln
                410                 415                 420
act ctg aga gaa gct cgc atg acc gag aag ctg gag aag cag cag aag 27787
Thr Leu Arg Glu Ala Arg Met Thr Glu Lys Leu Glu Lys Gln Gln Lys
            425                 430                 435
att gag cag gag agg aaa cgc cgt cag aaa cac cag gttcttagac 27833
Ile Glu Gln Glu Arg Lys Arg Arg Gln Lys His Gln
        440                 445
cctgggcttt gctcaccctc actttggcag agctgtccaa tgaattcatc aaatggggtc 27893
agaatgactg aaaaatggac ccttgtgggt ggtggggaca tcacagaaca gaacggttcc 27953
ttgacatgta cataatccaa ccacatcatt ttatagacaa agacgcttaa gccaggacgt 28013
ataaatgact gcccaaggtc atacaattgc ttagcagcac aatgaggttt agtattgtct 28073
tagtttgtct gggctgctat aaaaaaatag cacaaactgg gtggttgatg agcaacagaa 28133
gtttattttt caccgttctg gaggctggaa gcctgagatc agggtactag catggctggg 28193
ctctggtgag ggtcttcttc tgggttgcaa actgttaact tctccttgtt ttcttacgtg 28253
gtggaagcag gccaagaaag ctctccgggc ttcctttata agggcactaa tcccattcgt 28313
aagggctcta ccctcatgac cttatcacct cccgaagacc ccacctccta ataccattac 28373
attgggagtt agcatttcaa tatataaatt ttgggaggac acaaacattc agtccataac 28433
aggtgtcatg ttcataatcc catgggaagt ttttgaatgg gtgggcatag gtggatttcc 28493
tagagcaaaa ttctgggagc aggaacttgg aaaaggaaag tagaaagcaa aaagttattt 28553
tcataacaaa tataatagtg tcctggcttc tctgaagggc ctagttgaat gtttcacaga 28613

ggcaaatatt aaaaacattc ctttctgggg gtttggaaat attttgatgt aggatttgat 28673
ttcctgtcat agtcctgtaa attgtatttg ttttcctgtg cttaatttag tgcttagctt 28733
agtttccttt tgaaaaaatg ttggatttta gtgtaaatag agtatatatt taaaaagtgg 28793
atacaaattc tgcatggcaa ttttaacttt gtataaactt attttgtgtc atactctagg 28853
gtagttagag cagaaaaaca ttacttttgt gacatacttt ttgctggaaa tactcatgtt 28913
cctcaggttt attttggagt gaatgagtgg tttgctttac attttatcat tgccattttg 28973
taggtctctt tcagtcttcg atctttccat tctcatgtgg ccacaccctc aaggctaagg 29033
aggcttctct tgctaaagct tacaacaaaa ggaacccgaa gaggctatct ttcccaccag 29093
ccccatggcc cacaccttaa ctgcagagac accagggcac ctatccccaa acagattcta 29153
gtcttcctat gctgttaaac acacactgag agttttcttg gacaacactg atagctcaga 29213
ggacactggt cattggattt taagtatcct tttcttccct ttttggatct tctag gaa 29271
Glu
450
tac ctg aac agt att ttg caa cat gca aaa gat ttt aag gaa tat cat 29319
Tyr Leu Asn Ser Ile Leu Gln His Ala Lys Asp Phe Lys Glu Tyr His
                455                 460                 465
cgg tct gtg gcc gga aag atc cag aag ctc tcc aaa gca gtg gca act 29367
Arg Ser Val Ala Gly Lys Ile Gln Lys Leu Ser Lys Ala Val Ala Thr
            470                 475                 480
tgg cat gcc aac act gaa aga gag cag aag aag gag aca gag cgg att 29415
Trp His Ala Asn Thr Glu Arg Glu Gln Lys Lys Glu Thr Glu Arg Ile
        485                 490                 495
gaa aag gag aga atg cgg cga ctg atg gtaaggaact ccctgcagga 29462
Glu Lys Glu Arg Met Arg Arg Leu Met
    500                 505
gcccaggaaa ctactcaacc cacgtccgtc tgcaatgaga ccattaaata tggtggtagt 29522
agaaggaaaa aatgaaaact gcttatcaaa aattttagta aatttctttg aaccttaggg 29582
agatgaagta aaaacttttt ctctttcctg gagaatgcat gccaaaaatc tagggggatg 29642
actgatgcgt atagtatttc tgtgtcctgg ggtggcattg cgcggtacac attacagctt 29702
ctctcataaa agcaggtact gtattttgca tgagtttgat aaccagatct ggaaggatgg 29762
caaatgcagt tcttttaaaa atatggcctc tttgagatga atgaccacgt tttttggaat 29822
gaataacaca ttaagtcgtg tcttcaaatc taagaaggtc tgtgttttga agcctctggt 29882
gcttatcttt cttttttaat ggcagaaatt attctctaac agtgtaataa gtgattttta 29942
agttgatttt gaaatgttgc cttttttgtg atagccttgt tatggtcaaa ggctattcat 30002
ggattattac atttgcactg ttatttttta atgggcatat aagcacagaa gagataggtt 30062
tatatcattg gcctgagtat ttgtttttat tgaccattta tttttggaat tggacctcag 30122
agcacactgt ggattttaga aaagcgtgtg tgtgtgtgat gttataatta taggagacct 30182
gcagattcta tctaggacgt aatgcctgca tagatgaaga taaggtcatt agtctcagac 30242
cataattatg ttcctggaaa attctaagtg acactgtctt cctggctggt attacagttt 30302
gcattttttt cattttttgg attccagaga gtaaacacca tgttttaaaa gcctctgtgt 30362
gcaggtacta ctacatctta ctaccctatc ctgcctccct ctccatacca gggagagagg 30422
actgtccgag aggtttctga ctagaccgtt tttgtagtaa tttgatcact aagcagtcaa 30482
atttgtctga ctcaactgaa gtgaaactat attagaactt cagtaacaga ttggtttgac 30542
actaatgatt aggcatttgc aaatcattaa tttttgcaat taagtgatgg aatatacaga 30602
taatagagaa actgaagttt tttatcactc tttcttcaag tgagttcaac cataaccgaa 30662
agcaattaga gttataatag catttgaggc tcttgctcat ccacagggca gacggcagct 30722
agactgagtg atgaagctgc tgtcactcag gcagatgcta gagtgacacc cctggacaaa 30782
agatcccaag tttttgtttt tgtttttgca atggagttgc ctcttaaaga ttttgcagct 30842
tgctcctttt atcgtggcat tacatgataa aaataatttg gagtgtcctt ttattgggat 30902
acacaaagag atttctctca aaatggctta gagtacatca gagttttatt ctgtttaacc 30962
tcatcatgtt gcttaatggg aggtggaggt gggaattact gtatctattt tattgctcag 31022
cagtaccaga cttcaggtgc taagtgactt gtctccagtc acctgggccc attactcagt 31082
gcagatctgt ggccaaaaaa aaaaaaaaaa aaaaaaaaaa aaaaaatccc ccaaatttga 31142
tctttcctgt taaccacaaa gtttgtaatg ccaaaaagaa agacttttta aaatataaga 31202
cattatttgt taaaagccca tttgattcct tgcctttagt agtacttttt gaaatattta 31262
tgaatgaaag gatattatgt ctggaatttg cttcaagaca attcagtttg ggtttagaag 31322
aagagtagca atgaaacagg agtcatcaca agtgaatttt tgttgtttgg ggttggttat 31382
aggagaaatt aaactattct ctctgctttt gattatattt gaaaatgtct aaaatataga 31442
gttaaagata aagtttagat catgaattgt caaattctaa aatctttttg tacagttaaa 31502
gcaacacaga aaacaaagag cttggctcag agtgcttaat cacaccacac gtacagtgtg 31562
gctttgtgcc cggactttgc tgtaaactcc agtgtcctgg gatgtaggcg tcacacttct 31622
gtgtgtttgt cccacttcag actctgtgca atggcttgaa cagcccaact catccagttt 31682
gctacactcc taccagtcaa aatgcagact gtcaagggga ggacagagtg gagagtggag 31742
ttgtctgcac gcttatatta tgctctcatc ctgctcttct ttccttaata g gct gaa 31799
Ala Glu
gat gag gag ggt tat aga aaa ctg att gat caa aag aaa gac agg cgt 31847
Asp Glu Glu Gly Tyr Arg Lys Leu Ile Asp Gln Lys Lys Asp Arg Arg
510                 515                 520                 525

tta gct tac ctt ttg cag cag acc gat gag tat gta gcc aat ctg acc 31895
Leu Ala Tyr Leu Leu Gln Gln Thr Asp Glu Tyr Val Ala Asn Leu Thr
                530                 535                 540
aat ctg gtt tgg gag cac aag caa gcc cag gca gcc aaa gag aag aag 31943
Asn Leu Val Trp Glu His Lys Gln Ala Gln Ala Ala Lys Glu Lys Lys
            545                 550                 555
aag agg agg agg agg aag aag gtgcgtatcc tagtggtggt ggctgagtcc 31994
Lys Arg Arg Arg Arg Lys Lys
        560
agggtgtatg ggcagggata agtttttaag gcgagtattg ctctttaagt actacttctt 32054
acactggtgg aaggtttaga tgattgaatt gtgaaaattg cataaatggg tatggcatgg 32114
aagtgttgat taggtacact agacaactat tggaggtaag ggcataataa tgggatatac 32174
taaactaaaa caatgttgtt cataaatcat tgtttaatag aagattttaa attaagtaca 32234
tacagtcaat tgggaaagga aaaacagaca caaataagtg ttcgcttaag tttaatgcta 32294
gggtggcagg ggattactca attaagaatg aaatttaaga agaaaatgca gtgtacaggt 32354
tggaagaagc cagaggaaga tataagcaat agttacttgg acagatggca gtgccaaagc 32414
tgttcattca gttctgctgt ccctgtgtcg cagtgaggtg agtgtgcaca ttggggtttt 32474
aggtaattac ataaaaaaag agtcctatgt gaaatatctc ctaattatca tgagaaatga 32534
agggtaagat ttagaatctc tgcagctatt atatctcaat tcaagagtaa cctgctgagg 32594
ataggcctgt gaatatgatc ctctttgaag cttaagtcat ccagtcgtct agttagtggt 32654
tgatactgtg tgttcatgat aaattctgtt taggccaggt acaaacatca actggaagtc 32714
agaattgtaa aggttgagga ttggtagcat ttaatcattc attctgtata ctgaataagt 32774
gaacaaaatg cttttcaaaa aacaattaat ttcatggctc ctgaatcctc atacctgtct 32834
cataattaag gaggaggaat aaatgattaa gaaggcacct tcttgcactg atttctttag 32894
tataagatag cttacctcat tgtgaagatg aacgagagat gactcagggc ttgtgtttag 32954
acattttgtt ggtgaggaat acctcatttt cttgtcagtg tagcattata gactgtgcaa 33014
aactccagct ccaattccta ccttaaaaag atgttttcag agttagtgag gaacatatac 33074
gtaagggggg agggaagaat gatggggaaa taaatcatat aaatgtagtt attaccactg 33134
aactgtagac ttaagaatgg taaaggtggt acattttata tgtctaatga cttccagaaa 33194
aaatttttaa aaattaaaaa ggaattactg acatattgat agcagagaga gagatggaat 33254
tctggaatat tctgtaagtg gaccccatta aatggcagtt ttagtttttc ctaattcagc 33314
catgtgttct gcagaggttg tcactgttag tgtttttagt attaaaattt ttgtatttta 33374
catttctttg tgattaaaag ctaatgaccc agttttgaga ttatggctta caattaatag 33434
atttctgccg tggtgttaga tagttgcatc gtcgtaggca gagaaagata cacaattgag 33494
tgttgatgat tatgcctata tttttgtgtt catctgtaca gaacagggac ttgtcacaat 33554
tgtttttcat ggtgttattc tgcatgatct gtaagttggc acgccccttt caaacccttg 33614
ccttcaattt ggtaatagca cttattcttt gtgtcttatg cttgcactca acatgtgtta 33674
actgaacact tactatgagc taggtcgtgt gctagaatgc tgggagaagg aggggtacaa 33734
tggtgaggaa aagcatcctg ccttctctca cagtttaatc agagagagaa aaagagaggc 33794
tagactgata ttaatcaggg agagaaagag aggctagaca gatattaatc caacatgcac 33854
ccaactatac aatgacaagc tgggatgaat gttgtggagg gaaactgttt ccagttggag 33914
gtggggcctg atttcttggc aaacgcgtaa tagttgtcaa agcagctact gtctacctta 33974
cagatatctt tgagtatctc ccattgatta tctcacatga tcaatgtgca gaatttttac 34034
tttatggtca gccatctacc cttccttgca tgctcttggg tgcctagcat ttgtgccctc 34094
agcaggacta agtcggaggg gctctatttc cttccagggt atcttactgg gagaagtggg 34154
acaaagaagg gggaagaaaa aaagaaaaga aactgaggcc cttagccctt cctggtacct 34214
gtctcttttg cggggcttta agacttagtt cagagcgaat tatccagttc cagatgcact 34274
tttattcaag tgcaaaggaa ggggatgctt taagaagctg atttgacact ttggattatc 34334
tgtagttctt cttccctcca ttgcagtgaa tgattgggag ttgcttcttc tagcttctct 34394
gtagtggagg aaaacttctg tgtctatgag cataaaaatc tctgctatcc tttttagcac 34454
ttttccatag tattaagtat tatgctgtac ctatcaatat tttttaaatt aacaaatact 34514
tcaataaacc tttgttttga gaaaacctac tttactcagt gagctagtag ttaatagcac 34574
tgagctggta gcaacacttg aaggacaact aagctactga ttactctaag aaatgtgcta 34634
ttaaagatga atatggttga ctttttcact taatttaatc tgaaaaagat taccttttat 34694
tttcagcgtt aaactagtgt acaatttttt ttttttaatt ccctagaagg acagaggcct 34754
ctgtctttct ttgggagttt attgtgttga cttgttgctc gtgaataata gggtaggttt 34814
cctttaacat gttatgacct tgtttaattt tctcatcatt acagacattt ttaggaagta 34874
cacatttata aatcattttg taacttctca ttttcagcct ggtcttccat taaagccctt 34934
gaaatggaag aagaagaagt gaactacatg tatctcctta agtgtcagag gaggaataat 34994
ttttaaagta ttgaaaacct agttatacga aactaatttc tttgatgtta caagatgaat 35054
gtgctattat gtgcgtttag cgtgaccttg aggtttccta cagggtacaa aagagaatgg 35114
ggctagatca agccaccagg gcctgtagaa ctaggattga acatggctga attagaaaat 35174
tggaccactg tctgagtcgt gttagcaggt aatcttttaa aatcaaaact acatgagaaa 35234
atatttatac taatttgcta ttattctctt gtaggctagt tcgttaaaag atctattacc 35294
atagtgcttc agtctcatga tctgtaagaa ggcaatagga atgttgtcta ccccatagat 35354
ttgtagtgag gattacacgg gaaagtttat ctaaagcatg tagaatacac cctaacatat 35414
agtaagtgct cagtaaacat tatttcatca agcaattcta tgatacgaaa gtggaatcat 35474
aattataata cggttatgtt tttctgaaaa caaatgtctt tttaaacctt tactgcatat 35534
tctttgtgat gttgaatcaa taataattgg tcaccacttt ttttctagtt ggaaatcagt 35594

tgaatattag tgtgtaggat atctttgagg acagtgacag attctttaaa tactgaggca 35654
tgcagaagaa aaatgaggct ttatcacagg tgataaactc tgcctaccac cctgttcccc 35714
caactcctac aaagagaatt ttctaattac atggcctcta ctttactagg gttcctggcg 35774
attatgaggc atctctcaca gtactttagg gtacattttt ccttaagctg tggttaggag 35834
cccataccag gagcagccct gcctattgct taaaagttta gccatcagct gggtgcggcg 35894
gctcacgcct gtaatcccag cactttggga ggccgaggcg ggcggatcac gaagtcagga 35954
gatcgagacc atcctggcta acatgttgaa accccatctc tactaaaaaa tacaaaaaat 36014
tagccgggcg tggtggcagg cacctgtagt cccagctact caggaggctg aggtgagaga 36074
atggtgtgaa cccaggaggc agaggttgca gtgagccgag acggcgccac tgcactccag 36134
cctgggcgac agagcgagac tctgtctcaa aaaaaaaagt ctagccattt cagaggttca 36194
gtttccagag ccgttgacat cagccaaaac taaatattta attctccctt ccacatcttt 36254
cagtgggatg tataccagca gaactattct tgtgttattt atttgtgaca ttttttggaa 36314
acatatgata ctaatgggca catactcaga atctctgagg caacactaga ccagatatag 36374
tcttcattgg agtgttcgat tttctttctg aaagttcttt tatcctgtct ctctctctct 36434
ctctttctcc ttaccaaccg ctattataag taaatctcag gactgtgata ttttgcaggt 36494
tgaaccttcc tggcataatg tattggccaa catagtggta ggaaatgtgt ttttatcctg 36554
tttagatacc agaatgtcag aatagcctgc cgttggcaca gttttcagta aaaagagctt 36614
gaaaagcaaa gcagtaatat tgagacattt attttaaata caagctactc cctgattctt 36674
ctcatttttt ttaatctctt acagtttccc caaagattat atactttatt acagctttcc 36734
tctacaggat tttcatagat gaaagaaagg ttctaatgta attctggaca tttgagtgta 36794
ggaactattg taaggatagg cgaacacaat gccacagtga ttagatttat ttgattagtt 36854
gagtgtcaaa atgggggaga ccagaagttt cgagaaaagt cctgtttaac tggattgacc 36914
agttacttga gtcaaaatgt ttgaattgat tttctctata agaaacaacc caagtaattt 36974
gcaaatgcct gctaatttca tatccctggg cattttttaa aattacccga ttacctatct 37034
taatgctcaa aggtcccctt aaatgacagt aaaaataact caagtggttt ttgaaagctt 37094
aggtttccag caaaacagaa tggatacttc agataccagc acatttgaat gttgtttcag 37154
tatattataa taacacccac tgggggataa agtacattca gtttataata tagattgtaa 37214
aatatttgat ttaaaacttt atgtaatgag gctgtttata gcctatgtta aagtaatcta 37274
aataaaatgg cctactgaag tcttcttttg agttcagctt acacgggatt ttcatattta 37334
gagatttctt tccctcctca gccctgttgt tgattacttt ttataccttc tgcaaggaag 37394
atgggtagcc atgcacatac ctttgcttgt gaacattgct aaatggaaga tatgaattga 37454
cgttggcctt ggaaattggg aacacacact gtctttagtt tgagtaattt ttagttatga 37514
gtaatcttta gttatgaata attcctatca tgtcccctta catgattagg ttaattatac 37574
tcctcccttc ctacataagc ataattagtt ctaaactctt ttgcatgtag gaagaagcca 37634
gtacaaagtt tcttgttcag tgttttgttt tgttttcaat caatggaaat aagtgcaggc 37694
atcagccctt aatattgata gcatgtgtct gcaattaaac gatgcagata actacatatg 37754
aaaatttctc aggtggcagg agacgctgta gcagctgctg cattcacacc aggacatttc 37814
tttaggtgct tagagactgt ccgaagtcag ctcaaacagt agaccaatca caaactcttg 37874
ggtgttaatt ttgaaccaag aaaaagggac agttgtgttt gttttgcttt gtttttttaa 37934
gttttgagac acagcatgaa gttgatgtga gtacatgtgc gcaccaacca gcaatgtcat 37994
caaactgaag gaatccaaag aatgatggga ggagttgata tctcttgtca tagagtagac 38054
tgccacctgg cctctatctc ttagaataac atggcagaat taggtccaag gacagggcat 38114
gcttgcgagg aaagtcacac agagcaccaa ttcaatggct aattgtgtgc gtgcttttaa 38174
ctattttatg acttgcaaat gtattggcat ctatttatga tagtaataat ttaatgagca 38234
tatgctaagt ggaaagcatt gttctttgtg ctttcattca ttccttcaat caccaaatat 38294
ttatttagcg ccttttaagt gcctgatgcc cttgtaggtg tgggaatgca ccagtgaaca 38354
aaacagacag aaatccctga cctcatagcc ttatgaagca gttttcagga aatgcgacag 38414
ccagtcttaa ttatagacat aaaagaatag tcctcggact atcgttgacc cttgtacaat 38474
gtggagattg aggctggcag catatatatt acttgtgaca ctggattgta catgtcgaaa 38534
tagtgaccca gtcctgcaga agccctagga agcaggcact gtttgttacc gttcctattt 38594
tatgcaagag ggaactaaga cacaagagaa aatgatttgc tcaagatcat acagctaaga 38654
tctcttgcac ttaattacag agagctaaga ttgaaacaca gtttgtctaa ttgcagaacc 38714
aaatgtctta aatctaatat tactctgtct ctcagatttg atacctaata gaactttaaa 38774
aaatagtttt ggggatttta agtaatggaa gggcctgact gtgaggcagt ttttagataa 38834
caatctagct agtctatagg acattcatag gaaagtatcc ttcagaatag ttttgattat 38894
attaatatat gatgcttcaa gctttttaat gtactattca ttgaagtggt gatccaattc 38954
cacttgctag atatttgaaa ttggcttatt cattgtctca tttgattttc tttgttggcc 39014
aaatttgtag cttatttctg atttgtatgg aaagaatggt gggattaatt gactttcccc 39074
cattaattaa aacagtcccc tgagtataga ttctgagaac tccaaaaatg gaaccttcag 39134
tgactggtag cagggatggt ttcttttttg aattcaaggg ttctgttatt gttctctgtg 39194
tcactagctt agatgtagag cctgatgctc tttgaagctg atcatttctt ttgagcttat 39254
aaatgttttt gctctgaggt gacataaata gaaatttaat tacttggaaa atcataacca 39314
aagggagcat tttatttcct actgtttggt ggaagctgca ttttttttta tgccttgtgc 39374
tttggcatac atacatacat gtatttttgg taaaaattgc attgtaaagg aacttcagaa 39434
atttcaaatg ttgatggaac tgcctttctt cactatgact aaaaggctga ttttaaactt 39494
taagtgtttt cgtaaaagct attttgtcaa aagacagttg agttattttc atagaaggca 39554
aaacgtatta tataatcaat atcttaagta aacaaagttg agttcatatt gattctgtat 39614
cgtcattttt tatttttaat taactaaaga gcccaaggga ggaaagctca ttttctcaag 39674
catcttggca aaggaaatga ctaaagtttt ttttttttct ctcattatac tatgcagaaa 39734

tagtatagct ttccagcagt aactttaaaa tagtgattct gtagaggtga ttttaaagat 39794
ttcacttgtc agagaggtta tcgtaagtgg aagaaatttt agattatttt gcgtatatta 39854
cagatgagac tttcttggat atgtacaaga tatgacataa ccttttgttt tttttttgga 39914
gacacagtct ctctctgtca cccaggctgg agtgcactgg caccatctcg gctcactgca 39974
agctctgcct cctgggttca cgccattctc ctgcctcagc ctcctgagta gctgggacta 40034
caggtaccca ccacaacacc cggctaattt tttgtgtttt tagtagagac agggtttcac 40094
cgtgttagcc aggatggtct cgatctcctg accttgtgat ctgcccgcct cggcctctca 40154
aagtgctggg attacaggcc tgagccaccg cacccggccg atatgacata atcttaactt 40214
ttcatttagg ttgtttttga ggcacataga agttaatgga ctaagatatt gggatatgta 40274
agaaatggta gataggctgg gaggccgagg caggcggatc acgaggtcag gagattgaga 40334
ccatcgtggc taacacggtg aaaccccgtc tccactaaaa aaatacaaaa aaattagccg 40394
ggcgtggtgg cggtcgcctg tagtcccagc tacttgggag gctgaggcag gagaatggcg 40454
tgaacacggg aggcggagct tgcagtgagc cgagatcggg ccaccgcact ccagcctggg 40514
cgacagagca agactctgtc tcaaaaaaaa aaaaaagaaa tggtagataa cagaattctt 40574
catcagataa caatatctgc ataaaatttt cccatttaca catggtagaa atactatatg 40634
ttatgaaaga aatttaggtg gaattaaaaa catccaggga actcataaag ttttatatta 40694
tgtttttatg cccatggagt gggtcatagc ttattctttt ttttgagggt gcaatttgat 40754
ttttattatt tcaactttta ttttagattc aggggataca tgtgcaagtt tattacctgg 40814
gtatattgca tgatgctgag gtttgtgtta cagttgatcc tgtcacccag gtggagatca 40874
tcgtacccaa taattagttt ttcagctctt gccccactcc ctccttcccc cccagtagtc 40934
cccagtgtct attgttgcca tcagagctta ttcttgatgg acagttttag aatgttgttc 40994
ttcattgctg aagaaaactt gcttagggga taggaaagtt gagctaatta tgtcaggata 41054
tatcttccca ctcccaaaca cgagaagaac atttcagatt ctgaccactg tcttgatact 41114
gccctgtctt aagctctttg ggtccctgaa gacacaatct acaaatctct agtgtgctgg 41174
cctcagatca catctgactt taagtgactc tctacaagtt gctaataaaa tacatgtttt 41234
gtctcagcaa agacaaatga acataaaagg cattaacact taagctgtgt tagataataa 41294
tggggtaaca atgaaatcct attacatacc ggagagttac caggaagtcc tgtaccccat 41354
catcttggtt acttataaca ttcgacattg ttgttttgca g aag gct gag gag aat 41410
Lys Ala Glu Glu Asn
565
gca gag ggt ggg gag tct gcc ctg gga ccg gat gga gag gtaagggatc 41459
Ala Glu Gly Gly Glu Ser Ala Leu Gly Pro Asp Gly Glu
570                 575                 580
gtcatccttt ccactgtgtt ctgctgaatg gagtctgtgc catacctggc agcaccattc 41519
ttcatgcata ctattttatt cttactgtgc tatttatttt aagtaaaaat agtaatacat 41579
tagcatagta gaaaattaga atgatacaaa agggatacag tgaaaaataa tctacttaca 41639
tgtcagactg tcaatcaact gatattaaat gtcttgcata gtatttcaga attttctgtg 41699
cacatataat cgtatgttta caaatatata ggatgtgtaa gaatcttaaa tgattctata 41759
attatattgc ccaataaatg atacagaagc ataaaatatg acacagtttc tctagtttat 41819
tattaatttg aaattataaa aatacataaa ctaaagaagt cacatatttg gttaacttat 41879
ttttgaaatt tttaatgggc ttgccatata gcttctatgt gtatgttcat cagcctagtc 41939
ctaaaattca tttcctagta gttccagtat tctcctgaat tatgaggata caattaattt 41999
attggatttt tttctgaaga atttaaggtt ccctgccagc gattcacagt gaggaggcac 42059
actcccagag caggatggga agggagtttg tttcaaatta tcttcatttt gtctcataaa 42119
ttctgataaa cctacttgga ggatcagagt gggtgggagg ttgccacttc ccattctggt 42179
atcactctgc acacagttgt attgtgaact caaaacagtg gagatgcaga aaaaaaagat 42239
tgaaccacta ttctatgcag aaacctcttg aacaataaat gttacttaac cactataatc 42299
tatcacaagg aaagaaagga tcaggataaa tatctgatgt gtgagaaact ccgtgggctg 42359
ttactggtta agaatactat atattagagc acttagaaac aactctaatt tgcaaaagcc 42419
cagccttacc taagaatgcc tcccattggt ctataattaa caagcacaca gggagagatt 42479
taacatgtta gcgtaatagt gttcttatat cttattatct gttgggaaaa ttagctactg 42539
atcttggact tgtgacatac atcttttttt tcttttaatc ttaacacacg tgtcctgttt 42599
gctaaggatt gcaacgtgga catcttgctc tattttgaaa taatgttctt ctgttctcat 42659
tcagcgcttt tgccaaggtt ctaattccat ctacagtgat ttccagtttt cttagaggca 42719
tgacattagc acgaaaacaa gctgtattat aatggttcat gataacagaa tatgattttt 42779
ccaagggaaa tgatagcaaa ttgtagattt ccttctttat ctcttgagta tatagatgac 42839
cttttacatt aatgaggaag aagtgtcatg ttggcatgca aagtaatgat gttcaaaacc 42899
attgctttag cacacacaaa agaaaaaaac gacaatctgg agtaagcctt ttctcttttt 42959
aaggttattt ttgtggataa ttgctaatat attgtttctt gtgctgacag tgctttgttg 43019
agggtgtaag gggaaagaac cattgggtat gtgtttttca ttttgttttg ttttttggtc 43079
atctcctgta agatatagaa gataaacgat agcatactgt aggttatcct atcatttctg 43139
cttaattcca attgaaagga atctagcaaa aattttggaa ttcatgaatt gtctaaaaat 43199
tcaattttat cacttttcag gaaatagata acttgaacca ctcacagacc aagcatttcc 43259
aaacaaacat tttacttagg gatctgttct tagaagtaca gatcaccctg caatcaggag 43319
cagtcattcg ttactgatta tattcaagtg tgattcttgt gttccttgga tatagtagtc 43379
tagtaatgaa ctttagctta atttttggtc aatttgccag atttcagagg agcagagctt 43439
ttttctttgt cacactcaaa gataccaata gttgggttag acccctgggg ggttagtttt 43499
ggaaaaagga caccggttct gcagagacca aaccagccac tcatgagatg acataagtta 43559
taataccttt tctgtttttc attctctctg aaaccatgac tttgcaatag ttgttatctg 43619

ggaagtatta gttgaggaat ggggattgtt agttacaaat gaccaaaatc aagtctgcct 43679
gatttaaata aaaagggaat gtattgatag gatactgggc aactcatcag atctccaggg 43739
tttctgaaga acctgactca gggctatccc caatattatg ctctacaact ggtctagtga 43799
ggacaggctg tggctgggag caggcagaag tttgacagag ccattaccgc tttcctggca 43859
ttgttgccac tgtggtctgc cctgcaccgg tgcacctgtt ggtttccaca cccttgcact 43919
tctagctctt aactcacaac cttggtgatg tatctggttg ctggagctga ggtttgtgcc 43979
tgtatcttag ctaccaggga ggcagcattt tcagtttctt tagtgggagg tagcctctca 44039
cctcccacca acactcatta ggtagtgaaa tttccaaaca cagaataagg tttcacatgc 44099
tgggcagcaa aaactgctga gaaacgtcca gtacaggact gggggaaggt cttaacatga 44159
aaccgtgaca tgattttccc tcctttgtag ccc ata gat gag agc agc cag atg 44213
Pro Ile Asp Glu Ser Ser Gln Met
        585                 590
agt gac ctc cct gtc aaa gtg act cac aca gaa acc ggc aag gtt ctg 44261
Ser Asp Leu Pro Val Lys Val Thr His Thr Glu Thr Gly Lys Val Leu
                595                 600                 605
ttc gga cca gaa gca ccc aaa gca agt cag ctg gac gcc tgg ctg gaa 44309
Phe Gly Pro Glu Ala Pro Lys Ala Ser Gln Leu Asp Ala Trp Leu Glu
            610                 615                 620
atg aat cct gg gtaaggcatg aaagcagcgt tcatggtgtt cttttagctt 44360
Met Asn Pro Gly
        625
tttagatttt ggtaatcttg tacgatctcg aaactaaaac tacacagcct ttgctgagat 44420
ggttgaagtt gttacttgga tttgtctttt atacatagaa tgtgctatta agtcatgtgt 44480
gtctttattg tattgtaatt taaaaaacta agccccctcc tcttcctcac tctttttcct 44540
tag t tat gaa gtt gcc cct aga tct gac agt gaa gag agt gat tct gat 44589
Tyr Glu Val Ala Pro Arg Ser Asp Ser Glu Glu Ser Asp Ser Asp
            630                 635                 640
tat gag gaa gag gtatgtatgt atctctgttt ggggtttatt ggtttgaaag 44641
Tyr Glu Glu Glu
            645
ttatcagagg aagaaattct tcctgaatgt aagcccgggc cttgggtcct tctatcattt 44701
cttcctccag gattggcctt tgggtgacag ctggggctaa ggatttacaa atgctttgat 44761
ggtgtatgcc tagtggtaca gtgatctctt ggaaggccct ggctaatggg gaaacctggc 44821
acattctaaa ttcacttgaa accaagagaa gagaagctgg cattaacttg ggagatggtg 44881
aagtggtctt ttgggggaga attggagatg gattaccttg ggaaaggcca taccatacct 44941
ctaagtggtt cacattgaga attaactgcc aaaaagttct gaagtccttc agaccttcga 45001
gtctatttgc aattctgagt ctggaaactt tgacaaaaag ggcaacgtgg gcttggttta 45061
atgagtttat tgatcctctt gatttctctc caaatgtgat tcttgtttga attccttaga 45121
tatacttgtt atccaaagag aagcgctcag tctcttggaa ggaatactta gtataactag 45181
aacctaaaat gggaatcttg ggtattttta atagaaattg agaaataata aaatatttgt 45241
taaatcagcc acaaaatgtt taatttaata attacaaata gaaggattaa aagaagattt 45301
gctctccttt attagagcat tgttaatata ttgtcattag atctgatatt tttgagagtg 45361
ccgtagtata ggtgccgagg acaaggtaaa acatccttac ccataatagc ttaccagggt 45421
ctcaagacta tggctgtttt ccttctattt gtggacctgt gttttccaaa ttagtcttcc 45481
atgagcactt aatgcttttg taaagagaaa gaataataaa agttacatta caaaaaagca 45541
taatcattaa aaagcaaaca tggttgggca tggtgactca tgcctataat cccagcattt 45601
tgggaagctg acgcaggaga attgcttgag tccaggagtt cgagaccagc ctgggcaaca 45661
tggtgaaacc ccatttctac aaaaaacaca aaaatcagcg gggcgtggtg acgcatgccc 45721
gcagttccag ctactcggga ggctgaggta ggaggatggc ttgagccctg ggcagagact 45781
gcagtgagct gaggtcgtgc cactgcactc cagcctgggt gacagagcaa gatcctgttt 45841
tgttgtgttt tgttttttaa aagcaaatac tttttggggg atgctatttg tttcccccag 45901
ctactctatc tctaagatat ttaaaggtgt cttgtttact tggatgaatg ggcttataag 45961
tgacctactg gaagaagctt ctctttgaat aagagagcca gagagggcag agataagatg 46021
aagtaggccg cctagccagc aggtgccatc ctgagaatga caagcacaga cagcccttgt 46081
ccctgggccc atcacatggg tctgctgcag gtgagcctga gtgacatgtg gagagtcatg 46141
tcccctctca gcgttttatt tcctggcctg gaaaatgaag gagttgtgcc aaggccttag 46201
ctgactccct gcttctaaat gatctgctat cataaggttt gatttttctc cttgatagtt 46261
tgtcaattca tactttgtca tttaagagtt atggaaaagc atagttctct ttaaacctgc 46321
ttttcaagga aattctactg gattgtctgg aaagatttag ccttctacac caggttagta 46381
gtggacagaa tggacactta cttagtgatg ctttcaaatg ggtcagctgg atgtagcact 46441
cggttgagaa gggagaggta ttgaatcctt cgccaagtta ttgagagttt ttaatctcag 46501
tactctgcaa tgtgtgatgt gaatcagcca cagaaagcag tgtgtttaca agatccaggt 46561
cctctacagt ggacatatat ggggccatcc aagtcccatg ggcattatca gagagagtag 46621
attttttttg ttttgttttg tgttttgttt tgagacagag tttcgctctt gttgtccagg 46681
ctggagtgca gtggtgtgat cttggctcac tggaacctct gcctcctggg ttcaagcgat 46741
tctcctgcct cagccccccg agtagctggg attacaggca tgagccactg cacccagcca 46801
tcagagagag tgtttttatg agaaggagga tcctaagagc tctacaatct ggctttggag 46861
agagtgctac tgagtgagag cagatatgct ctacaggact ctgataacca aataatgata 46921
aacatttatg tttctggcat gatatttaca tactcattct tgggagcaag ggttctaggg 46981

gatcctcaga gagttccctt cattatctca ttcatgattt caacagcagt tgaattgggg 47041
gtattacctt acttaagtag atgttacatt tacttcctcc ctgaatttta tgatcttact 47101
tcatttgtga agttcaatac tagtttatgt cttttcagaa aatgtcaatc atagagattt 47161
ccttgttaaa atataatttc ctccctatct ttgttccttt tccag gat gag gaa gaa 47218
Asp Glu Glu Glu

gag tcc agt agg cag gaa acc gaa gag aaa ata ctc ctg gat cca aat 47266
Glu Ser Ser Arg Gln Glu Thr Glu Glu Lys Ile Leu Leu Asp Pro Asn
650                 655                 660                 665
agc gaa gaa gtt tct gag aag gat gct aag cag atc att ga 47307
Ser Glu Glu Val Ser Glu Lys Asp Ala Lys Gln Ile Ile Glu
                670                 675
gtatgtactg catttttccc ttggaaatgc attgcatgag gatgatgctt aatgaatttt 47367
cttttaggaa attttgttca aggaaggcaa ctaacttcac gcctacactc tgcttgaaac 47427
tgtgctccta gaggtcacca ctgagttctg ggttcccagg tctcggggtt tcgtgttgat 47487
tctccatccc tccctccctt ccttccttcc ctttcttccc tttctttctc acttccttac 47547
ccttctctct ccttaatccc cacctccagc tcaggctcac tcctgagcct cacacctgtc 47607
gtctgcacct cagtcccaga ttgaactaat gaaacttatc tccctaactg gctgcttcgc 47667
tgcctttcct gcctccctta atgatatcag cagtctccct gaaacccaag tgagagacac 47727
cattcctgtc ttactcctgc tgccatagcc aagttgacca ccaactctcg cccagtccct 47787
gaaccctctc cagccctccg agtgccctgg ctctgtgtct catccattcc tgtgggccct 47847
ctacgtcaag ggtttgcaca tttctttgct tgtatagctg tactattaaa agaaaactgt 47907
tgtgaaacaa tataaaagat gaggtatttt aaaaaccaga aatagacatc ctaatatttc 47967
tcccttttat tccatggttg tgtgccccac tttagggacc cttcctccta gcagtctgcc 48027
ctgatccttc actgaaggtg ttaagaatcc ccctcagtgg ttcctcattg cctgtagaat 48087
agcagagtag ctcatgaaca aggcacagaa gatagaagcc tacctttcct ccttcctacc 48147
cctttaatgt tgggttgccc tctccccctg ccctcccagc tttagccagt gttctgtgaa 48207
taggatttat ctttgtattt aacagtctag gacagcttca tcggcaaagt attcatctgc 48267
aggtgtaggg ttcaggctcc tatctgtctt ttgggtcaag ggggcctgca ttcgaatcct 48327
ggtttcacta ccttttatgt gatccaaaca aatgaagtaa cctctctaag cctcgggttc 48387
tccacctgca taaaatgggg atgatcatca taacgccttt tgtgcttgct ttttgagagg 48447
attacagata gtgtatatta actgcatggc aagtcgtggt tgataaataa aacacactta 48507
ttgcagctat tttaggttct caaatcattt tttgagtgaa gtaaaacaac acatgcacgc 48567
acatgtctgt gtgtgattct ccacttttc ctttcccag g aca gct aag caa gac 48622
Thr Ala Lys Gln Asp
680
gtg gat gat gaa tac agc atg cag tac agt gcc agg ggc tcc cag tcc 48670
Val Asp Asp Glu Tyr Ser Met Gln Tyr Ser Ala Arg Gly Ser Gln Ser
685                 690                 695                 700
tac tac acc gtg gct cat gcc atc tcg gag agg gtg gag aaa cag tct 48718
Tyr Tyr Thr Val Ala His Ala Ile Ser Glu Arg Val Glu Lys Gln Ser
                705                 710                 715
gcc ctc cta att aat ggg acc cta aag cat tac cag gtaattggca 48764
Ala Leu Leu Ile Asn Gly Thr Leu Lys His Tyr Gln
            720                 725
tggtttcagt ttccttggca agttgtaacc atttacctaa ttttgattga agtatgtcgt 48824
gcacatgttg actaagggct tttgatgata ttttaggcca catcacttat aatactgagg 48884
ttcctttcat tgtgcctatg attcttcttt tagagtactc catgttacca tctttactag 48944
ggcatgccat acccattttc tcttttatgc ttttgggatg tattttaagt gatctctgca 49004
aaatggaaaa cccagccaca ttctattaaa ctggagaaca tccacgcctc atcttgaagt 49064
atttctttaa aatttttagg tgtggctgtg gttttatgcc gtggattgaa cccacctagg 49124
agtagctgtc tactacattt atgctttttc ttaagacaca ataggccagg catggtggct 49184
cactcttgta atcctatcac tttgggaggc tgaggtgggt ggatcacttg aggtcaggag 49244
ctcgagacca gcctggccaa catggtgaaa ccctctctct actaaaaata caaaaattag 49304
ctgggtgtgg tggcacatgc ctgtaatccc ggctactctg gaggctgaag caggagaatc 49364
gcttgagctc cggaggtgga ggttgcagtg agccaagatc ttagcactgc actccagcct 49424
gggtgacaaa gtgaaactcc atctacaaaa aaaaaaagaa acaataaaat tgtgagcatc 49484
tgtgctttga ttaaaaatta ggccattgtt ttatttgacc tgtctggatt gaggctaatt 49544
ccctgtgttc taccttctac cacttagtga cgtttcccaa attgtgttcc atgagacatt 49604
tgttctatga attgttcctt aaaaattaaa aaaggaggcc aggtgcggta gctcacgcct 49664
gtaatcccag cactttggga ggctgatgtg ggccgatcac aaggtcagga gattgagacc 49724
atccttgcta acacagtgaa accctgtctc tactaaaaac acaaaaaaat tagctgggtg 49784
tggtggcggg cacctgtagt cccagctact cgggaagctg aggcgggaga atggcgtgaa 49844
cccaggaggc ggagcttaca gtgagccgag atagcaccac tgcactccag cctcggcaac 49904
agagtgagac tctatccaaa aaaataaata aataaataaa aataaaaata gataaataaa 49964
aaaagaggtt tgtggccaaa tactgtaact aagattacca tataaattac tatttctaac 50024
ttgaatactt gtgaacgtga gtcgagggcc ttattattga taataggcat aaccctggac 50084
tgtccctgaa caacaaccag atacggtcac tctggcagtg acccctcttg ttaattcaca 50144
tgcccgtcag cacacttggg actctgcgga aatcaacatg taaccataag gcacatcagc 50204

atcctgcagt aaaatcactt ctttaacatt gtttaacttc atatttccca aatttctttg 50264
actaccaaac cctattttta tatcaccaat taacatcctg taagactagc gtcttctaga 50324
atacacttta ggaagtgtac ttgttggcat cgggtcctct ctactcttaa cacttggtac 50384
tgtgtctgct gcccttgcat aggagtgcag aatacaatga ctgccagtac agtactgcct 50444
tgatggtgct gaagcactag cagtttttac ctaccaatgc ttttgcatca tcagtgcaga 50504
cgtcaacaca gtaaaaatga caagtaatgc cttagtattg tcatgctaat cattttgacc 50564
tcgtggatct ccttccaaga tcttgggggg ccctgagaac ccctggaaca cattctgagg 50624
accactgctc tggccttagt aagatcatgt tttctccaaa gaccatgtca tagtcctgag 50684
atccaaagca ggtttcatct tgtttggcct acctgacata gatgttaact catagcatgc 50744
tctaataaac ttttgtatat ctgcatatgg gagatccatc ttttgaatta tttgtgaata 50804
acttgtaatg ccacatgggt tttcttctgt catccaacac ccttctaagc agtcttcatc 50864
actctctttt caaacctagc tccttcgact tccatatgtg agttttatgc ctctgtgttg 50924
ctgagctgct gtagattccc cgaacatgcc ttgcagtgtc tctctggatt tataccttat 50984
gaagtgcctt cttcctggaa tcttgactag ccatgtccgc ctgatgagat ctcacaaacc 51044
agttcaagtt gtggctcaaa ccttccctga tcatttctca taaaccagtt caagttgtgg 51104
ctcaaagcct tccctgatca ttctctccat gagtaagctt tctatcttct tgcttcttga 51164
caggtttttg tgtcattaat atttacttaa ccttgacacg gctgaggtct tcctgctact 51224
cctagagagc cacataagct tggttcaata tctaatcctc cccctggcca attctgccag 51284
tttccagcct gagaaaatcc aaattacttc ccacaatttt tgtgaatttc tagtctcctc 51344
tgctgctagt ttctacttct gttttgtatt gaattagcac aaacgcaaaa ttcagcccct 51404
ttaccaattt tctctaaaaa aagcagaaac aaaaattcat cctacagagt atccaaaatg 51464
tttcttgtat ttagttgact tttctaaagc tctagtggtt tcagggtcac gttgcaggct 51524
gacagcttct cctgtctacc actgtttgtt ttataataat ttcccctctt tccctctatc 51584
ttttgcccat tctcttggag gttcttcctg tttctctact tatctcttgt tgttgttgta 51644
gttctgcaga aacctttagg atgactttgg aatcttgcat ggtacaaagt atctcaagtg 51704
gcttatggaa gtattgcttc aaaccaatgc ggtgtctccc taagaaaaat tcttgtcaat 51764
acagttttat atgtatacat atacattggc actgtcaacc acttcttggc atatgtgtat 51824
atgtgcctca ttcattgttt tctcttttat ttgagcttct gaagggaggc atgcatctta 51884
cagaccttgg tatctatctg ctacagctcc ttgcatggcc ccttgcacag aatagtcatt 51944
taataaatat tttaaataaa ctaattgctt gctttgttct tacataaaaa attaacattt 52004
ccctaagcat aacatcatta gcaagctaaa ttgactttag caaacatata tataattgat 52064
cctatcattt aaatccagca tctgtggtat gatggtggaa gggaattgtt ggttggggga 52124
tggaggttta acaattttgt attttcaccc aaccttactt ttgtacttaa cgtggggaat 52184
ctctggtaca gtgtttttgt acttcagaaa tacgttgagc atgtttccag ttgccagctt 52244
ccagtgtgca gcctttgggt agtactcaca ttacaaagtt cccatgtgct gtctgggaac 52304
tttctctctg ccaggaagag aaatgtcctc ctgtggtccc agcagagtgt ccctggctcc 52364
ctggtcagtc cgccttttca ctgaggggac ctcacccata ttaccgaaaa ggctgttttt 52424
aataatctgt gatctgtgtg ccaaatctgc cttcacattg gctttttatt cttggagtgg 52484
ttggattaga ttttggtttg ggcttttttc atttataata attctagaat gtcacatact 52544
gtgaaatact aagtgtggat gagcggcaaa gctgctttaa aactcacctg tcaagaagca 52604
agaagagaga aagcttactc atggagagaa tgatcaggct catattttac attctttttg 52664
cattggtgct tcccccattt tcctactgtg ggaaacccaa catacagcag tgaggagtta 52724
agaagtcaca ccctcacttg ggttgtatta ggattaatta cctgtgcaga tcttggataa 52784
ttgaagcaat tactcttcat ttcag ctc cag ggc ctg gaa tgg atg gtt tcc 52836
Leu Gln Gly Leu Glu Trp Met Val Ser
    730                 735
ctg tat aat aac aac ttg aac gga atc tta gcc gat gaa atg ggg ctt 52884
Leu Tyr Asn Asn Asn Leu Asn Gly Ile Leu Ala Asp Glu Met Gly Leu
        740                 745                 750
gga aag acc ata cag acc att gca ctc atc act tat ctg atg gag cac 52932
Gly Lys Thr Ile Gln Thr Ile Ala Leu Ile Thr Tyr Leu Met Glu His
    755                 760                 765
aaa aga ctc aat ggc ccc tat ctc atc att gtt ccc ctt tc 52973
Lys Arg Leu Asn Gly Pro Tyr Leu Ile Ile Val Pro Leu Ser
770                 775                 780
gtaagtaaag tcatttattc cacagtcatc gttctgtatg ttgtagagtg cccgattagt 53033
agcttgtcct tgttttttac ttaagacaga attgtagcat gtactaacct aaatccagat 53093
taccaagagc atgtaatctg gatttaggtt ttaatttccc acccctcccc atcctagcct 53153
gtttgattgg ctatttaagc acggaccgaa gtgattttat cggccatcag cattacaggt 53213
ctgcacagcc tgaacactgc atagtgagtc ttgctcatca ttattcctta agtggctcac 53273
aaaggggaaa ggtgtgtgtg tgtgtgtgtg tgtgtgtgtg tgtgtgtgtg tgtgtgattt 53333
ctttgatttt ttccttattc catttccaaa agatgatata ttcttaaatc ttccttttaa 53393
aagcagaaca taaatcaaaa tcataactag gagttttgca ttaaaataac cttagtatgt 53453
gcactgaggt tatgtttcaa tgagtttgct gcttagtaag gacaggagtt tgctgttcgc 53513
cattacgttc ccagtgcctc acctgctagg tggttcttag aaggtcctaa gtgggcacta 53573
ccgagtgagg gcacagtgat tcacaagcac cctcctggga gttcagtgtg tctgcctgtg 53633
ggtgttaggt tgcagttttg ttcaccaagg tcttgttata gatctcaatg ctgatatgaa 53693
cttggacctt cagtaagaag aaggattcta ttaatattat tgtgttttct ttgaattttt 53753
tctaatgaag actatttgca gccctttttg tatgaattat ctgtatctgt aatatttagg 53813

catcttttgc atttgatcta tattttgcgt gttctgaatc atatatacag tgacactttc 53873
agggctttaa aaaacgtcat tcaatatttc ttcccctgtt ccctcttgac agatttattt 53933
tatgttttca tgtggtttta taatttcttt ttcctgactc atcttttttc tgaacctata 53993
tggctgttta tgaagattcc taaaatcatt ctggcaattg ctctttttta atatcatatt 54053
acagtttagt tttttgctct ctatatttat gtattcttta gtttttttct tacaagattt 54113
ttttatgaac cacaaacttg tcatgttttg tgacagtttg ttttgaactt atctcctatt 54173
tccacacctt taaaataaat atcttgttga gatatgttat attctgtagc ccctttcaag 54233
gtgaggcctg aacatgaata agaacatctt tttaaccatt gatttttata caaatgtctt 54293
ttttctgttg tttttttttt ttgttccata g g act cta tct aac tgg aca tat 54346
Thr Leu Ser Asn Trp Thr Tyr
    785                 790
gaa ttt gac aaa tgg gct cct tct gtg gtg aag att tct tac aag 54391
Glu Phe Asp Lys Trp Ala Pro Ser Val Val Lys Ile Ser Tyr Lys
                795                 800                 805
gtttggaatg ctgtatttat atataaatgt ggaaaagcaa aaaatagacc ctatttcttt 54451
cattccatat gcacatctgt gaactgtatt tggttgtaaa gttttgtcat gtacatcatg 54511
tactaaaccg tgagagttaa tcatcccaag aagattactg taataaacca taattacttt 54571
cagatagtga aatccagaag aaaattgtta attatcatat tgcaaggttt ctgatgagag 54631
taatacaaat gacaaatatc acacattctg cgtgctgctg actggctcgc tgtgggctgc 54691
tactgtgctg actgttcctg caaagtcagg caattacccg gatgcccggg gctgcctgct 54751
gtactttctc aacagaactg gagcctttca gcagagcggt gggttctcta ctaccacaaa 54811
ttcactgtca tttccatttc cctttgccct cccactgtct ttggtgggaa aacctttcta 54871
aaaaaagatc tgaaaagggt caagacgtgt aatttagcca aaacttcctc gtgtataata 54931
gatcagtcta tgagaatgtg tgtctgtgca ttcttcagtc acatgtctgg gcatcattaa 54991
gctatgaaaa gtgagaaata atgcacatat atgtccatag gatcatgcat gtattttttt 55051
ctttccattc ag ggt act cct gcc atg cgt cgc tcc ctt gtc ccc cag cta 55102
Gly Thr Pro Ala Met Arg Arg Ser Leu Val Pro Gln Leu
                810                 815
cgg agt ggc aaa ttc aat gtc ctc ttg act act tat gag tat att ata 55150
Arg Ser Gly Lys Phe Asn Val Leu Leu Thr Thr Tyr Glu Tyr Ile Ile
    820                 825                 830
aaa gac aag cac att ctt gca aag gtatgttttt aaaaaattat tttctctcta 55204
Lys Asp Lys His Ile Leu Ala Lys
835                 840
attaggacca ttgcactgga atgtgaagta ttgcccaagt cagtagtgta attggatcct 55264
tagatggctt gtccttttct ttatttttct aaaatttttt cagagtttga tatgttttgg 55324
tcgtggattt gatttcatgc atgctgtgtg tgtgtgtgtg tgtgtgtgtg tgtgtgtgtg 55384
tgtgtgtgtg tttatgattg atcctggggc ggctcttgtc tatcccctca gagttgatga 55444
gtttacatct cttaaaaaaa ttcacctggt tgcttctgca actctttttc tctctaacag 55504
ctttgcacat tctcgggaaa gaaataattg tttttgttgc cctgagatat tgtcagtgta 55564
ccttcattct tcttgtgcct ttgtttctcc aaagatgagc cagacacctc tccatatggt 55624
ctccaccata tgaacattta tccaagccgt ggcttttgtg gtagtcttgt ttcaggtttt 55684
tcctggtgaa ataatagact atcattgttg gagttactta aggttgtttc tgtgactatt 55744
tatttgaaac tgagaaagag gtgtttctgt gacttacatg tctcctaatt tttatgtcct 55804
aagttgttcc ttagccacaa agtagaaatt tttactccca tttatatcat cttgtcttct 55864
aatacgttca tgtagaaaga tggaagtgct ctctttcgtg gaacaaataa tgaaaactct 55924
tttttttcct ctttagtaat tttttttacc ccatgacgtt ttgaaagcac tataatctga 55984
aactaagtta tgtccaaaat tgaatagaac ctctatttct ggggtctatt ctacctctgc 56044
attaaactct tgctctggga actgagagtt cccttcatgg catcatagga aataacaaaa 56104
aggaaatgtg ttcccagggg aggtttaata attgagatgg cactggcaac aatgctgtgt 56164
tccacctcct cacactcaga gatgaatttg gcagtgtcaa aaatgatgtg ttttcaatat 56224
tactatggga atgtgtttgt acatttgaga tccttttaaa aaaattcatc ttgtgtcttc 56284
tgcatatgta tttcaagatt cattggagac gcctggtaat gagatgcact gcttttattt 56344
gttactggag aaatggagct atagaaagag gtagtaattt tgtccaagtg gaagacctaa 56404
ttagtagtag accccatagc aaaatcaaag gcctctgatt ctttattatt tcataagcta 56464
aattaggcag gaaagtagat ttaggatatt tatttaaata tattttttgt ctgcttgcct 56524
cttttcatgc tgttggtaag tctgaggcag ttgattgtac agctttaatt atccttaaac 56584
acttttctta gaggtggtgg aggaaggaaa aaaaatcgag gtagtttttt ttttgttttt 56644
ttcttacctg tacatcccag cctgactgat gattttattc cttttctcct ggcacgtctg 56704
atgcttctaa tgaggaaggc aaggactgta tagaatactt tcctcatctc ccttctctct 56764
ctaggagctt tgcagcagga tctggggaca tctgagcacc agatgttttt cttggccatt 56824
gccactgttt tgtagagaat ggataggctt agtggaacat tcctcattgt tctcacttta 56884
agaaactgaa cagtgctctt aagaggactg aagggtaagg tatgagagag ggaacgtact 56944
gtgtagacag gaaggaggac tgggccatat aagcattctg gacgttttac gcaatcgtca 57004
tgttggggaa gtatgctgta tttaattctg cagttttggc ataaatacag gagtgatatt 57064
tttgcagtgc gctttctgcc agtgagtata ttcttttatt ttagataaaa caggggtcct 57124
agatctggca ggtaaggaat gtgcgtgtat tgagggagct tcgttgtcag acctaatttt 57184
tgaaaagccg tatgtacaaa tttaatgtga aatagtccaa cttttaaatg ttagcaacca 57244
gtccaaacaa aacaaaacac acttccgggc caagccctaa aggtagtaac cagccaggct 57304

aatagaaaag aaggcatgga gccaagaata tctcagctgt agaacctgct agggccatct 57364
tggaaaaatc agtcattaac ctctgcttca ggtgggtacc tatattctgt gcctgtcatg 57424
agctgaaata gcatgaccat gaaatctggg atagctgtca tgataacgta ttaatagaag 57484
gggcattgga tggggagagg caggatccac acgtggaaac aaccatgtca ttctggaccc 57544
aaatttcctt ctttgtaaaa agttgatttg gaattacgtg gtctgtaagg aacattgttc 57604
ctttaacatt ctattattat aggatgtctt atgcggccta tcaggcatga gacattgttg 57664
agattccctt gtctcaaagg taatcacagc atatcatata ccaaggatgg gttctttggt 57724
tttccgcacc accacttgct tgttggaaat agttgtattt tcccttgctt actacacgtc 57784
cgtccttcct cttgtgttat ag att cgg tgg aaa tac atg ata gtg gac gaa 57836
Ile Arg Trp Lys Tyr Met Ile Val Asp Glu
        845                 850
ggc cac cga atg aag aat cac cac tgc aag ctg act cag gtc ttg aac 57884
Gly His Arg Met Lys Asn His His Cys Lys Leu Thr Gln Val Leu Asn
        855                 860                 865
act cac tat gtg gcc ccc aga agg atc ctc ttg act ggg acc ccg ctg 57932
Thr His Tyr Val Ala Pro Arg Arg Ile Leu Leu Thr Gly Thr Pro Leu
    870                 875                 880
cag aat aag ctc cct gaa ctc tgg gcc ctc ctc aac ttc ctc ctc cca 57980
Gln Asn Lys Leu Pro Glu Leu Trp Ala Leu Leu Asn Phe Leu Leu Pro
885                 890                 895                 900
aca att ttt aag agc tgc agc aca ttt gaa caa tgg ttc aat gct cca 58028
Thr Ile Phe Lys Ser Cys Ser Thr Phe Glu Gln Trp Phe Asn Ala Pro
                905                 910                 915
ttt gcc atg act ggt gaa agg gtactggtct gagttctgtt ttttccactg 58079
Phe Ala Met Thr Gly Glu Arg
            920
catgataatg acatgaaatg aaaggcccta aagctgagaa cattagagcc acagaacacc 58139
cgcagggtgg tttccactgt tgtttatttt atgaaaccca tcggtgggtg gacatggtct 58199
tgtggtgggg ttattttccc ctcctaggaa aatggatcta gtggtacatg gggcatctaa 58259
cccctttgtc ttttgccagc atgtttacca ggctaccaga aaagggtccc aaccggtgga 58319
atgcttaact gcagtgggcc acagggagga tgaacatgga gatattttgc tgaagtaatt 58379
taaccagacc aaattaacca actaaataca agctttaaac aaatctttcc ttgttacaaa 58439
cttggagttc cctttaaaat atttccacat aaagattttg tgtgtaaaac agaactagtt 58499
ttcacacatt ttctgcagac cttttttttt tctttttagg ttttaatgtg attttcagtt 58559
ttgtgtgtac ataaatagtc ttcctggagt agccatttat tttccaattt cttattgttc 58619
cttccactgt cttctcttaa gagctcattt atgaaaggta gacaatgggc cattgcaaga 58679
aaagaaatgg gcacgtgtca ttttcatagt ggttcctcat tcataggaat gaaaagccac 58739
taaaaatgct ttagtaaaat ttcggtttga attatttggc cctttgatgt tgaggcacat 58799
gggcccatcc tcagtagtta actcactggc ttgcccattt gtagaaagta gtgtcaatca 58859
actctgcccc ctagaggtta ataggagaag gtgaaagaga aagccgggaa atgtataaga 58919
tggactaaac ttgaactaga tagggctgag aaaaggaaag gagtgaagtt agagggactt 58979
agaggtccac cagcataaac tggatcaact cttgttcctc ttctttatat gttcaagccc 59039
ctgttccttt agtatgtttc tgtgtctgag acaaccaaga cgaagggcct aacttaaggc 59099
tgccacttta aatagggaac atattaactg ttgcccacct tctcccattc tatctcagtc 59159
ttcagtctgt cttcattgac agtcatatcc catacaattt gaggctgttg acactactga 59219
taaaaaactg gtggcttccg ggctcttttc catagaactc agcgttaaga caaagacacc 59279
tatttagagg tcattttgtt accacttggt aggcaggtgg gaagataatt cagaggtaga 59339
tatgacaggc ttaaacttgg ctttaatgca tgtaaaatgt caatcatgta ttgaaccaca 59399
catatgtaac ataaagcttt attgtatgaa acatcctttt ctttaaaaaa tcaaattcat 59459
aataagcctc tcttacag gtg gac tta aat gaa gaa gaa act ata ttg atc 59510
Val Asp Leu Asn Glu Glu Glu Thr Ile Leu Ile
    925                 930
atc agg cgt cta cat aag gtg tta aga cca ttt tta cta agg aga ctg 59558
Ile Arg Arg Leu His Lys Val Leu Arg Pro Phe Leu Leu Arg Arg Leu
935                 940                 945                 950
aag aaa gaa gtt gaa tcc cag ctt ccc gaa aaa gtatgttgca caaccaaaag 59611
Lys Lys Glu Val Glu Ser Gln Leu Pro Glu Lys
                955                 960
ttgtgggttt tttttttcct ccagcaaata tatttgaagt acctgcctct gaaattgtgt 59671
ttctgaaaca gcagactcat attgtagtta atagtatctt gaaaatatct taaattgttt 59731
ttttgtttga tagggctaga gagagcaggg aaattatagc atttgcagcc tgttttccaa 59791
aagcgttcga gcagatttta tgaaattatc aaattcactg agtctcattt acattttaat 59851
tttagatttt tttttttttt taaattacgg acctcattga gttcactgtt gcaggcactg 59911
tagaattgtt ctgcttataa gtgaatggaa gatttggcgt tggggacttg tatcatcatt 59971
gaagggggtg gtaaacattt acaggttttc tagaaattta tttaacattt gatgtttgtg 60031
atgtcaccat tctgacagta agtgaaaaaa cagagttcct gcaaattaca gacacatcat 60091
ctaattattc ggggacatta tttttgttct gtgccataaa aattaagttc ctgtgaacat 60151
tttaagccag tatttttcgg actaactttt ggaaacttag tctgcagtac tttagaacac 60211
cttttgagcc cttagctcca tgttttgggg ttggatgtga gtattagttt aatatgtttc 60271

tgatcatatt ttggaaccat gcatatttga attctaagtt tcattctctt agagacagtt 60331
tagcattgtg tactatttgt tgcatgaggt attttgattt gagttttttc tttagaagag 60391
ccaattctga aatgtcttat ttttgcatga ttgtggtttt tctgggtaac gactctattt 60451
ttggacagat tggttcatcc tgttcatttt taagtgggag ttttcaagat ggtgtacaac 60511
gttgatcact ttgttacgag agaattttat tccctaatcc tttatgcatt gtcagaaaaa 60571
aaattaaaaa tgacttttat accatgcata tctcagtgaa agtctacgta tttaagtatt 60631
ttattctgct ggtgaatgca tcttggacgt agaatgctat tgatattccc ttggacttcc 60691
tgtggtcttg gcctctgggt agcacccttt ctgttcaaga tggagtttag ttactgagta 60751
gataaattga tgaatgggta tagtggccat gtgggtgcct tgaattactt cctagcatac 60811
agttacggtg attctgcaag tgatagagat ggaatgttaa acattttaat ttatcccata 60871
atttcttttt ttttctttac aagaggtata aaataaaagg caattaactg aagcccttca 60931
tttattcatt ccataagact tcttaatctt gttcattcac ttctaagact ggctaataca 60991
gatagatttt taaaaatata ttgcaagttg aaagtatgtt atgttgtaag aaaatccact 61051
agagaaaaat tgaaattttt gcagcttaag aattatgagg tgattaatta tatcacaaag 61111
gaattctttg ctttacaaaa atatttattt tatggttctt tctacaagag aagttcctga 61171
gtatacttga aggatgataa aatttttatg aaatgtatct accctcaaat gatcacgagt 61231
atgtctcttg cataaagtat acatgtattt attaacaccc agagtattat atttgaggta 61291
tgtaaaatac tcagtgtgtg gtattatcta cctttagagg tatctgtacc tttaaaggca 61351
gccatgttat cttttccttt ggtggagtgg aaatcatggg ctctgcctgt cagagtaggc 61411
atgggtttca ccacattctc catctcttac tatgtgaact tgggcaagtc ttctaaccta 61471
aatgggtctt agtgttctca tcagtgaact ggaagataaa tgactctgtc ttatggagtt 61531
gtgaggatta aatggatgta gggctcctaa taccatgcca ggcacatggt gggcacccaa 61591
ccaatgacag ctgcttctac catattcctt attttccact catgttgctg tctctgttat 61651
taatgggagt tccattttcc tattcttgaa gctctttctc gctgttctct tgtctttgct 61711
ctccttccgg tagccccccc acatctctct ccataaactt ttcaagcact ttgtgtgtgt 61771
atgtggttgt tgttgatatg gctgggctga tgttcatttt ctactttttc agccttgtta 61831
ggattttttt tttttagcct ggggaaagtt ggaaatttca ttgccatcct agggggtaaa 61891
gaagtttctt ctaccaccac tgcttacagc cacaaacaat gtgcatgtct tacttagcct 61951
ggataaagaa tccagaaagg aactacggca gatggaggaa aaggctaaat actttcacag 62011
cagtaattct caaatgtttt tatgaaaaaa gagccatata ggaaagaggc ttttactttt 62071
attttttaat ttatgtgtag caaaatttgc ttcttttggt gtatagtttt atgagttttg 62131
acaaatgaag agttgtgtta accatgccca caacaaaaat tcaaaacaat gtcatcatcc 62191
accgctccaa aaaaaattcc ctggtatttc ctggtactac ttcttttcag tgaaaccctc 62251
cctcggctcg tagcctcatg cagccactca tccgttctcc atccctgtct atagctttat 62311
cttttccaaa atgtcgtatg agtagaataa gacaatgtat aaccttttgc atggtttttt 62371
ttaatttggc aaaatgtgtt tcagattcat ccatgttgtt gcatgtatca atagttggtt 62431
catttttatt gttgaataat attccattac atggatgcag cgcagtttgt ttgacatttg 62491
ggtcatttct agtttttgac aattatgaat agagctgcta taaacttttc atgtacaggt 62551
ttttgtgtga acataggttt gcatttcact tgagtaaacg ccttaggtgt atgactggtt 62611
gatcgaatgg tcagtgtata catagatttg taagaaactt ctgcattgtt tcttagactg 62671
tctgaagcac tgggtagtcc caccagaaat atatgaccat tccacttcct ccacattctc 62731
attagtgctt gctattgtca gtttttcaag atttttagcc attcaatagg tgcatagtgg 62791
cgtcttgtaa ctttgattgg cattctctaa tgtctaatat tgttgagcat cttttcatgt 62851
gctttctcag gtgaaactaa agcaggtata taaaggccac ggttttgtaa atgcaggtca 62911
accgcatagg aaaattagct ttaatattgg tctaatgaaa cacttaatga gcaagggaat 62971
atacccaccc cccaaccacc aacaccgcta agctgttgtc ataaaatcca tttcttatgc 63031
acgggaaaga atgccaaata gaaaagggat tatgtaagaa caacttaagc taaataaaaa 63091
ttgatttttc tatttagctc acatttaagg ttgtctgtgc tgactctttt gttaattcat 63151
atcatcaaga accaactata cttgcaattg gctgggtatt tctttatgtc taaatatgct 63211
aaaatatgta tataatatct gaggttaact agaggttgac ctacctaaag tcaatcctga 63271
ttctgatata atttgtgaaa aacaccaaca aagctttgct tacattggca atttgtgtat 63331
tcattatatc aaattttgga atgcttagca taattaatca ttactttgtt cctctctcat 63391
gccatagcgt ttagaacctg agacttttca taaattgaat catattgttt acaaatctca 63451
gttcacaaag tggtgatata tcgcatgtag tttaggttta ttattccaat aaaacatcct 63511
ttaggaaggg gaattataaa tgaagaatag tattcaggca gaagctatga acaaagttca 63571
aatacttcta caggggtata taagaagaaa tgctgagcca gtataatgac ctgatttgcc 63631
tacttttagg gaccagttag gccatttact aacataagaa tgcttaggag ataattacca 63691
gatacatgag gtcagcacct cagttttaca tatgatttaa acaaaaatag ctccagtggc 63751
tctcacaaac ctaacttact gggagaatca cccccgtgaa gaagtcacaa gtttcatttg 63811
ctatagcacc agtatatttt gaggagcttt cacatgcatg ctctaaacag atgccatcac 63871
attttttggg ggatgaactc agactcaacc gagttggagt tggcacgatt attataaaaa 63931
cttctctcgt cactgtgatt cagtgtggtg gctgtataat tgttcagtcc atcccttttc 63991
ttgatacaaa cctgttgatt gcctgctctg tacgttatac tgcactcagg ccaggtggga 64051
ggcacaatat accagaaatg ggaggcatgg tgtgagaagt caacagactt ggctgtggag 64111
atgagggcta ctcacactga ataatagtgt ggcaatgcaa ggcagcatca tcccaccaat 64171
cagaatgaat gatccaggca ttcaggtggt ttaggacggg gtacccagta tgtacacatg 64231
ggtgtatgtg ttgggaaatg cttattagta atccattggg tggagaaaga tgttagaaca 64291
atcatatata ttgatctttc tcttaaaaag tcttcactga tatttaacat gcagattgac 64351
accggtacct atgtggtagg tacgtatgtg tgtctgaaag cgagtcatgt gctgggagta 64411

cactgtgtag gttatcccag gggaagcgtg agagtgggtt tccaccactg ggagcatctg 64471
atagtccttt catccttgct gcctggttca tttactttct gccaatggtg gtgaggcctt 64531
atgggtttcc ccgtgcccag ataaggagat ttagtatgat ctgattttaa ctaaactaac 64591
aaccctggtc cccagaaggg cttaaaagga ttctgcaaag gagtgcaggt tgaaatcaca 64651
taggcataga tgaacaatgg gaaaaaggcc atcttgtctc ttttcccatt cctagtattg 64711
gttttcaaca gccacatcat ggtacatggg ctggagttga ccaaaaatat tagatatcat 64771
caaattggca gctttaaata gggctataaa tcaacttcct ttaacattga cctgtgccct 64831
gaaagtaact catatcttga gacattaata ttagtgcatg catatagtaa ttcataaata 64891
aatatgtaga tatatgttgg gaaaatgagc ttcagcatgt cttcttgttt ttcttctcca 64951
ttatcaaaaa agcttactga caaacttctt tgagagaaat gaaagggaaa ttggctttgg 65011
ctgatagtgt gattgggaaa gatcttaaaa agggccctca aggtagagta gagttagaaa 65071
ggagccttaa gactaagtaa ggatatccca ggcctggaga caagtacaaa aacaaaccaa 65131
gtggcctgga gttgtttgag aaggctctgg gagcaaacat aaaatatagt tgtgaagctt 65191
atgtgatttt tgatcctgtg tattaaatgc cctaattgtg tgaggaaaga gactctgcct 65251
tctaagagca gccatcaggt ccttgagagc tgcttgcctc tctgctgact tgacctcgtt 65311
gagaatgctt ggcatccctg tctccatggt atcaccatgc atgcctcctg ctttccaaac 65371
tccttcacct ccagccctta gcagtggctg actcatagga aggtgcttga gtgttctttc 65431
ctgaattctg gatctgcctt ccccaaatgc acaagcatca gccatgcctt ccacattcag 65491
gaaggtttga tcaagccagc agggaggtgc aggtgaaggt gaacctcagg cagagctagg 65551
aagcctgaaa atctcctcag tcgtcctaac gtggagctta aaagagtgga ttctatttca 65611
aatcctaact gtgccattca atagctatat ctttgggtct gtagctaaac ctctgtggtg 65671
ctcagattcc taatctataa aatgagaagg ctaaatgtgg ctatcccagt acagctcttt 65731
tgtgtgttaa atgagagaag gctcagaaag cactttgcac agggcttttt ctgtggaaag 65791
agcccagtaa tattagtcct tgatgtcatt ttaccattat acttgttaaa gtctaaagtt 65851
gttcagcgtt tttctttacc cttatcaaat gtcactgtga gttaacagct cttgaaaaag 65911
tagaagcctg gggtaatcat ttcacaatgt ttatgtatat caaaatctca cagacgtacc 65971
ttaaatatac acaatatgta tttgtatata ttataccttt ctaaaatatt ataccttgat 66031
acaattatac ctcaatacag ctgaaaaaat tagaattttt tttttttttt gagacagagt 66091
cttgctccgt cacccaggct ggagtgcagt ggcgtgatct cagctcactg caacctccgc 66151
ctcccgggtt ccagcgattc tcctgcctca gcttcctgag tagctgggac tacaggcaca 66211
caccaccatg cctggctaat tttgtatttt ttagtagagc tggggtttca ttatgttggt 66271
caggctggtc tggaactcct gacctcgtga ttcgcccgcc tcggcctccc aaagtgctgg 66331
gactgcaggc gtgagccacc gcaccgggcc aaattataga aaattttcat aaaaaaaaaa 66391
ggagaagtac tttaaacata taacatgatc agttatctaa aacacaaagc ctgtattaaa 66451
tgcatactta ttgattaatc ttggagattt ttctttcacc tggccaagaa ggtcattaaa 66511
gactaaagca tgtttttgga gggttactta cacttctcac cttttctgta aaagagcggg 66571
cttttctcta atctcatttg atgcatgaaa atgtgtggcc agaaactatt aattgtgctt 66631
ccatgtggcc tgacagattc cttcgaaagc aacaattgaa ggaatattca agttctctgt 66691
gacctcttct ccctggccct cagagctgca aataatgtga tcattttaca tcagtttatt 66751
aaacattagt cagcttgctg atttattttt aatcacattc tgcatatttc taggcattaa 66811
tctgatgttt attatgagaa ttgcacattt gtattattgg gattctttat taaataaaga 66871
aaaacaatgg taggaaataa ggcagatttt atgtattagg agtatgataa agtacgcagg 66931
tgtgtgataa accatgagca tgtgaattcc ccttaacttt ttaaaattgg attttgatgg 66991
tatttcaact ggaagcacag gaggccttgt cctcctttag atgaccataa agtagtgtcc 67051
aattgtggat taatttctct tccacctgtt ctcactatgt tgtatcctgc catttctcaa 67111
gagtagaagt taatggtagt caccctaggg atgttataaa tctcacttta agactagtat 67171
tagagatacc acatgtgacc atttaaaaat gtttcacaaa gaaaatcaca ggatatttgc 67231
cagtcactgc ttttcctaaa accgttctgt tcataggaaa ggaagtataa ttgggaagtg 67291
aaggaaatgc taaaaatatc ccatattgaa catttttgaa tttaaacagt ttgtaagcca 67351
ttctctctct caaatttgga accaagtcaa gaaatttcag aaaacatgac cctagcatca 67411
attacatata accttcagaa ggccccccca tcataatggt accttgaaca atgatgactc 67471
tggtctcttc taacaccccc atctcacccc gcactccgtg aatccaagaa agagaaagag 67531
agacaccttt gtgcttccag gagtgacact gactcagcag tcttcttaga acaggcgcct 67591
tctccttcct gctcttgcct acttactgtt ctcttttctg cag gtg gaa tat gtg 67646
Val Glu Tyr Val
            965
atc aag tgt gac atg tca gct ctg cag aag att ctg tat cgc cat atg 67694
Ile Lys Cys Asp Met Ser Ala Leu Gln Lys Ile Leu Tyr Arg His Met
                970                 975                 980
caa gcc aag ggg atc ctt ctc aca gat ggt tct gag aaa gat aag aag 67742
Gln Ala Lys Gly Ile Leu Leu Thr Asp Gly Ser Glu Lys Asp Lys Lys
            985                 990                 995
gtacgttgcg aaagatgatg caactcaagg tgctgtggta tgtcttctca tggctgtgac 67802
attgaatatt cacaggaagc atccctgcta atgctgattt gttaaagtaa atcactggac 67862
ctcctttgag ctattatagc tgccaaagat aatctcttca cctcttagct gcattggggc 67922
aggaagaaag aaggaatatg ttcatattac caaaatagtt agctctgctt atgtcaattt 67982
tgcagcaaat atattctgcc taagtgtggg gtggcatttt agaaggtttt aaaatccagt 68042
ttcactccct gggcatctgt tctgtgcttc tgtgttccgg agtcctacgg aatagacagt 68102
catgtcgcaa agccatatcc ctcactggac ataatccttc aaggttcact ttttttgtag 68162

cgtaagttaa tcacaagaaa gacattatgt ctaactcagg aactgctata taattaaaga 68222
agctttgaac cacaaggtgt gtaggtgact gaaaaaacct atggtccttt gtcctttgta 68282
taagaagccc agtgtgaagg atctgaaatg tctgaccagt taataattaa ttctattttt 68342
ccctttccac tggttcttag ggg aaa gga ggt gct aag aca ctt atg aac 68392
Gly Lys Gly Gly Ala Lys Thr Leu Met Asn
        1000                1005
act att atg cag ttg aga aaa atc tgc aac cac cca tat atg ttt 68437
Thr Ile Met Gln Leu Arg Lys Ile Cys Asn His Pro Tyr Met Phe
        1010                1015                1020
cag cac att gag gtaagtctgt attgtgtgtt ttgagcctga ttgtgctaat 68489
Gln His Ile Glu
        1025
catactaatg ctagttaaaa aaaaacaaac aaacaggaaa aaaaaaaacc aaaatagatt 68549
taaaacatgt tggcggaagt tgagatgaca tgatctgata gctcgacaag cagaattaac 68609
aaacatgagt attctggtta tgggttctca ttggcaccaa gcatttgagc atctggaatt 68669
ttataaccaa gagtttaaag tggagagaaa atgctataag cagtggctca cttgtgtggg 68729
tttgggtaag tactgtgaat taccttgtta tactgggagc tctgaaattg aagataatga 68789
taaatacatt cagcttcctc aaaagaatgc cgtgaaaaat attaaaataa tgtatttatg 68849
acttctaaag agaaagattt tctttaaata ttttctttgt aaaagacact ggctttagtc 68909
tgttactttg atttatattt ttcctttctt gtttctgtca tgatgatgta aatccaggct 68969
ttcatcctga tgctcaggtc tctagaaagc tattttgcag ggagaggaca ttttctgcca 69029
tgttcactcc atgtgaataa gtttcctaaa ttgacagtgt ttcaccccag tgggcacaat 69089
tgactggagc aggaatgaaa aggagccaca attgtattcc ccatttgtca ttgctgctgc 69149
tgaggcaaat ggtcctggtg gcaattattt gccagggtag tgcctacttg ctactgcaaa 69209
atgggcagga gacatagaga acatcactgc agggctccag tgtctgcacc aatttatttt 69269
aaaaaataca tagaaacttc tggaaagtta aaacttaatc acagtataaa aattgagttg 69329
gagcactctt cttttcgaat attctttttt gtcccttatt tttggcagtt gtacatgtca 69389
ggtcagagta ttcatggcac accatatcca catgtcagag ttctttccta gctgtgctgg 69449
ataattggaa agatttctga cactgctatt caaggagtcc ataggagaaa gtatccatag 69509
acactgggga gtgtacccat agacatagga gagagtgtgt gggcatagag gagaatatcc 69569
atagacacag gggaaagtac ctacatactg gagtaaaatg acaacagcaa caacacttta 69629
aaaatctcat ggctgggtgc agggggctca cccctgtaat ttgggaggcc gaggcaggaa 69689
gatcacctga ggtcaggagt tcgagaccag cctggccaac atggtgaaac cccgtctaaa 69749
actacaaaat tacaaaacta caaaaattag ctgggtgtcg tggcaggcac ctgtaatcct 69809
agctattcag gaggcttagg aaagagaatt gcttgaaccc gggaggtgga ggttgcagtg 69869
agccgagatc gcaccattgt actccagcct gagtgacaac agcgaaactc ggtctcaaaa 69929
aaaaaaaaaa agtcacagtt atttacatgt attgttgaag gggattgcaa tggccaaaaa 69989
tgagttcata tccaccaggt cactttgtaa ctgtgaactg ggtaagtcaa ctcagattct 70049
tcatgttgta atgtgcttga tttgtaaaca ggggtaataa tagattatac tactggcagg 70109
aggaccagct tttaaacaat aacatgtgaa gcatcttgta aactgatgat gtgttatatc 70169
agatttaagg taccaatgaa ttggtggtgg gtgtatgttg ggtttagagg agctgggaga 70229
aaagtctaat gatgaaactg tctgggattt tcttagtcta acctgttacc cactgtccca 70289
tttcttctga aaacaatttt aagacatgta tggtaatcta tgggggcctt tggggactta 70349
ggaattttga agattaagca tcaatcttac ttcgttttgg ttatcataaa agaaatgacc 70409
ccttgagcca tgctgggcta agtagggaag gaagggagat gatgccttct gtcctccagc 70469
ttcagtttgg agggtgggaa aggatagagg aggaggggga atcatgaagg aagcgaagtt 70529
gaaggtgcat gcccttcagt acctacatat tgacatttta agtagcagtt ggtagaaagg 70589
cagactctga tttgcaattt ttttccctag aaacaagaga tccacagtta ttggcaaatc 70649
tattgacaac tatggaacag agatggtgtg tgaatttgcc tttgttgtgt ttcgtttgcc 70709
acacaccctt tgttcatact aaatcctgcc agatgctgag cctcaaaatg ggtctcactg 70769
cctccctggg gaccttaaac tcaagaaatg gaaacagtat ttttaacatg atgctgttac 70829
ctaatctcca acagcatcca ttgacccctc tctttggttt tgacaatcct gcttggagac 70889
agggatggag cctaatggag accatgggtt tggttctagg ggatggagtt gaggtattgg 70949
ccacagtcca tcctgttaat tttgccacag ggctggagcc taccgtagat catgagcata 71009
tttgagagta ctgttgagcc cagagggaaa gatctggtcc ccttggagga aaagctgcag 71069
ggatccaggg aggagggaaa gcagaaaagg gagacagccg gtggctgatg tgaaggtgta 71129
acctgctctc cattcccaca gatggttgcc attcccataa agtcagaatg gagaaatgca 71189
tggagaacag tcatttctca taaagtcaga atggagaaat gcgtggaaaa cagtatttct 71249
ttctgtgaat cagaaaggac ggagttatgt gattacgttc tgtattctga aaacagaagc 71309
tggacacttg aggggtgctg tacatctagg accttatttg taatggagga aagcacagta 71369
tagaggagac attaggattt cacctaactg gccaggtgtg gtggctcatc caataagcct 71429
agcactttgg gaggaaaagg caagcaggtc acttgaggta aagagttcaa gaccagcctg 71489
gccaatgtgg cgaaaccctg tttctactaa aaatacagaa aattagccag gcatagtggc 71549
acatgctgta gtcccagcta ttcaggagtc tgaggcagga gaatcgcttg aacctgggag 71609
gcagaggttt gcaatgagct gagactgcgc cactgcactc cagcctgggc aacagagtga 71669
gactctgtct caaaaaaaaa aaaagaaaag atttcaccga actaagaatt ataatatatt 71729
atataatcac aattattttt attgctgaat ttttccacaa tacatattgt attagtccag 71789
tttgatgctg ctgataaaga catacctgag actgggtaat ttgtaaggaa aaagaggttt 71849
aatggactca cagttccaca tggttgggga ggcctcacag tcatggcaga aggtgaaagg 71909

aacgtcttac atggtggcag acaagagaga atgtgaggca agtgaaaagg gaaacccctt 71969
acaaagccac gagatctcgt gagacttatt cacaaccatg agaacggcat gagggaaacc 72029
ttcctcatga ttcagttgtc gcccccaggg tcccacccac aacacatggg aattatagga 72089
gctataattc aagatgagat ttgagtaggg acacagccaa accctatcac atatatttat 72149
atatacatat taattctgat ttgcttgttt ttgtgctctt ctagtctaga ctctgtattc 72209
cttgagttca gatccaccct agagggtgga tctccagaaa taactattgg ctccgctatg 72269
ggccagtggg tgctcagtga ctgtgtgatg ggtaaagtaa ccctgttacc tgagagtcta 72329
gagactagaa gaaactcaca ggatactttt ctgatggaac aaaagtctgc atgcaatata 72389
ctgaatttct ggttcattac gtgtgtaagt tttttgctta ttcattaatt attttgtttt 72449
aactatagaa ataggtagga taacctcact aaaagagtaa tcattaagtt aataattaca 72509
ttttttaaaa tcattctttc tatctctctc ttttaaag gaa tcc ttt gct gaa 72562
Glu Ser Phe Ala Glu
            1030
cac cta ggc tat tca aat ggg gtc atc aat gg gtaaatctca 72604
His Leu Gly Tyr Ser Asn Gly Val Ile Asn Gly
            1035                1040
aatttttttt tcttttaaaa aaaaatgttg ttggccttac ataaactcct cctctctaca 72664
gtgtttcctc ttgtgcaaat acttacttcc agccctctaa gggctcttca gtggagaagt 72724
taaccaaaac ggtatcagca ttctctttta ctttactggc ataaattatt acttgcctta 72784
agcaagtggc tattcaaagg acatagatct tatttaatag acttaatagt atcctagtgt 72844
tctaaaaatg ttgttgaagc ttcaaaggtt tgttatttat aaccaaaagt atagcttggg 72904
cttgaagaga atgtatctta ttaaatttca cattgacatt tgacattaat gtacatgaga 72964
agaaatctct catctgctgt aaatgtttac agttttatag tcatttgtta gagtgtggtc 73024
tgtgtaagaa atgtttttca ttagcaatgg atgttaaaga gaatcttgac ttgcaacaac 73084
aaaaatgaac actgcattaa cagaaagtgt gtgtgtgtgt gtgtgtgtgt gtgtgtgtgg 73144
tgtgttgcaa atttgaaaaa tttattatta ggatgtagtc atcatttttg gatcccaagg 73204
gcggacagtt tcactgggct aagaaccagg aactatagcc tatcctgaag cagtgctgct 73264
aagctttccc tacctctcct tgcctctctc ttctctgggt tctgtggtta ctcctgctcc 73324
catcagcttg gccagggttg catggtgaaa acacagctat agggtatttg gagaacagtt 73384
ctcagagtgg aggatggatg tcgggaagac atacgtttga aaatgtgtct gttagaaata 73444
acatatcctg tgtcataaaa tcaactacca gatgagacat ttcagtacta tagattacat 73504
gtgcactggc aagttttcca aacagtatca cctaaaattc ggttttaatt gtataacaca 73564
gtcctgttga taaaagaaaa aaatgggtga actgtagagg gaaaaggccc tgctttagcc 73624
tttctccata tcacagcgtc ttctgcatct catactccca acgtgttttt gacttgtact 73684
tgtatagtaa tccaagactc ttgtcctccc ccagtccatt cttacatgac gtccatagtg 73744
atcttataaa aaggtacgcc agacctcatc actcccctgc ctaaaaccca ttgatgactt 73804
ttcattgcac tgaaaatgaa aacgttcagt atagctcata gagccctaaa gagtctgttc 73864
ctagtcccat cctccacctc tctgtcttca tctcccagta ttctctcctc acttctgttt 73924
aagccatgct ccctgttttt tttttttttt taatttctca aatccttctg tctcctttac 73984
ccctcaggac ctttatgcat gctctttcct ctccctggaa tctcctcctc tccaccccag 74044
tgcccccgtt cttcacctgg atgccttttt actcattatt tcaggtttta gctaaaatgt 74104
tatctcttca gggaggtcac ctctgacact ctaaaccagt ggttggcaaa ctttttctgt 74164
aaagatccag attgtaaata ttttcagctt tgtgggccat aaagtgtctg ccacacctgc 74224
tcagcgtagc ctctgtaaca tgaaagcagc cagacaatag agatgagtga gtgtggctgt 74284
gttctgataa aaccttattt gtaaaaacag acagcagacg gggccaaagg ttgccaaact 74344
ctgctccaat ctaaagaagg catcacatgt tgctgtgagt tttctcattg gaacttgtta 74404
ttttaatagt atttattaca gtttgtaatt atccatttag taaaatgaga ctctgttttg 74464
cttattatta tatacatggc tagcactgtg cttggtacat agtaggtgat cagtaagtat 74524
ttagatgaat cttaagtgcc ctgaattaaa aatttaaaaa gttatatact gaataaggag 74584
aaatttacag aaacatggca tatacgtgtg tgtgtacgtg tgtgtgtgtg tgtgtgagag 74644
agagagagag agagagagag ggcgaggaga aagaataagt tgagagaaag attgaaagta 74704
ttaagaacat gaaagtggtt ccttttcttt ctagaacctg tcatcatacc tagtatatgc 74764
aatgcacttt tcatgggttt tatatgaata tattttcctt ttctgctaaa tattctcatt 74824
gctttggagt tcatattcat tcattcattc atttcacagt tacttattga atgtttacag 74884
tgtacaaagg actatatgaa aagcaaagca atgctggatt ttttaagaag acagaagtaa 74944
tactgtcttc ttgtttttgc atttttttgg gttcag g gct gaa ctg tat cgg 74996
Ala Glu Leu Tyr Arg
        1045
gcc tca ggg aag ttt gag ctg ctt gat cgt att ctg cca aaa ttg 75041
Ala Ser Gly Lys Phe Glu Leu Leu Asp Arg Ile Leu Pro Lys Leu
        1050                1055                1060
aga gcg act aat cac cga gtg ctg ctt ttc tgc cag atg aca tct 75086
Arg Ala Thr Asn His Arg Val Leu Leu Phe Cys Gln Met Thr Ser
        1065                1070                1075
ctc atg acc atc atg gag gat tat ttt gct ttt cgg aac ttc ctt 75131
Leu Met Thr Ile Met Glu Asp Tyr Phe Ala Phe Arg Asn Phe Leu
        1080                1085                1090
tac cta cgc ctt gat g gtaagtgcat aaggcattag gctcggaagc 75177
Tyr Leu Arg Leu Asp

        1095
catactactg aaaatgaagg gataatgggc acttaggtcc aatctcagcc aaaaagaagg 75237
ggtaaaattg aagaattgac tagaagcatt gggagcagtt tttctagata gcaatttttt 75297
gcatccttaa gctttaaata agctgacctc atttgtgcag ctgcatagcc cacaaataaa 75357
ccaacacaaa tgagtttaga gtcacttttg agtacctaaa taaaataaaa ttaaactaaa 75417
ttaaaatttt aaaatcttcc ttgctcttaa aattgttacc tatgtgccaa agatatagaa 75477
tacattgaca cacctttaac tttttcagtt aaggcatatt ttaaggggat aaaggctatg 75537
tcacttttct ttgtaacaat acaatcattc atttaagatt atatttatca ttaaagcccc 75597
tctcttccta aataagacgt atccacatgt tacattgtta agagacatga cttcattgtt 75657
tcctggattt tgcaagctat gtaaaatatt tgcctggttt taagagtgaa atttaaatac 75717
cgtgtcctaa aaattagtaa gaaaaaatat taatggaagc aaatatgatt ttagggataa 75777
tccctaaatt attttacgat ttggatatca gccataatat attagaggtt gaaagtatca 75837
gcatttatac acaatggaag ggcataaata tggcattcaa agagtggtaa gtaggaatag 75897
aagttaattt acagtcagga tgctatttaa tccctgggta gaaaagagga tactgctaat 75957
accagtattt atctaatgca gtattactat tgttaaatgg gaaacttaag gtttatgttt 76017
cattaaaaga ctttattttc aatccaacat tttgttttcc tttttgcctt aattgaacgt 76077
aattggatag ataccaaact cagacttgcc gtatgttctc tgacttaagg ttttaggtga 76137
attctcttta tggtctgcct cttagggtat tgttaattaa ttttcaaatt aattatacca 76197
tttgacaaac ttgatttttt tttttatttt attttgtttt tattgagaca gagtctcact 76257
ctgtcaccca ggctggaatg cagtggcatg atctcagctt actgcaactt ctgccacccg 76317
ggttcaggtg attctcctgc ctcagcctcc cgagtagctg ggattacagg catgtgccac 76377
cacgcccggc taattttttt tttttgtatt ttttagtaaa gatggggttt caccatgttg 76437
cccaggctgg tcttgaactc ctgacctcaa gtgatccacc tgcctcggcc tcccacagtg 76497
ttgggattac aggcatgaaa cttgattatt taaacccctt tacctctccc ctccaaatta 76557
cgtaatgaat gaatggttac gtaaatgtca atttttagtt cacaaataca gtcctattag 76617
gcacagtttt tctctgttgg caaatttcct ttattgtctc ttctcctttg actggatccc 76677
tgcctccgga aagtggaaaa ttcggcactc ttctttcaaa agacaaactg tcctttacct 76737
gctgctcaag tgaggggctg tggggttgtg gggctgtatt ttcccctgaa ctgacagccc 76797
cacacaggcg tcctgcgtaa ctatcacttg ctactttgtg gcatttcatt tccaatctca 76857
ggtctctgac gactgcttct ttggaatatt tgcatctctc aaactggtaa gaagtgactt 76917
cctggaaaca aattgctttc atgttttctt gtcagcctga gaggttctct ttaatagttt 76977
attggctgct gtccttttgt tagtaatagt cacaacagct aacatgtatg gaacaccgct 77037
ctgtgggact ctgggccaag catttcatgt gcattattaa tttgattttc tcaataaata 77097
agctactgtt atccctgtca accagtgtgc cattatgatg cccatttaag gatgagaaaa 77157
caaaggcata tggtgaaacc aggttctaac ttggatctgt ttgattccaa attagctgag 77217
ttttagcaga tccagagagg tataccccat ctctgaaaat acaggcttct tctctagcta 77277
caacatttta agaatgattt tgaacttatt taaaattttc aattgactaa ttgcaaacca 77337
ccatgcacta gagatacagg atggatgaac tattcactgt tgctgctctc agagtcctta 77397
ccattaggtg gaaggagaca gacaagcaaa ttgcctaaca tgttacagat ggaattgaaa 77457
aggcatcttt ccgtaagaaa cagcagccgt tccttttaag ggtggacaga actaatggac 77517
aaacaatcaa tatacacaca gataagttag tacacacact tccagctgaa cccccttctg 77577
tcccccatcc caccacccaa ttattaaaat gacttcagat tttcattgct gaatttgatg 77637
tctcagattt tgactttagg ggaaaaaaaa tcacttgatg agagctaagc agaaattttc 77697
tgcaaagatt atggttgcat gaatagattt agctaatact ccatatagtt tttgttttta 77757
ctggaaatga tttttgagtg agagcaagtg gtgaggtttt atcccagaag acaacacttt 77817
ctaaaagcag gtgaaactaa gttaaaatta gtcatcatga aagtctgaca gtgtgttcct 77877
tctggtaaat tctgtaattt tcaacacagt tgccctatta attgtatgct tataattggt 77937
tatgtatcaa agtctgaaag accatgaaac tgatgttagt attgagcacg ttaaatcttg 77997
agccacttgc aaggagtgtc tgcctttgga tagactgtta catggtagtt tgaatctccc 78057
tgaacaatat taaaaatttt gttaaattta acaaaaaaaa tttgttaaat taaaaaaata 78117
tttgttaaaa agattgctga tgatcagcaa agatcttgac ttggaaatct ttgacagaaa 78177
gggttttcta gcaaattgca aaccttatga taagaaatgc catagctttg tatgaaaaat 78237
gacttaatct tttttgtttt ttataatttg tcaagttccc agctatttat acagtttatt 78297
taatttaatt ttattttatt ttattgagac ggagtctcgc tctgtccccc aggctggagt 78357
gcatctcggc tccctgcaac ctccgcctcc tgggttcaag caattcttgt gccccaccct 78417
cccaagtagc tgggattaca gatgtgtgcc accatgccca gctaactttt atatttttag 78477
tagagatggg gttttaccat gttggccagg gtgatctcga acccctgacc tcaggtgatc 78537
cgcccacctt ggcctcccaa agtgctggga ttacaggtgt gagccaccat gcctggccta 78597
tacagtttaa atacacacca gatatatggt tggcctgttt gcttgaggta tctgttatag 78657
ggtctgaaaa ctcctttgac caaataattt tatttggata ttccttttgt ttttcagtgc 78717
tttattttgc atgtaaggtt atttcatctc agtaggcatt ggttgtattt atatcttatg 78777
ttactggcca gatccacgtc tgttctgccc acgtgcagta aatcaatcac tgtgacacag 78837
gttttgcaaa agagaaaaga tttattcaca agggcaccaa gcataaagat ggaagaacag 78897
ccctcaactc tacctccctg aagataaggt ttagggatat ttatggggta aggaagtggg 78957
gtggtgtaag gcacagggaa aggtgattgg cagtggggaa aaatgaaatc acaggttcgt 79017
tctacacagg catagccagg gttcatggca tttcctagga catacataca aaaaatggca 79077
gagtcagcat gatctgatgg tggagttttt ggctctccaa catcaaaagg ccatctcttg 79137
ggcacttgcg ctcaggccca gtttaagggc cagtggtgtc aacgggtttg aactggacag 79197
agctggccaa agttcctgaa aaacaactga agtaaccatt accatggtga cgtatgcatg 79257

ttatctgtaa agtagccagt gaaggtcaag tttgagcatt cagcagcaag accttcagct 79317
actgcagcct tcagcttcat ggaaaaagag aaaacaaaaa taacaaaaag caagaaacca 79377
gaagcatgca gggcaggcag acctgatgag attaactgtt cggtttcatt aatccgtgtg 79437
attatcagct ggttcatttt cagttaatca aaactgttga acttctatcc tgagagtata 79497
gatgtatagt tttgtaccca gttttcagaa acacagatgc catgatctct tctttcccca 79557
ttttagaaga aatttttggt ttctgtggct tttcattcag tttaaggcag aggagaaata 79617
tgtgaaccat taatacatgt aatttttaaa aaaattcttg tagctggaaa gatgatagag 79677
aaggtttcac agcagaaata tcttccatta gcgtcgtgaa cagatagttc accatgatct 79737
cagaaagcca attctgaaaa taaaagaaaa aagagcccca catttgaaag ctcactcatc 79797
aaagcatgtt tagtgctcct cctccaaaat ctaaactcgg acagttgatt gtgtcggacc 79857
tcaccagttc actaaactcg aaacctaatg tagaaagtaa attgggtcta aagaatgtga 79917
ctccatgtga ctcaatgaga gcccccagac ccctcatttc taatccctgt ttgctgtatg 79977
cagggaagtg atttgttttc tccctcttca ggagggcatt tgttattttt attaaatgtg 80037
aaaaacagat cgtccccatg tttgagaact tggccctcat agtaacttga tttcattagc 80097
tatagtcact taaatgtttt aatcctctga aataaattgc tggtatgttt tcatccttaa 80157
aaaatgaaca gcatgtagga ataatatttg aagagcagct ggtcagagac tgtcatgtaa 80217
catgcataca tatttcccaa cagcctaaag ccttctttca aaattcagca tctaagcttt 80277
tctggtgaaa tatgttcaga tggaatactg gtgttttttt tttctcccct tccacaatgc 80337
atttcatgaa atggctgggt aaatacatta cacatacata atatgtgatt gagtggctca 80397
tctggaatgc aagaatgccc caaaagtgag caccagactc tgaggtttgg tactgtctgc 80457
ttattcacta ttttggagcc tcttcagacc ccatggggca aaccaccccc aagtatttaa 80517
catgctgctc tgtttagttg gttttgattt agcatgcttt gaactcaaca gagcaggatg 80577
ataacgtgag aaacagacta agagatttct tgtggggggg gtggggatta ttgtggcttt 80637
aagtcttgct aacaaggata aaattattcc atttgcaaaa aaaaaattac attcataatt 80697
tatgttttta ccaaccagac catacttagt agtaatccaa atggtaaagt agtagaaaag 80757
aactttcctt ttttttctat tttcttggca taaaaaaagt ccttgcttta atgcctattg 80817
gcaaagttta tttaagccat tattacagtt ttaagtgcac gtgtgtgtgt gtgagagtga 80877
aagagacagg gcgagagagg aagtgtgtgt gttcttaaaa ttatttgttg ttataaatca 80937
cagaaataaa tccaaaaaat gttttcattg ttttaggtga gagtaaggat tttgtaaaat 80997
ttgtgaagtt gcgaagatgc ctcattagaa atgtttaagc tgtttctttc tttttttttg 81057
taattgcaaa atgttcaaag ttttttatcc agaagtacaa agccctggaa attacctcac 81117
cagtgttagg aggagtctgg gtatatttct tgaaggaagc aagccttttt gtctcattct 81177
gtgccatttt cagacaagag ttaattggca aattaatttt tctgatcccc tcag gc 81233
Gly

acc acc aag tct gaa gat cgt gct gct ttg ctg aag aaa ttc aat 81278
Thr Thr Lys Ser Glu Asp Arg Ala Ala Leu Leu Lys Lys Phe Asn
    1100                1105                1110
gaa cct gga tcc cag tat ttc att ttc ttg ctg agc aca aga gct 81323
Glu Pro Gly Ser Gln Tyr Phe Ile Phe Leu Leu Ser Thr Arg Ala
    1115                1120                1125
ggt ggc ctg ggc tta aat ctt cag gca gct gat aca gtg gtc atc 81368
Gly Gly Leu Gly Leu Asn Leu Gln Ala Ala Asp Thr Val Val Ile
    1130                1135                1140
ttt gac agc gac tgg aat cct cat cag gtctgcatgt cccactcagg 81415
Phe Asp Ser Asp Trp Asn Pro His Gln
    1145                1150
tgcccaggcc tccctctgga gagcaactaa aagatgatca gtttcattat tcacatttac 81475
aggacagagc aaatatctaa acgagtgggc tgttgctttc ttggagccaa ttcttctggc 81535
tgttttctta tctcccttag agcatagata cgaggtccca catatgcctt tgaagagctg 81595
aagtttgagg tagaaaaact tagtttctcc ttagttttaa tcccatctct tgggattgcc 81655
attgaacaaa gtatatttaa tgagggataa gtcaaaaatg ttgcaaaatc atagcagtaa 81715
gaacaatagc aaccatcatt catgggaccc ttaatctgtg tcagcctctt gggcattttt 81775
tcattcagtt ttacgacaac cctgtcagac ggttaatatg atttgaatct ttgcagtcaa 81835
ggaaactgaa tcctaggcag ggtaagtaac ttccccaagg ccaaatagta ttacagtagt 81895
taacctttta ttttgtgttt tatttaaagt catcatcaaa acatattcta atgagcattt 81955
attgttgtaa agctctttta gccaggtaag ttcagggcta tccttttaaa gcagtacttt 82015
gatgttttgt tttttttttt ttaaattgag cgggtctctc atggcactgg ttttctaagt 82075
tactctggga ggtgaggcat ctgcctgtag gatattgggt agggtgatat tggtcaagat 82135
ttgattcata ctggagcaaa tgttgaattc aaaacagtaa aaatgaagag caggtgtggt 82195
ggctcatgcc tgtaatccca ggactttggg aggttgaggc aggaggattg ctttgagccc 82255
aggaatttga gaccagcctg ggcaacatgg tgaaaccctg tctctacaaa aaaatacaaa 82315
aattagctgg gcacagtggc acttatacct gtagtcccaa gttcctcagc aggctgaggt 82375
gggaggattg tttgagtctg ggaggcagag gctatagtga gccatgatcg taccaccgct 82435
ctccagcccg ggcgacaaag caagaccgtg tctcaaaaaa aaaaaaaaaa aaagaaaagc 82495
aaaaagaaaa tggcaactct cacgtaggag tatgaattct acatcagctt ttcctaggct 82555
cctcgttgag ccctggtttt ctcaacagta acactatctc actgggttgc gaagactgag 82615
tggcagtgcc atatagcgtc acttttggca ggaaatcaca ttcaggggta cattaaaatt 82675
ctaagggagc ttttaaaaaa tatgcatgcc tgctttctgt tccctgccac cgcaccccag 82735

ttttcttatt cggtggagct aggtgcttcc tttgagcgct cccaggagat tctgaaatag 82795
attctcatac agatgaggag cactgattta aagactcaac tgtgggccca aaccaccaac 82855
actgggccac tgcgtgtact taccattctc tgtaggtgaa taaattaaag cgactattaa 82915
aattttctga tcttgaaaag gattgggagc caccagcaca gaatatatta gttctaagtc 82975
ttcaggaaat atggttacgt atttttataa atacataaaa gtgtttcatt ccaaggctca 83035
ggtagaattc atattgatgt gagagatctg cagtttttct ttttagcatt atggattcag 83095
tatttgttcc acaaacattt attgagtgtc tgttatgcag caggcagtga gctaggctgg 83155
gtatctaagg gggaggaagg aagacgtgat gtgactcctc tcctcctggt gtgctcaatg 83215
tgattggatg tttagattat ccttgctggt acctaagttt tgaggacgat ctataggtga 83275
atggagagtt tgcttgtatt ctgtcttcgg aggtaagcat ttccactgac tcttagatgt 83335
ttaccttcac tggcacagta tacagtataa atcctaaaag ccactgttcc aggaaatgct 83395
aatttaacat ggaaaataac tctgggacat ctgtatgatg ttccacccca ccccgcctct 83455
catttttttt tttcgtactt tctctctagc tctgttaaaa actttgaact ctagaatcaa 83515
agtaaattgc agtaacatga aatagtcccc tcttaactac atgggaacta aagctttggc 83575
agcaaaacac aggatcccag attttctctg gtggaattta caacaggttc ctcacactgg 83635
gcctctgcaa gtatttctag ctgccatagg tagattcact tccacctgat tgaataaact 83695
gagctgacca gatttttcat ctgtcactga acaggaccat tagtttggtc aaatttttct 83755
ttttcaggaa tcagtgattt tatagtgata aagatccatc tgagttttca tttaaaaggt 83815
acagaactct actaaatatt gttaggcact ttgcatacct cacctcaagt aatcgtcaaa 83875
atatcttcta cataaggaaa ccagtcaatg ttagcttgct catgaacacc ccaccaggat 83935
cattcacacc catgtctgtt taggtttcag tttgaatact gggtttgagc tctttctacc 83995
ttgcaccctt gcatatagat tgtatagccc ccaaaatgaa aattgtgaag gtctttgtat 84055
caggttaatt ttagcatgca ttgaagaaaa atttctataa gtatgcatta tcatgttgaa 84115
tcaaaattaa ggcacattgg tgaaaatagg ggatttgaca taggttgaca gatagaggcc 84175
ttaatgttgc tgattgagga ccctacctag aagcaaagat agaactcgtg attgtggaat 84235
taacaaaaaa aaatcccacc aggttgccaa gactctcatt tccaaactgt tggttagaaa 84295
catgggttgg tactcgtgtt ttttacccac ggagctgcag ctgtgttgct attacggaat 84355
ggtcgaccac atttagcttg cagaccctct tctcagacca ggctcttagc ttcaagtatt 84415
atcagaccta agtcagtgtg atgaagtttc agaccaccga agtgagactt cagaaacatg 84475
gagtaatcca tccaataatt acagcacttc tttgtcacaa tattcccccc acattttggc 84535
ttagggactc aagctcacat ttcgttaaac ttctccagtc tagaaatatt agcttttgtg 84595
accttccaat tagatgggtg atctgaaaca cacaaacaca catcactgct accaccacca 84655
aacgaaactt aactgcagca aattcccaag gattctgtaa tgtctgaccg tgttcctatg 84715
ttgtccatgt gccctgcttc ctcacctgtt ctattcatat tctgctgccc cctgcaatta 84775
gtatttttgt ggttttgtca gcatcttctt ggacaagggt cagcaatcta ctactcgcgg 84835
gctcggtctg acctgcccct tgtttttgca cccccacagg tgaagaatgg ttttcacttt 84895
ttaaagtata aattagaaaa tggtggggaa aaagagtatc agccttctgg tgagaacagt 84955
tgttcaattt ttgcttctgg gccagacagc atagaacgtt tactattgcg cccattttag 85015
agactgttgg cctctgttcc aggagatagt gaaaagcctc atgacacaga ccttgtcatc 85075
ttgactgctg ttagcaggtc tggtgaagat atgtggagag ggacggttga actaaaagct 85135
taaaaaggaa ctttagggtg ttttaaagtt ctttgtcccc tttggatttg aatgatccac 85195
cttacattct cctctttaga aaataatcta atatagatgg gtcccagctg ttttcatggc 85255
ttagcactat gatatttata ttaacttatt caaatatctg ctgaggaaga agttttgcct 85315
aattggctac ttaattttaa aggtccctgc agcaaaaaca aatctccagg gcagcctttt 85375
gtctgctgcc ttcttgtgct acaacaggtg accaggagac ttgtctggtc aagaagacgg 85435
gtttacagct gttgagcatc agctctctgc aaattatggt gccagggatg tgggatacag 85495
aaaacagact tttgccctaa aaatctatac agtctaatgg gagacatgtg taaacaacta 85555
attagcatca gctcagataa catcacctgg accacaatga ataaaagcca catgccaaga 85615
agtttttatt acgtagctat aaaattaggt aattctgttt catgggctca tgaaggtttt 85675
tatttaaaaa tttcatgaaa gcttttttct tattattaat atatgagtaa catttaaagc 85735
agaaaaaaag caaaatgtaa gcaaagtgaa gaaagtgaaa taacacctat aatcttatca 85795
cctggagatt tacgttgttg gcatgttggt gtttgtcctc ctaccttttt ctttatatag 85855
ataaatttgc acatgtaaga tcagaatgca catggttctt tttaacttgt tttcacttat 85915
gtcattaaat ctcctacaat ataattttta tgatactttg ttgatacata catatagtag 85975
gatactataa ttcatttaac taattcagta ttgttgaatg tttctttttt taaaaaaaaa 86035
gccattagag agagtaaatt attctgagaa ggtacatcct tatttctaaa tctttgtgca 86095
tatctatgat tatttgttta ggatacattt ccgaagtaat atttctaagt cggagacata 86155
gacagtttta acacttctaa taggtagtga caaatttctg tccccaaaag tttaccagtt 86215
gactacccaa ctagcaatcc ttttgatttc ccagattgaa gaccgagtct ttgataaggt 86275
taggttcgag gaaattgact acctgagcag tcagtgtgtg tgtatgtgtg tgtgtggtaa 86335
gatgtatgta tgtataatac ctgaatgtac aactcagtga tatttttaaa ataatcttca 86395
acacatcacg ccaaagaaaa ggcgtttctt gcaacttaag gttagttgta ggtgttatgt 86455
aagtcataat tctgacattg gacattggtg tcttgtttaa tgttttagat atggactaaa 86515
tctgatgggg aacctagaga cagataaaaa caatatgata cctggatttc aaaacctgag 86575
atgtatttca gttggcttgg ttaatttcaa cccaggtttc ttcaactagg aatgacactg 86635
aatttttcca aacgtagctt gtgtgttttt aaaatgtagg caaaatctta ccttagtgaa 86695
ggtgaaatac agaacccttc catatttccc tctggggtgg ggtccggttt tggatgccta 86755
tgccaggcat ctcagtcctc atagcatatt gaccccccaa acag gat ctg cag gcc 86811
Asp Leu Gln Ala

        1155
caa gac cga gct cac cgc atc ggg cag cag aac gag gtc cgg gta 86856
Gln Asp Arg Ala His Arg Ile Gly Gln Gln Asn Glu Val Arg Val
            1160                1165                1170
ctg agg ctc tgt acc gtg aac agc gtg gag gaa aag atc ctc gcg 86901
Leu Arg Leu Cys Thr Val Asn Ser Val Glu Glu Lys Ile Leu Ala
            1175                1180                1185
gcc gca aaa tac aag ctg aac gtg gat cag aaa gtg atc cag gcg 86946
Ala Ala Lys Tyr Lys Leu Asn Val Asp Gln Lys Val Ile Gln Ala
            1190                1195                1200
ggc atg ttt gac caa aag tct tca agc cac gag cgg agg gca ttc 86991
Gly Met Phe Asp Gln Lys Ser Ser Ser His Glu Arg Arg Ala Phe
            1205                1210                1215
ctg cag gcc atc ttg gag cat gag gag gaa aat gag gtattagaga 87037
Leu Gln Ala Ile Leu Glu His Glu Glu Glu Asn Glu
            1220                1225
aaaccccaag tttatgaaat caaacagtgg ccttttgtct ctgaaggact cagaataatc 87097
cctttgttta aaaaacttcc tgggctggcg ttaatgaaaa tgaagaaata aactgctgtt 87157
ttagatccca accacagaaa gcctgtgaaa gttatccaaa atgagtgtct taagagctat 87217
ggctggagga acctcaaggc ttggcctgtg ggtgctccct cctttttccc agggtggtaa 87277
aaccagctgc tttattgaac catcagtgct gccttctgta gggcagatgc agaactgcag 87337
aggatacttt agggacaaaa tcagccggac gagacaagac ataaatggaa ggggtcctct 87397
ttctctcatc ctccagagcc ctctctctct tttgcctttt gatcagggat cacagagctt 87457
caacatgttt gtaggacaaa ccttcacagc catcttccgc acatttagct atacttgctc 87517
ttggcaagca tgaccttaac ctctacttta aatttttctt taagagctgt ctgaaggttg 87577
ggaaaaattc tggttaaaca gcctttattt gtcagggttg tatgaagaat gctttgatta 87637
ggtttttatt aggggaatgt tgagttgtca tttgtattat ttttagattg ccaacgttag 87697
taattttata aatattcatt ccaaaatgga tttgaagtag atttttaaac gtatgtatat 87757
agtgtagcaa actacagaat atcagcagaa gtccttatca taaaactggc tgaacatatt 87817
ttttgtgtgt gcccatactt acaaattgaa gagtaagtct accttacata ttcatgtgga 87877
atactgtttt cattgcatat attagatata atttgatttt tgccttaaat aggatatgtg 87937
tttttgcatt cttctataaa ttatgaacct gagtctttta aaagtcttat aaacaaatac 87997
tcctcaactt acaatggtgt tacatcccga caaacccatc ataaattgaa aatatcagga 88057
catggcccca tcttaagtct agtggagtac tgaatgtgca tcccttctgc accattataa 88117
agtcgaaaaa tcctaagtcc agccgttgtt aagccagaga ctgtctgtat attttttttt 88177
tctaaaatga atctaaatgt gaagtaaagc ttgatattta aaggtatcct gaaatgaacc 88237
catttgcaaa ttgaagagca cttttgtatt atgcctccta atttgtccgt cttatatttt 88297
tgattagcta tccactgagt ttcatgaaaa tgataaaaaa tacatatgca catgcactca 88357
ctatccacac acatttgaaa aatttttatc tcaaattatt tatagtttct gattctagaa 88417
attgaatatt tgatacagat atggagtcaa attgtcaggt gatttttttg ttgtttatac 88477
acttggacat ttccaaaaaa acaatatggc gaatgatcta tcctgtgaca aagcagatat 88537
gagcagagct gacactcagc cagtcgtctc cactacattt ttgcttattt gttttgtgta 88597
ggccttgtga aaatagtacc tgatctggtt gagaatcaag gaagcctgga gttttccagc 88657
cttcctaagc cgcagggagt tgctgatctc ccctgggccc gagggctgcc aggtggggcc 88717
tgacgtgcac tcccctccca gttgtcctgc catcaaggct agctgtgagc agccttgctg 88777
aggctcctgt cccttcttgg ctgccagtgt gtcagaaaaa tgttgccgtt ttcagggggg 88837
cttgtgtcct gatgtaaaga tgtttgaaaa cactgagaga ctgcagtcaa agactctggt 88897
tctgaaagat caggacaaca taggctggca tcaccatggt tgatgcgacc atgaagtgct 88957
ggggcagaga acccaggaag atgcattgtg cacttccagc tatttggttt tccatgaagg 89017
tcataaacag gcactttaca agagaatctt ttcatcttgg gcatggggtt gtctgagccc 89077
ctcatggtcc aggtgatgag agggtaatgt gtggttctag gaagaaagta ggcgaagcgg 89137
gacatctgtt caccagagga gacattacca gatattttgt actgtgaagt ctgaacccac 89197
tcccagctag tctgtgtcct tgtcagatga aatgtaatat ttagacaaat tgactttaat 89257
caatctggag gcaaactaga atagaaggtt tttcagaaat aattattatc ttgaattgta 89317
gattttttta ctttattgga ttgcattcat ttctaacact tattaagttc ctgcagtgcg 89377
aaagacgtta tcttgggagc cgacacagtc atctcatttg agccgctagt acactcatac 89437
tctttctcta aaacaagttg cctctttttg aatatacagt ttgtgaaatt ggatgtctga 89497
agttctgtgt tttcctcttt cttccctcga ctgatacact gaaattcccc gtttgatttc 89557
taacaagccc actcagcata tgataaaaat aatttgggaa gataaatcct gaagcatatg 89617
ccgaactttt agaatcattt tttaagtgtg gaagtcatcc tataacattg ctaaaaatcc 89677
tgagaactgt tggtttctga ggaattagca gagagataat gtacctttgt ttgacgtaat 89737
ataaaatgaa aattttagcc catatgtaat tgctttctct gcttcattta aattatttta 89797
attaagtata attttgaggt tttttttgtt actgatacct ttttagttat cccatccata 89857
gctccaatca ttaacaataa tacccataga aatatattaa gcacacttca aaggctatat 89917
atttggcaca tatatgtgat ttttattgtc ttagtgccta gtaacaaggt ttcacatact 89977
tatgtctttt gtaaggattt gaaaataagc atttatattt ggcataaggt aaatggaatt 90037
aaaagaatta atgttaggta ctttttcata tggatgtcct tattaaaatc ttacatcccc 90097
atgtgaaata gatgtcatac ccatatgacc aatgaggtca ctgaagcgag gagacattaa 90157
gtaacttgtt gtaaaagtaa cagaatcaag atacacatcc aggagaactc atatttctta 90217

tagtggacca cagtttcctc cctgaacgga ttttgctctg ggaagatggt tttaatagct 90277
tgtaaaatag taacgtcaag gactaaatcc agcaacccct tccctttctt ttctgccttg 90337
agaaatggga cccctctggt ctggaggaca gatcacttac ttgtttgtac cttgccccag 90397
ctgtccactg gttaaaatca ctctgttttt aaccccag gaa gaa gat gaa gta 90450
Glu Glu Asp Glu Val
    1230
ccg gac gat gag act ctg aac caa atg att gct cga cga gaa gaa 90495
Pro Asp Asp Glu Thr Leu Asn Gln Met Ile Ala Arg Arg Glu Glu
    1235                1240                1245
gaa ttt gac ctt ttt atg gtaatgttac agaaaatcat gaacacaaat 90543
Glu Phe Asp Leu Phe Met
    1250
gctttatacc tctgcccctt tttctgttaa gcagaatgtc tgcagcctgc gtgcctggca 90603
gaacttcata cgtaaagcct atgcctttcc ttcagttcag aggctgcaaa ctggctggag 90663
ttagcctgca gacatatttt gtttggccca cgcagcgttt tttaaaattt cccattcatt 90723
gccaacataa aaaagaatcc tgatttctgg cttctcttga aaaatgagat cgggcagtac 90783
caggcccaca tctttgtgtg gaaacagttg gccagggctg agtagcagct gcactctctg 90843
gacaggcctc gcggtctaca gcttaccata gacgccctcc tgttcccagt tcgtttctaa 90903
tcccaggcca gtgtcattca ggaagcctac ctggctccta taggcattgg aacttacaac 90963
agtgccctta caggatcaaa gcaacatgct ccttagggag ataggtggac aacagctaaa 91023
acaacacaca ggtgtgcctt ttcagattgt tgacgagagc ccgcttaaat gaatgggaac 91083
cttcaaggtg tgcataaagg tgtagccttc atttaaggat caaaatcagt tggatttccc 91143
tccagtatct tcaatcattt tcatcttcat caaagactga ttacagagag agtaaatata 91203
aaccacaact ttggtggttg aatgctgcag aaccctaaag ttagaggtcc caaatttgag 91263
tgccatcaga tcttacatga gtaatttgca tgtgtgaagc aagtgggcag aggtaggtgt 91323
actggagatg ctttgtctat ggggggacag agctgcaagg tagaaatgtg agacctatgt 91383
cgccagaaat gacaaatttt caaggggagc ttgaagtcta ggtgtttata tgtgatctct 91443
agattttaaa acactgcaac taaatccaac tttttaagtt attatataac actgcatgcc 91503
aaacaaaaca caactgtggg ccaaatatga tccacagggt ttgcaatctc caccttttta 91563
gcctagacag aatggaactc cacctgtttg gagaggctta tgttattttg gggttttgtg 91623
tgtgtggctt ttttctggga acattgattg tatataatga tccacaataa aattttcttt 91683
ctcaggaaaa ttcaggtagc tgtgggaata actactaaac tggaatatgc acttcccttt 91743
ctccctccgt ccccgaaatg aaccaccctt cccaagtaaa gctatttcag agaggaatgg 91803
tagtaagaaa gacttaacag catggtcaat gtagggcatt cataaattct agatttctta 91863
actcacactg gtcagcataa tcaaagatta caatattctc tatttaggga ggttaaagat 91923
acccatcaga aatagacatc ccctctgtcc ttgaagaact aattaaaagg cagattactg 91983
gggaacaccc aagcaagaga ccctcagctg atcactgtta ggcaataatt cagcactgct 92043
aaagccgcag atactctcaa gacactggag aggacaagct gcttgccatt atacaaatta 92103
gattaggccc agaaatggag aattctttca gcagccctga agtaaggggg tgagtgtact 92163
ttcctgcctt acttttgggc tatttgtaga aattgaaaac ctcagaaaca atctgaatat 92223
cttggaaggc acagccttct tttaatttgg gactatttta ccactcaaag aacagggttg 92283
taacacaggt gaggctggct agggaacact ttttgtaggc actttgattt tgtgaatttt 92343
ctgctggttt cttaaaggct ggcttctatt ttgaggatta gacagatttt aggtttggtt 92403
tagtttcttc taatccaccg gccgcctttg gccaattcac tattccttag cacctccact 92463
tgggggcagg aaataaaatt tgaatctctg ccagctacag attaaaaagt ttggtgagaa 92523
acaagaatgg aatgtgagct agaataaatc tttggatttt aaatatttaa actctttatc 92583
cattattaga aaagagtgag ttctctataa aattaatgca aacttattat ttgattttgt 92643
atcatagaca ctggtttaca catgataagc agatagttta tttaaaataa cgtcttttaa 92703
aaaaagcatg tcttgctttc tctttctaaa aatgtaactt cagaaaaagt atcttttata 92763
ccaattactt aggaatttta ccaaaaagat ctttggcagc tcaagataag tatctgagac 92823
attttgctaa catacaaaca ttaagctttt agatgctatg ttctctacaa agggaaaaag 92883
cacttttaaa actaattgta cttgtaagca taaggtttat aatcagatct caagaccaat 92943
tattcacaaa gtaactaaat agtaagtttg acttttgcca taatgttgaa ttaattatat 93003
tggtattttg tgggtagaaa tctctaggga gagatgcagt tattaagact ttaagcttat 93063
cttatgtaat cgaatctacc tttcctaaaa gaaaaaagaa acaagtgcat aatttacttt 93123
tctatcagaa aagatttatt ttctcaattg tggacaaata tattagcaag aataatgtcc 93183
atagaaatgt attgattaaa catacctcaa agactatttt tttgctgtag ataatagtag 93243
cagtaaaacc aaagattgaa atggcagtat tttgtcatct cagtatagga cattaacaaa 93303
attttttttt ttcttattag ctctctaata actattagga ggttgcctca cgaagcccct 93363
ctttagaact gctgtaaaga aggcaacctc tgtgtgccag ttatgaaggt ttggtggatc 93423
catttctcat accaaaacat tagaagacag gattaagcat cgtgagctaa ttatagcaca 93483
tgacagagca aggtggccac atttatcaac caatgggact tcacctgtct cccaagccta 93543
ccagattctg gaaatgtaat gacttccaaa aatgtggaag aagtataaag agctttgacc 93603
actaatttta aaataggttc tagaggtcat atttgggttt gcgtagatat ttcatttaca 93663
ttttatcagt tttagctttt aagaaaacat aatattgaat cccaatttca gtatggtaga 93723
accaaaggaa gaaggaaaag gtaaataaca atgattgatg gtccatttca ttgtggaaat 93783
gacacatctc gctagtgttt atacataatg tctgtggata tatagtggca gtttattttt 93843
taaaaatcca tcgtattgtt tagttataga ttaaagccaa gtgtgtggcc cttagtttat 93903
ttacaagtga actggattat taaaaagaga gaaggtgggg aagtaggcat tttgaaggct 93963

ctgaaaggaa tccatttcac ttacaactag atcagttggg atgcgattac ctctgagctc 94023
tctctgtctg tggtataata tattcataag agcacaaagc ttaaaatgta aagaattgaa 94083
gagcattcct cagacagtgc ccatgaggta ttgaaaagtt agtatattca tggccaatgg 94143
accagtgaaa ccatttatgt ctcttccaca tgtccaactg tgggtagcta ttctgtactg 94203
ataccttggg tccccctttt ttttttattg ctgattgttt agttcaaagc acaagcccta 94263
ttgaataaaa ggaaactttc ttaaagatct ttcttttctt tactcaaaga ataagtttct 94323
tccagggaag caatgagttg tatagccaag aggtcatatt tttaaaatgc attcccaatt 94383
tgaatttttc cactattact ttaaagaggg agctttgcat acacacagac taagaaaagg 94443
aaattcttta aaagtacttt ttaaatgtat gaataagttt caaaaggtgg gtacagaaaa 94503
agggagggga ttttacttaa tggaatctct ccctagatat ttagggatga gctagaacaa 94563
gccaaccagg atgagagagg ttgaaaggga ccctgcagcc ataggaagtg acttggggaa 94623
gttgtgtagt gctgggaagt ctgcaccata cagaagccct gactttcggt gaccctctta 94683
ttaatgtctc cag cgg atg gac atg gac cgg cgg agg gaa gat gcc cgg 94732
Arg Met Asp Met Asp Arg Arg Arg Glu Asp Ala Arg
1255                1260                1265
aac ccg aaa cgg aag ccc cgt tta atg gag gag gat gag ctg ccc 94777
Asn Pro Lys Arg Lys Pro Arg Leu Met Glu Glu Asp Glu Leu Pro
            1270                1275                1280
tcc tgg atc att aag gat gac gct gaa gta gaa agg ctc acc tgt 94822
Ser Trp Ile Ile Lys Asp Asp Ala Glu Val Glu Arg Leu Thr Cys
            1285                1290                1295
gaa gaa gag gag gag aaa ata ttt ggg agg ggg tcc cgc cag cgc 94867
Glu Glu Glu Glu Glu Lys Ile Phe Gly Arg Gly Ser Arg Gln Arg
            1300                1305                1310
cgt gac gtg gac tac agt gac gcc ctc acg gag aag cag tgg cta 94912
Arg Asp Val Asp Tyr Ser Asp Ala Leu Thr Glu Lys Gln Trp Leu
            1315                1320                1325
agg gtaagcctag cttttctaac ccgctctcac taggtggagg gtttttggtg 94965
Arg

gcttggagaa accaggggcc tagagctggg attttctgag gaggttgagt tcgcaatcaa 95025
agggaagggg ctcttgaaca cagaagataa agtcattctg ctgttgcata aagtcattgt 95085
gctgttgcat gaaaagataa aggcgttctg cacttcaagg cggtgcttgg aaaaaaatat 95145
gcaggatttt ttgctagagc agaattggcc attggaagca caggattatg agagctgtga 95205
gacccactaa tgttttatga ccaattgcct ggtcatgcct cactctctcc aactgagaat 95265
caaaatgtgg aagggcacct tagagatctt tgtaattgtt ccccctcatt tcatggtctt 95325
taatcgaggt tttacttctg caaggtctgc acagatcttt cagggatagc agagttttct 95385
aactgtgtgg ttattttgta gacccatagt acttttagag ctggaaggta cctttgtact 95445
tgcttggttt gatgaagaaa ctgaggccca gagaggtgac atggctatgc aggacacaca 95505
gctggttaga aagcacagaa tcggtgggtg cttatgtgtc tacatgctcg atttccgctt 95565
gatctgatct gtccatcaca aacacagatt agagcctggg actcgggtca ccgatgcttg 95625
gaaatgttat tcgggagttt gtttatgttg tttttccttt ggaaaatggt gatttctcat 95685
tttgattgag gactgaaatc tcagaacgtt ccttgggcag cacagaaata tcaaaatgct 95745
cttaggagtc gcgtgtgtga acactgaggc ccgggtgact gttccattcg aagggttagc 95805
tctacttccc atttttggga gtagacttag tacctagaaa ggcacagcaa cctgggacgt 95865
gcattccaga cacagccagt aaatgcatgc tggaaatagt aagtgtagtt gggtaaaaac 95925
caagcaaaac aaaagctttg gggagttgtt cgttttatcc aaggggagta cagttcccaa 95985
gctttgtgaa acatggattt taggacccag gttttaagta ggtagatggg tagaaaggca 96045
gtgggcagaa ttttgagatt agataccaaa ggcaaacagg tcttgtcacc gcttgagatg 96105
tgatttactc aggatgttgt atgatgagag ggacgtgacc tgaattttga aaagatggga 96165
gcctgtgatt ctgaaaagca ctgctcatta tttgtggttg aaccgttgtg tataaaatag 96225
aacatttgtg tgattagttt gtcgtatcca gtcaaatatt ccagtgacct cagaatatga 96285
ttctgtgaat tttctttgtt tcactattga gattatagtt atgtaattag gattcattct 96345
gtaatatgaa gagttttttg gctaatatat atttctgagc ttgggaaaat tttatttaag 96405
caataaattg agtacctgga gtattattac agcagacggg acaataataa tctgggccaa 96465
cctttttttt tttttttttt gagacagagt ctctctctgt cactgaggct atagtgcaat 96525
ggtgtggtct cagctcagtg caacctctgc ctaccgggtt caagggattc tcctgcttca 96585
gcctccccag tagctgggat tacaggcgca taccaccatg cccggctaat ttttatattt 96645
ttagtagaga cggggtttca ccatgttggt caggctggtc tcaaactcct gagctcgggt 96705
gatccacctg ccttggcctc ccaaagtgct gggattacag gcgtgagcca ccgtgcctgg 96765
ctgggcgaaa tttttaaagc taagctttga tatgtttact ctccagacat taaaagcaaa 96825
ataaccttgt ttattgggca agaatgtagc tgaacttaac ttcttaaaaa taatcattgg 96885
gaattgccct gaagaaaaac aacattatga atctcaggaa gttttccagt ttaagatatc 96945
tgttttgttt tctctttttc attcagtttc tatccctgat gagtagcaca gtgcctagca 97005
tagagactca gtgaatgttt gtgaaaatga aagtacagcc ttaagaggaa agggaaagtg 97065
actaacttat cagcagtgtt aggagagcat ttctcttttt agcgcatcta gtaaacttcc 97125
ttttatctac aacatgcaca tttaaaaatg gaaaattagt ttgttttaca aaactgcact 97185
gtgaaaattg gtttttataa ttgtaattct gaaatgccat gcaacatact aagtcatcca 97245
agaaggacga aactgttctc aaaacagctt tattgtaacg tatctttttg taatctaatc 97305

ttttgaaaac tgcatgcccg tatttggtat gaaactggca acttgttatt aatcaggaca 97365
tgcaatggat gcaacatgcc ccatggccta agcattcaaa gtagatgctt cctgtctttg 97425
gtgttcacca gcagcaatcc tatttaatat cagcctgtta agcttttttg atgatgaaat 97485
gatattgaag gggttctcat gggcctggat gtgaagagat gttctctact ggcttatgat 97545
ttaacacatt gcatgaaccc agccaacatg gccttgaaag ggagtttggc aaggaccgtc 97605
gtgtaagaaa tgctgtaaca gggctaaaat aattcttctt tttcctggca atgttgtaca 97665
aagcctgctt ggcagagaca tccaagatgc atagcatcgc agagctgcaa ggcagttgaa 97725
tggaacagct tcccatccct tgcttaaatc acattctctg caatctctgt gccaggaggc 97785
tggccacccc tagaaggaaa aactaccttt ttgtctgtgt agcccctcca gttcgtggtc 97845
agctctgatt aatagttctt cctccctttg aatagaatct tctttccttg tagcttctcc 97905
tcagaggcct tgattctttt ccctggggtg aagagaacaa tatttttttc tgtctttagt 97965
atcacagccc catggataaa atgtttgcaa actcatgttc agtctggatt gaacttttct 98025
accttcctct ttctcttgaa ctttcctcct ccttcaaggc ccaattcagt tgctacccac 98085
tctgtggcac cttcacagat aaatattaat tgcactttta attgtcctct taacatttct 98145
ttgcttgtcc ttcagtcaca gtttcctagt attctcggta gttgccaacc tgcctgcttt 98205
cctgactagt tgataagctg gctctgtatc ttgttagaat ttaggacttc tgtgtacaaa 98265
tactaagtgc ccatcagttg ccaaattaaa gtagcactgg attgacagat aagggtatag 98325
atagatgtgg gtaatgttca ctgctgccac agatggacct caacatgccc tgtccatgga 98385
cctatccaga aaaataaaca gaagttgatt tctcccttcc ctgaaagtcc atggtaggta 98445
tacctgattg gtgaggacag ctgttctcca tagggtcatt cagggctcca ggctgacaga 98505
gcctctgccg gcttggtcat ctccatccac ttgagctgga agtggggaaa acaacatgga 98565
gtagtgggta tggaagtcta atgggccctg cctaaatggt tataattcca ttggctagaa 98625
cttggttatg tggccactcc agctgcaaag gagatggaaa tgtggtctgg ctgtgagccc 98685
aggaaaacaa aattttggtg aatatttagc catccctgcc tcagcacact taggttaaat 98745
ttaagtgtta tggtcacttt ctttgccagt ggtgttttgg tttcattgaa gacaggtttt 98805
attttgtaat catggcatgc taaaaattcc tacagtgtcc tttagagaat ctgtttagtt 98865
ggtggaattt agaatttttt tctgcattca taatgttgtt tttaatcagg aggatcacaa 98925
tttcaaacac agttcagttt tttttttaaa ggtattttac attgttttga aattcctatt 98985
tccactgagg aaacttagat gtccttagtg ttagctggtc tcaagttaac gtcatctttt 99045
tgtggtagtc agttcaacat tcacattctg ggctgtttct ctcttccccg gggctcccca 99105
tcccatcctc ctctccaaga tggcatctgg ataaagacgt ggtgtttaag cacggcattg 99165
ggggcgagca tcctggttct cttgctcccc atgggtctcc actgatctca gcatatagct 99225
gctggtctgc tcctttcctg ggaggtcttt acatgcagct ggtttcatct cattctttta 99285
gagaccttga tgacatgtgc tgttgaaatc tggagcctgg ccacaggcac tagttagaag 99345
ctgtcctgca ttctcacatc tcataagacc cctggcctct ttggcctcat ggtgctctta 99405
gctcttttgt gttctgtttg gaagtctgca aactctcctt gactgatttc tccacactgt 99465
gctagaagat gcagggtgca gtgaaactgg ccccttcctc agcagttgct aagtcagcac 99525
caggggttgt caaacctgaa gcttctagaa tctgatggtt gggtgggtcc tggagctttt 99585
taaagaaagc aattcaaaaa tgtcttaagt ttgcaaattg tataaaagta tgtaactgta 99645
tttgggatca catagtgagg gatcctccca gagctttgga aggagcttgt gagtgagagg 99705
cctaaagcct aagcttcatt agctttccaa taaatccacc tttagcagtt ttagtgcctc 99765
attatcttcc tttccactca gtctctgcct ccaaacaccc caggcttgga ttctgaaggg 99825
ggagtattgc atttcaaccc agctgcaatt cagtgctggc acaaactagc cagacttagc 99885
acagactcca taagttaaag tgcatagtcc ccaacaacac tgccctgact tcagacacca 99945
gccacacttt gggggtcccg aggcccccca cacttttgac tgactacaaa ttcaggggct 100005
cgtgaaccct ttaggcttga taatttgcta gaacaactca cagaactcag gcaagtgcca 100065
ttagcaacgg attcggattg attatgaagt atacaaatca agagaactag gccaggcact 100125
gtggctcatg cctgtaatcc cagcagtttg ggagggcgag gcgggtggaa catgaggtca 100185
ggagatcgag accatcctgg ctaacacggt gaaaccccat ctctactaaa aatacaaaaa 100245
attagccggg cgtggtggca cgtgcctgta gtcccagctg cttgggaggc tgaggcaggg 100305
gaatcgcttg aacccgggag gcagaggtta cattgagccg agatcgtgcc attgcactcc 100365
tgcctgggcg acacagcgag actccatctc gaaaaaaggg gactagccaa atgaatggaa 100425
gcacagcatg cagtctggga ggaaacctgg gctcctctgc ctgctcctcg tggagtcagc 100485
gtccatcagt cctccagctc atcagtgtgt tcactaacca gggagtgcta ctgggccttg 100545
atgtccagag ttttcaatgg ggtttcacaa tgtataggca tgattgatta gattgtcggc 100605
cacatgcttg aactcattcc ccagcctctc tgccctccct ggaggtcaga ctggctcaca 100665
tccccaaccc tgtagtcatg tggttggtct ttctggtgac cagtgcccac tggagctgtt 100725
taggggccta tcacacatga cacttcatta tcagaacaaa gttactcctg tcactacgga 100785
gatgtcaaac atagaaactc catgtcagga acccaggaca aagaccagac aaattctttt 100845
ttttgaggca ggatctcact ctgttatcta ggctggagta tagtggtgta atcttggctc 100905
acagcagcct tgacctccca gactcaagca atcctcctac ctcagcctcc tgagtaccta 100965
ggactacagg tacatgccac catgcccggc taattattgt acgttttgta gagtcggggt 101025
tttgccccat ttgccaggct ggtcttgaac tcctgagctc aagtgatcca cctgcttccg 101085
tctcccaaag tgttgagatt acaggcatga gccaccatgc ccagcctgac agattcttta 101145
ttatataata ggtagtaata tattcaatcc tgaccttact tctcttcctc ttgactcctg 101205
tttcctccat ggaagaaggc tctcctcctt gccttcctgt tacgtggcaa cttcctgttc 101265
ccgagtgata gcaccatgtg ctgtggcttc ttgtgccatt ctgagaatcc cggtcctcgg 101325
ccttgtcgtg gccgagtggt taaggcgata gatgagaatc ccagtccgct aggtttggct 101385
cttgatcgta aaggtggtga agggaattca ctaccctatt ttggaagtca aagatgttgt 101445

catttttgtc tctgcatacc aactttatcc taattctgct caaggcaatg agtaactctc 101505
ccgaacaagg gatggttctt ttagaaagtg tgaaagagaa tattaacatg gttaacaaat 101565
attccatcca ttacagtttt gtaactttcc tatggtgtgt gcccaaggtg ataacagtgc 101625
tatattacag attacatata tatatatttg catgcgcaat tatacatata catacataca 101685
tacatatata tatacacaca catatatata tgtataattg cgcatgcaca agaaatggcc 101745
catcttgata atttcttgga aacatatatt tgcttcttta tatgtaagag caggcattcc 101805
atctacctaa attctgacaa gcccagtggg ccacggtttt ccggtttaat gtcagccctg 101865
tgtacactgg ctgtgctgtt tctagcaccg taaggcagtc atcattattg agccaggggc 101925
tgtgcaaaat tggggtgatt gcagagccaa aaagcgggct ggagggtggt ttctggaatg 101985
cctgttttac tattcttcct gttcctttgc tctttctgcc aacttcagaa tccttccttt 102045
ccttaaaaca tttagtgata tccctgaatt tatttaattg ctacgtgagt gatttttgcc 102105
ccacagcact ggaagataat tggaatgcat tctagcttat gtgctctgga taatatgcaa 102165
ttaccagcca gctggagagt agtatgcctt tgtctcacaa tgtcttgtaa ttttaaaaag 102225
tttctcctat ttgctgttgt ctcttgttac ctttcttacg gcatcatata tctactatgg 102285
ggtccttatg tctgaatgtg tagacacttc ttttggtata ctgaataagc tcctggcgat 102345
gagggaagac accgctttgt tttgtgatgt cctgtgcagt tgggccccag taaacgctac 102405
ggaaatgatg gtgatgagac ttatttcttt agatctttaa attgggcgat agataacagc 102465
aagttttaga agccatagaa tttcaagagg aactttgcag aaagacagaa caccctcctt 102525
ttccttgacc agtctgtcac caggtgacag cctgagcttc ccatggtgaa cgtgcctaaa 102585
tggggttccc atcatcatgt ctgctgacta ctaaaataat tttggggttt tcttcttccc 102645
aaagtttaac aaagtagaaa aaacaaaatg aggccgggtg cggtggctca cgcctgtaat 102705
cccagcactt tgggaggtcg aggccagcat ggcgaaaccc cgtctctact aaaaatgcaa 102765
aaattagctg ggcgtggtgg cgggtgcctg taatcccagc tactcgggag gctgaggcag 102825
gagaatcact tgaacctgga ggtggagccc gcagtgagcc aagatcacac cactgcactg 102885
cagcctggcg acagagcaaa actccatctc aaaaaaaaaa aaaaaggaaa aaaaaatgac 102945
atttataata ctaggtgcta atcagtaagg taaaatgtga tacatctcaa gagaatttag 103005
tgtctttaaa aagctatgta ataccaagct gcaataagct ctctgagttg gcctctcatc 103065
acaggaagcc aaactgctaa actctacaca ggaaaaggca gacaaatcca tggtgaatta 103125
gtcacaaaaa actggggaaa atacgaagaa aggttaaaga acaagacatt tgaaattcta 103185
tggaaataca caatgataaa taaaatattt cctcacaggt gtaaagaggt gggctgtgga 103245
tttttctctg cctagagatc ttgcaagcaa gctaatgcct cctctgggct gcattaagta 103305
tgatccagcc tgaaatcaca gacaggggct tgaatcactc agctctgttt ctcctgacag 103365
cattagtgat tctcgtagat gtacttttcc cctaaaatat tttattttac actggcaaga 103425
tttttttgtg gggggggatc ctttttctaa agcataatgt gcatcatgtt tgtaatattt 103485
ggctgcattt gttttgtcat ttaaaatctt ttcttgcttt ttctgtaaga tgtgagaagc 103545
tttgtaaaca tggacccttt tttcttttat caaatggcat tttaactttt aaaattgggt 103605
catcagattt actggggcaa aattttctag caagctgcat ttccgttttt atacacaagg 103665
gagagctaag gccctagaaa atagatttgg gttttgccaa attccaagtc cttcaatact 103725
ttagggtgga agtactgaac taattaagag tggtaacagt tgtgaattgt ttggaaaaat 103785
acttgatagt aataaaataa ttttaagaac tttgtataat ttttattttt ttctgaaatc 103845
cagtgactag attgctttaa cagcccattg gacttaacat ttcccatttt atataattcg 103905
ggagaataaa tttaatagaa tagtcctcag aggtctgacg gaaatatact aaaataaata 103965
ataaaggttg aggagtgaga gggaattgtt ttgattttta aaactttgat gcttggaatt 104025
tctggtcttg agttatgtcc tctgaaatca ggattcagct ctgaaatcaa actcactcat 104085
cgaaagtaac catgggaaac tgaatttggc ctacagatac taaggagccc caataatttc 104145
acattatctt ttaatggagc aaactattac agagaaatca gagaaataat attttaggaa 104205
tttgtattat attttagaaa taatatttta ggaatatgtc tatctgaatc aagatttggt 104265
ttttgacgtt ttctgctttt ttactttatt tttttaagag acagggtctc actctgtcac 104325
ccaggcttac tgcagcctca aactcctcgg ctcaagcagt cttcctgcct cagccttttg 104385
agtagctgag actacgggta cgcaccgcca cacccagcta atttttgtat ttttttgtac 104445
agacaggatg tcactcaatt gcctaggctg gtcttgaact cctgccccta agtgatcctc 104505
ctgcctcagc cctgcaaagt gttgtggtta caggaatgag ccaccgcgcc tggccagttt 104565
ttgttttgtt tagttttttt aatcaataag gctacatgtt tcagtcagtg tcaggattta 104625
aaaaaaaaaa aaaagtctac atgaagaaaa tcttcattaa ttttcttaat aaagaaatgt 104685
aattattcat taagaggtgg aggtggggaa gagtacagtt taggaagcag agacagggca 104745
gggtggcctc atcatcatta tgcctcaggg ttcgggtgag catcagaaaa ataactgtga 104805
cttttgatgg ctctctatat ggagaattta ggatagtgat taagagtaca tgccttgaaa 104865
tctgatgaac caagaggttc agattccggt tccatctaat tagctgtgtg accttgggga 104925
ccttgtgtca cctctcatag ctttgctttc ctctcaggga aaatcataga acctacttga 104985
tagggtattc tgagaattaa aggtgtgtaa catagaatct ggcatataag aaggactcag 105045
aaatacgtat tagctattaa gaaaaagttt ccaattattt cgaagaagac ctaatggata 105105
atagaaaaag aagaaaatac gttctctttt gtgtcttgat gacaagactc ggggtgacaa 105165
gagaaaggta aaattgggaa tcaagaagct cctttcgctt actcactaat ggaattaaaa 105225
gagaacaata tgtttacttg ctgaatatgt tacaggacca caggtttatt catctcaagc 105285
actccagctt gtactcattc agccattaag tctctttacc ctggttttta cctaggtgca 105345
caccaaccca aactcgctcg gccattaact ctctttaccc tggttttctc cctagctgta 105405
caccaaccta tactcacttg gttattaact ctctttcccc tgactttcta cctagccgcc 105465
tgccaaccta tacccacatg gctattaact gtctttactc tggttgtctc tgtaaccctg 105525
catgcctgca ggaaaaacaa tggggctagc agtcaggagc ctggaatgtg agttctgacg 105585

ctgctaccag ataaatatgt gatcttggac aggtctcctg accttcctgg gttgttatgg 105645
actgatgttt atgtccctgc aaaatggata tgttgaaatc ctaaccccca atgtgatatt 105705
aggaggtggg ccctttggaa ggttattata tcatgagagc cctcatgaat ggaattagta 105765
ctcttataaa ggggacccta gagagccccc taggtctttc tacaacagga ggtcagcaat 105825
ctgcaagctg taagagggct ctcacagaat ccaatcatgc tataccctat cctcagactt 105885
ccagcctccg aaactgagaa ataagtgtat gttatttata agctatatta gggttatcca 105945
cagaaacaga accaatggga tatgtggagc catgtataag aggagatata ttatgggcct 106005
tgactcgtgc aagaaatccc atgagatgcc acctgcaagt tggagaccca ggaaagccaa 106065
tggtataatt gagtccaagt ctgaaggact gagaaccaga gcagggacca ctgctggtat 106125
aaatcctgga ttacgaagac ctgagaacta ggagctccag tgtctgaggg caggagaaga 106185
tggatgacac agctcaagaa aagaaggaat tcccctttcc tttgcctttt tgttcaagtc 106245
cagccctcaa tggattggat gattcctacc tatgtgggtg agggtgaatc ttctttactc 106305
agtatgtaga ttaaaatgca aatctcttcc agaaacaacc ttacagacac atcacaaaat 106365
aatattttac caactatctg ggcatccctt agcccagtca agttgacacc caaaattaac 106425
catcacatca gtcacccagt taatggtatt ttgttatggc agcctgaact aagacatgcg 106485
ttgagttata ttatctttaa tttgcaggat cccttccatc tatctaacag taacctctaa 106545
agtcacctga agcagctatt atggagtaca gtggtgcaat cttggctcac tgcaacctct 106605
gcctcctggg ttcaagcaat tatcctactt cagcctccca agtagctggg attaaaggtg 106665
tgtgccacaa tgcctggcta cttctttgta tttttagtag agatggggtt tcgccatgtt 106725
ggtcagcctg gtcttgaact cctgacctca ggtgatctgc ctgccttggc ctcccaaagt 106785
gctgggatta caggtgtgag ccacagcacc cagcaacttt tacttttttt ttgagacaga 106845
gttttgctct gtcgcccagg ttggagtgca atggtgcaat cttggctcat tgctacctcc 106905
gcctcccagg ttcaagtaat tcttctgcct ccgtctcccg agtagctggg actacaggcg 106965
agtgccacca cgcccggcta atttttgtgt ttttagtaga catggggttt caccatattg 107025
accaggctgg tctcgaactc ctgatctcgt gatccgcctg cctcagcctc ccaaagtgct 107085
gagattacag gcatgagcca tcgtgcctgg caacttttac tttttattgt ggtttttaat 107145
aataattatc ccctgcttct tttttttttt ttttttttaa gatacagttt caatctgttg 107205
ccccggctgg agtgcagtgg cacgatcttg gctcactgca acctgcgctt ctcgggttca 107265
aacagttctt gtgcctcagc ctcccgagta gctgggatta caggcacgtg ccaccacccc 107325
tggctaattt ttatattatt ggtagagacg gggttttgcc atgttggcca atctggtctc 107385
gaactcctga cctcaagtga tctgcccacc tcagcctccc aaaatgctgg gattacaggc 107445
gtgaacactg tgcctggcct attccctgct ttctcatctt acataaatgt actgtgagta 107505
catttatgga gactgttgaa aagcaaattc aattcaacat ccatttgtca aacattgatt 107565
aggttgagca acacactgtg attggcctgg ggaaagcagt attgtttgga ttccttacag 107625
tcatggcaga cccaccttat tagtgcaatt aggagccagg tttgagagag actattaata 107685
gaggtaatat tgcacagtta gccaccttga agagcttgtc aagagcaggt ctccaagtag 107745
acacagatgg aaattgatag agattaaaat aaaccatgag atgctctgac ttttaggggt 107805
ttaagctgtt caggaaatca gagtttaaac aacacatatt ttacttaatg ctagaatttt 107865
agtgtcattc ctctggatgt tatttgcaat tttcccttcc accatcctca aactcccaga 107925
ggaggaaagg gccactgcct ctttggccaa gcagtctggc ctccagacac agcaggcaac 107985
tgggtttatc ttcctggcta ctgctaggag actcaagcct tcttaacacc gccagtgctc 108045
gtgtggtttc taccaaccct acttggccca aaatgaaaaa aatgtacagc acggcccatt 108105
cattcacctt tgagaaaaga aagattcagg gctttaggtg ccaaagtaat aaggatctgg 108165
agtgtgtaac atttgcagat ttttggcatt ttggaggata cctttcctcc ttgttcacaa 108225
cagttcaggt ggctacccag agaggtcagt gggaacttac caaaaatcgg ccctgatgac 108285
cactcctgca tcaggttctc cagtggcctt ccattgtaag tgttattaaa tctaaacact 108345
tgaacatggt ctataaggcc acaatcacca ggcctttgcc tggtgctgga ttcccacctc 108405
cttgccttca tctctgggtg gctgctccac tgaactctcc ttctagtcct tggcacttct 108465
agaagtcttt ctcccagatc ttcatgtggc tgtctcctcc ttgtcccaat gatctccttc 108525
cagagagacc cttcctggcc aacctagctg aaatagatag cccctggcaa caagtcatat 108585
cacctatgtt aactttcttc atggcatttg ccactatcta aaagttatct tactgttcct 108645
ctccactggt ataaagctcc gagaaagcca gggcctttgc ctgtcttctt gactgctatg 108705
tcctggtacc tgcagcagga ctttatgcgt ggtaagcact ctttaaatag gtgtggataa 108765
gtgaatatac taggcagaaa tgagggctag gagggatttg gttgcagccc catatccttt 108825
tggcaaagac agtagaatca tgtttttgta actaagatga gtcaaggtag aaattgtcat 108885
tctctcctgt tagattccag tgatctgtga cttcatgtaa agatttctta aaataattct 108945
aaaggcacac atttttgatg taacagtgct tttgagcttt aagaatttga ggcctccatt 109005
ctaatttcag aacatattaa tgaagattta aatatgcatt aaaaagtaca agcattgctt 109065
caacccagct atttctcaga aattctcatt gattttataa cagctgtctt ctagcttgag 109125
ccttctaatt tcttcagcaa aaaacaacaa caacaacaaa aaaaaaacag ataaatacat 109185
ctaacttaaa attaagacaa aaagtattaa ttggattttt agttttgtgt gttttccgta 109245
tttaaaatgt gatgctatat gccttttaga tggcttccct tcttttatgt taaagaatga 109305
attgtcagaa tcatggaaaa ttgtggatga caccatgacc ctttccttga ggcaaaactt 109365
gattcttgtt ctgtcactcc tattgaaata agtatttaag gtcactatgc tcttaggaaa 109425
gcatattcct aacatatcat gctgttttga agaatatctt tcccttggac ctttgtagaa 109485
cctttttcca aagattattt taaatatcat cctcaaaagt tgagctttac caaggcttcc 109545
catagtccgt tcttctcttc cacagctgcc tctgataact tatgaaagat gggctcaggt 109605
gtcagatgat cagggttctg cttgcagatt tgtcactgat ttgctctgca acatcttgga 109665
taaatcactt tacgtctcag agcttcagtt tccctgtctc tacattggag tcatgaaatg 109725

gggaaagggg tagggaagag agcaccatct ctcctgctct gtcattctcc agctttgcct 109785
ttggcggccc tagaaagaac catgtgggtt tgccttggcc tggctccatc tccgagaact 109845
cagctcttag gaactgaaac tcagaggggt gtgggtgaga gatttttctc cctgttctta 109905
gccggagttt ctttcaaaaa tgcaggcttg acaactaagg gttccctagc atatctgtag 109965
cttagtaact ggtcaaatgt gtagagcttt tggggactca cctgtagcat ctgaagagtt 110025
gagatgagtc ctgtgtgctg acaggatgtg atggacagga caccaacatg gtggagtcag 110085
taaagggagc acctgcttgc cacaggggtt tactgtaatc acccaacagg tcttccttct 110145
ggctgcacaa agccaggcca ctgagaccgt ggcattgcgg taaagaaaga gtttaattga 110205
cgtgaggcca gccacaccat gtgggagatg gagttagtac tcaaatcagt ctcccctaag 110265
acttgtaggt ttgggttttt caaggattgt ctggtgggca ggggggtaga gaatggggaa 110325
tgctgattgg tttgggatgc agttatagaa gtgtggaaaa tggtcctcat gtgctaagtt 110385
agcctctggg ttggggagcc acaagactgg tcgagtcacc aacactggtg tgatattgtg 110445
aagtccagca cgctagagtg tcaacattcc aggaagatca ctaaaatgaa catttgttct 110505
gcaagagaat taataagtcc agcatagggg ttactggtat atatttttat ttgctgccat 110565
tttatctgat gattgactgt tttgttctat ttttctccat ttttataatc actaccccca 110625
atatagtcgc tactccaggt ggagtcagct ggtcgtcaga aatgcagaag tctgaaaaga 110685
catctcaaga caccaatctt aggttctacg atagtaatat tgtctacagg agtaactggg 110745
gaagtcacaa atcttgtaac atcagggaaa aatacctggt tattgtttca cttacataca 110805
tcttagcaga attcaggccc ctctcataat cctaacctgt gggctttcat tagttttaca 110865
aaggttgttt agttttggga agggctatta tcatccttgc atgaaggtta aactacaaac 110925
taaatttctc ccaaagttag cttgacctat tcccaggaat gactaaggac agcttggcag 110985
tcagaagcaa aatggagtca actatgtcag atttctctta ctgtcgtaat ttgcaaaggc 111045
ggttttgcta ctttccttgc aaattagttg gggacttcag cagttctgca tagaccaaga 111105
ccaagagttt tctagtactc tcatcaagaa gtcacctact ctggtcccat tttaaatccc 111165
ctgcatactg tctagaagaa tatataacag atgctaacag gatccaattt gttcattgaa 111225
atatatcaag ttttctccaa attttcaaga ccatattcat ttgcatttaa ttttaaaata 111285
tcaatacact gatgtcatgg tatatcattt gaaggcacag ggaaggatat tgtgaagtcc 111345
agcagactac cgtgtcagca ttccagggag atcaccaaaa tgaacatttg ttctcaaaga 111405
gaattaataa gcccagcata ggggttcact ggtatatatt tttatttgcc accattgtat 111465
ctgatgattg actgttctgt tctgtctttc tgcatttttg taatcacaac ccccaaacgc 111525
ctgaagcttt gggtttttat gtaaactgta aagtagaatt tatttggaac cagttccctg 111585
tcataaaagc taatgaaaaa tacagaagag tgaagaaaga aactagaatc acctataatc 111645
tcattgacct tcaggtatcc aaggttggag aaggagccaa gaaaaatcat ggacagagtt 111705
attttggtgc cattacattc agaattattt ttaacctgtt gtgaggctca gctcttttgt 111765
agtagcttca gtaaatccta acccatgatg cacaccgagc ccctgcccgt tagtggtaat 111825
gtgcatgtct ttccatttgg ttgaggacac cttgctgtac tgggcacttg gtgccagagg 111885
actttttttt ttaagggcca ttaagggata catggagacc tgcattctca cactggcgca 111945
gtgatttttg cttcctctct gaaccggcct ttggtatttc acaggctgaa cccttctctt 112005
tttaagtgac ctgtcacctc tcatgtagtc aatgctttct tactgccacc aagttggcaa 112065
gtgtcatgcc attcctctcc ccaggggaga gaattcttaa tccaaattta ttcccatagg 112125
attttcagga cttttcctga gccaaattag accctttctc tcagtatatc tactatctcc 112185
tcctttccct gtattggcca gtgctagact ggaagctata aatagacagc accttttaat 112245
tcctttaaag atttggagta ggtattattt ctcccattct gcaggtgagg aactaagcct 112305
cacaggtagg gagtatggtt tttggatttc gcatttagtt aatgacaggt gtatttgttt 112365
agtgaatgtt gaatgtagag ttttaccttc tttcatatat tgaagatgca catctatgca 112425
agtctgtgca taatgtaatg gcacaaggta atggcaccaa tggcatccta taaattttat 112485
ttaatagcct gtagcattga acgtcttcaa gccttttaac tctcaaagct aaaataagaa 112545
gattgtgtag ttactagtta aaataaaggc agtttaaaga ggtggggctg gagtagggca 112605
aactgagaac aaagatcttc attttgcccc atttctctgg cctattttta ttcactatgt 112665
agttgtgtgg ccctcccttg agcagtaatc atccaagcca tctatgcaag tctgtgcata 112725
gatgtgcctc ttcaatatgt taaaaaaaac tataggacta tgggagttta gtgaaagtca 112785
atcacatttt ggtttacagc ccctttgccc actgagctga tctcctggct tgtgaattct 112845
ggcctcttct tgaaaaggtg caggagcgcg tcagatcagc aacaggtggt ctagctggcc 112905
cggggaggac ttacgcagaa ttgaagacag cctcctgggg gcaggtgatg agacctgccc 112965
gtgaaggctg ttaacctggc tgctcctaca gtaggggccc cttagtggac ttccttctgc 113025
caagcagtta ggacctacag gttagtttcc ctctccttgg ttgtctctgt ttctcgagaa 113085
ggtagggccc ttagctccat cagtcaagtt gcagatgctc tacctttggg ctagtgggtg 113145
ttggtcttca ctgctcctcg tcttgggtga cactggacaa tcttttttac tttgtcaaaa 113205
gacaaaatga catcaaattt aaagttttta attggctttt cctgttctag aatcagacaa 113265
cgtcaagcaa aggaagttgg cttaatagaa agggtagagg aaagcagaaa tagaacaaaa 113325
agcatattgg ttgtttcaaa gttaattttc cttttaaagg ttaaagcaga gggaacttcc 113385
ttatgcttgc ttaaactggc ctgtttgggg atttggtatt ctctctctct ctcctgattt 113445
ctcagaaggt cagataaaca gcttggtttc agtggtgtgt aacttcagca agggtgattc 113505
cattttggtt tggcctgttg agtctagtgc aggaggtcag tccaaaccaa tggcctccta 113565
taaatgttat ttaatagcct gtagagttga acgtcttcaa gccgtttaac tctcaaagct 113625
aaaataagaa gattgtgtag ttactagtta aaataaaggc agtttaaaga ggtgaggctg 113685
gagtagggca aactgagaac aaagatcttc attttgcccc atttctctgg cttattttta 113745
ttcactgtgt agttgtgtgg tcctcccttg agcagtaatc atccaagcca gttgttagaa 113805
cgcgtttccc tagcaagctc tgtcatcctg agaaagcaga ggggtaaaca ataaagactt 113865

aattgtatct caccaacatt tttgggtccc attggtgagt aaggcatcaa tttaagtgcc 113925
tttggaagca attttgatta aaatgatttt ttttaagttc ttaaacagat ttcgggatga 113985
taggagattc ggacatattt taaaacaaaa gaaacttata caagcagaat taaacaacct 114045
ggataaaagt acctgtaatg tattgtgcta gtacagggga aggagggaac tgcgtaggat 114105
gttataaaag aaatggtatt ttagcctttt cttaaaagat gaggatgctt tgggtaggaa 114165
agagggatca ggagagttcc aggccaaggg aagtatacga aggcttggaa atacgtgacc 114225
cctgcttact gtgactgtgt gtttgggaca gagtttttaa cagcagtaga tgtggggagg 114285
aaaaatgagg ctagggccag accctggagg ctatgcatga ccaaagaatc tgggctttat 114345
tctatttcac agaaagagtt caaagcctga gcgtatttga aatgagagaa tgaatgatgt 114405
gtccctctgt ttaagcagag agaagatacc atttgcattg aaaaagatta attgaagagg 114465
tcaaagaaag ggcagggaaa acccataagg gaggccattt aaatgtgacc ctgtgggaga 114525
ggagacccgt ctgacagcag ggagaccagg gggaaaagat tgcttatgaa ctatttcaga 114585
ggtaaactat atgatgacaa ataggactgc agggatgtga taagggaaac tgatcatttt 114645
aaagatttcc aggagagata actgggaaat gatgctacct ttagatgaga cggacaggat 114705
ccacaaagag aaatatttgc ttggttgaag gaaaagtgat aaacttgatt tgtgatgttt 114765
ggtaggacag tggaattccc aattggtgac tccaaaaaga agcgcaggct agagagataa 114825
tttttgaagc catatgcatt gaggttgtag gggccaaggg agaacttccc cctttcccct 114885
ctgaaggttc gttgagaatc aactgacaaa ggcagattaa taggagaaaa gacatacaaa 114945
atgttaacag gcatagcacg gggaactcac aggaggatga ttacccaata acctagtgca 115005
atacagaatc gtacataccc ttttcatagg ggagccggga gatggggggg gatgtaggca 115065
tttcttttga gggggcagtc aatcattaga ggaaatgaat ggacagaagt taacttggaa 115125
atgatttttt tcttgaaatt tgaatgagcc caagaggcag gtattatctt gtgaaggagt 115185
cttctcagat gtggttgcat tcctcagtct tcttttctga gataatgaaa tttcagggag 115245
gagagggaaa cagttgtgtt tctgttggcg acccctgcgt actgtgactg ttaaactttc 115305
ttggccagat gaggaaattc cagagcaagt ccgttcttgt gtccttcgct ggatgggggt 115365
gggacaagac caggttagaa ggacctcgat tctgaggcag cttctaaggc ctctaagcat 115425
gtcaaggcac cagtctttgg ggtatcactt tctgagtccc gacaaggtca tgtttgaagc 115485
catagaggca agtaggatcc ccatggcaga ttttacaccc aggaggaggg cttagattcc 115545
tctcaggagc ctcccatcag tagttaggag ctgctcagaa agcactcacc atatggagga 115605
ttggagtcta tgatctaggc tgctcagcag gagtccacaa aagagccccg tctccaggat 115665
cagtaaaatt aacagccttc ctgaattatg gcttatgtcc cctccagggg ggcagtagtg 115725
aagttctcta gcagcgaggc ctccagaggt cattaagaga ctcacctgtt gctgatcaca 115785
actcatctca cctgttgctg atcacaactc atctcacctg ttgctgctga ggacatccag 115845
acttagtggt gcctggtgtg tactcagaaa gggtaattgc ccacatttga ctgcatattc 115905
acaatctgtg aatgaaaaaa aagccaaaag aaaagttggg gaatctgcat ttaaggagag 115965
aaaaatgtgg ctagtaaact tagagaaacg cgctggttat ggtggctcat gcctgtaatc 116025
ccagcatttt gggaggccta ggcggacgga tcacttgagg tcaggagttc gaaaccagtc 116085
tggccaaaat ggtgaaaccc atctctacta acaatacaaa aattagccgg gcatggtggt 116145
gcatgtctgt agtctctctg agctacttgg gaggctgagg caagagaatc atccaaactc 116205
gggaggcgga ggttgcagtg agccgagact gcaacactgc acttcagcct gggtgacaga 116265
gtgaagactc ttgtctcaaa aaaaaaaaaa aaaaaaaaaa aagagagaga aacccaaaca 116325
ctatagaaag gagagatatt ttaagtaagg agcaatggag gagaaagtaa ataaaagagt 116385
ttagacaaga ctaagatgtg ggaattagaa agcttttggc aattttttta aaattattaa 116445
tgtccttctg ggtgccaagg aattttattg tgtggtcagg cggtagaatc atccgtaaag 116505
gtgaccctag acacctgaca gtaaagggga ggtgaggaat gggtgaagta gggaagatgc 116565
agagagtttg atgctagagt ttatccttta gtacttgaag attgacatta aaatacctag 116625
aacaaataca tttggactta gtgttccata aatggatata tatctaagag cagttttccc 116685
agggaaccct atctaaaagt tgaattcgtt ctcaactgtt tttacagtat ctagtttccc 116745
tccctcatta ctgtgctatg tactctaggg gaattgagaa aaacgaaggc attatctttg 116805
taagaagaaa aaatcaagat gatctggcac acctggaggg gtagtggaat gggagttgat 116865
cactgaacat gtatgcaatg tgcataggca caggggattg ggaaagacat cccaggcgag 116925
aagtaagagt aatcacaaag gtgggaataa atatggcatg ctacagtgtg gcatgggctt 116985
tgtgttccta ttatttgcag aatgttcgca tagctgggtg tcttagtctg ggatgctata 117045
ccaaaatacc atagattggt ttataagcag ttctagaagc tgggaagtcc aagatcaagg 117105
caccagcaga ttccatgtct ggtgattctg tttcctggtt catagacagt gcctttttgc 117165
tgtgtcctca catggtagaa gggacatggg agctctttgg ggtctttttt aataagggca 117225
ctattcccaa tcatgaccta atcacctccc aaaggcccca cctcctaata ccatcacctt 117285
gtgggctagg atttcaagac aggaattctg ggaggacaca atcatttagt ccacagcaat 117345
aagaaatatg cgaaatcagg atgggtagga tcagatcata gagatctcta gattctgtta 117405
ccggatccca ccacttactc aaagttagcc tttggatcgg gggtttccta ggtattgtgg 117465
ctttgtggtc accagaaaga tgttgctgaa aaggggtccg gatccagatc ccaagagagg 117525
gttcttgaat cttgcacaag aaagaatttg aggcaaatcc atacagtaaa gagaaagcaa 117585
gtttattagg aaagtaaagg gataaagaat ggctactcca taggcagagc agcttcgagg 117645
gctgctgctt gcccattttt gtggtggttt tttgatgata tgctaaataa agagtggatt 117705
tttcatgcct ccccttttta gaccacatag ggtaacttcc tgatgttgcc atggcatttg 117765
taaactgtca tggctctggt gggagtgtag cagtgagggc caccagaggt cactctcatc 117825
accatcttgg ttttggtggg ttttggctgg cttctgtact gcaagctgtt ttatcagcaa 117885
ggtctttatg acctgtatct tgtgctgacc tcccatctca tcctgtgact gacaatgcct 117945
taactgtctg agaatgcagc ccagtaggtt tcagctttat tttacatagc ccctattcaa 118005

gatggagttg ctctgcttca aacatctctg acagttccat acaggcaggc ttggaattga 118065
tagactgagg agaatttaag aaactgagta gtgcagtagc tcatgacaga agtgtttaag 118125
atctgagcaa taggatacag ctcataacta tttagaaaaa gtacagaatc gagctattaa 118185
gtattattac agggaaaaaa tgtactgagt gaccaaaaaa aaaaaaaaaa aaagcaaggt 118245
actatgacct tctttatgac ccccattcca aaaagatgga agagcagatg ccttcttaaa 118305
taatcagaac cagtggtgca ttggtcaacg aaaacattca ttagggtgag aaaataatat 118365
gtcacttata tgtacatgta atttttaatt aaaagcatat atctgtttgc cacatctttc 118425
attgtaaggc tgagattgcc tctttgagta aactggctag aacacataaa tcacacataa 118485
tctttctcac agaaataaca cctataatgt ttcacttagg atttccagtt ttggtgttct 118545
gggcgtggtg gctcacgcct gtaatcctag cactttggga ggccaaggcg ggagtatcag 118605
ctgaggtcag gagttcgaga ccagcctggc caacacggca aaaccctgtc tctactaaaa 118665
atacgaaaat tagcggggcg tggtgggcgg gcacttgtaa tcccagctac ttgggaggct 118725
gaggcaggga aaattgcttg aacccgggag gcagaggttg cagtgagctg agttcgcacc 118785
attgcactcc agcctgggtg acagagccag actccgtctc aaaaaaaaaa agtagggcct 118845
cacccaggct gtagtgcagt ggcatgatca cagctcattg cagcctccct caaactcctg 118905
gccttgagtg attcttgcac ctcagcctcc cagagtgctg ggattacagg tgtgagccac 118965
tatacccagc catgaatgag aatcctaata ttaacaaagc atgctgagtt tacacttgcc 119025
tcccagttgt aacagtctag ccctcaccaa attcaacagc atttctgttg ctgctgctgt 119085
ttttaaagaa actcaccttt ttggagtctt gtattgttca tgctctttat agaactaaga 119145
acccaatttc ttctcacagt tacattatat tcatttatgt aagtactatc taattgaatt 119205
aatgtagctc cagtaataag tataactaac atcaacatgt ttgggaacac agctgatcat 119265
tggtatttga gccaaatggc aagagcagag tggcctagac aaggaaggtc tgtgcagtag 119325
gcagagtcat aagtagtggt cagttgagtg agcctcttaa tttccttcag atttattgac 119385
tatcagttgt ataccctcta aaattttctc tagatactcc atttctggaa cacattttag 119445
gagatgtttt ctttttttta ttattatttt attattatta tactttaagt tttagggtac 119505
atgtgcacaa tgtgcaggtt tgttacatat ctatacatgt gccatgttgg tgtgctgcac 119565
ccattaactc gtcatttagc attaggtata aggagatgtt ttctttcttt tttctgagac 119625
acacgcacat gccaccacac ccaactaatt tttttatttt ttgtagagac agagttttgc 119685
catgttgcct aggctggtct ctaactcatg gcctctagta atcctcctgc ctcagcctcc 119745
caaagcactg agattacaag tgtgagccac ggtgcccagc ttgttttcta tagatcttgc 119805
cattgcccat tagagcactt tgtgtttgtc tctcctgcgc aaatccttga ctgtgcttca 119865
gttgacctgg tctcctggtg tctccttctc tgccaccaac tcttcatgtt gatttcagcc 119925
atgtattacc agcaaaacgg ctagtcctgg cttggaatac caaaaagcta tggaagccat 119985
gcagggccag aatatagcta agtgtgttta ttagtaaaac ctgcctgtat tagtctgttt 120045
tcacactgct gataaagaca tacctgagac tgggaagaaa aggaggttta attggactta 120105
cagttccaca tggctgggga ggcctcagaa tcatggcagg aggcaaaagg cacttcttac 120165
atggtggcgg caagagaaaa tgagagagat gcaaaagtgg aaacccctgg ctgggtgtgg 120225
tggctcacac ctgtaatcca agcactttgg gaggctgagg caggtggatc acctgaggtc 120285
aggagttcaa gaccagcctg gccagcatgg tgaaacccca tctctacaaa aatataaaaa 120345
aaattagcca ggcatgatgg tgggtgcccg taatccaagc tactcaggag gctgaggcag 120405
gagaattgct tgaacccggg aagcagaggt tgcagtgagc cacgaacatg ccattgcact 120465
ccagcctggg cgacagagtg agactcctca aaaaaacaac aacgaaaaag gaaacctctt 120525
ataaaactat caaatcttgt aagatttatt cactaccatg agaacagtgt gagggaaacc 120585
gctcccatga ttcaaattat ctcccaccgg gtccttcgca caacatgtgg gaattatggg 120645
agtacaattc aagatgagat ttgggtgggg acacagagcc aaaccatatc actggccatg 120705
ttcacctttg ttcttatttt tttgcgcttt agagattcat tttaaagttt attggatagc 120765
aaattgtctt tgccacatat atgtagctct tagctaacca aaactcaaaa ttttgtttgg 120825
cattttagct ttattaaata aagtgggata gacatacttg acaaataacc ttttatatac 120885
ttagcaacag catacagact agatctgaaa tgagtgtaca tttcaagttg gcttctcaaa 120945
tttaactaga accagacact cctctactga cactctaata atatgtgtat gtgcatctat 121005
gtgtatttat gtatgtgtac atacatgtat gtgagtacat gcgtctgtat ctattgctaa 121065
ccaaatactt gtgtgtgagt gcaaggaatt agaacctccc tgggaagagc tagtcatctc 121125
cttcacacag tatacttttt gcatatctcc tgttggcctc tgcttattca tggcttctac 121185
tcataacatc cctagcataa tgctacctta tgacctatat tcaacttcta ctgccatacc 121245
aactggacat gttctttcac acctgcagct caaattctta atagaagtga actgactggc 121305
tcagccaatc actctggctg tgcttgtgca gagaccctca tgccaggccc cacacaagct 121365
tagagacagg gtactctgag aacaggggac cattcccatc tccatcaggt gagactagga 121425
cagatagcag ctgcctgata cagaagatgg cagcgcaggc agcagatgct gtgggtgggg 121485
cagtttccct tagattatag gatggcagga aagataaagc ctgccatcgt ttactccgag 121545
gggtgtgtta tatgtgccta cgagcagcgt tgctttgcac cattccatat gtacacattc 121605
ccatcactgg atatagttaa acaccagtcc ctcaatgaca aattttagtt accctatgta 121665
atagtttgct aggactacca ttataaaata ccacagattg gtggtttaaa caacagagat 121725
ttattatctc actgtgtgga ggctggaagt ctgagatcaa ggtgtcagca ggtttggttt 121785
cttctgagac ttctcccctt ggcttgtaaa tggctacctc cttggtgtat gtatgcgggg 121845
ttgttcctct atgtgcatct gtgtcttagc agccttttct tagaaagaca gcagtcatat 121905
aggcctaggg ccattttaac tctttaaaag ccctatctca aaatacagtc acaatctaaa 121965
gtcttgggga ttaggacctc aacgtatgaa ttttagaggg atgcagttca gcccataaca 122025
ctatagtata gcaattccga gtcactgcac ataaaacaca gactttgggg ctaacttcac 122085
aaatcactat gtacatgaca catgtacatt ttgatcaata actaatcata tcacttctca 122145

gaatctggtg gtgatttgtt agcgagcgtc tgttcagttc atgcacagac agcaaagtat 122205
gtagctgtgt tgctgccttc tttctcagtg aaaacatgac atttaaaaaa atagttcatc 122265
aggagaggga attggccacc aaagatgaaa gtgctgcaaa gaaacaggta gtagtaatga 122325
aggaagtgaa atttgttgtg atgagatgtg aaaatggcaa caggataacg aagacagtac 122385
aagacccagg ccagcatgaa gctacagtac gaaccagatt gaggctgggc acagtggctc 122445
atgcctgtaa tcccaacact ttgggaggat gaggcaggtg gatcacctga ggccacgaat 122505
ttgagaccag cctggtcaac acagtgaaac cccgtcttta ctaaaaatac aaaaaaattg 122565
gctgggccca gtggtgcctg cctgtaatcc cagctattgg gaggctgaga catgagaatc 122625
tcttgaaccc aggaggtgga ggttgcagtg agccaagatt gtaccactgc attccagcct 122685
gggtgacaga gcaagacttc gtatcaaaaa taaaaaataa atgaattaaa aaatatgtaa 122745
accacactga aaatgtccaa tgaatataaa aaaaacacag taaagtcatc tcaatatatt 122805
ttaatttaaa ttgcgctagg aacagaaaac cacttatgat tgaaattgag tattttactt 122865
tagttggaag accataatga tttttaaaaa tcctgcttag tttgactagt attcaggcca 122925
aagtgtttaa aaatcctgct tagtgtgact agtattcagg ccaaagaagt tagtttctga 122985
attaaaagaa aatggtaatt acatggagac tataggagga cttttgactg ccagtaaaga 123045
ctggtttcat tgtctcagaa gtcaccatga actgatgaat gttaaatagt ctgatgacac 123105
cactagtgta aatcaggatg cttccgtgaa atttcacccc gaatcccaag atggaggcag 123165
gtttatgatg atcctggaat atttcatgct gatgagatag atcttttgga agacaaccct 123225
atcaagaact taagtgaaga aaggctgggt gccatggctc atgcctgtaa tcccagcact 123285
ttgggtggcc ggggcaggtg gatcacctga ggtcaagaga gtttgagacc agtctggcca 123345
acacgatgaa agcccatctt tactgaaaat acaagattag ccaggtgtgg tggctcatgg 123405
ctcatgcctg taatcccagc tacttggaag gctgaagcag aattgaacct gggaggcaga 123465
ggctgcagtg agctgagatt gtaccgctgc actccagcct gggcaacaga gcgagactcc 123525
atctcaaaaa tttaaaaaat aaggtcgggt gcattggctc acacctgtaa tcccagcgct 123585
ttgggaggct gaggtgggcg gatcacctga ggtcagaagt tcaggaccag cctggccaac 123645
atggcaaaac cccatctcta ctaaaaatat aaaaattagc caggcgtggt ggtgcacacc 123705
tttagtccca ggtgcttgag agtcttgggc aggagaatca cttgaacctg ggaggcggag 123765
gtttcagtga gccaagatca cgccactgca ctccagcctg ggcaacagag ggagcaagac 123825
tgtctcaaaa aataaaaata aaagaattta taaaaaataa aaataattta tgtgaagaaa 123885
gatgtaggat cacaaagtgg ccatacagga gagattctag atattcagct agggaaattg 123945
gtgaaggtga acttacccac ataaatgagg gatgtggcta tcaagaaaac gatgaagatg 124005
acccagagga agtgataatg gcaaaaacaa acaaacaacc aaaaaaacaa agtcacagta 124065
aaggaactca gctaattcac gacatggaaa tggcaaagga taaaatgttg gaagccaacc 124125
caaatttagg agtatggcag ttttccaagg catagaaaag acgtgtattc tatatcgtaa 124185
gttatttgac aaggagaaga aaagcactgt tcaaagtgcc ctcgataggc cagatgtgat 124245
tgcttgctcc tgtaatccca acactttggg aggccaaagc aggaagatcg cttgcgccca 124305
ggagttcaag acaagtgtaa gcaacatagc ctgaccctgt gtctacaaaa aagaaaatcg 124365
ccaggcatgg tggtgtgcgc ctgtagtccc agctctcagg agggtgaggt gggaggattg 124425
cttgagccca caaggttaag gctgtagtga gctgtgattg attacaccac tgcactccag 124485
cctgggtaac agaatgaggc cccgtctaga ataaagcaaa gcaaagcaaa atactctgga 124545
tagagttttt ttaataggga aatgaaacat tttaatgctc aatgttttta atattttaaa 124605
ttactgtact aagcaaatgc ttattttact agttttttcc ttcccctcta catgtatgag 124665
tttttaatgt ttagacataa attttttaaa gttgtggaac agtaatccca ttgactatta 124725
aaatgacttg tgcactttga acttgcatgg ttacttttat ggttccacac gtctatgcaa 124785
agctgtgtgt gtgtgtgtgt gagagagaga gatctttgct ggaagtgtgt ttatctgtgt 124845
gtgatatttg ctgaaagtct tcctaagatg tatcatgtta tgtttttact tgaattattc 124905
acctaagcct ggcaagtata acttagcctt ttcctgatgg tgcagtcatc attaataata 124965
agttattgtt ttctgctata acgtagaata atctggtagt taagggtgtg gtttatgcag 125025
ccagactttg agttcaaata ctgagtcatc ttcttactat gatatgatct ggaacaagat 125085
gctatatctc tctgtgcctc agtttcctca tctgcaaaat gcagagagta agagtggcta 125145
cctttttagg gttattgtga ggtttaaatg actaaatgta cccaaagcac tgagtggctg 125205
tcaacacagc aaacgtgtta gccaatgttg ctgctgctgc tggcagggac aatggtggta 125265
gtagtagtag ctgcatcagt agtagtagaa gtattaatga tgctgtgtat ctgaagattt 125325
attaaaagtt ggggctattt ttttctatat tagacagcct caaactacta tgcttttgat 125385
ttcttctgtt gctttattta cgtgttttcc tctctcatga ttgggctgtt aatggacatt 125445
ctttgcattg gactcaagca gaacaactac ctcattctca tcagtatctt attaatttga 125505
tgttcttaac cacgatgggt gtagtgacct tctgaaagtc tgacttgttg cagtagcgtg 125565
gaaccctgga ttgtggctca gttcctcatg gacggcctat cccagggaag cccaggaaag 125625
aaccatttat tttgctcagg cctcacacat tcatgaaggt gcttccaagt tgccatttga 125685
cattatctcc aaatgaggtt atgctcttaa tagttgtatt ttatctgtct taaaagcatt 125745
aagttattct ttgtgagcat taaaactata ttagtttgtt accctctttt ggcatttcat 125805
ttttaatata ctgagaacct cgaagtacag aagtggccca ggcccttctg aggagccctg 125865
ctcaacagac aagcggatgg agttcctgac tgtgttaaga gtagatagta acttgattga 125925
tgatctcgga ataggaaagc agaactgtat ttcttgcagt tctccattgt ttctctaagt 125985
ctcttgcagg gtctcaagat catctctcag gttttttgat tctaccccag ggagctgagc 126045
atagcatgtg cctaacttgt aagaacaaag gtgtaaatac ttcacattta taatttggta 126105
gggtcttcag ggccagacgt tttgtgggcc aacctccact aaatctaaaa gtcattctcc 126165
aaatagagta tattgtgttt aatactgctt aaaaggtatc agtcacatgg gttcttagtt 126225
gaacttatgg ctaaaataat ttttaaactc atttttcttt ttattttttt tttgagactg 126285

agtcttgctc tgttgcccag gctggagtgc agtggggcaa tctcggctca ctgcagcctc 126345
cgcctcccgg gttcaagcga ttctcctgcc tcagcctccc gagtagctgg gactacaggc 126405
acgtgccacc acacctggct aatgtttgta tttttagtag agatggtgtt tcaccatatt 126465
ggccaggctg gtctcgaact cctgaccttg tgatccaccc gccttggcct cccaaagtgc 126525
tgggattaca ggcgtgagcc actgcgcggg ggcattttcg tttttccaac caaattgctt 126585
ttaactaaaa tagtgtatta attgaccttg tctcagatgt gtaagccgta gctccccttt 126645
tcccttcgtc acccacctcc atgtgcttat ggcatatggg gaaagagaaa accttttttt 126705
tttttttttt tttttttttt tttttttttt tcaaaagtga tctgaagtta ttgatcagta 126765
aactggaaaa cccactgtac cagaaattca gttaagctga ggctcaattt tcttcccctt 126825
tatggtcagc tttgtacatt tcagtcccaa atcagggaga actgggagtg cagttggcat 126885
cccagttgcc tgacgaatga aatacgaaac agagtctgtt gagttaatgt catcagttta 126945
ttgaaccaca gtcggaggaa aaaaactata tccaaaaaag tccttaccct tcagaccaac 127005
tcatctcttg aatatgggaa gcatttgtta acaaaagtat aaggtttgtg aatagttatg 127065
tgctatacac agttattttc ttacatcatg gatattccct taataacctt tgaaagaata 127125
aatggggatt ttcgaaaagt tctagccatt ggccagggca agactgaacg tgctatctgt 127185
tgaaatgtta aaagggttga attctgaagg aaagctagca agatttcgct aatatgtgct 127245
gtgtgtcgag gtggtaagaa catgtatgaa cctgcagttg ttggggttta attggggtta 127305
agttgttcct taatttagat aatttggttt cttttcaggg actttttttt cttatgggaa 127365
aataccataa agtttaccat tttagacttt tttttttttt tttttttttt tttttttttg 127425
aggcagagtt ttgctcttgt tacccaggct ggagtgcaat ggcacaatct tggctaattg 127485
caacctcagc ctcccgaggt tcaagcaatt ctcctgcctc agactcctga gtaggtggga 127545
ttacagggca tgcgccacca tgcccggcta attttgtatt tttagtagag atggggcttc 127605
tccatgttgg ttaggctggt ctcaaactcc ctacctcagg tgattgacct gctttggcct 127665
cccaaagtgc tgggattaca ggcatgagcc accgtacctg gcccattgta gccatttcaa 127725
gtgtacattt cagtaacatg aagtacattt acattgttgt gcaaccatca ccaacatccc 127785
atcttcagaa cttttccgtt atctcaaact gaaattctga cctcattaag cactaactcc 127845
ccggccgtct tccctcagcc gctggtaacc actgttctct tttctcatct ctgaaatcat 127905
acagtatttg cttttttgcg tctagattat ttcactttag ggaatttgat tttatgtcag 127965
tttttctaac aggttgcttg tggagacagt aataatcaaa aacttactgt ccaaagccct 128025
gagtctgaaa ttctgcatcc aaaagtgcta cagtatgcat taaaccgatt aaaataacgt 128085
agcatctttg aggacgagct ctatgttcaa ctttgttgtt gtttaatatt tatattgtaa 128145
gaaaatctct catatatgaa aatgtaaatg taaaattcca ctattttgtt gccattgctt 128205
ttcttgtttg ggggatcatt actgtattat tggcttccag gatctcagca gggcacgagg 128265
gaggtttagc ctgtctgaat tttggaatca agtttggtga tgtgtgagct gtccccagaa 128325
agcaccagta taatgtagtg gtcttaacct gcattcttta acgcttagcc ctgggctcta 128385
taaccccacc cccatgccca gcagcatccc agggtcctct tctggaatcc aactagagtt 128445
cctaagggag gaggatcccc agagggagaa agagaaagtg tcagtggaca gaaatatgat 128505
actgaaatag cagagttagt gtttgagtac tgacttttcc cgttgcacat ggaagaaatt 128565
tccatgcctt gtctggatat ctttagaatt gctcctttca gtgttaagat ggtttgttcc 128625
actgtaagga ctgtgccaca tggcttggtg gacttggaga ggcggaggtg gaaacgatgc 128685
gcaggagttg gcttggggct ttttgtttgc gtgtccctgt ttacctattc ataatcatgg 128745
atcccctctg ctttgtgata ctgtgaacca cgcataacag caattcttta caccaccggg 128805
ttgagaagaa ggcgcctgag gctgactttc tggacctgcc gtcacgcagt aaagatgtgg 128865
ttggtgtgtg tcaggatgag agggccacga ctggacccac gtgtggctgc ttcccgggtc 128925
tgccatgtgc tttgctagct gcaaagcgtt tcctctagaa cagctaatcc tgcatgactg 128985
tctcctgcat tttgctgtca gaacgtgcac gccgctttca taagaactaa ttgaagaggg 129045
agctagtttg catctcgttc ttgcacaccc tgagtcatga aaaacatttt cttttaagca 129105
aaattgaaag aaagaaaaga gagaaagagg gccaaaaaaa aaaaaaaaaa aaaaaaaggt 129165
tgccatccag tggaggaagg agtaattccc agcttcattc aggaaaaacg caggctgaaa 129225
tatgcttaac tgttttaact tgactgggga tttttaccac catatgggtt ttttttcgtg 129285
acatccggag ccaaacgggt gcagtgtgcc tttaagaaaa gaggtgcctc actaaacttc 129345
gctgtagttg tgtttcaccg tctcggtagg gaaggaaagt gttaatttct tatttgtaca 129405
ctttttttcc ccgacttcct tcctattcac ttcattaaat ctagaggcag ttgagcatgg 129465
gagccgtctg tatgttgaat tagggctcgc actcttgcgc aacacgtcac cagtcggaaa 129525
ctgggggttt gcttctgtga tttatttcat tattgtgctg gtaaaaggtt tggaagggaa 129585
ttctttttgg gggtagtact ttagcattgt gtagcaagtt ttggggtttt ttttgtgtgt 129645
gaccccccag cccccagcgc tgagtttgag tcagttgagc cagtttagta aataattttt 129705
taaaataaaa gaacagttta aaatctccat gaataatttt acttacatgc aggagtaatc 129765
ttactctact ctttatgtgc gaaaagcatt gggaagtgtt tagtgaattg atttccatta 129825
gaaaaagacc cttagaaatc acagaacata aagcactgca tatggatgtg tttggggtct 129885
ttggggagga gggaagatgt tttgtagctc tctgcattcc tgcataaaac cttagtttga 129945
ggggaataat ggtcagtact ttgtgtgttt ccttgtaatt ccaaaaccta aatttaataa 130005
gaatctgcac gtattagcag ttgtaataaa aattgtttca gttgttctgt ttgcaattta 130065
atttgttttc gcttcttagc tactcacaga tttagagatt taaaagtcca aatatcacaa 130125
gcttatgctt tttccctctt ttcaaaatta aggcggggtg agagtagaaa tatccttttt 130185
acaactcccc ccaacttcat ttatctcagt gagaagaaaa acaatgaaaa attttacagc 130245
atgaaactga aagtgaagtg ttacagttgt tctgaatttt ctttaaagag cagaataaca 130305
tattatgtta aaatatttga atcttagaac atgtacattg cttttattta catgcattta 130365
attttcttaa aaaattgtct tctgttttaa aaatcattta gactgctcca aaccatgctg 130425

ctatttttta aaaaataagg tacaaaatac caaaaaagca taccttgttt taatttttta 130485
aaataaattt gaatattaat ttgttttctt actggcttaa ttgttaaaat gcgagcaaga 130545
ctcagaaacc atctgagcta aaatctctaa ttctgctgcc tggaataata aggggaaaaa 130605
aatatgataa gtagttgtca atattaaaac aggctgaagg aggaagcctt cgggccttgt 130665
agtaggtagc atgaaacagc agatttgagt atccacaatc ctttcagtat taaattttca 130725
gtaaaataaa attacttgta tatgaaaaca cagcctttcc ccagctctct tttttttcct 130785
gatcagctga tgaagagact agcagctcgc tgctttgctg gcttgttaat tttatcccca 130845
ctaactgtga tttctgatag ccggcctgct gatagtggta aggtaaatat cctttttcgt 130905
tgccgtcttg aagattcagt agaactacat cccaggcatg tgtaggtgtg taacacatca 130965
aaagccggtg ttctccgtat ttctgtgtgc aactgggtgc ttgaggagag caataccatt 131025
aactgttgac agccttgcat gctgtattta ttattagcat gttcagcctc caagatatgc 131085
gggacaattt ccttttctta atcttcgctt agctgcagtg tgtttagctg tatagtgggt 131145
gtcctgcttt taggcaaatg aatattaatg caataatgtg aagctttttt tttttttttc 131205
cttttccttt ttcctcataa ctcatcagat ctcgcctcct tcatagtggt tatctgaaca 131265
ctgtaggatc gtgatggaaa ttgtcttttg attattaagg cctaggctca gactgtccct 131325
taggaatctg tctgtgtgca tgttgcttct atggatttgc acattggagg atgcgtagaa 131385
aacatatttt tttaaaaatc ccactggcat ttttaacatg ccaggttgtt ttgtgttctg 131445
caccaggttt tcttcacaaa acctttcttt ttcaggaagc gttgtacata agtgaaagaa 131505
attatgtctg agctgtaatc actctttata ttgattgtta tactcacatg atcataaata 131565
ttagccatgt ttggtgctgt ggagaaatga aggtaattgc cttatgatta gagctctttc 131625
agctacgaga aaggattgat tatcatttgc atactggagg caatattgag aggcaggagg 131685
aacaaataac aacattagtt attttgtgtg agagagcaat ctcaaaacgt aatttaaaga 131745
tctttttttt agaaaatttc ttttctatgt tcaaatttta tgttcttttt ttttttcttc 131805
cttggcagta tttttcccct ttatttttct tcttttgagt tttgagattt accaggaaaa 131865
aaaaaaaaag tttaaacttc tgggtctgtc acatggatct tttagccctt tatgaacagg 131925
tttcttttat acctggttgt ttttatagct attttattat ctctctgcat tagtgctgct 131985
ggctctggtt taaagcaagc gtttgcaggt aattggaaaa aagtgtttat tgtttgtgag 132045
tgtgtgtgtt ctttatcgaa tgatttaagt gcgtttacca aggaatgtgg ctttgtactt 132105
tcccgtttcc ttgtttacta attgccacgt tagaacaaaa atctagatgg aattttgatt 132165
tatattgtta atcatgaata aattgatgca aaacctgaag aaaaggtcaa agcttatatg 132225
atcgtgtgga tgtattctta gggattgttt tttcataacc cacccctcgt tttgtttacc 132285
ttttccttcc cttacagtaa tgagaacatt agggtcttgt tcttaaactg agctaaaatt 132345
tcatctggat tcagttatca tcaaacactt gaatttatag atcttttaat atttgtcata 132405
ttctccttta ctgtgagtgt tttgggcctt cagtgtgttt tgctcatgaa acagacttga 132465
gttaaagatg catttatcca atatggtagc attccttttt tcttcttcct ccactgatta 132525
ctgttaactt ttactttttt ttggttaatt tctttcattt tattctaatt gttggagcta 132585
tatataaata tacacatact ttttttgtct tggttattct cttgtcttga atttgtctcc 132645
tttgtttcca acgaacag gcc atc gaa gac ggc aat ttg gag gaa atg gaa 132696
Ala Ile Glu Asp Gly Asn Leu Glu Glu Met Glu
        1330                1335
gag gaa gta cgg ctt aag aag cga aaa aga cga aga aat gtg gat 132741
Glu Glu Val Arg Leu Lys Lys Arg Lys Arg Arg Arg Asn Val Asp
    1340                1345                1350
aaa gat cct gca aaa gaa gat gtg gaa aaa gct aag aag aga aga 132786
Lys Asp Pro Ala Lys Glu Asp Val Glu Lys Ala Lys Lys Arg Arg
    1355                1360                1365
ggc cgc cct ccc gct gag aaa ctg tca cca aat ccc ccc aaa ctg 132831
Gly Arg Pro Pro Ala Glu Lys Leu Ser Pro Asn Pro Pro Lys Leu
    1370                1375                1380
aca aag cag atg aac gct atc atc gat act gtg ata aac tac aaa 132876
Thr Lys Gln Met Asn Ala Ile Ile Asp Thr Val Ile Asn Tyr Lys
    1385                1390                1395
gat ag gtgagtgttt ggttccttca ccttgatcat ctctcaccaa gacgccgagt 132931
Asp Ser

ggcgctccct gaggagcagg agttgttaag ttgtgcactt aggttttatt aaggttcttt 132991
ctagtttgtg ttttatggct ttctctcttt gtacctgttt aaaatggcca ctgttgctat 133051
tttgtatgat gtttcatcag gtattattta agatagttga aaatttagtt tagattacaa 133111
aatgtttaaa atatctaaat tagaaaagga ttcttaagga cctctgaaat accatctgaa 133171
aatgaagata tttcagatga aagggattgg aatgatggag taatattatt ttttttaatt 133231
gaattgttag agaaagaaga atcctaatat atttaaggta aatatttcat gttaagactg 133291
tttaaagcaa aatagatgct tcatagtaaa tacagtttct cctttccttc caacacttga 133351
atttctgagc acttgaatta tctctttctt cctaatacca catagcatgg tctttcagca 133411
aatttacctt ttttatggga tttttggata cagactttga gaaagctgtg tgggacactc 133471
ctcccaggcc atttaaaaca gtgaataatt atttgaaaac caaagacact gtagggtgtc 133531
agtcagatcc atacagtaga tgaactagtt aggctacgtt ttggttagtt gacttggttt 133591
ccactttatt atcacgtggt aatgttttta tttgaagatg gcttctcgtt tcagttgaaa 133651
aattgacagg aaacagacat tttcactctc tcagttgcat agtggtatta atcagtttta 133711
aattaccaat tgaaaaaaat tttttcttaa gggggtgcat cattcctgga ggtactgcag 133771

taccaggtcg atgtgtggag tggatgaagc aagctcctct tctagctccc tgcttccaaa 133831
atccacttaa catattgtcc tcggatagag gaggaatcag atattaagca tgtaacaaca 133891
gatactacac ttggtcttag ccaaaaggct gagaagcagt tagttaccag aaatgtttga 133951
cattaactag tgttgctctg tgcacctgta agtgtttaac atgaataact acgttgtgtt 134011
tttagttttt cattatggct ttgccgaata catcatagca ggaaggaaaa attattataa 134071
aaattaagtg gtataaaaag caagaaactt tccaccatgg ggttttgctg ttaagccttt 134131
cttatctgca aggagttttc agaaatgggg aaagaggagt aggtggagat gtttgtcaaa 134191
aggccagaaa ttcttgctcc atttgcccca ccaggtctct cagattttgt gctgttttcc 134251
ttttttcaaa aaataaatta agttcatccc ctttaaggct tctgaagtgg ccagaatggc 134311
aaccccattc tcttgggttt ggaccaacgg ttcattaatg aaggggaatg acagcaatcc 134371
aggaaggaaa ttttgaccgc aagtccatgc tttgtccaga gtgaacatga aaacttcctt 134431
gtaaaccatg aatcagtaac tcttacgttt ttactcaccc ccaagctccc cttgtatatt 134491
tgccacagaa agggacagta ttctagaagc cagcttggat tattatgccg tattttctca 134551
gtccctggtt taaaacaaaa tattactaag tagagagcat cactctgtat atacatcaaa 134611
cttttttgcc tttgtctcag gagagtgtaa cgtctgtttc tctgaccaga tgggctgtga 134671
tgtgtgacag gcattttgtt cattacttgc tgagtgagtc aagggctgtt ataacactga 134731
gaagggccag gaaagtcctt ggaggttgag aagctaggcc tttcattcta aggtgatggc 134791
tgtgtctttc tctagtgaca taaaagaagt caccaacagg tggatgacat actgccaaca 134851
gattcgtttg gttttatgct tttttttgag gggtgggggt cagcaaatac ataaacctgg 134911
agagatttct tgcaaaaatt tccctcccat cccccaccct tttgaaaaac cagaaactct 134971
ggcagcactt tgcccagctc tccctaaggt aacaatcagt aggcatgaag aagtggccat 135031
cctttcattg gcagcatcgc gctctaggtt gtcacaatcc ccgtcactct gtatcatctc 135091
ttcagtatgg agtgcgtcgc catttaccgt catgcttttt acgcccgact cacttcattc 135151
atttgtatta cctgctggct ccatagcctt tgagtttatg acttcctggt atacatggct 135211
gtgggctgac cactttccta gaaaccatcc agaaatacag attggtagga gattatatgc 135271
cttaaacctc agctaagcat tgcacagtgg tttagagtac agactcttgt ctgtcttgat 135331
ttaaccacct gcatttgaat cctgactctg acattcacca gctatgggac agtggagtaa 135391
tttacttaat ctccctgagc ctcagtttcc ccatcccaga atcccagaaa tgggattcat 135451
tactgcgcct gcctctagag tccttatgtg tcatcaggga tttaatgtaa ctcaggttct 135511
ttgaacagtt tcattattaa taatcagcct ttcagacttt gaggctaatc aggtaggtga 135571
atggacctct gagcaccaga aagaaagtgt ccttgctgat gctgatttcc atggttttat 135631
ttctagaaac agaaaatgcc gtttcagtgg tactgtttat tgagtatgtc tttagttgta 135691
ctgtaggtaa agggtatttg aatatttcag catgcagata ttgagtagct ttttcttttt 135751
aacttttcct tgttttattt gcttacttac ttttccttaa aaagtgtcct ggtaagacac 135811
attctattag ctcccacatt tagatgcttt ctggggagta tgtactaaat ttcccaccac 135871
tgaatgtttc atatacattt catgttggta atcctctaga aaaagcacta actatttata 135931
ttttgtattt ctctttaact tggtactcaa gtaggaggca ctcaaattag gagagcctta 135991
attagataca gtatcatatt tttggttggt tttagaaaat gctgctggga gatgtacatt 136051
tcactgtgtt gagatttttg caccattaaa gtcagttatt taaatagtaa ctcaacccac 136111
ctgtgaaaga aataccacct gtgctaacct ccttcccatc actttggtca aatgttaggg 136171
ctctgacagt catgtagatt aacagcatat taaataagaa cagccattta tatttaactg 136231
atatataaga agaattaatt agctctcatg agacagttgt ttaaaagttc atgcatgatt 136291
ctggggcaga tgtatcttga tattttcata aagaactaag atcatttatg tgtgtgttcg 136351
ttgcttttta gtcagtgata ggacttgttt tttaaaaagg agggtgcttc taaaaatctg 136411
acccttaaga gccagatatt gtgtgattca taagtgtaaa atccttctgc agtaactgct 136471
gtctattcag aggtagtact ggtctgttgc tactggcccc catcatttta tttgcacaaa 136531
tagaaaactt tatatcaagt agtaccagaa ggctttgttt tgacttgtta agcatgcttt 136591
gttttctgct tatttagaag cttttaaaaa ctcatattct caatttatgt aatatttaac 136651
ctaagactgt tctttgagaa gggattcatc ctgtttgcat ttgtaccctt gaaaattgcc 136711
cctgggatga gatgtagagt tagacggtaa agagtagagt ctgtggagct attccaactc 136771
tagagacatg gctttcagtt acttggataa agaagagaga gagattccta gaggcattat 136831
taacgttgga agggatgttt agctctgcct ggcccaactt cccacaatac aagtaagtcc 136891
cctgtaccac ctctggcatg tggtcatcca cctccatgtg gacactgcag tgtgagctcc 136951
ctgcttcaga gcagttcttg acaccaaagg acagttctaa acacatcacc gtttcctaca 137011
ttaaaaggga tgcagccatc tgacccctag ctatttgttc tacttggaac ctcagagaat 137071
aaatctgctc tcattctgag aaatatcctt taaaatgtat caacaagcat atctacctca 137131
ggacattgtc cttcaggtag gaacacttca ctttgctctc tgagtgatgc ctagacattg 137191
gccacattcc agtctcctag tgtcccattt aaaacatggc gcccagaact ccaatggggt 137251
attctgatca ttaattacag ggtggttagc ttccttattc tggacaccag aattctgtta 137311
gcataactca agggtgtgtt tactttttcc agcaaccttg acacagagtt aactcattga 137371
atatgtgact tcttgaaata acccccactc cccaccccag aacacacata cactgctctg 137431
atttcacatg aatactgcca aggttagctt tcagataagc ataatttttt tgaacctatg 137491
cctactgatt gtattcttat ccctgtgtag ttccagcctt actacttttc atcccatcat 137551
ccttgcatga tgctgtacct ctaaattttt atctatgcaa aaattatgtt tactatgttt 137611
atgtatctaa gttttttcat ccaaagattt gatataagct attgcttaga aacttagaca 137671
aaggacaggt ctctgtgaca tacccctgaa aatcacttca ggttgacatg aatatattaa 137731
ccagtaatcc ttaaggttgg ctacccatca gtgaactcat atagccatgc tctcatgtca 137791
cccacattta tcctttctca gcattttcaa gaaaggctaa aaccatgata caccatggct 137851
agcctttcca atctatcacc ctagaaaccc tgccgaaaaa gtgaaatgag gttagcttgc 137911

catggctttt tctaagagac ctcctgctgg cacttaatga tgttatttat cttttctaaa 137971
tagccctgtg gcatctgaat aatttcttct aaatttttac caagggtcaa catgaagtgt 138031
aacagtctca aattttcttt ctcatattga aattgggaca tttgcttatc tctagtgttc 138091
tgattccatt tttgtgctct gtgatttcct aaatgttatt agcagaagca atctacatgc 138151
ttttccagga tttaattagt ttgcaactgg acacttgaac ttacttgaag tggcttgata 138211
tgctctttct atcaactctc ttatcaggag cttttgttca ccatttatta tgccttgctt 138271
ttcaaatctg agcattgttc tcattcctga agaatctgga aacagaaagt gaatagggtg 138331
tcatcacctc ctccctctct atcaattgct cgaagacaat tctttcagaa gcagtccaca 138391
ggaagaccag ccctgctgtc tgtaaaaggg tttggacatc aggtctagcc ttgaagatgg 138451
atgcagcaaa gtgatggagg aggcaggaga tacctgcctg gtcagtgcct ggtcagtcca 138511
attggtgaac acagctccgg ggcacagagg ggtggcagcc acgtggctcc tgcctccctg 138571
acactcttca gtctctgctg tcttccccta gctgggaatg tgttcttctt gcccctaaat 138631
ttaataagcc cttgtggtat ctctgggaat tgtggtaaga ctcagcttgt cctgaatttt 138691
tagttctcac attggagaaa atcattcatt ctttttagaa atatttcctt cattgtttgc 138751
ttatttccag gactgtactc ttgaatataa ctatagggta aaaaattcca ggtacttacc 138811
cccactagct catcatgcag tggaagagat agctttccta tgggatatta ctgaagaggt 138871
agctggcctg gggaattcag ggaagtaggg gtcgtatgag ccccattatc tgaggataca 138931
atgacattac ctggtactta taatcaattc aggacattat aataatattt acctggggaa 138991
atgagctttt tttctttttc tttttttttt ttttttgaga cagtttcgct cttgttgccc 139051
agctggagtg cagtggcaac atctgcctcc cgggttcaag caattctcct gcctcagcct 139111
ccagagttgc tgggattatg ggcgtgcacc acgatgcctg gctaattttg tatttttggt 139171
agagacaggg ttttgccatg ttggtcaggc tggtctcgaa ctcctgacct caggtgatct 139231
gcctttctca gcctcccaaa gtgctgggat tacaggcgtg agccaccatg cccggcaaat 139291
gatctgatat tttattacat tatagcagct ttccaagaat ttggtaaact acaaacctgt 139351
ggactaattg tagttaattc ctatacacac tgaaaagtaa tttgcagcat ttttctcatt 139411
ttgagtaggg ctgaaactct gctgaaggcc tacatataat cagcccattc tccagaaggg 139471
cagggctctt atttcaaaat attgccttgt tccaaggctt cctctgtctc ggaagttctt 139531
tatttgaagt gcgttaggta tttactgtgt cgaaggcttt cccacactga ttatcatctc 139591
aaggcttcat caaaccaatg gatctttaca ctggcattca tactgtcttt ctaaaataag 139651
ttttcgtaac aaacatttta aaccaaatct atgtcattat gtagttatta ttttttgttg 139711
ctttgtttta gggttttaaa aatttgtatt tttttttccc caagccaatc agatggctga 139771
cctgcatgga ctccttatca gaatgatttg aaattattct gctgggatct ttggttgttc 139831
agaacccact gagttgtaat ctgcgtcagt cttagatgac agagccatac ctagcttctc 139891
tgagttagtt acaatctact ctgtaaagcg ttctaaggta gcacttgttg ttttcaggtt 139951
ttcctcccgt cactcgtgca cattcggaga tgacatggtc tacttaagct tctgtgtctc 140011
cttggtcagc accatcatta gcccaaggtc ctaaaagtga aataatgaag actttcccaa 140071
ttctgtgcct tattgctgac actcagagtc ccctcaacct gctcctaatt gtccctacaa 140131
gacacccgtt cacctgcctc ctcaccccct gtcttcctga ggcccttgca ctgctggcct 140191
ggcctggcaa gctgcagggt ggactgcgtg ccctgcctcc agtctcttcc tgtcctggct 140251
tctttctgag gcacctaact tgtcatttca tattgtaact ttagaactct tcacagcggc 140311
cccgttgcct acccgatgat gaccagactt cttagcgtag gagaattgtt atcttccaca 140371
gtctgaacct tcggatcttc ccaacttttt ttttccaacg cacccctccc agctctcctt 140431
atatttcaga cattccaaag aattctgtgt attttgaagc gagcacatgc gcttccaccc 140491
ctctgttgat ttgtttatgg gtttatgctt ttccctctct gcaacacccc gaccccacac 140551
tgactctaga gcagaatggg gcagtgactc cgagaaggga tttatacatg gacaggcaca 140611
ggctgtgaat cccaaagggt gctgtctaca cctggaggga gatctagagg gtatggaggg 140671
tcttcccagg atctgtgaaa cctggcagct gcttccctgc caccacaaaa agggacacca 140731
acctagcagt ttcaaaaatc ctcaactgaa gaggcttttc tagaggcttg tagaagtgcc 140791
caagacgcca ggtggtggtt tattttcatt tcccactaaa gatcatgaat tcagtgatct 140851
ctcgaaaagg aatagagctc ttggaagcag agctgactag ttagatggtg ttcagaccca 140911
aattggctga tgcttggaat accttctttg tgcaggctac tgtaagaaag cactttatcc 140971
tatttaattt ttataacttt ctgaggtaag tatttccccc attttacaaa tgaggggcta 141031
aactcagata gactagcact accttcccaa gatcacacgc acctctagcc agtggctgcc 141091
tgtctcccac cttctggaac agtgaatgga acatgtaaga gggaacaggg taacaggaat 141151
ttctgtgtgt gacggaagta ggaaaatccc agaaaggtta ggaaatccac ttcccccacc 141211
atttttagag aactatgtta tttttccgaa tgaggttcca aacataaccc cgtgtaatct 141271
tcctaacaag gcaggttggt gaggagactg aggcttggcc aggtcaccca gcctaggaag 141331
aaggagccgg gcggggacga gaacccaggt cttctgactc tagtgttctt tctactctac 141391
cgcaggtgta acgtggagaa ggtgcccagt aattctcagt tggaaataga aggaaacagg 141451
tcaggatctg tcttgtattc ccctatctct aaatacaggt atccctcgtt acgtgaaaca 141511
gattgaatca tataatcggc ctttggaagc aaatttcttc ggtcacctcc tgatcacccc 141571
tacttggaga gcgggataga ggcacagata ctcttaaaca gctgtcatgg cttctgaagt 141631
tgttggtgct gtattttaat ctggctgtgc caattctata catgcactcc ttacaagggt 141691
attgggagct cctggaaagc ccgccctaac tgcagcatct tggagatggg tttctgttgc 141751
ttccccctcc cttccagcgc agcttctgtt gtgtgttcct tgggcctctt taactctgtg 141811
ctgtcttggt ttagaagctt aatgcgaagc acctgtagtg acttgatctc ctgcgagaca 141871
tgaagaagcg tgcatgttca cgctgtgtct gcattccaga gttgatcatg gcatgcagag 141931
ggcaattgct gagttgtctc ttgtttgtgt gatgggttgg gtttcagggt gtgactcctg 141991
ggcctacctg ttgtgtgtag tcccaagcat ctaaagccag tctctttcaa cctctgtgtg 142051

cctttgcttc tgttgggtag aaagcagaaa tgcttttctc ctagagatag tgtgaatatt 142111
agatgacatt ggttgagctg gagagatgga tgtgcatgat acgccaaggc taggagctct 142171
tattaatata agagagataa catttctttc tccatttcgg tcaacctgta cttaaaaaaa 142231
aagttgatac agtgctttac catccatatt tcaatttttt cagctgaccc aataacatcc 142291
tttacaacat tttttccacc agcataggat ccaatctagg actggagaga gagaattgag 142351
gatgtatcgt gtatttcaga atgaacacag atcactggtt gtggtattct tctttatata 142411
aggagattaa aaactagtgg tgatttatac ttatacttat ccatttttgt gtggagatga 142471
aactgtcggg caagattgtc aatttttgat atttcttatt gcagtccagt aaacaatgac 142531
taatcctgga aaccaggcac ataaactata atgtccattg agaaacgtca ggaattttgt 142591
tctgtatttg ccattatgag aaagaactca gtctggattc tgacttggag attgaaacaa 142651
cgaatagttg acttcataag tttgggcagt ttaaataatc aaccaacttg ggtaaatatt 142711
tccaccccta atacctagaa aggaggcctt agggctaaat ggcatgatgt aagctgaatg 142771
ccctgcccag gaaacaggga caggcaggag ctcctttccc tctttgccct cttccatcaa 142831
gatggtcatg tgaccacgaa aagtagatca ataactttaa aggctattta ttgcaagtcc 142891
ccagtgccat gcccaggctt tttgagggga gatgacatga cggtatatgg ttaggaacta 142951
gataaattca ttttcactaa agacacattt aaactgtaat ttccagagac atagcagtgg 143011
gcttggtgtt tgcttcctca gggctctctg gagtctgtct tgtcccttgc tgtctgtcac 143071
acacacttcc ttttcccata agcctgagct gacaacatgg tgtttggaga ttgtattatg 143131
atgaattaac cctttcttca gaagagcttt taaaaaatct ttgcttcagt ggtcaaaaga 143191
ggctctttgt aagtactgaa aatggaaaaa aaaaatgaat taaatggagt tatttaaagc 143251
acaattggaa agagctatgt caacaaataa tgcaagtacc tggtatgaat gacacatata 143311
cacagggtgg gatgggaagg gatggggcag gagtggagag cgcttctaac tggctgtgag 143371
cagagtggga gtttctcaaa cagacgggtg aagggaggat gggtgggggg gcagagcttg 143431
tggtggtatg tgcgggaagg ggggatgtag ccatggaggc ttggaaagca gtccccctct 143491
agagactaca ccatctcagt gtgaccagct cattggagac gaaggaagac tgggacagcc 143551
tggatgaggc tggagaagcc tgagaacaga tcccggaggg ccttgaacac caagctgagg 143611
aggaattttg atgtactctt ctaggagtgg ggtcttgagg gcaacctgta gtcatcgcag 143671
tgactgttac gagcactgtt tcaaggaggc caacggagag cacacctgag ttctgggcgt 143731
gagcgtgcag ggaccagtgt gtgatgtctg tcatgtggga gaggggaggt gacacccagg 143791
gagccgacat cagccctgca gtggaaagta gatagccaag agaggatcat ggggatacag 143851
tgagatttgt gttcggaaat gtcactgata gtctgtgaag gatggatcga aggaggcata 143911
cattaggaga atgttgtctg gtagtggagg aagatagtcc ttaaaataag gaagtggcag 143971
taggtaggaa gacaagggaa taaatattca gaggtaggat tgacaagact taggggcagt 144031
ctggatgtgt gaggaggagg aggaagagaa ctcacatagc tcttggatct ctgccctgag 144091
tgatcagtga acagcggtgc catttacaga aatgaaggtg gcaggaaaat tggtgtgtgt 144151
gaaggcactt gtatgtatgt gtgtgtgcac atgtgcatgt atgtagttac ctggccctgc 144211
atacaccatc acaggacagc tgaactctct acttggccat tagcagccct tgatgttgtg 144271
atgatgttgt tagtattgac tgctctttca ggaccagcca acaggctctt gtaggcaaaa 144331
tttattttta gtaccagaca tcttgtatta ctgtaaatga gagcgagagg ctgtaatgat 144391
agctctcaag cccaagcacc tctgcattta gtggctctaa tttcattata gagaaattac 144451
catgaaatag cactgtgacc aattagatac acttctggga aattttaggt gtaggtgaag 144511
cacctttttt cattcattct ctgtttctga tagtttggac ccagaatcaa gctgacagtt 144571
tatcgtgtag agtagcagcc tcctgaggtc agcgcctcat gcattgtctg gtgcatgagc 144631
atttcatgat gcttcccaga gtgagttctt ccaactctag ccttcctact ccttcttgga 144691
gattcccagt acagccagag gccctgggaa gtcttgctgt aaagtcagct gtttaactgc 144751
ttaacccagc atctcccaaa cttatttgac caaagaaccc ccttttcacg ggacactgtt 144811
actaacccat taagctagtg ttctgtggag cacactttgg gaaatgctga attggagccc 144871
aaatattaca ttcagtgcag agagaatctg ctcggagatg tggactccag tgctttattt 144931
tgccctcatt acaaatattg atactttaaa tgtttgctga aacaggctgc tttgactctt 144991
ggtgactcga tacttattta tggtgctcag ccagttatgc ccacgtacag caggtgggca 145051
gcccacaaat aaacttagtc cgtctgtttc ccgagacaca gaatgcttcc ccttatcttc 145111
cagcattagc ctggccctga agcccctctt tcgagggtct ctgcctctca agctccaggc 145171
caacaccagc ataaaggacc cctctctgaa ggaagggaag gaggctcagc tggaaataac 145231
aaaatggatt ttcttacagg gctgacacac tgttttccaa accactctat aaatgttttg 145291
attaaaaagg gattgctctg agagatcgca gttatttgga tatatatatg tccttgtata 145351
ttaatggggg aaaaaaatct gtctcactcc tttagaatca cacctaatat gtcttccttg 145411
aggatttatt acaacagcca ttgtaggaaa aagcagcact ccggtgcctg ccacgaccca 145471
cccatttaac aaagcagcgt gtgggacttg ggcacagatg gagaggcatt tgattttgtc 145531
tgcagaagct ttgtgctttt taatttggtc gcaattcagc caaaagcttt gaagcatttt 145591
accattaaga aatggggaag caaaggccag gcacagtggc tcacacctgt aattcccagc 145651
actttgggag gccgaggcaa gcggattgct tcagctcagg agtccgagac cacccgaggc 145711
aagatggtgg aagcctgtct ctacaaaaat gcaaaaatga ggtgggtgtg gtggcatact 145771
ccagtagtcc tggctactta ggaggctgag atgggaggat tgcttgagcc tgggaggtcg 145831
aggctacagt gcagtgagcc ctgatcgtgc tactgcagtc cagcctgggg gacagagtga 145891
gaccctctct caaaaaaaaa aaaaaaaaaa aaaaaaaaag gaaatggaga ggcaggaaag 145951
gatcaaagga caggatattc agtggaaggg aacgtgtggg ttcttacatt ttgaaagaca 146011
gggacgcttt gagtcatgag cgcgtgtaac gtctacaagg tggggtggga aaaactgtag 146071
aaatggccgg ctgaggaagc tgcgggtcat ccggtgtctg tctgtcatca catttagtca 146131
gatgggcagt ggagttctgt acctgcaccc actgttatga aacgtcaagt gagaaagagg 146191

gcagaaaagt agccatccta agacagtatt tccgaaagct gagtgatagc tgacaaatct 146251
actcaaaaac attttttgaa aatgcttgtc cccgcccccc cccaacaata cacacacaaa 146311
aacactctct ggatcttacg tcctgtttca agcacctttg ctttgtagta ataaatgcaa 146371
tgggagaatc ctgaagaaat gcatagaaac tgaaaagaag attctgatgg ttgcaacata 146431
caacatatta aaacttttgt gtaatgattg tattctttta tttgtttggc tttatgtgat 146491
agttaaaaag tgccagttgg taaaacgtgc tgcttatggt gataaaaata aaggttggtc 146551
ctatgatcct taaacgtaat ttctttggaa aaataaaatt agatatttga cttatggcat 146611
gttttcaatg aaggtaaccc aattaaagtg tttgttggga cctactttat ttatggataa 146671
aatggttcta aattacattc aatcataaat cttcattttg gtgaataaga gtaaattatt 146731
atttttcatt atgctttggc atatctcctt ctggtcgttt ttcaatgcct tttttaaatc 146791
cttgttgagg ttgaagttgt actgcactac aatttgatat cctctgtttt tttgggtttt 146851
ttttgtttgt ttgtttgttt tttgagatga agtttcgctc ttgttgccca agctggagtg 146911
caaatggcac aatctcggct ctctgcaacc tccgtctcct gggttcaagc gattctcctg 146971
cctcagcctc ctgagtagct gggattatag ccatgcgcca ccatactcgg ctaatttttt 147031
tatttttggt aaagatgagg tttctccatg ttggtcaggc tggtcttgaa ctcctgacct 147091
caggtgatcc acctgcctcg gcctcccaaa gtgctgggat tacaggcatg agccaccgcg 147151
cccggcctgt tttttttttt tttttttttt ataatgacgt aacaagcatt tttttcttta 147211
aaatacataa ttttagactc tcttgcattc tattacttgt tctttccaca actgttaaac 147271
gttttggctg ctttgaatta tttattttaa gtaatcctgt catgaacatc ttgatgatta 147331
tctgtttcag cccagagtag attaccaaaa gtaaaatcat gcatcagagt gtgattgttt 147391
ttaagactct agacaaagtc ttggtgcctt tcttattgag ctgagtgaac ccttttcagt 147451
ctcctaacag ggaactttgt ttgtcttggt aactcaggga cattatttat ctgtagtcct 147511
ttatgactat tagggtgatt tttttggtgg tgttgagaca acaaagtcag ctctgttgac 147571
caggctggac tgcaggggca tgatctcagc tcgctgcgac ctctgcctcc tgggctcaag 147631
cgatcctccc acctcagcct cccaagtagc ttgggactac aggcacatgc caccacgcct 147691
ggctaagttt tgtatttttt tttttgtaga gatgaggttt ctccatgttg tctgtgctgg 147751
tcttgaattc ctgagctcaa gtgatcagcc tgcctcagcc tcccataggg cggggattgc 147811
aggtgtgagc cactgtgccc caacattggg gtgattttaa cagctactga ttccatgacc 147871
caaacaagtc tactcccatc ccaccctgtt ttgctcttgc ttcaagggtc taggtgatga 147931
tatttccaaa cttgttagga attatcacca atcaatactc aataaggctg agatgagaga 147991
tctcagtagc aaatatggag tgcagatgct tgtaacacct gcttcatgac tggctcaggt 148051
ttctgcaggc tctcttgata agtaggggca ctaatgtggt ctagtaaatt cttcaatgat 148111
atatattaaa cttcatagga acctgaacct gaaaagaagc agcacacttc acctactgcc 148171
ctgcagtgtt gacccaattt ttaaagtttt cttcccaaac gactttgcta tgtttgattc 148231
ctttaggatt gtgatagctt ttctgtaaaa tccttccaac ctcaagtgag tattctaaat 148291
catgacatca ttttctaatt gaagtaagat agtcacattt taacccttat taatataaac 148351
aatttatttc agctatggtt aagatttctt tctcagcact atctattagc tattctggtc 148411
aacagatcag cttgagcagg taaaaaagaa aaaccataaa agaccctcac taattatcaa 148471
ttttcgcctg gtcctggaaa aaaaaatcat taaaagacat aaccagtatt ctagaagtag 148531
atttcttttt ttttttgaaa ctgagtttcg ctctcgttgc ccaggctgga gtgcagtggc 148591
gcgatctcag ctcaccacaa cctccgcctc ctgggttcaa gcaattctcc tgcctcagcc 148651
tccctagtag ccgggattac aggcatgcgc caccacaccc agctaatttt gtatttttag 148711
tagagagggg gtttctccat gttggtcagg ctggtctcga actcctgacc tcaggtgatc 148771
ggcccgcctc tgcctcccaa agtgctggga ttacaggcat gagccaccgt gcccggccag 148831
aggtagattt cttaactagc atatcagcct tcagcatgtc tctaagctta aaatataatc 148891
aaagctaaag ctgacattta tgtaaagttt ataatgtatg cagtcaccag tttagagtca 148951
tagtatagat gtggaattat acttttggtt tgtggaggta atgaggaaaa aaaaaaacgg 149011
ttcccagatg aagcataaca aattaaatct cagtagtgtg tgtatataag ccatgcatac 149071
aaggagttca aactaccact tgcacagtgg tatgtggctc ttttccatct cgtactctat 149131
ggtctcatgg ccacgtcgcg gtgtctgttc acttccatga agaggagagg ttcttgtcca 149191
ccaacacctg tgaaccgtaa gaccagtgag tgcctttaaa cttgtatttc aagttgtacc 149251
cctcccccac ttaccccatg tctcatcaga gccatacttt ctagcctgag gtagatcatt 149311
acgtaaaatc catccgatgt cacaggccat cagtggtctt ggggaggggg tctcattttt 149371
gtctgggaag tagtgtactt tccaagtagc aatttatatt taggccttaa attgtgtaaa 149431
atgacataat ggaaaacacc ttgggatagg ataagcattt caacagaaac agaacacctt 149491
ctgtaaacaa cattgtacaa ttagactatt gttctgtact taaaaaggaa aaaagcaact 149551
ttctggccgg gggcagggtg gtgcatagaa aaccaccaca tttcaaaagg tgtattaaaa 149611
aaaagccctc gctcagctca aattagagaa agaaatgaca agaattcaga actgtcaggc 149671
tgcttcactt ccttttttgt ttgtttgttt aatttttttt taacctgtaa tttaggaaaa 149731
aaataaatta ctccagcaag tgttcgtgct acctcaaggt attctatagg taatgtggaa 149791
gctaggtact gcccaggacc tcctggcttt gtctgccgaa gctggccatc caatcccttg 149851
ctctgggagg gagggggatg agtttgatgt ctgtaagaga accccaccct gtagggagga 149911
gagaaacaat aacacacatg gaagaattag gttctgagca atgaggggac tgagagtaag 149971
tgtagttgat atttaggaga atttcctcca tttttctgga caatgggttc ttggtcctgg 150031
agggataagg aaatacttgg agaggagaaa aaaccacaga gaactttcca gctatggagt 150091
gagagctagg tcaggcctgg agacctggaa tgggtcataa agaggggatt gcccatgaaa 150151
gttgggggta cacagtagag actctcctga ctagaagaga agtgtgttgg tggtaccagt 150211
tgggttttta gagaatgagg acatatcctg gagggctttg gaagccagac tctgagatat 150271
ttcagtgatg atggaaacaa catatcccaa gaagtcttcc atagataact tccaggtcct 150331

ccttttgcct ttgtccttta ttcttaggag aggaaaaaag aaagtgggat aggtatatta 150391
agtccagtga gcctttattt tttaaacaga ataaatgtgt ttatttctca ttttacagta 150451
ttggccccaa gtctcagagc attcagctct cttctttatg agcatagaga actctggact 150511
tgtctcctta tggggtttag aatttttagg gtcttactga agacagagat gtcataccta 150571
gattgtttta gccttacccc ttagagcttt gttgcactat cggtcacaaa ctgcttattt 150631
cttatttatt tcaccagtag cacagttaaa attaagagtg tgtccatcac aggtgtacag 150691
agatgtgcct gtactccatc aatagaaaca tgacaccatc tgttggcaat gtagaagatg 150751
acaccatgaa gatgtgtcat tgagggagag tcaggtgtta aatccgcgat tacaaagagg 150811
tctgactttt ggggtgctgc tagctgataa agaatgctca ttaagcaagt tgctctctac 150871
aaattactct cactgcattt cataggcacc ttttcaaaca gataaaatgt ttgggtgtat 150931
aaatctcaga atggtaggaa aagaacagac cagaaattgt ggccctgtgt tgaaggtgat 150991
ggtaaataaa atagttcttg tcatatacta tgtggcataa gttagggaag gacgaacgca 151051
tttctcgaag agatgtgagg ggtaggggtg gttgagaggg tgagagaaca ccttggttct 151111
tgctgcagtg atccagaaat ccaattcctg agtaaaatgc agtcttctta aacctcttgc 151171
gttcacccct gattttcgta ctcatgaggc ccatatgtgc attaaaaaaa taaaacaaaa 151231
acgatgaccc agtgtagcag aaaaactagc ccatctcttt gacagaggat tttcggttaa 151291
tttatgcagg cctctttgca cttcagcatt ttttccactg gttgctaagc aactgtttga 151351
gttactgcgc atgccttttt tatgataact gtcattatga ttacttttta attatacaac 151411
tgggactgtg gattaaaaca gcatgttgtc actcatgctt tgccactgcc ttctagtatt 151471
ctcacaagct cctagaatca ccttctgaaa tttttatggc agtgatttat gattaatttt 151531
tgatatttct ggcttcacaa aggaagtaga gatggcatcc taaggggagt gagacttttg 151591
tgaaaaatga ctgtatcaca caccacaagc tgttattgat ctgagtagac caaatcagat 151651
attaggatct tgaacctaaa ttgtgaatct ctttgtgcct ttaaccacat ctgcttccaa 151711
cagtaagtga tgttaaaagc tcatatgctt tgtagcctgt gtttgaaaat tacaaaaatg 151771
ctaagcattt gttgctatag aacctgcttt caaataaact taccatgaat ttgggggagg 151831
gtacagcact aatcccagaa atgtgaagca tccacactta agcttgtgaa acatgttctt 151891
ctttcaccca aattgctcta cacacacaca atggcagtta ggcaagatga aggggttcct 151951
ctggcaagta ataataaagg tatgtgtggt ttacagtatg taaaataccc taaaggaata 152011
agaccaagaa aatagtcagc cagtaggtac gtacgaaggc ttttcacttg agtttccaga 152071
gtatattcta acatattatt ttgagttact ttctaaatac aatgtgaatt tgaaaacaaa 152131
ggagtatgga aatacaatgt ttaaatatgt atatttttat atatatgtac gtgtatatat 152191
atacatatat atttaccaat tcatttaaaa atgaaatttt tagctaacaa ggatatttta 152251
aatatttcta tcaaaataaa gagatatatt tgctctgaaa gggaaatcca cagtgtattt 152311
cccatattgt atgtgtgaca gaagtgggga ccaaattgta ttttactgtg atcttaaaat 152371
tatcacaagg gacatcaatc tccaatagag ttgtgggatt tttcctgact taaaaagctc 152431
catatctata tgcgtggaat ataatttact ttgttgacat gttctgaaat aaaaataaaa 152491
cctttcctca gtaatgtttg atgtggcttg tttttgtttg tttcacccat cctacag t 152549
tca ggg cga cag ctc agt gaa gtc ttc att cag tta cct tca agg 152594
Ser Gly Arg Gln Leu Ser Glu Val Phe Ile Gln Leu Pro Ser Arg
                1405                1410                1415
aaa gaa tta cca gaa tac tat gaa tta att agg aag cca gtg gat 152639
Lys Glu Leu Pro Glu Tyr Tyr Glu Leu Ile Arg Lys Pro Val Asp
                1420                1425                1430
ttc aaa aaa ata aag gtagatattt tgtttaccaa ctttattctt caagtaaata 152694
Phe Lys Lys Ile Lys
                1435
aaggagcagt gtaaagtcat tgaaatggtt gtagttggag ctgaccgcca ctgatgggta 152754
cccagggctc gcgacaggaa aggttgggat gtccttgtgc ttttccgtga gtgttactta 152814
atcatgttga gagctcacgt tatattgcat tttattgaag agctttcagg ctccctgctc 152874
cccctgccca cattattgct cttctcattt cccatcatat gctcctcctt ggcaaaagga 152934
atgacagtgg gagtgggagg gtggacttgg tgaatggcgc cccctggggt tatgcagtcc 152994
cagatccctg gcagggcttt atgctgtgaa gacaaaggga aaatcaggct ttgttaacta 153054
agtaatgatc gctgaatttt cctctccctc tttttttctc cattttctcc aaaatttcca 153114
tcag gaa agg att cgt aat cat aag tac cgg agc cta ggc gac ctg 153160
Glu Arg Ile Arg Asn His Lys Tyr Arg Ser Leu Gly Asp Leu
                1440                1445
gag aag gat gtc atg ctt ctc tgt cac aac gct cag acg ttc aac 153205
Glu Lys Asp Val Met Leu Leu Cys His Asn Ala Gln Thr Phe Asn
1450                1455                1460
ctg gag gga tcc cag gtctgtcttg ttaagttgtc taaaagttct taactgtaaa 153260
Leu Glu Gly Ser Gln
1465
tgtgcccgtt gttcttttta agtagaatat ttcattttct acctcttgaa tcattgctac 153320
tggcagaaga aaaataacta aaagcctatg ttccttgcta cctgtgtaag aaaatagagg 153380
ctttggcatc tgcagattac ctttgtacca cagctgtgct agcgcctact cacacttctt 153440
ttcaaagctt atagtctttt tttttttttt tttttttttt tttccttttt tgaggcagaa 153500
tttcactgtc tcccaggctg gagtgcagtg gtgccttctc ggctcatggc aacctccgcc 153560
tcccaggttc aagccattct tctgctctca gcctcccaag tacctgggat tacaggcacc 153620
caccaccatg cctggctgat ttttgtattt gtagtacaga tggagtttca ccatgttggc 153680

caggctggtc ttgaactcct gacctcaggt gatccgcctg cctcggcctc ccaaagtgtt 153740
gggattacag gcatgagcca ccacacccgg cctcaaagca tatatttttt tttcgagatg 153800
gagtctcgcc caggctggag tgcagtggca tgatctcggc tcactgcaac ttccacctcc 153860
ctggttcaag ggattttcct gccttagcct cctgagtagc tgggattaca ggcatgcgcc 153920
accacgccag gcaaattttt gtatttttag tagagacggg gtttcaccat gttagccagg 153980
ctggtcttga actcctgacc tcaggtgatc tgcccacctc agcctcccaa agtgctggga 154040
ttacaggtgt gcaccactgt gccaggcctc aaagcttata ttctaattag aggagggatt 154100
caagtaaatc attgaaggta atgctgaaag gctggcagta ccatttttgc ttttttggag 154160
attgtatatt tgattacatt tacctttatt tttctgattg tactcttgga tcaggagctg 154220
aaactactag aatttaagac aaaacaaact ataggaaaaa aagaaacact gcctctaatt 154280
gctttttaat cctttttatc agcccaattg caattctctt tttactcccc ttctccacag 154340
tcaacaattc taagagtata taaatgaact gaatttatta aaataaccaa ggtgagggca 154400
ctttcatttt aattacacct caattactgt caccttatgt aggacttttt tcctgtctgt 154460
atctctggcc tgatgccttc agacccatga agaagaaaga aaaagacaaa gatgtacccc 154520
tcaaccaatg cagtgtcaag ccacctttaa attccttaag cctgtttcct gatcataccc 154580
ttctgcctgc acatcaattt cagtcatcaa acaaaggaat agagaaatct tcagaatgtg 154640
ccttgaacag acttaaacat ttccaagtta atagtagtag tatcttgacc aaaacaggag 154700
gtcttgaaag caagccttct gttactcaag acactctcca aggaaaaagt taagcttgaa 154760
gccaggacat agaaagtaga agaatcaaca aagaaggttt tgtgaatgac agacagtttg 154820
ttttagtctc tggatagata tttggattct ttatgtcaat atggcattat tatgatatac 154880
atccagtcct tttttctaca accttcctat ctgttttcac tctgactgtc tcacaactga 154940
aataaggtgt caagtggcct cttgactggt ttctccccca ggccatctgg acactaattt 155000
gtctttctct agccaccttc ctatcctgtt catttactgc ctctcttccc aacctttgag 155060
gttcccctgc actgtctaga atgaatttca tctctgtaga ctgcttgctg aggtctaagg 155120
taagtcttta gcttcatctg ctactcctca acctaaacag cccatagtcc attcggactg 155180
atactatgat cacctgcttc cccagttaca tttcttttac tctttaagat cattcgagac 155240
tcacatgcag ttgtaagata cagtacagag agattctgtg taccttttac ttctcaagat 155300
ggaaacatct tgcaaaacat aggataatat cttttttttt ttttttttga gatggagtct 155360
cactctgttg cccaggctgg aatgcagtgg tgccatctcg gctcgctgca aactctgcct 155420
cctgggttca agcaattctt ctgcctcacc ctcccaagta gctgggatta aggcatgcac 155480
caccatgccc ggctaatttt tgtatttttt ttagtagaga tggggtttca ccatgttggc 155540
cgggctggtc tcgaactcct gacctcgtga tctacccacc tcagcctcct aaagtgctag 155600
gattacaggt gtgagtacca cacctggccc aagacaatat cttaagcaga aaattgacat 155660
caatacaatt cccagatgca tctcttttat tcttgcctcc tcatctttct catttgtttt 155720
cctttctaaa aagcccctca tttctcaccg ctgaaggaat tcttctcatc tttaagatcc 155780
agctcaactc tgatgtctct ggaaaattct gccctgatgg tcccatcctc tctctctctc 155840
gcgcgggtcc tttgttctgt catactcata ctcactgcct atattgttca tttggtgcaa 155900
actgcaattt gcccagtcac ttttttgaac gagtaggtct tctttacaat aaaaggaata 155960
tgttcctaaa gagcaggggt tgttttattt tcctttaaat ttttaaaaca ctgtgattaa 156020
gagacataac aaaatttatc ataatttttc agtatacagt tcagtagtgt taagtatatt 156080
cactttgatg ggcaacagat cttcagaact ttttaatctt gcgactctga aactgcgtgc 156140
ctacgaacaa ctaactctca tctccttccc ccagcccctg gcacccacca tggcactttc 156200
tgtttctatg aatttgccca ttttagatac ttcgtatgag tagaatcata gagttacttg 156260
tctttttgtg actggcttat gcacttatgg acttagcgta acaatcctcc agttacagga 156320
tttccttcct ttttaagact gaataatatt ccattaaatg tatagactat agttgcttat 156380
ccactcattt gtcgatggac ccttgggttg cttccacctc ttggctattg tgaataatgc 156440
tgctaggaac atgggtgtgc caatgtcttt tcaagacgcc tcttgtgatt ctttatatac 156500
ccagaagtgg ggctgctgga tcatatagta gttctatttt caattttttg aggaaaagga 156560
ccatttttga acttatatct ttcagcacct acccagggcc atgctcagac atgtgtatcc 156620
attaaaaata gagtgttgat tggataagtg ttggctgcta ctgataaact cagtaataag 156680
gaatagcccc cacttgctag gggcactgca ttttaaagaa gattgcttta ccacagtctt 156740
actacagcat ttggtagaac aattcaaata agaaatgtag ttttaaaaaa taccattttt 156800
gttgtccaat atgaggatat tttatctact attctcctcc aagcatccat gcactttgaa 156860
ataatttcaa ctgcatttgg actttctgta tgaatgtgta tgtctgtatt tgggcttggc 156920
gttttgctac tgggttttca tgtgggcata aaagctaacg gtgacatttc tactaaaatt 156980
catgtgaatt aagctggtgt attgcataag gaagagttca ctgccatggt aactcagctt 157040
gcagttttaa cagatgcccc tttgaccatt tag atc tat gaa gac tcc atc 157091
Ile Tyr Glu Asp Ser Ile
1470                1475
gtc tta cag tca gtg ttt aag agt gcc cgg cag aaa att gcc aaa 157136
Val Leu Gln Ser Val Phe Lys Ser Ala Arg Gln Lys Ile Ala Lys
                1480                1485                1490
gag gaa gag agt gag gat gaa agc aat gaa gag gag gaa gag gaa 157181
Glu Glu Glu Ser Glu Asp Glu Ser Asn Glu Glu Glu Glu Glu Glu
                1495                1500                1505
gat gaa gaa gag tca gag tcc gag g gtaagcccag acattcgggt 157226
Asp Glu Glu Glu Ser Glu Ser Glu
                1510
cctgtacatc tttgcccctc ctcacctgca tagctgtctc cacagatgtt cacagaagag 157286

actttagagt ggggcagatc tggattggag cccttattcc actttgtcct tgctgggcaa 157346
ccggtggcca tgtccttatc cctgctcatg ctcagttttc tcatctataa aactgaggtc 157406
ataaaaatga cctccaagca tgtaaaatgt ttagcatagg ctgggctcct agcaaacact 157466
gttttatttc ttttatttat ttatttttga gacagggtct cactctgtca cccaggccgg 157526
agtgcagtgg catgatctta gctcactgca acctccacct cccagtagct cactgcaacc 157586
tccacctccc aggttcaagt gattcacctg cctcagcctc ccgagtagct ggaattacag 157646
gcgtgtgcca ccacgcccgg ctaattttta tatttttagt agagatggag tgtcaccatg 157706
ttggcaaggc tggtctcaaa ctcctggcct cacgtgatcc acccgcctcg gcctcccaaa 157766
gtgctgggat tataggcaca agccgccaca cctgacctgc aaacactttc tgaatgttgc 157826
ctttgaatat tagcctacta cacattttct acaacactgc tgctaatgcc acctatcttc 157886
tcttctttac cacggtggtt ctcaaacctg gagggcttga agaatatgcc agtgtttgat 157946
ccagtagcct gttgtggggc ctgagaatcc gtatttctgg caaattccca gggggaactg 158006
atgatgttgg gccaaggacc acaccttgag aatcacagct ttagaaaata attgtcaaac 158066
tgagttggaa ctgtgaattt tgcatagaat tttttttttg ccaggggaag aggggtgatg 158126
acagggtact gtctttattt aaaatgttga tagtttgttt atcatggatt tggggggcat 158186
taatttgggt ttttaaaata tttacctttt aggatattag ttgatgactg ggttttggtg 158246
cccccttaaa ttttacaccc aaggtgcgtg cctccattgt ttgaccctag ttccagccgt 158306
gactgataac ccaaatattc caaaggttaa gggtgctgtc agaagttgaa aatttcttct 158366
tagtgaggca tgttggctca caaatataat ccgagcactc tgggaggctg aggcagaagg 158426
atagcttgag gctaggagtt caagaccagc ctggataaca tagcaagacc ctatctctac 158486
aaaaatgttt gtaaaaagaa atagccaagt gtggtggcac gtgtctatag tcccagctac 158546
tcaggaagct gaggcaggag gattgcttag tctaggtgtt caaggctgca gtgagctatg 158606
atcataccgc tgccctccag cctgggcaac agaacaaaac tctatctaaa aaaaaaattt 158666
taaatataat taataaatat tttaaaacat aaaatttctt ctttcctatg tgtgtcttca 158726
ctgtatacct ctcaaagtaa agtactaaaa cctatgtttt acagtataag ccgagcccag 158786
tggctcatgc ctgtaatcct agcactttgg gaagctgagg tggctggatc acttgaagtc 158846
accagtttga gacaagcctg atcaatatat tcataataag tacaaaaatc atgccacaaa 158906
actgtgtgct atgtttttat aaacaaattt atgtgtggaa aaaaaggtaa cgtctcaaaa 158966
tattacatgt atgtagagat atagaattac acgttttttt accttttcaa atatattaaa 159026
agtatagaca tgcttttaaa aaattattag gtttctaatt acatgttaat acagatgatt 159086
tggaaaatgt aatgattttt gtacttatta tgaacacttt ccatggcaat gagggttttt 159146
ctacagcaac atatattaat atgtataata catattaaca ttcattaatg aatgtgaatg 159206
tactgtaacg tgttaattat actctgttaa tcaggctgtt tcccaataat gctgtcctga 159266
gcattttacc catagattat tgaaaagatg taaaatgtat gtgatgtata cacattgcca 159326
cagagaatgg agtttccatt cttgccctca acatacataa gcgactcttt cccaacgtca 159386
ttttctatta tctatattct agttagtgat tctgcctttt atttgttatg aatttaaact 159446
gtcccaaatt catgtagtta ttcctttgtt ctagcatttg aatacatctg catcatctat 159506
tttagctata tatttcatta ttttagagcc agatttctaa tctgttttca aagagctgct 159566
ctcttccccc atccatcaat ttagtttaat ttctcaagcc ttggtatatt aattttctat 159626
tgctgctata acaaattacc acaaacttag tgactttaat acaacacaaa cttattacct 159686
cttgtgggtc acaggttcag catgagtctc cctggactaa gatcaatgtg tcagcagcct 159746
gcattccttt ctggatgctc tagataggaa tcccatttcc ttgttcatcc tggttctcag 159806
cagagttcaa tttcttgtgt tcgtagaaac aaagttccca ctttcttgct ggctgccagt 159866
ggaaggctgt tcccaacagt gttctttggc tcattgcctc cccaccccta ccccttgttc 159926
caactttaaa gccagcaatg acaggtacag tccttctcat gttgtgtctc catgaatctc 159986
tcttttgcgt cttcctcttc cacgtttaag gatgcagatg attagactgg gcccacctgg 160046
ataatccact ataatttccc catctcaggg tcagacaatt aacagtctaa aattcctttt 160106
gccatgtaat ataacatatt cagaggttct tggattagga tatggacatc ttgtgaggag 160166
atgatattct gtccaccaca cttggtagct agaacttttc ataggtgcat atggttgtat 160226
cataaaacct taggaagctg tttatagggc tcacctaatg gtccagttta ttagcctgct 160286
tgtccatagt ctgtcaggtg ggttttttgg agagacatat ccactgacgc gtgcctgaca 160346
gcattagagt gcgccacact ccaaggcaca tttagattac gctctcaagg atacattttc 160406
ttacccctat tgcctgtctt ctgcccattc atttcctata ctcctgtgtc agggagagcc 160466
cacacctgta taaatggaca tatttcactt tgtctttcca cactgtttag aaatgcccat 160526
tatgactggc ttctttcatc tatttctttc gacgtcctac atctttccgt tttcattagt 160586
aggtaggcct tttcatctcc agtctcttct tcttctccta agttactcca cccaaagcaa 160646
agggccccac cttatagctt gtcaagcagc agacagtggg tataatagat agaagccttg 160706
gctaggagta ctattttgtg tattaggatt tggaatggag gaaaggcaaa tgctaccacc 160766
acacttgagt tcataacagc acagctctca tctcttctgt tagcttatgt ttctgtggga 160826
gattttagtt ttggaaatat tttgtgggtc cctctacttc acaaaagaag tctgaaacaa 160886
acactctcat ctccttctgg agctaggatt ccctctatga caacatctat ttctagatgc 160946
cttgagaccc accaaacatg gtagtctagc aaagctagct tgaatgagag acccacattt 161006
tcagtgcctt catcatcaaa taccaaaaat ggtattctgt ggatacctat cccaaatagc 161066
taccatggag cagattgtct ttgttctttg ggatatgtac ctatatttgt gatattgtac 161126
cttgaactct tccaagtgtt tccattggta tccccagaat agtcctgtga aggtggaaat 161186
gggtgcagtc tttctgaagg ctaatctgac agagcactgt tagaaattta ccatatggac 161246
atgtccacca tacctccctc aaattctacc aagatacatt gaaataggtt cacagcagct 161306
ttgtttatac tagcaaaaaa agcagcaaac ttcagccctc atcatatgag actgattaaa 161366
taaattatag tataccatac gatggaaaat tttcaggtat ttaaaagaat taggcaggta 161426

tgtatgaggc tttacctata aatatcccac atatgtgagt aaacagccaa gtggagaata 161486
tggacatgta taaagatccc tttgggataa ctacataaag aattcaagta tgtacgtgtg 161546
tatatatact tacttgaatt ctttatattc tatagagaga cttttataga atatgtagaa 161606
tcacacacac acacatacac acagagagag agaaatggat gtacttgcag gtatggacat 161666
aaacattttt tttcctggaa ataattagtt atagggaaag agactaggaa tgggggaaag 161726
gaagcattca aaagtgtttt ctttttactt tccattttgt acctttatgt taaaaatatg 161786
attgaactat ttgaattttc tttttaagtc aatggagtta tctacatggt aaaattattg 161846
gcctttaaac tctttacatt tatttatatt tttaatgtaa aagttctatt aaatgattgc 161906
ccaacacctc cacaagtaga aaacacttcc cctcattatc cttggtcagc cattgggaga 161966
caggtgaagg atccagcccc actgtcctcc atgagacctc ggtccctctt gttgcctgtt 162026
tagcgtgact acatcattta tcttccaaaa gaagacaggt tggcaagtga aaggggtcgc 162086
tgactagaca gaaccacaca gaacaggcat aaaccgggaa tgttctgggc actctgggat 162146
gtttgtgttg tctgtaggtt ggtgatagtc ttaccaccta tacaaagatc tccttgtgat 162206
tactcactgg tgtctatttc atttgctttg gttttag ca aaa tca gtc aag gtg 162260
Ala Lys Ser Val Lys Val
    1515
aaa att aag ctc aat aaa aaa gat gac aaa ggc cgg gac aaa ggg 162305
Lys Ile Lys Leu Asn Lys Lys Asp Asp Lys Gly Arg Asp Lys Gly
1520                1525                1530
aaa ggc aag aaa agg cca aat cga gga aaa gcc aaa cct gta gtg 162350
Lys Gly Lys Lys Arg Pro Asn Arg Gly Lys Ala Lys Pro Val Val
1535                1540                1545
agc gat ttt gac agc gat gag gag cag gat gaa cgt gtaagtgtag 162396
Ser Asp Phe Asp Ser Asp Glu Glu Gln Asp Glu Arg
1550                1555                1560
ccgactggga ctgaaggcgg agacgccctc tcccctgctt gctggcctct tgcatttcca 162456
tgccccttca gccttttcgc tttattaccc aggactggaa atgtcaggat ttagtgagat 162516
ttcattattg agggatgtga acggagctgt atgatttaga acaaaggatt gggggctttg 162576
tttattgcct ttttaatagt cttcaaaaat gaaaaactac caaatcaagc ttccctttcc 162636
ccctttttaa tcttcccaca tgctttctta taaatatggt tttccccgta gccagttaat 162696
attttacatg catcacagcc agcatggtgc ctggtacata acagtagatt agatggaaga 162756
atgagaaaaa ctgttatgac gtcagagtta ttggctcgag ttgctactta agggaccatg 162816
taaaaactca aaggactttt taagtaccta ggggtgttat ttttcatatt tgaaaaattc 162876
agtgagcatc tattgagcca ggccttgtaa taggtacctc cctgtgtggt agcatattaa 162936
atactgatgt cggcatcgtg agatagatgt cactctcaaa taacatgtta tggaaatggt 162996
taggttatgg gtgttttgct acagatcacg tcgcagaggt ggaattcgga cccgagtagc 163056
ataactcagt ctagtgtgct atgtctgact gggcagcaag gtagaagagt gggcagaagc 163116
tcctaatacc ttgccttttg aaataccctt catttcatcc attcactttc cagaggatgg 163176
tttcctggca cccacattat tttctttctg gaaagataaa tttccctttt cctagtttga 163236
ttttccttca acacattacc attttggtaa aatttcagaa ttatgatctc accccagcct 163296
taacccttga tgcaagcatt ttcaaataca tttttcttta ggagatgtca aatgcagggt 163356
attttttttt ctcccatgaa ttttctaaaa cctggggctg aaaaagagaa aatattagcc 163416
ataaaccttt caagccaaag tgaaagggca caaacaatag cttagagggt gggctttgct 163476
ttggggtttt tcagtaagaa aaacttctca gcttagagac tgtcgatgcc tctttaatgt 163536
gtttctgtcc tcccacggaa agagatttgg cgagttgttt ccaaaatgtc attttttcct 163596
actggcctct tgatggtttg ttgttatatc ttctttttct tgcatgtgat gttacttcat 163656
tttatcttct tatttttact tttag gaa cag tca gaa gga agt ggg acg gat 163708
Glu Gln Ser Glu Gly Ser Gly Thr Asp
            1565                1570
gat gag tga 163717
Asp Glu

<210> 5
<211> 4719
<212> DNA
<213> Homo sapiens
<220>
<221> CDS
<222> (1)..(4716)
<223>
<400> 5
atg tcc acg ccc aca gac cct ggt gcg atg ccc cac cca ggg cct tcg 48
Met Ser Thr Pro Thr Asp Pro Gly Ala Met Pro His Pro Gly Pro Ser
1               5                   10                  15
ccg ggg cct ggg cct tcc cct ggg cca att ctt ggg cct agt cca gga 96
Pro Gly Pro Gly Pro Ser Pro Gly Pro Ile Leu Gly Pro Ser Pro Gly
            20                  25                  30

cca gga cca tcc cca ggt tcc gtc cac agc atg atg ggg cca agt cct      144
Pro Gly Pro Ser Pro Gly Ser Val His Ser Met Met Gly Pro Ser Pro
        35                  40                  45
gga cct cca agt gtc tcc cat cct atg ccg acg atg ggg tcc aca gac      192
Gly Pro Pro Ser Val Ser His Pro Met Pro Thr Met Gly Ser Thr Asp
    50                  55                  60
ttc cca cag gaa ggc atg cat caa atg cat aag ccc atc gat ggt ata      240
Phe Pro Gln Glu Gly Met His Gln Met His Lys Pro Ile Asp Gly Ile
65                  70                  75                  80
cat gac aag ggg att gta gaa gac atc cat tgt gga tcc atg aag ggc      288
His Asp Lys Gly Ile Val Glu Asp Ile His Cys Gly Ser Met Lys Gly
                85                  90                  95
act ggt atg cga cca cct cac cca ggc atg ggc cct ccc cag agt cca      336
Thr Gly Met Arg Pro Pro His Pro Gly Met Gly Pro Pro Gln Ser Pro
            100                 105                 110
atg gat caa cac agc caa ggt tat atg tca cca cac cca tct cca tta      384
Met Asp Gln His Ser Gln Gly Tyr Met Ser Pro His Pro Ser Pro Leu
        115                 120                 125
gga gcc cca gag cac gtc tcc agc cct atg tct gga gga ggc cca act      432
Gly Ala Pro Glu His Val Ser Ser Pro Met Ser Gly Gly Gly Pro Thr
    130                 135                 140
cca cct cag atg cca cca agc cag ccg ggg gcc ctc atc cca ggt gat      480
Pro Pro Gln Met Pro Pro Ser Gln Pro Gly Ala Leu Ile Pro Gly Asp
145                 150                 155                 160
ccg cag gcc atg agc cag ccc aac aga ggt ccc tca cct ttc agt cct      528
Pro Gln Ala Met Ser Gln Pro Asn Arg Gly Pro Ser Pro Phe Ser Pro
                165                 170                 175
gtc cag ctg cat cag ctt cga gct cag att tta gct tat aaa atg ctg      576
Val Gln Leu His Gln Leu Arg Ala Gln Ile Leu Ala Tyr Lys Met Leu
            180                 185                 190
gcc cga ggc cag ccc ctc ccc gaa acg ctg cag ctt gca gtc cag ggg      624
Ala Arg Gly Gln Pro Leu Pro Glu Thr Leu Gln Leu Ala Val Gln Gly
        195                 200                 205
aaa agg acg ttg cct ggc ttg cag caa caa cag cag cag caa cag cag      672
Lys Arg Thr Leu Pro Gly Leu Gln Gln Gln Gln Gln Gln Gln Gln Gln
    210                 215                 220
cag cag cag cag cag cag cag cag cag cag cag caa cag cag ccg cag      720
Gln Gln Gln Gln Gln Gln Gln Gln Gln Gln Gln Gln Gln Gln Pro Gln
225                 230                 235                 240
cag cag ccg ccg caa cca cag acg cag caa caa cag cag ccg gcc ctt      768
Gln Gln Pro Pro Gln Pro Gln Thr Gln Gln Gln Gln Gln Pro Ala Leu
                245                 250                 255
gtt aac tac aac aga cca tct ggc ccg ggg ccg gag ctg agc ggc ccg      816
Val Asn Tyr Asn Arg Pro Ser Gly Pro Gly Pro Glu Leu Ser Gly Pro
            260                 265                 270
agc acc ccg cag aag ctg ccg gtg ccc gcg ccc ggc ggc cgg ccc tcg      864
Ser Thr Pro Gln Lys Leu Pro Val Pro Ala Pro Gly Gly Arg Pro Ser
        275                 280                 285
ccc gcg ccc ccc gca gcc gcg cag ccg ccc gcg gcc gca gtg ccc ggg      912
Pro Ala Pro Pro Ala Ala Ala Gln Pro Pro Ala Ala Ala Val Pro Gly
    290                 295                 300
ccc tca gtg ccg cag ccg gcc ccg ggg cag ccc tcg ccc gtc ctc cag      960
Pro Ser Val Pro Gln Pro Ala Pro Gly Gln Pro Ser Pro Val Leu Gln
305                 310                 315                 320
ctg cag cag aag cag agc cgc atc agc ccc atc cag aaa ccg caa ggc     1008
Leu Gln Gln Lys Gln Ser Arg Ile Ser Pro Ile Gln Lys Pro Gln Gly
                325                 330                 335
ctg gac ccc gtg gaa att ctg caa gag cgg gaa tac aga ctt cag gcc     1056
Leu Asp Pro Val Glu Ile Leu Gln Glu Arg Glu Tyr Arg Leu Gln Ala
            340                 345                 350
cgc ata gct cat agg ata caa gaa ctg gaa aat ctg cct ggc tct ttg     1104
Arg Ile Ala His Arg Ile Gln Glu Leu Glu Asn Leu Pro Gly Ser Leu
        355                 360                 365
cca cca gat tta aga acc aaa gca acc gtg gaa cta aaa gca ctt cgg     1152
Pro Pro Asp Leu Arg Thr Lys Ala Thr Val Glu Leu Lys Ala Leu Arg
    370                 375                 380
tta ctc aat ttc cag cgt cag ctg aga cag gag gtg gtg gcc tgc atg     1200
Leu Leu Asn Phe Gln Arg Gln Leu Arg Gln Glu Val Val Ala Cys Met
385                 390                 395                 400

cgc agg gac acg acc ctg gag acg gct ctc aac tcc aaa gca tac aaa 1248
Arg Arg Asp Thr Thr Leu Glu Thr Ala Leu Asn Ser Lys Ala Tyr Lys
                405                 410                 415
cgg agc aag cgc cag act ctg aga gaa gct cgc atg acc gag aag ctg 1296
Arg Ser Lys Arg Gln Thr Leu Arg Glu Ala Arg Met Thr Glu Lys Leu
            420                 425                 430
gag aag cag cag aag att gag cag gag agg aaa cgc cgt cag aaa cac 1344
Glu Lys Gln Gln Lys Ile Glu Gln Glu Arg Lys Arg Arg Gln Lys His
        435                 440                 445
cag gaa tac ctg aac agt att ttg caa cat gca aaa gat ttt aag gaa 1392
Gln Glu Tyr Leu Asn Ser Ile Leu Gln His Ala Lys Asp Phe Lys Glu
    450                 455                 460
tat cat cgg tct gtg gcc gga aag atc cag aag ctc tcc aaa gca gtg 1440
Tyr His Arg Ser Val Ala Gly Lys Ile Gln Lys Leu Ser Lys Ala Val
465                 470                 475                 480
gca act tgg cat gcc aac act gaa aga gag cag aag aag gag aca gag 1488
Ala Thr Trp His Ala Asn Thr Glu Arg Glu Gln Lys Lys Glu Thr Glu
                485                 490                 495
cgg att gaa aag gag aga atg cgg cga ctg atg gct gaa gat gag gag 1536
Arg Ile Glu Lys Glu Arg Met Arg Arg Leu Met Ala Glu Asp Glu Glu
            500                 505                 510
ggt tat aga aaa ctg att gat caa aag aaa gac agg cgt tta gct tac 1584
Gly Tyr Arg Lys Leu Ile Asp Gln Lys Lys Asp Arg Arg Leu Ala Tyr
        515                 520                 525
ctt ttg cag cag acc gat gag tat gta gcc aat ctg acc aat ctg gtt 1632
Leu Leu Gln Gln Thr Asp Glu Tyr Val Ala Asn Leu Thr Asn Leu Val
    530                 535                 540
tgg gag cac aag caa gcc cag gca gcc aaa gag aag aag aag agg agg 1680
Trp Glu His Lys Gln Ala Gln Ala Ala Lys Glu Lys Lys Lys Arg Arg
545                 550                 555                 560
agg agg aag aag aag gct gag gag aat gca gag ggt ggg gag tct gcc 1728
Arg Arg Lys Lys Lys Ala Glu Glu Asn Ala Glu Gly Gly Glu Ser Ala
                565                 570                 575
ctg gga ccg gat gga gag ccc ata gat gag agc agc cag atg agt gac 1776
Leu Gly Pro Asp Gly Glu Pro Ile Asp Glu Ser Ser Gln Met Ser Asp
            580                 585                 590
ctc cct gtc aaa gtg act cac aca gaa acc ggc aag gtt ctg ttc gga 1824
Leu Pro Val Lys Val Thr His Thr Glu Thr Gly Lys Val Leu Phe Gly
        595                 600                 605
cca gaa gca ccc aaa gca agt cag ctg gac gcc tgg ctg gaa atg aat 1872
Pro Glu Ala Pro Lys Ala Ser Gln Leu Asp Ala Trp Leu Glu Met Asn
    610                 615                 620
cct ggt tat gaa gtt gcc cct aga tct gac agt gaa gag agt gat tct 1920
Pro Gly Tyr Glu Val Ala Pro Arg Ser Asp Ser Glu Glu Ser Asp Ser
625                 630                 635                 640
gat tat gag gaa gag gat gag gaa gaa gag tcc agt agg cag gaa acc 1968
Asp Tyr Glu Glu Glu Asp Glu Glu Glu Glu Ser Ser Arg Gln Glu Thr
                645                 650                 655
gaa gag aaa ata ctc ctg gat cca aat agc gaa gaa gtt tct gag aag 2016
Glu Glu Lys Ile Leu Leu Asp Pro Asn Ser Glu Glu Val Ser Glu Lys
            660                 665                 670
gat gct aag cag atc att gag aca gct aag caa gac gtg gat gat gaa 2064
Asp Ala Lys Gln Ile Ile Glu Thr Ala Lys Gln Asp Val Asp Asp Glu
        675                 680                 685
tac agc atg cag tac agt gcc agg ggc tcc cag tcc tac tac acc gtg 2112
Tyr Ser Met Gln Tyr Ser Ala Arg Gly Ser Gln Ser Tyr Tyr Thr Val
    690                 695                 700
gct cat gcc atc tcg gag agg gtg gag aaa cag tct gcc ctc cta att 2160
Ala His Ala Ile Ser Glu Arg Val Glu Lys Gln Ser Ala Leu Leu Ile
705                 710                 715                 720
aat ggg acc cta aag cat tac cag ctc cag ggc ctg gaa tgg atg gtt 2208
Asn Gly Thr Leu Lys His Tyr Gln Leu Gln Gly Leu Glu Trp Met Val
                725                 730                 735
tcc ctg tat aat aac aac ttg aac gga atc tta gcc gat gaa atg ggg 2256
Ser Leu Tyr Asn Asn Asn Leu Asn Gly Ile Leu Ala Asp Glu Met Gly
            740                 745                 750
ctt gga aag acc ata cag acc att gca ctc atc act tat ctg atg gag 2304
Leu Gly Lys Thr Ile Gln Thr Ile Ala Leu Ile Thr Tyr Leu Met Glu
        755                 760                 765

cac aaa aga ctc aat ggc ccc tat ctc atc att gtt ccc ctt tcg act 2352
His Lys Arg Leu Asn Gly Pro Tyr Leu Ile Ile Val Pro Leu Ser Thr
    770                 775                 780
cta tct aac tgg aca tat gaa ttt gac aaa tgg gct cct tct gtg gtg 2400
Leu Ser Asn Trp Thr Tyr Glu Phe Asp Lys Trp Ala Pro Ser Val Val
785                 790                 795                 800
aag att tct tac aag ggt act cct gcc atg cgt cgc tcc ctt gtc ccc 2448
Lys Ile Ser Tyr Lys Gly Thr Pro Ala Met Arg Arg Ser Leu Val Pro
                805                 810                 815
cag cta cgg agt ggc aaa ttc aat gtc ctc ttg act act tat gag tat 2496
Gln Leu Arg Ser Gly Lys Phe Asn Val Leu Leu Thr Thr Tyr Glu Tyr
            820                 825                 830
att ata aaa gac aag cac att ctt gca aag att cgg tgg aaa tac atg 2544
Ile Ile Lys Asp Lys His Ile Leu Ala Lys Ile Arg Trp Lys Tyr Met
        835                 840                 845
ata gtg gac gaa ggc cac cga atg aag aat cac cac tgc aag ctg act 2592
Ile Val Asp Glu Gly His Arg Met Lys Asn His His Cys Lys Leu Thr
    850                 855                 860
cag gtc ttg aac act cac tat gtg gcc ccc aga agg atc ctc ttg act 2640
Gln Val Leu Asn Thr His Tyr Val Ala Pro Arg Arg Ile Leu Leu Thr
865                 870                 875                 880
ggg acc ccg ctg cag aat aag ctc cct gaa ctc tgg gcc ctc ctc aac 2688
Gly Thr Pro Leu Gln Asn Lys Leu Pro Glu Leu Trp Ala Leu Leu Asn
                885                 890                 895
ttc ctc ctc cca aca att ttt aag agc tgc agc aca ttt gaa caa tgg 2736
Phe Leu Leu Pro Thr Ile Phe Lys Ser Cys Ser Thr Phe Glu Gln Trp
            900                 905                 910
ttc aat gct cca ttt gcc atg act ggt gaa agg gtg gac tta aat gaa 2784
Phe Asn Ala Pro Phe Ala Met Thr Gly Glu Arg Val Asp Leu Asn Glu
        915                 920                 925
gaa gaa act ata ttg atc atc agg cgt cta cat aag gtg tta aga cca 2832
Glu Glu Thr Ile Leu Ile Ile Arg Arg Leu His Lys Val Leu Arg Pro
    930                 935                 940
ttt tta cta agg aga ctg aag aaa gaa gtt gaa tcc cag ctt ccc gaa 2880
Phe Leu Leu Arg Arg Leu Lys Lys Glu Val Glu Ser Gln Leu Pro Glu
945                 950                 955                 960
aaa gtg gaa tat gtg atc aag tgt gac atg tca gct ctg cag aag att 2928
Lys Val Glu Tyr Val Ile Lys Cys Asp Met Ser Ala Leu Gln Lys Ile
                965                 970                 975
ctg tat cgc cat atg caa gcc aag ggg atc ctt ctc aca gat ggt tct 2976
Leu Tyr Arg His Met Gln Ala Lys Gly Ile Leu Leu Thr Asp Gly Ser
            980                 985                 990
gag aaa gat aag aag ggg aaa gga ggt gct aag aca ctt atg aac act 3024
Glu Lys Asp Lys Lys Gly Lys Gly Gly Ala Lys Thr Leu Met Asn Thr
        995                 1000                1005
att atg cag ttg aga aaa atc tgc aac cac cca tat atg ttt cag 3069
Ile Met Gln Leu Arg Lys Ile Cys Asn His Pro Tyr Met Phe Gln
    1010                1015                1020
cac att gag gaa tcc ttt gct gaa cac cta ggc tat tca aat ggg 3114
His Ile Glu Glu Ser Phe Ala Glu His Leu Gly Tyr Ser Asn Gly
    1025                1030                1035
gtc atc aat ggg gct gaa ctg tat cgg gcc tca ggg aag ttt gag 3159
Val Ile Asn Gly Ala Glu Leu Tyr Arg Ala Ser Gly Lys Phe Glu
    1040                1045                1050
ctg ctt gat cgt att ctg cca aaa ttg aga gcg act aat cac cga 3204
Leu Leu Asp Arg Ile Leu Pro Lys Leu Arg Ala Thr Asn His Arg
    1055                1060                1065
gtg ctg ctt ttc tgc cag atg aca tct ctc atg acc atc atg gag 3249
Val Leu Leu Phe Cys Gln Met Thr Ser Leu Met Thr Ile Met Glu
    1070                1075                1080
gat tat ttt gct ttt cgg aac ttc ctt tac cta cgc ctt gat ggc 3294
Asp Tyr Phe Ala Phe Arg Asn Phe Leu Tyr Leu Arg Leu Asp Gly
    1085                1090                1095
acc acc aag tct gaa gat cgt gct gct ttg ctg aag aaa ttc aat 3339
Thr Thr Lys Ser Glu Asp Arg Ala Ala Leu Leu Lys Lys Phe Asn
    1100                1105                1110
gaa cct gga tcc cag tat ttc att ttc ttg ctg agc aca aga gct 3384
Glu Pro Gly Ser Gln Tyr Phe Ile Phe Leu Leu Ser Thr Arg Ala
    1115                1120                1125

ggt ggc ctg ggc tta aat ctt cag gca gct gat aca gtg gtc atc 3429
Gly Gly Leu Gly Leu Asn Leu Gln Ala Ala Asp Thr Val Val Ile
    1130                1135                1140
ttt gac agc gac tgg aat cct cat cag gat ctg cag gcc caa gac 3474
Phe Asp Ser Asp Trp Asn Pro His Gln Asp Leu Gln Ala Gln Asp
    1145                1150                1155
cga gct cac cgc atc ggg cag cag aac gag gtc cgg gta ctg agg 3519
Arg Ala His Arg Ile Gly Gln Gln Asn Glu Val Arg Val Leu Arg
    1160                1165                1170
ctc tgt acc gtg aac agc gtg gag gaa aag atc ctc gcg gcc gca 3564
Leu Cys Thr Val Asn Ser Val Glu Glu Lys Ile Leu Ala Ala Ala
    1175                1180                1185
aaa tac aag ctg aac gtg gat cag aaa gtg atc cag gcg ggc atg 3609
Lys Tyr Lys Leu Asn Val Asp Gln Lys Val Ile Gln Ala Gly Met
    1190                1195                1200
ttt gac caa aag tct tca agc cac gag cgg agg gca ttc ctg cag 3654
Phe Asp Gln Lys Ser Ser Ser His Glu Arg Arg Ala Phe Leu Gln
    1205                1210                1215
gcc atc ttg gag cat gag gag gaa aat gag gaa gaa gat gaa gta 3699
Ala Ile Leu Glu His Glu Glu Glu Asn Glu Glu Glu Asp Glu Val
    1220                1225                1230
ccg gac gat gag act ctg aac caa atg att gct cga cga gaa gaa 3744
Pro Asp Asp Glu Thr Leu Asn Gln Met Ile Ala Arg Arg Glu Glu
    1235                1240                1245
gaa ttt gac ctt ttt atg cgg atg gac atg gac cgg cgg agg gaa 3789
Glu Phe Asp Leu Phe Met Arg Met Asp Met Asp Arg Arg Arg Glu
    1250                1255                1260
gat gcc cgg aac ccg aaa cgg aag ccc cgt tta atg gag gag gat 3834
Asp Ala Arg Asn Pro Lys Arg Lys Pro Arg Leu Met Glu Glu Asp
    1265                1270                1275
gag ctg ccc tcc tgg atc att aag gat gac gct gaa gta gaa agg 3879
Glu Leu Pro Ser Trp Ile Ile Lys Asp Asp Ala Glu Val Glu Arg
    1280                1285                1290
ctc acc tgt gaa gaa gag gag gag aaa ata ttt ggg agg ggg tcc 3924
Leu Thr Cys Glu Glu Glu Glu Glu Lys Ile Phe Gly Arg Gly Ser
    1295                1300                1305
cgc cag cgc cgt gac gtg gac tac agt gac gcc ctc acg gag aag 3969
Arg Gln Arg Arg Asp Val Asp Tyr Ser Asp Ala Leu Thr Glu Lys
    1310                1315                1320
cag tgg cta agg gcc atc gaa gac ggc aat ttg gag gaa atg gaa 4014
Gln Trp Leu Arg Ala Ile Glu Asp Gly Asn Leu Glu Glu Met Glu
    1325                1330                1335
gag gaa gta cgg ctt aag aag cga aaa aga cga aga aat gtg gat 4059
Glu Glu Val Arg Leu Lys Lys Arg Lys Arg Arg Arg Asn Val Asp
    1340                1345                1350
aaa gat cct gca aaa gaa gat gtg gaa aaa gct aag aag aga aga 4104
Lys Asp Pro Ala Lys Glu Asp Val Glu Lys Ala Lys Lys Arg Arg
    1355                1360                1365
ggc cgc cct ccc gct gag aaa ctg tca cca aat ccc ccc aaa ctg 4149
Gly Arg Pro Pro Ala Glu Lys Leu Ser Pro Asn Pro Pro Lys Leu
    1370                1375                1380
aca aag cag atg aac gct atc atc gat act gtg ata aac tac aaa 4194
Thr Lys Gln Met Asn Ala Ile Ile Asp Thr Val Ile Asn Tyr Lys
    1385                1390                1395
gat agt tca ggg cga cag ctc agt gaa gtc ttc att cag tta cct 4239
Asp Ser Ser Gly Arg Gln Leu Ser Glu Val Phe Ile Gln Leu Pro
    1400                1405                1410
tca agg aaa gaa tta cca gaa tac tat gaa tta att agg aag cca 4284
Ser Arg Lys Glu Leu Pro Glu Tyr Tyr Glu Leu Ile Arg Lys Pro
    1415                1420                1425
gtg gat ttc aaa aaa ata aag gaa agg att cgt aat cat aag tac 4329
Val Asp Phe Lys Lys Ile Lys Glu Arg Ile Arg Asn His Lys Tyr
    1430                1435                1440
cgg agc cta ggc gac ctg gag aag gat gtc atg ctt ctc tgt cac 4374
Arg Ser Leu Gly Asp Leu Glu Lys Asp Val Met Leu Leu Cys His
    1445                1450                1455
aac gct cag acg ttc aac ctg gag gga tcc cag atc tat gaa gac 4419
Asn Ala Gln Thr Phe Asn Leu Glu Gly Ser Gln Ile Tyr Glu Asp
    1460                1465                1470

tcc atc gtc tta cag tca gtg ttt aag agt gcc cgg cag aaa att 4464
Ser Ile Val Leu Gln Ser Val Phe Lys Ser Ala Arg Gln Lys Ile
    1475                1480                1485
gcc aaa gag gaa gag agt gag gat gaa agc aat gaa gag gag gaa 4509
Ala Lys Glu Glu Glu Ser Glu Asp Glu Ser Asn Glu Glu Glu Glu
    1490                1495                1500
gag gaa gat gaa gaa gag tca gag tcc gag gca aaa tca gtc aag 4554
Glu Glu Asp Glu Glu Glu Ser Glu Ser Glu Ala Lys Ser Val Lys
    1505                1510                1515
gtg aaa att aag ctc aat aaa aaa gat gac aaa ggc cgg gac aaa 4599
Val Lys Ile Lys Leu Asn Lys Lys Asp Asp Lys Gly Arg Asp Lys
    1520                1525                1530
ggg aaa ggc aag aaa agg cca aat cga gga aaa gcc aaa cct gta 4644
Gly Lys Gly Lys Lys Arg Pro Asn Arg Gly Lys Ala Lys Pro Val
    1535                1540                1545
gtg agc gat ttt gac agc gat gag gag cag gat gaa cgt gaa cag 4689
Val Ser Asp Phe Asp Ser Asp Glu Glu Gln Asp Glu Arg Glu Gln
    1550                1555                1560
tca gaa gga agt ggg acg gat gat gag tga 4719
Ser Glu Gly Ser Gly Thr Asp Asp Glu
    1565                1570

<210> 6
<211> 1572
<212> PRT
<213> Homo sapiens
<400> 6
Met Ser Thr Pro Thr Asp Pro Gly Ala Met Pro His Pro Gly Pro Ser
1               5                   10                  15
Pro Gly Pro Gly Pro Ser Pro Gly Pro Ile Leu Gly Pro Ser Pro Gly
            20                  25                  30
Pro Gly Pro Ser Pro Gly Ser Val His Ser Met Met Gly Pro Ser Pro
        35                  40                  45
Gly Pro Pro Ser Val Ser His Pro Met Pro Thr Met Gly Ser Thr Asp
    50                  55                  60
Phe Pro Gln Glu Gly Met His Gln Met His Lys Pro Ile Asp Gly Ile
65                  70                  75                  80
His Asp Lys Gly Ile Val Glu Asp Ile His Cys Gly Ser Met Lys Gly
                85                  90                  95
Thr Gly Met Arg Pro Pro His Pro Gly Met Gly Pro Pro Gln Ser Pro
            100                 105                 110
Met Asp Gln His Ser Gln Gly Tyr Met Ser Pro His Pro Ser Pro Leu
        115                 120                 125
Gly Ala Pro Glu His Val Ser Ser Pro Met Ser Gly Gly Gly Pro Thr
    130                 135                 140
Pro Pro Gln Met Pro Pro Ser Gln Pro Gly Ala Leu Ile Pro Gly Asp
145                 150                 155                 160
Pro Gln Ala Met Ser Gln Pro Asn Arg Gly Pro Ser Pro Phe Ser Pro
                165                 170                 175
Val Gln Leu His Gln Leu Arg Ala Gln Ile Leu Ala Tyr Lys Met Leu
            180                 185                 190
Ala Arg Gly Gln Pro Leu Pro Glu Thr Leu Gln Leu Ala Val Gln Gly
        195                 200                 205
Lys Arg Thr Leu Pro Gly Leu Gln Gln Gln Gln Gln Gln Gln Gln Gln
    210                 215                 220
Gln Gln Gln Gln Gln Gln Gln Gln Gln Gln Gln Gln Gln Gln Pro Gln
225                 230                 235                 240
Gln Gln Pro Pro Gln Pro Gln Thr Gln Gln Gln Gln Gln Pro Ala Leu
                245                 250                 255
Val Asn Tyr Asn Arg Pro Ser Gly Pro Gly Pro Glu Leu Ser Gly Pro
            260                 265                 270
Ser Thr Pro Gln Lys Leu Pro Val Pro Ala Pro Gly Gly Arg Pro Ser
        275                 280                 285
Pro Ala Pro Pro Ala Ala Ala Gln Pro Pro Ala Ala Ala Val Pro Gly
    290                 295                 300
Pro Ser Val Pro Gln Pro Ala Pro Gly Gln Pro Ser Pro Val Leu Gln
305                 310                 315                 320
Leu Gln Gln Lys Gln Ser Arg Ile Ser Pro Ile Gln Lys Pro Gln Gly

                325                 330                 335
Leu Asp Pro Val Glu Ile Leu Gln Glu Arg Glu Tyr Arg Leu Gln Ala
            340                 345                 350
Arg Ile Ala His Arg Ile Gln Glu Leu Glu Asn Leu Pro Gly Ser Leu
        355                 360                 365
Pro Pro Asp Leu Arg Thr Lys Ala Thr Val Glu Leu Lys Ala Leu Arg
    370                 375                 380
Leu Leu Asn Phe Gln Arg Gln Leu Arg Gln Glu Val Val Ala Cys Met
385                 390                 395                 400
Arg Arg Asp Thr Thr Leu Glu Thr Ala Leu Asn Ser Lys Ala Tyr Lys
                405                 410                 415
Arg Ser Lys Arg Gln Thr Leu Arg Glu Ala Arg Met Thr Glu Lys Leu
            420                 425                 430
Glu Lys Gln Gln Lys Ile Glu Gln Glu Arg Lys Arg Arg Gln Lys His
        435                 440                 445
Gln Glu Tyr Leu Asn Ser Ile Leu Gln His Ala Lys Asp Phe Lys Glu
    450                 455                 460
Tyr His Arg Ser Val Ala Gly Lys Ile Gln Lys Leu Ser Lys Ala Val
465                 470                 475                 480
Ala Thr Trp His Ala Asn Thr Glu Arg Glu Gln Lys Lys Glu Thr Glu
                485                 490                 495
Arg Ile Glu Lys Glu Arg Met Arg Arg Leu Met Ala Glu Asp Glu Glu
            500                 505                 510
Gly Tyr Arg Lys Leu Ile Asp Gln Lys Lys Asp Arg Arg Leu Ala Tyr
        515                 520                 525
Leu Leu Gln Gln Thr Asp Glu Tyr Val Ala Asn Leu Thr Asn Leu Val
    530                 535                 540
Trp Glu His Lys Gln Ala Gln Ala Ala Lys Glu Lys Lys Lys Arg Arg
545                 550                 555                 560
Arg Arg Lys Lys Lys Ala Glu Glu Asn Ala Glu Gly Gly Glu Ser Ala
                565                 570                 575
Leu Gly Pro Asp Gly Glu Pro Ile Asp Glu Ser Ser Gln Met Ser Asp
            580                 585                 590
Leu Pro Val Lys Val Thr His Thr Glu Thr Gly Lys Val Leu Phe Gly
        595                 600                 605
Pro Glu Ala Pro Lys Ala Ser Gln Leu Asp Ala Trp Leu Glu Met Asn
    610                 615                 620
Pro Gly Tyr Glu Val Ala Pro Arg Ser Asp Ser Glu Glu Ser Asp Ser
625                 630                 635                 640
Asp Tyr Glu Glu Glu Asp Glu Glu Glu Glu Ser Ser Arg Gln Glu Thr
                645                 650                 655
Glu Glu Lys Ile Leu Leu Asp Pro Asn Ser Glu Glu Val Ser Glu Lys
            660                 665                 670
Asp Ala Lys Gln Ile Ile Glu Thr Ala Lys Gln Asp Val Asp Asp Glu
        675                 680                 685
Tyr Ser Met Gln Tyr Ser Ala Arg Gly Ser Gln Ser Tyr Tyr Thr Val
    690                 695                 700
Ala His Ala Ile Ser Glu Arg Val Glu Lys Gln Ser Ala Leu Leu Ile
705                 710                 715                 720
Asn Gly Thr Leu Lys His Tyr Gln Leu Gln Gly Leu Glu Trp Met Val
                725                 730                 735
Ser Leu Tyr Asn Asn Asn Leu Asn Gly Ile Leu Ala Asp Glu Met Gly
            740                 745                 750
Leu Gly Lys Thr Ile Gln Thr Ile Ala Leu Ile Thr Tyr Leu Met Glu
        755                 760                 765
His Lys Arg Leu Asn Gly Pro Tyr Leu Ile Ile Val Pro Leu Ser Thr
    770                 775                 780
Leu Ser Asn Trp Thr Tyr Glu Phe Asp Lys Trp Ala Pro Ser Val Val
785                 790                 795                 800
Lys Ile Ser Tyr Lys Gly Thr Pro Ala Met Arg Arg Ser Leu Val Pro
                805                 810                 815
Gln Leu Arg Ser Gly Lys Phe Asn Val Leu Leu Thr Thr Tyr Glu Tyr
            820                 825                 830
Ile Ile Lys Asp Lys His Ile Leu Ala Lys Ile Arg Trp Lys Tyr Met
        835                 840                 845
Ile Val Asp Glu Gly His Arg Met Lys Asn His His Cys Lys Leu Thr
    850                 855                 860
Gln Val Leu Asn Thr His Tyr Val Ala Pro Arg Arg Ile Leu Leu Thr
865                 870                 875                 880

Gly Thr Pro Leu Gln Asn Lys Leu Pro Glu Leu Trp Ala Leu Leu Asn
                885                 890                 895
Phe Leu Leu Pro Thr Ile Phe Lys Ser Cys Ser Thr Phe Glu Gln Trp
            900                 905                 910
Phe Asn Ala Pro Phe Ala Met Thr Gly Glu Arg Val Asp Leu Asn Glu
        915                 920                 925
Glu Glu Thr Ile Leu Ile Ile Arg Arg Leu His Lys Val Leu Arg Pro
    930                 935                 940
Phe Leu Leu Arg Arg Leu Lys Lys Glu Val Glu Ser Gln Leu Pro Glu
945                 950                 955                 960
Lys Val Glu Tyr Val Ile Lys Cys Asp Met Ser Ala Leu Gln Lys Ile
                965                 970                 975
Leu Tyr Arg His Met Gln Ala Lys Gly Ile Leu Leu Thr Asp Gly Ser
            980                 985                 990
Glu Lys Asp Lys Lys Gly Lys Gly  Gly Ala Lys Thr Leu  Met Asn Thr
        995                 1000                1005
Ile Met  Gln Leu Arg Lys Ile  Cys Asn His Pro Tyr  Met Phe Gln
    1010                1015                1020
His Ile  Glu Glu Ser Phe Ala  Glu His Leu Gly Tyr  Ser Asn Gly
    1025                1030                1035
Val Ile  Asn Gly Ala Glu Leu  Tyr Arg Ala Ser Gly  Lys Phe Glu
    1040                1045                1050
Leu Leu  Asp Arg Ile Leu Pro  Lys Leu Arg Ala Thr  Asn His Arg
    1055                1060                1065
Val Leu  Leu Phe Cys Gln Met  Thr Ser Leu Met Thr  Ile Met Glu
    1070                1075                1080
Asp Tyr  Phe Ala Phe Arg Asn  Phe Leu Tyr Leu Arg  Leu Asp Gly
    1085                1090                1095
Thr Thr  Lys Ser Glu Asp Arg  Ala Ala Leu Leu Lys  Lys Phe Asn
    1100                1105                1110
Glu Pro  Gly Ser Gln Tyr Phe  Ile Phe Leu Leu Ser  Thr Arg Ala
    1115                1120                1125
Gly Gly  Leu Gly Leu Asn Leu  Gln Ala Ala Asp Thr  Val Val Ile
    1130                1135                1140
Phe Asp  Ser Asp Trp Asn Pro  His Gln Asp Leu Gln  Ala Gln Asp
    1145                1150                1155
Arg Ala  His Arg Ile Gly Gln  Gln Asn Glu Val Arg  Val Leu Arg
    1160                1165                1170
Leu Cys  Thr Val Asn Ser Val  Glu Glu Lys Ile Leu  Ala Ala Ala
    1175                1180                1185
Lys Tyr  Lys Leu Asn Val Asp  Gln Lys Val Ile Gln  Ala Gly Met
    1190                1195                1200
Phe Asp  Gln Lys Ser Ser Ser  His Glu Arg Arg Ala  Phe Leu Gln
    1205                1210                1215
Ala Ile  Leu Glu His Glu Glu  Glu Asn Glu Glu Glu  Asp Glu Val
    1220                1225                1230
Pro Asp  Asp Glu Thr Leu Asn  Gln Met Ile Ala Arg  Arg Glu Glu
    1235                1240                1245
Glu Phe  Asp Leu Phe Met Arg  Met Asp Met Asp Arg  Arg Arg Glu
    1250                1255                1260
Asp Ala  Arg Asn Pro Lys Arg  Lys Pro Arg Leu Met  Glu Glu Asp
    1265                1270                1275
Glu Leu  Pro Ser Trp Ile Ile  Lys Asp Asp Ala Glu  Val Glu Arg
    1280                1285                1290
Leu Thr  Cys Glu Glu Glu Glu  Glu Lys Ile Phe Gly  Arg Gly Ser
    1295                1300                1305
Arg Gln  Arg Arg Asp Val Asp  Tyr Ser Asp Ala Leu  Thr Glu Lys
    1310                1315                1320
Gln Trp  Leu Arg Ala Ile Glu  Asp Gly Asn Leu Glu  Glu Met Glu
    1325                1330                1335
Glu Glu  Val Arg Leu Lys Lys  Arg Lys Arg Arg Arg  Asn Val Asp
    1340                1345                1350
Lys Asp  Pro Ala Lys Glu Asp  Val Glu Lys Ala Lys  Lys Arg Arg
    1355                1360                1365
Gly Arg  Pro Pro Ala Glu Lys  Leu Ser Pro Asn Pro  Pro Lys Leu
    1370                1375                1380
Thr Lys  Gln Met Asn Ala Ile  Ile Asp Thr Val Ile  Asn Tyr Lys
    1385                1390                1395
Asp Ser  Ser Gly Arg Gln Leu  Ser Glu Val Phe Ile  Gln Leu Pro

1400                    1405                    1410
Ser Arg Lys Glu Leu Pro Glu Tyr Tyr Glu Leu Ile Arg Lys Pro
    1415                    1420                    1425
Val Asp Phe Lys Lys Ile Lys Glu Arg Ile Arg Asn His Lys Tyr
    1430                    1435                    1440
Arg Ser Leu Gly Asp Leu Glu Lys Asp Val Met Leu Leu Cys His
    1445                    1450                    1455
Asn Ala Gln Thr Phe Asn Leu Glu Gly Ser Gln Ile Tyr Glu Asp
    1460                    1465                    1470
Ser Ile Val Leu Gln Ser Val Phe Lys Ser Ala Arg Gln Lys Ile
    1475                    1480                    1485
Ala Lys Glu Glu Glu Ser Glu Asp Glu Ser Asn Glu Glu Glu Glu
    1490                    1495                    1500
Glu Glu Asp Glu Glu Glu Ser Glu Ser Glu Ala Lys Ser Val Lys
    1505                    1510                    1515
Val Lys Ile Lys Leu Asn Lys Lys Asp Asp Lys Gly Arg Asp Lys
    1520                    1525                    1530
Gly Lys Gly Lys Lys Arg Pro Asn Arg Gly Lys Ala Lys Pro Val
    1535                    1540                    1545
Val Ser Asp Phe Asp Ser Asp Glu Glu Gln Asp Glu Arg Glu Gln
    1550                    1555                    1560
Ser Glu Gly Ser Gly Thr Asp Asp Glu
    1565                    1570


<210>  7
<211>  71
<212>  DNA
<213>  Artificial
<220>
<223>  shBRM oligo sequence
<400>  7
cgcgtccccg gaggtgctaa gacacttatg ttcaagagac ataagtgtct tagcacctcc      60
tttttggaaa t                                                           71


<210>  8
<211>  69
<212>  DNA
<213>  Artificial
<220>
<223>  shBRM oligo sequence
<400>  8
cgatttccaa aaaggaggtg ctaagacact tatgtctctt gaacataagt gtcttagcac      60
ctccgggga                                                              69

## Description

### [Technical Field]

The present invention relates to a method for predicting responsiveness to a cancer treatment with a compound capable of inhibiting BRM and a method for selecting a cancer treatment target on the basis of BRG1 function suppression. Moreover, the present invention relates to a method for treating a cancer having a suppressed BRG1 function by using a compound capable of inhibiting BRM. Further, the present invention relates to a reagent, used in these methods, for detecting whether or not a BRG1 function is suppressed. Furthermore, the present invention relates to a screening method for a compound on the basis of BRM inhibition, the compound used in a treatment against a cancer having a suppressed BRG1 function.

### [Background Art]

Tyrosine kinase inhibitors are effective against solid tumors with activating mutations in tyrosine kinase genes (for example, EGFR mutations and ALK fusions observed in lung adenocarcinomas) (NPL 1). The present inventors and other researchers have recently identified RET oncogene fusions in lung adenocarcinoma (NPL 2), which supports the importance of tyrosine kinase genes as therapeutic targets. Two other tyrosine kinase gene alterations, the FGFR1 amplification and the DDR2 mutation, have been identified as therapeutic targets in squamous-cell carcinoma, which corresponds to another major type of non-small-cell lung carcinoma (NSCLC) (NPLs 3, 4).

Meanwhile, inactivating somatic mutations of genes that encode subunits of the SWI/SNF chromatin-remodeling complex, such as BRG1/SMARCA4, PBRM1/BAF180, ARID1A/BAF250A, and ARID2/BAF200, have attracted much interest. This is because these mutations have been identified by genome-wide sequencing analyses of cancer cells (NPL 5). These mutations are thought to interfere with the functions of the SWI/SNF in transcription (NPL 5) and DNA double-strand break repair (NPL 6), and believed to be critical for cancer development and progression. In addition, there is a report that BRG1 and BRM play complementary roles in this complex (NPL 7).

However, no therapeutic strategies have yet been developed for specifically targeting cancer cells harboring these inactivating SWI/SNF mutations.

### [Citation List]

### [Non Patent Literatures]

[NPL 1] Lovly CM, Carbone DP. Nat Rev Clin Oncol. 2011; 8: 68-70.
[NPL 2] Kohno T, et al. Nat Med. 2012; 18: 375-7.
[NPL3] Hammerman PS, etal. CancerDiscov. 2011; 1: 78-89.
[NPL 4] Weiss J, et al. Sci Transl Med. 2010; 2: 62ra93.
[NPL 5] Wilson BG, Roberts CW. Nat Rev Cancer. 2011; 11: 481-92.
[NPL 6] Ogiwara H, et al. Oncogene. 2011; 30: 2135-46.
[NPL 7] Reyes JC, et al. EMBO J. 1998; 17: 6979-91.

### [Summary of Invention]

### [Technical Problem]

The present invention has been made in view of such circumstances as described above. An object of the present invention is to develop a therapeutic strategy for specifically targeting cancer cells harboring inactivating SWI/SNF mutations. More specifically, an object of the present invention is to develop a therapeutic strategy for specifically targeting cancer cells in which a normal BRG1 function is suppressed, preferably cancer cells in which normal BRG1 is lost.

### [Solution to Problem]

Synthetic-lethality therapy is a very promising cancer treatment method. For example, the BRCA1 and BRCA2 genes have a synthetic-lethal relationship with the PARP1 gene. The growth of BRCA1-deficient or BRCA2-deficient cancer cells is dependent on PARP1 protein function. These findings have been translated to the clinic through the development of PARP inhibitors to treat BRCA1/BRCA2-deficient tumors (Chan DA, et al. Nat Rev Drug Discov.2011;10:351-64). Besides, a synthetic-lethality therapy has also been proposed for treatment of cancers deficient in genes involved in DNA mismatch repair and cell metabolism (Muller FL, et al. Nature. 2012; 488: 337-42., Chan DA, et al. Sci Transl Med. 2011; 3: 94ra70, Martin SA, et al. Cancer Cell. 2010; 17: 235-48). The present inventors have earnestly studied in order to apply synthetic-lethality therapy to a therapeutic strategy for specifically killing cancer cells harboring inactivating SWI/SNF mutations.

As a result, the present inventors have found that suppressing BRM expression in BRG1-deficient cancer cells remarkably suppresses the cancer cell growth. This growth suppression is complemented with the introduction of the wild-type BRG1 gene, but not complemented with the introduction of a BRG1 gene with a disrupted ATP-binding pocket. Hence, it has been revealed that a loss of the ATPase activity of a SWI/SNF complex particularly contributes to the growth suppression. Moreover, it has been found that cancer cells whose growth is suppressed by suppressing the BRM expression exhibit a flattened morphology, and that the proportion of cells exhibiting G1 arrest in the cell cycle is increased. Further, expression of a senescence marker p21 has also been observed. These facts have revealed that senescence is induced. On the other hand, other driver gene mutations (for example, EGFR mutations, ALK fusions, and so on) serving as targets of molecularly targeted therapies have not been found in BRG1-deficient cancer cells. This suggests that current molecularly targeted therapies based on tyrosine kinase inhibitors are not suitable.

From the above results, the present inventors have found that BRG1 and BRM have a synthetic-lethal relationship, and that a treatment for inhibiting BRM (particularly its ATPase activity) is a promising approach for treating a BRG1-deficient cancer having no mutation in known therapeutic target genes. Additionally, it has also been revealed that, in this therapeutic strategy, a BRM inhibitor may be administered to a cancer patient after the selection based on BRG1 function suppression, thus enabling an efficient treatment based on the companion diagnosis.

Further, the present inventors have also successfully developed a novel screening system based on ATPase activity inhibition in order to develop a BRM inhibitor useful in this therapeutic strategy. These have led to the completion of the present invention.

Thus, the present invention relates to a synthetic-lethality therapy for specifically targeting cancer cells harboring inactivating SWI/SNF mutations, and a companion diagnosis for the synthetic-lethality therapy. More specifically, the present invention provides the following inventions.
(1) A method for evaluating a cancer, the method comprising detecting whether or not a BRG1 function is suppressed in a biological sample derived from a cancer patient.
(2) A method for predicting responsiveness to a cancer treatment with a compound capable of inhibiting BRM, the method comprising:
   detecting whether or not a BRG1 function is suppressed in a biological sample derived from a cancer patient; and
   determining that the patient, from which the BRG1 function suppression is detected, responds to the cancer treatment with the compound capable of inhibiting BRM.
(3) A method for selecting a target of a cancer treatment with a compound capable of inhibiting BRM, the method comprising:
   detecting whether or not a BRG1 function is suppressed in a biological sample derived from a cancer patient; and
   selecting the patient, from which the BRG1 function suppression is detected, as the target of the cancer treatment with the compound capable of inhibiting BRM.
(4) A method for treating a cancer having a suppressed BRG1 function, the method comprising administering a compound capable of inhibiting BRM to a patient from which BRG1 function suppression is detected.
(5) A method for treating a cancer having a suppressed BRG1 function, the method comprising:
   detecting whether or not a BRG1 function is suppressed in a biological sample derived from a cancer patient; and
   administering a compound capable of inhibiting BRM to the patient from which the BRG1 function suppression is detected.
(6) A reagent for detecting whether or not a BRG1 function is suppressed in the method according to any one of (1) to (3) and (5), the reagent comprising as an active ingredient any one of the following molecules (a) to (c) :
   (a) an oligonucleotide primer capable of specifically binding to a BRG1 gene;
   (b) an oligonucleotide probe capable of specifically binding to a BRG1 gene; and
   (c) an antibody capable of specifically binding to a BRG1 protein.
(7) A screening method for a compound used in a treatment against a cancer having a suppressed BRG1 function, the method comprising the step of selecting the compound based on whether or not BRM is inhibited.

### [Advantageous Effects of Invention]

The present invention makes it possible to efficiently evaluate a cancer and predict responsiveness to a cancer treatment with a BRM inhibitor on the basis of BRG1 function suppression. According to the present invention, a cancer treatment with a BRM inhibitor would be performed for a cancer patient having a suppressed BRG1 function after the patient is selected by detecting whether or not a BRG1 function is suppressed (for example, inactivating mutation or expression reduction) in a biological sample derived from the patient. This makes it possible to greatly improve the treatment outcome of the cancer patient. The uses of the probe and primer for the BRG1 gene and the antibody against BRG1 enable such an efficient companion diagnosis by detecting whether or not a BRG1 function is suppressed.

### [Brief Description of Drawings]

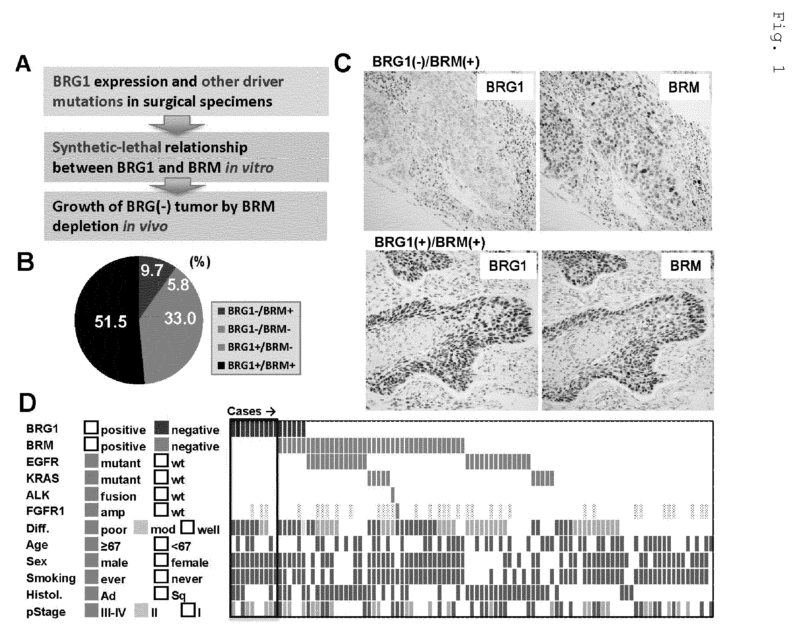

[Fig. 1] Fig. 1 is a figure for illustrating BRG1 and BRM expression in surgically resected non-small-cell lung carcinomas. A: shows the strategy of this study. B: shows proportions of specimens expressing the BRG1 and BRM proteins. C: representative images of immunohistochemical staining. Upper panel: BRG1-negative/BRM-positive specimens. Lower panel: BRG1/BRM-positive specimens. D: BRG1/BRM protein expression and other clinicopathological factors.

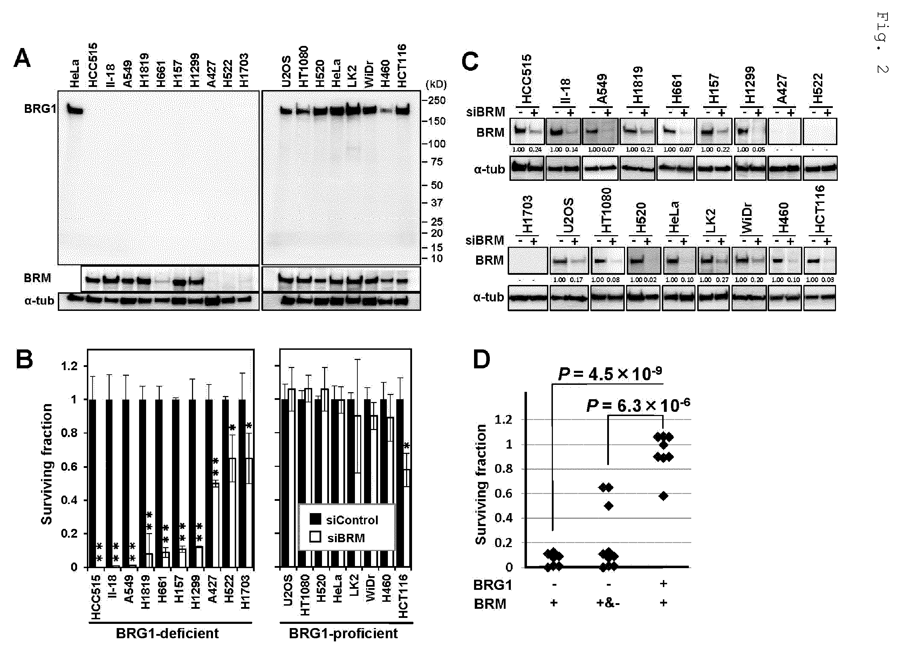

[Fig. 2] Fig. 2 is a figure for illustrating differential sensitivity of BRG1-deficient and -proficient cell lines to BRM knockdown. A: shows BRG1 protein and BRM protein expression in human cancer cell lines : BRG1-deficient (left panel) and BRG1-proficient (right panel). B: shows survival of the BRG1-deficient and-proficient cancer cells after the BRM knockdown: BRG1-deficient (left panel) and - proficient (right panel). Data are shown as mean±SD. Asterisks; significant differences in surviving fraction between siBRM-treated cells and siControl-treated cells, as determined by Student's t test (*, P<0.05; **, P<0.001). C: shows BRM protein expression in the cancer cells after the BRM knockdown. D: shows surviving fraction of the cancer cell lines according to BRG1 and BRM expression status.

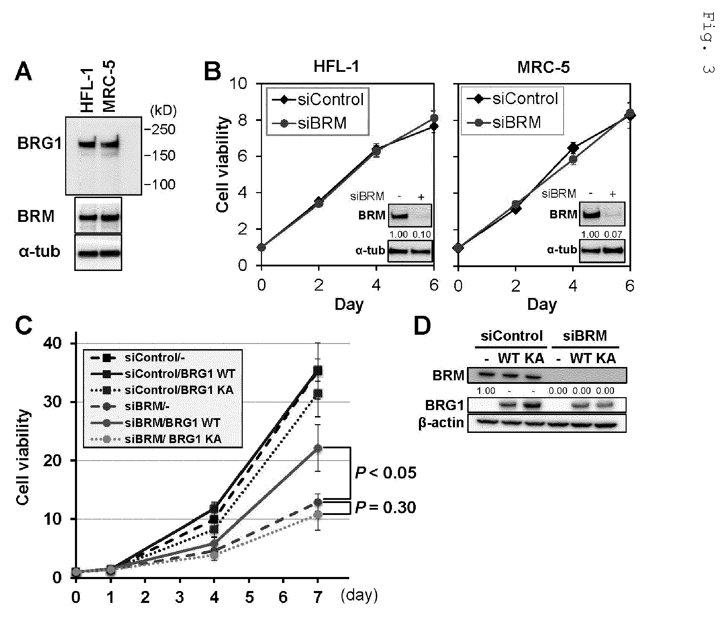

[Fig. 3] Fig. 3 is a figure for illustrating the effect of the BRM knockdown on cell growth in noncancerous cells and in NCI-H1299 cells complemented with wild-type BRG1 cDNA. A: BRG1 protein and BRM protein expression in HFL-1 cells and MRC-5 fibroblasts. B: shows surviving fraction of the HFL-1 cells and the MRC-5 cells after the BRM knockdown. Data are shown as mean±SD. C, D: illustrate BRG1 cDNA complementation assay. WT; wild-type, KA; ATPase mutant. P values were calculated using Student's t test. Data are shown as mean±SD of three replicates per treatment condition.

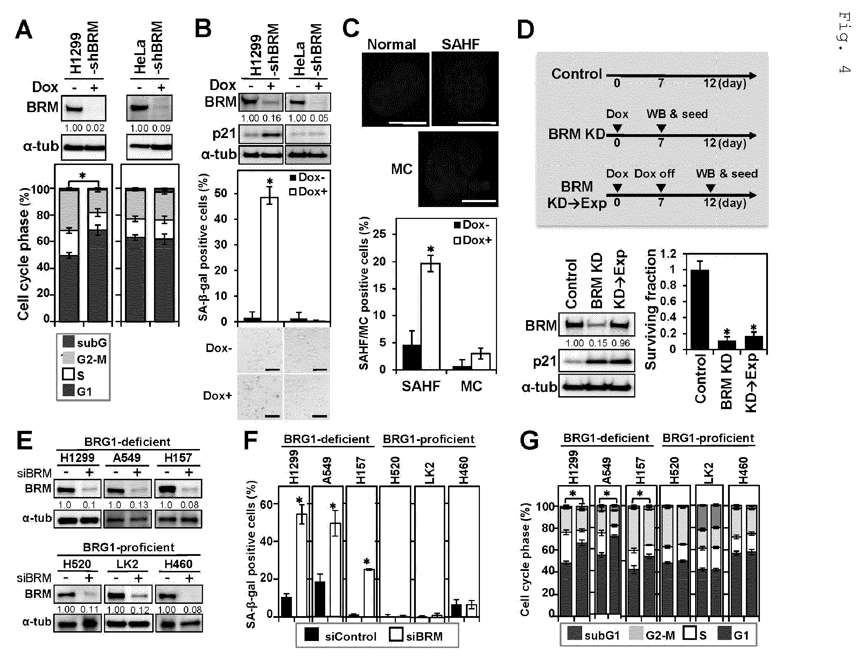

[Fig. 4] Fig. 4 is a figure for illustrating the effect of the BRM knockdown on growth inhibition in BRG1-deficient cancer cells. A: cell-cycle profile. Upper panel; immunoblot analysis of BRM and α-tubulin. Bottom panel: cell-cycle profiles determined by flow cytometry. Asterisk: a significant difference in the fraction of cells in G1-phasebetween dox-treated cells and -untreated cells as determined by Student's t test (P<0.01). B: shows expression of senescence-associated (SA)-β-gal and p21. Upper panel; immunoblot analysis of BRM, p21, and α-tubulin. Middle panel; percentage of SA-β-gal-positive cells. Asterisk; a significant difference in the percentage of SA-β-gal-positive cells between dox-treated cells and -untreated cells as determined by Student's t test (P<0.001). Bottom panel;representativemicrographs of cells stained for SA-β-gal (blue). Scale bars, 100 µm. C: shows senescence-associated heterochromatic foci (SAHF) and mitotic catastrophe (MC) as revealed by nuclear DAPI staining. Upper panel; representative images showing normal nuclear (Normal), SAHF, and MC patterns in dox-treated NCI-H1299-shBRM cells. Scale bars, 10 µm. Bottom panel; percentage of cells exhibiting SAHF and MC. Asterisk; a significant difference in the percentage of SAHF-positive cells between dox-treated cells and -untreated cells as determined by Student's t test (P<0.001). D: shows p21 expression and clonogenic survival of NCI-H1299 cells after BRM knockdown followed by reexpression of BRM. A schematic of the experimental protocol is shown at the top of this panel in the figure. NCI-H1299-shBRM cells were cultured in the presence of dox for 7 days (BRM KD) and then cultured in the absence of dox for an additional 5 days (KD→Exp). Bottom left panel; shows immunoblot analysis of BRM, p21, and α-tubulin. Bottom right panel; shows clonogenic survival assay. Data are shown as mean±SD. Asterisks; significant decreases in the surviving fraction relative to that of control cells as determined by Student's t test (P<0.001). E to G: BRG1-deficient (NCI-H1299, A549, and NCI-H157) and BRG1-proficient (NCI-H520, LK2, and NCI-H460) non-small-cell lung cancer cells were transfected twice (days 1 and 3) with BRM-targeting siRNA (siBRM) or control siRNA (siControl). E; immunoblot analysis of BRM and α-tubulin (loading control). F; positivity for SA-β-gal staining. Asterisks; significant differences in the percentage of SA-β-gal-positive cells between siBRM-treated cells and siControl-treated cells as determined by Student's t test (P<0.005). G; cell-cycle profiles determined by flow cytometry. Asterisks; significant differences in the fraction of cells in G1-phase between siBRM-treated cells and siControl-treated cells, as determined by Student's t test (P<0.005).

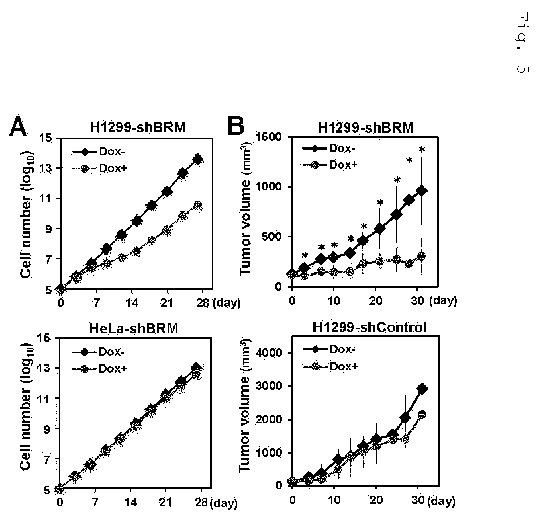

[Fig. 5] Fig. 5 is a figure for illustrating suppression of BRG1-deficient cancer cell growth by BRM knockdown both *in vitro* and *in vivo.* A: shows long-term proliferation of NCI-H1299 cells and HeLa cells after BRM knockdown. B: shows growth of NCI-H1299 xenografts in mice. Data represent mean±SD. Asterisks; significant differences in the tumor volume between the dox-fed mice and the controls, as determined by Student's t test (P<0.05).

### [Description of Embodiments]

### <Method for Evaluating Cancer, Method for Predicting Cancer Treatment Responsiveness, Method for Selecting Cancer Treatment Target>

In the present invention, tumor, malignant tumor, cancer, malignant neoplasm, carcinoma, sarcoma, and the like are collectively referred to as "tumor" or "cancer". The present invention has revealed that BRG1 and BRM have a synthetic-lethal relationship in cancer cells, and that inhibiting BRM in cancer cells having a suppressed BRG1 function can suppress the cancer cell growth. Based on these findings, it is possible to evaluate responsiveness to a cancer treatment with a compound capable of inhibiting BRM on the basis of the BRG1 function suppression. Thus, the present invention provides a method for predicting responsiveness to a cancer treatment with a compound capable of inhibiting BRM, the method comprising:
detecting whether or not a BRG1 function is suppressed in a biological sample derived from a cancer patient; and
determining that the patient, from which the BRG1 function suppression is detected, responds to the cancer treatment with the compound capable of inhibiting BRM.

In addition, a cancer treatment with a compound capable of inhibiting BRM is suitable for a patient from which BRG1 function suppression is detected as described above. On the basis of BRG1 function suppression, a patient who should receive the cancer treatment with the compound capable of inhibiting BRM and a patient who should not are selected, hence enabling an efficient treatment. Thus, the present invention also provides a method for selecting a target of a cancer treatment with a compound capable of inhibiting BRM, the method comprising:
detecting whether or not a BRG1 function is suppressed in a biological sample derived from a cancer patient; and
selecting the patient, from which the BRG1 function suppression is detected, as the target of the cancer treatment with the compound capable of inhibiting BRM.

In the present invention, the "cancer patient" may be not only a person who has a cancer, but also a person who is suspected of a cancer. In the method of the present invention, the cancer patient to be targeted for the detection of whether or not a BRG1 function is suppressed is not particularly limited, and all cancer patients can be the target. As cancers from which BRG1 function suppression is observed, for example, lung cancer, medulloblastoma, clear cell renal carcinoma, liver cancer, rhabdoid tumor, and the like have been reported. Nevertheless, such cancers are not limited to these, and maybe, for example, breast cancer, colon cancer, prostate cancer, stomach cancer, ovarian cancer, cervical cancer, endometrial cancer, uterine cancer, kidney cancer, thyroid cancer, esophageal cancer, squamous-cell carcinoma, leukemia, osteosarcoma, melanoma, glioblastoma, neuroblastoma, ovarian cancer, head and neck cancer, testicular cancer, colorectal cancer, blood cancer, retinoblastoma, pancreatic cancer, and the like. Since it is known that a BRG1 function is suppressed in 5 to 10% of cases of many cancers including lung cancer, the method of the present invention makes it possible to select a cancer patient with such a frequency as a "treatment target."

The "biological sample" used in the present invention is not particularly limited, as long as it is possible to detect whether or not a BRG1 function is suppressed. Nevertheless, the "biological sample" is preferably a cancer biopsy specimen, and may be protein extracts and nucleic acid extracts (such as mRNA extracts, cDNA preparations and cRNA preparations prepared from mRNA extracts) obtained from such samples.

In the present invention, "BRG1" and "BRM" are subunits of a SWI/SNF chromatin-remodeling complex. SEQ ID NO: 1 shows a typical base sequence of a human-derived naturally occurring BRG1 genomic DNA, SEQ ID NO: 2 shows a typical base sequence of a human-derived naturally occurring BRG1 cDNA, and SEQ ID NO: 3 shows a typical amino acid sequence of a human-derived naturally occurring BRG1 protein. Moreover, SEQ ID NO: 4 shows a typical base sequence of a human-derived naturally occurring BRM genomic DNA, SEQ ID NO: 5 shows a typical base sequence of a human-derived naturally occurring BRM cDNA, and SEQ ID NO: 6 shows a typical amino acid sequence of a human-derived naturally occurring BRM protein. It should be understood that even if BRG1 or BRM has no mutation, there may be a difference in the sequence case by case due to polymorphism or the like.

In the present invention, the phrase "BRG1 function suppression" includes both BRG1 inactivation and expression reduction. The "BRG1 inactivation" is typically caused by an inactivating mutation in BRG1 (including BRG1 deficiency). The inactivating mutation may occur, for example, by a missense mutation in a BRG1 helicase domain (positions 766 to 931 or positions 1084 to 1246 of the amino acid sequence of SEQ IDNO: 3), a missense mutation in an ATP-binding domain (positions 779 to 786 of the amino acid sequence of SEQ ID NO: 3) in the helicase domain, a nonsense mutation over the entire region, a complete or partial deletion of the gene, or the like. However, the cause is not limited to these, as long as BRG1 is inactivated. The BRG1 mutations have been reported in the literature (Rodriguez-Nieto S, et al. Hum Mutat. 2011; 32: E1999-2017) and so forth. For example, many of the BRG1 genome abnormalities are homozygous truncating mutations and deletions, and normal BRG1 expression is lost in many cases. Moreover, the loss and reduction in BRG1 expression are also observed frequently. In the present invention, this two are collectively referred to as "BRG1 deficiency."

In the present invention, the phrase "BRG1 expression reduction" includes both expression reduction at a transcription level and expression reduction at a translation level.

In the present invention, the method for "detecting BRG1 function suppression" is not particularly limited, but examples thereof include the following methods.

### -Detection of BRG1 Mutation-

In the present invention, the phrase "detecting a mutation" basically means detecting a mutation in the genomic DNA. In a case where a mutation in the genomic DNA reflects a change in a base of a transcription product or a change in an amino acid of a translation product, the phrase also means to include detecting the changes in these transcription product and translation product (i.e., indirect detection).

A preferable embodiment of the method of the present invention is a method for detecting a mutation by directly determining a base sequence of a BRG1 gene region of a cancer patient. In the present invention, the term "BRG1 gene region" means a certain region of the genomic DNA including the BRG1 gene. This region includes an expression control region (for example, a promoter region, an enhancer region) of the BRG1 gene and a 3' untranslated region of the BRG1 gene, and the like. A mutation in these regions may influence, for example, the transcription activity of the BRG1 gene.

In this method, first, a DNA sample is prepared from a biological sample derived from a cancer patient. Examples of the DNA sample include genomic DNA samples, and cDNA samples prepared from RNAs by reverse transcription.

The method for extracting a genomic DNA or an RNA from a biological sample is not particularly limited, and a known method can be selected as appropriate for use. Examples of the method for extracting a genomic DNA include : an SDS phenol method (in which a tissue is stored in ethanol or a solution containing urea, a protein of the tissue is denatured with a protease (proteinase K), a surfactant (SDS), and phenol, and a DNA is precipitated and extracted from the tissue with ethanol) ; and a DNA extraction method using Clean Columns (registered trademark, manufactured by NexTec), AquaPure (registered trademark, manufactured by Bio-Rad), ZR Plant/Seed DNA Kit (manufactured by Zymo Research), AquaGenomic Solution (registered trademark, manufactured by Mo Bi Tec), prepGEM (registered trademark, manufactured by ZyGEM), and BuccalQuick (registered trademark, manufactured by TrimGen) . Meanwhile, examples of the method for extracting an RNA include: an extraction method using phenol and a chaotropic salt (more concretely, which is an extraction method using a commercially available kit of Trizol (manufactured by Invitrogen), ISOGEN (manufactured by Wako Pure Chemical Industries, Ltd.), or the like) ; and methods using other commercially available kits (RNAPrep Total RNA Extraction Kit (manufactured by Beckman Coulter), RNeasy Mini (manufactured by QIAGEN), RNA Extraction Kit (manufactured by Pharmacia Biotech) or the like). Further, the reverse transcriptase used to prepare a cDNA from the extracted RNA is not particularly limited, and examples thereof include reverse transcriptases derived from retroviruses such as RAV (Rous associated virus) and AMV (Avian myeloblastosis virus), and reverse transcriptases derived from mouse retroviruses such as MMLV (Moloney murine leukemia virus).

In this embodiment, a DNA containing a mutation site of the BRG1 gene region is then isolated, and the base sequence of the isolated DNA is determined. The DNA can be isolated, for example, by PCR or the like using a pair of oligonucleotide primers designed to flank a mutation in the BRG1 gene region with the genomic DNA or the RNA as a template. The base sequence of the isolated DNA can be determined by a method known to those skilled in the art such as the Maxam-Gilbert method or the Sanger method.

The base sequence of DNA or cDNA thus determined is compared with that of a control (for example, the base sequence of DNA or cDNA derived from a non-cancerous tissue of the same patient). Thereby, whether or not the BRG1 gene region is mutated in the cancer cells of the cancer patient can be determined.

As the method for detecting a mutation of the BRG1 gene region, various methods capable of detecting a mutation can be employed, besides the method by which the base sequence of DNA or cDNA is directly determined.

For example, in the present invention, a mutation may be detected by a method as follows. First, a DNA or cDNA sample is prepared from a biological sample. Then, an oligonucleotide probe is prepared which has a base sequence complementary to a base sequence containing a BRG1 gene region mutation, and which is labeled with a reporter fluorescent dye and a quencher fluorescent dye. Subsequently, the oligonucleotide probe is hybridized to the DNA sample, and the base sequence containing the BRG1 gene region mutation is amplified using, as a template, the DNA sample with the oligonucleotide probe hybridized thereto. Thereafter, fluorescence is detected which is emitted by the reporter fluorescent dye through degradation of the oligonucleotide probe associated with the amplification. Then, the detected fluorescence is compared with that of a control . Examples of such a method include a double-dye probe method, and so-called TaqMan (registered trademark) probe method.

In still another method, a DNA or cDNA sample is prepared from a biological sample. Then, in a reaction system including an intercalator which emits fluorescence when inserted between DNA double strands, a base sequence containing the BRG1 gene region mutation is amplified using the DNA sample as a template. Subsequently, the temperature of the reaction system is changed to detect a variation in the intensity of fluorescence emitted by the intercalator. The detected variation in the intensity of the fluorescence due to the change in the temperature is compared with that of a control. An example of such a method includes a HRM (high resolution melting) method.

In still another method, first, a DNA or cDNA sample is prepared from a biological sample. Then, a DNA containing a mutation site of a BRG1 gene region is amplified. Subsequently, the amplified DNAs are cleaved with a restriction enzyme. Thereafter, the DNA fragments are separated on the basis of the size. After that, the size of the detected DNA fragments is compared with that of a control. Examples of such a method include methods utilizing Restriction Fragment Length Polymorphism (RFLP), PCR-RFLP method, and the like.

In still another method, first, a DNA or cDNA sample is prepared from a biological sample. Then, a DNA containing a mutation site of a BRG1 gene region is amplified. Subsequently, the amplified DNAs are each dissociated into single-stranded DNAs. Thereafter, the dissociated single-stranded DNAs are separated on a non-denaturing gel. The mobility of the separated single-stranded DNAs on the gel is compared with that of a control. An example of such a method includes the PCR-SSCP (single-strand conformation polymorphism) method.

In still another method, first, a DNA or cDNA sample is prepared from a biological sample. Then, a DNA containing a mutation site of a BRG1 gene region is amplified. Subsequently, the amplified DNAs are separated on a gel where the concentration of a DNA denaturant is gradually increased. Thereafter, the mobility of the separated DNAs on the gel is compared with that of a control. An example of such a method includes the denaturant gradient gel electrophoresis (DGGE) method.

Still another method is a method that uses a DNA containing a mutation site of a BRG1 gene region prepared from a biological sample, and a substrate having an oligonucleotide probe which is fixed thereto and capable of hybridizing to the DNA. Examples of such a method include the DNA array method, and the like.

In still another method, first, a DNA or cDNA sample is prepared from a biological sample. Moreover, prepared is an "oligonucleotide primer having a base sequence complementary to a base located on the 3' side of, and away by a distance of one base from, a base at a mutation site of a BRG1 gene region and a base sequence on the 3' side therefrom." Then, using the DNA as a template and the primer, a ddNTP primer extension reaction is carried out. Subsequently, the primer extension reaction product is subjected to a mass spectrometer for the mass measurement. Thereafter, the genotype is determined from the mass measurement result. After that, the determined genotype is compared with that of a control. An example of such a method includes the MALDI-TOF/MS method.

In still another method, first, a DNA or cDNA sample is prepared from a biological sample. Then, prepared is an oligonucleotide probe having 5'-"a base sequence complementary to a base at a mutation site of a BRG1 gene region and a base sequence on the 5' side therefrom"-"a base sequence incapable of hybridizing to a base located on the 3' side of, and away by a distance of one base from, the mutation site of the BRG1 gene region and a base sequence on the 3' side therefrom"-3' (flap). Moreover, prepared is an "oligonucleotide probe having a base sequence complementary to the base at the mutation site of the BRG1 gene region and a base sequence on the 3' side therefrom." Subsequently, the two types of the oligonucleotide probes are hybridized to the prepared DNA. Thereafter, the hybridized DNA is cleaved with a single-stranded DNA cleavage enzyme to release the flap. In the present invention, the single-stranded DNA cleavage enzyme is not particularly limited, and an example thereof includes cleavase. In this method, an oligonucleotide probe having a sequence complementary to that of the flap and labeled with reporter fluorescence and quencher fluorescence is then hybridized to the flap. Subsequently, the intensity of emitted fluorescence is measured. Thereafter, the measured fluorescence intensity is compared with that of a control. An example of such a method includes the Invader method.

In still another method, first, a DNA or cDNA sample is prepared from a biological sample. Then, a DNA containing a mutation site of a BRG1 gene region is amplified. Subsequently, the amplified DNAs are each dissociated into single strands. Only one of the dissociated single-stranded DNAs is separated. Thereafter, an extension reaction is carried out on each base from one near the base at the mutation site of the BRG1 gene region. Pyrophosphoric acid generated in this event is caused to emit light enzymatically, and the intensity of the luminescence is measured. After that, the measured fluorescence intensity is compared with that of a control. An example of such a method includes the Pyrosequencing method.

In still another method, first, a DNA or cDNA sample is prepared from a biological sample. Then, a DNA containing a mutation site of a BRG1 gene region is amplified. Subsequently, prepared is an "oligonucleotide primer having a base sequence complementary to a base located on the 3' side of, and away by a distance of one base from, a base at the mutation site of the BRG1 gene region and a base sequence on the 3' side therefrom." Thereafter, a single base extension reaction is carried out using the amplified DNAs as a template and the prepared primer in the presence of a fluorescence-labeled nucleotide. After that, the polarization of the fluorescence is measured. Then, the measured fluorescence polarization is compared with that of a control. An example of such a method includes the AcycloPrime method.

In still another method, first, a DNA or cDNA sample is prepared from a biological sample. Then, a DNA containing a mutation site of a BRG1 gene region is amplified. Subsequently, prepared is an "oligonucleotide primer having a base sequence complementary to a base located on the 3' side of, and away by a distance of one base from, a base at the mutation site of the BRG1 gene region and a base sequence on the 3' side therefrom." Thereafter, a single base extension reaction is carried out using the amplified DNAs as a template and the prepared primer in the presence of a fluorescence-labeled nucleotide. After that, the type of base used in the single base extension reaction is determined. Then, the determined base type is compared with that of a control. An example of such a method includes the SNuPE method. Note that if a mutation occurs together with a change (for example, substitution, deletion, insertion) in an amino acid in a BRG1 protein, a sample prepared from a biological sample may be a protein. To detect a mutation in such a case, it is possible to utilize a method using a molecule (for example, antibody) capable of specifically binding to a site where an amino acid changes due to the mutation. A method for detecting a protein using an antibody will be described later.

### -Detection of BRG1 Expression Reduction-

In the present invention, the term "BRG1 expression reduction" generally means that the expression level is low in comparison with a control (for example, an expression level of a non-cancerous tissue from the same patient or a healthy person).

In the method for detecting the amount of BRG1 expressed at a transcription level, first, an RNA or a cDNA is prepared from a biological sample derived from a cancer patient by the above-described method. Then, an oligonucleotide primer or an oligonucleotide probe is each used in an amplification reaction or a hybridization reaction, and the amplification product or the hybrid product is detected.

As such a method, it is possible to utilize, for example, an RT-PCR method, a northern blotting method, a dot blot method, a DNA array method, an *in situ* hybridization method, an RNase protection assay method, mRNA-seq, or the like.

Those skilled in the art can design an oligonucleotide primer and an oligonucleotide probe suitable for each method on the basis of the base sequence of BRG1 cDNA (for example, SEQ ID NO: 2) by conventional methods.

In the method for detecting the amount of BRG1 expressed at a translation level, first, a protein sample is prepared from a biological sample derived from a cancer patient. Then, an antibody specific to a BRG1 protein is used in a antigen-antibody reaction, and the binding of the antibody to the BRG1 protein is detected. In a case where the antibody specific to BRG1 is labeled, the BRG1 protein can be detected directly. Meanwhile, in a case where the antibody is not labeled, a labeled molecule (for example, secondary antibody or Protein A) capable of recognizing the antibody is additionally caused to act thereon so that the BRG1 protein can be detected indirectly by utilizing the label of the molecule.

As such a method, it is possible to utilize, for example, an immunohistochemical (immunity staining) method, a western blotting method, an ELISA method, flow cytometry, imaging cytometry, a radioimmunoassay, an immunoprecipitation method, an analysis method using an antibody array, or the like. The immunohistochemistry also has such advantages that additional information such as the morphology and distribution state of cancer cells in tissues can be obtained at the same time.

The type, origin, and so forth of the antibody used are not particularly limited, but the antibody is preferably a monoclonal antibody. As long as BRG1 can be detected with sufficient specificity, it is also possible to use an oligoclonal antibody (a mixture of several types or several tens of types of antibodies) or a polyclonal antibody. Moreover, it is also possible to use a functional fragment of the antibody such as Fab, Fab', F(ab')2, Fv, scFv, sc(Fv)2, dsFv, and a diabody, or a multimer (for example, dimer, trimer, tetramer, polymer) of the antibody. The anti-BRG1 antibody may be a commercially available product, for example, Clone EPNCIR111A (Epitomics 2822-1) or Clone G-7 (SantaCruz sc-17796).

The BRG1 protein can also be detected using a mass spectrometry method (MS). Particularly, the analysis with a mass spectrometer coupled to liquid chromatography (LC/MS) is advantageous because of its sensitivity. The measurement according to the mass spectrometry method can be performed, for example, by: preparing a protein from a biological sample, labeling the protein, fractionating the protein, subjecting the fractionated proteins to mass spectrometry, and identifying the BRG1 protein from the mass spectrometry values. As the label, an isotope labeling reagent known in the technical field can be used, and an appropriate labeling reagent can be obtained as a commercially available product. Moreover, the fractionation can also be performed by a method known in the technical field. For example, the fractionation may be performed using a commercially-available strongly cationic column and so forth.

### -Others-

It is known in the technical field that promoter hypermethylation is one main cause of gene expression reduction. Thus, in detecting whether or not a BRG1 function is suppressed, it is also conceivable to perform the detection on the basis of a methylation of a BRG1 gene promoter. To detect the promoter methylation, known methods can be utilized such as, for example, a method in which a change in a base sequence is directly detected by base sequencing after a treatment with bisulfite having an activity to convert methylated cytosine to uracil, or an indirect detection method utilizing a restriction endonuclease capable of recognizing (capable of cleaving) a base sequence before a bisulfite treatment but incapable of recognizing (incapable of cleaving) a base sequence after the bisulfite treatment.

After BRG1 function suppression is evaluated as described above, in a case where the BRG1 function suppression, for example, an inactivating mutation ablating the BRG1 function, is detected, in a case where BRG1 expression reduction is detected, or in a case where another phenomenon (for example, excessive promoter methylation) causing BRG1 inactivation or expression reduction is detected, it is possible to determine that the cancer patient responds to the cancer treatment with the compound capable of inhibiting BRM, and the cancer patient can be selected as the target of the cancer treatment with the compound capable of inhibiting BRM. Herein, the "responsiveness to a cancer treatment" and related phrases mean indicators that are used to evaluate whether or not the compound capable of inhibiting BRM can exhibit a therapeutic effect against a cancer. The determination of the responsiveness may include not only whether a patient responds or not, but also an evaluation of the degree of the responsiveness in the case where a patient responds (for example, evaluations such as that a high responsiveness can be expected, a moderate responsiveness can be expected, and so on). Thus, depending on the degree of BRG1 function suppression, a patient who may become a treatment target can be selected, for example, at such a level that a moderate responsiveness can be expected.

On the other hand, in a case where no BRG1 function suppression is observed in a cancer patient, it is possible to determine that the patient does not respond to the cancer treatment with the compound capable of inhibiting BRM, and the patient can be excluded from the target of the cancer treatment with the compound capable of inhibiting BRM. This makes it possible to improve the responsiveness rate of the treatment.

It should be noted that in a case where BRM, which is the target of the present treatment, is not properly expressed, the cancer treatment with the compound capable of inhibiting BRM may not be performed effectively. Thus, in evaluating a cancer, in predicting a responsiveness to a cancer treatment, and in selecting a cancer patient, it is also possible to further add normal expression of BRM as the indicator. The method for detecting BRM expression is the same as the above-described detection BRG1 of expression.

### <Cancer Treatment Method>

Moreover, the present invention provides a method for treating a cancer having a suppressed BRG1 function, the method comprising administering a compound capable of inhibiting BRM to a patient from which BRG1 function suppression is detected. BRG1 in a patient may be detected by a person other than a doctor, or may be detected by a doctor himself/herself.

The "compound capable of inhibiting BRM" used in the treatment method of the present invention is not particularly limited, and may be a known compound or may be a compound identified by screening which will be described later.

The compound capable of inhibiting BRM can be administered in various forms such as tablets, powders, granules, capsules, and liquids, depending on the characteristics of the compound, by oral administration or parenteral administration (for example, intravenous administration, intraarterial administration, local administration) to a cancer patient. The dose is not particularly limited, as long as the amount is effective for inhibiting BRM to treat the cancer. The dose should be selected as appropriate in accordance with the age, weight, symptom, and health state of a cancer patient, the progression of the cancer, and so forth, in addition to the properties of the compound. How often the compound is administered is not particularly limited, either, and can be selected as appropriate in accordance with the purpose. For example, as the dose administered in a day, the compound may be administered once a day, or may be administered separately multiple times a day. When the compound capable of inhibiting BRM is administered to human, the range of the dose per day is approximately 0.01 mg/kg body weight to approximately 500 mg/kg body weight, preferably approximately 0.1 mg/kg body weight to approximately 100 mg/kg body weight. When administered to human, the compound is administered preferably once a day, or preferably separately two to four times a day such that the administration is repeated at appropriate intervals. Meanwhile, the amount in a day may exceed the above-described amount in accordance with the diagnosis of the doctor as necessary.

This makes it possible to further inhibit BRM in cancer cells having a suppressed BRG1 function in a cancer patient, and to treat the cancer owing to the synthetic-lethal effect.

Examples of the cancer as the treatment target include breast cancer, colon cancer, prostate cancer, lung cancer, stomach cancer, ovarian cancer, cervical cancer, endometrial cancer, uterine cancer, kidney cancer, hepatocellular carcinoma, thyroid cancer, esophageal cancer, squamous-cell carcinoma, leukemia, osteosarcoma, melanoma, glioblastoma, neuroblastoma, ovarian cancer, head and neck cancer, testicular cancer, colorectal cancer, blood cancer, retinoblastoma, pancreatic cancer, and the like, but are not limited to these cancers.

### <Reagent for Detecting Whether or Not BRG1 Function is Suppressed>

Further, the present invention provides a reagent for detecting whether or not a BRG1 function is suppressed in the above-described methods, the reagent comprising as an active ingredient any one of the following molecules (a) to (c) :
(a) an oligonucleotide primer capable of specifically binding to a BRG1 gene;
(b) an oligonucleotide probe capable of specifically binding to a BRG1 gene; and
(c) an antibody capable of specifically binding to a BRG1 protein.

The polynucleotide primer should be designed on the basis of base sequence information on the BRG1 genomic DNA and cDNA (for example, SEQ ID NOs : 1 and 2) in such a manner as to make the primer appropriate for the aforementioned methods and amplification region, and to prevent an amplification product of genes other than the BRG1 gene from being generated as much as possible. Those skilled in the art can design such an oligonucleotide primer by conventional methods. The length of the oligonucleotide primer is normally 15 to 50 bases long, preferably 15 to 30 bases long, but may be longer depending on the method and the purpose.

The polynucleotide probe should be designed on the basis of the base sequence information on the BRG1 genomic DNA and cDNA (for example, SEQ ID NOs: 1 and 2) in such a manner as to make the primer appropriate for the aforementioned methods and hybridization region, and to prevent hybridization to genes other than the BRG1 gene from occurring as much as possible. Those skilled in the art can design such an oligonucleotide probe by conventional methods. The length of the oligonucleotide probe is normally 15 to 200 bases long, preferably 15 to 100 bases long, and furthermore preferably 15 to 50 bases long, but may be longer depending on the method and the purpose.

When used, the oligonucleotide probe is preferably labeled as appropriate. Examples of the labeling method include: a method in which the 5' end of an oligonucleotide is phosphorylated and labeled with ³²P using a T4 polynucleotide kinase; and a method in which a substrate base labeled with an isotope such as ³²P, a fluorescence dye, biotin, or the like is incorporated using a DNA polymerase such as Klenow enzyme, and random hexamer oligonucleotides as primers (random priming method and the like).

The oligonucleotide primer and the oligonucleotide probe of the present invention can be prepared, for example, using a commercially-available oligonucleotide synthesizer. The oligonucleotide probe can also be prepared as a double-stranded DNA fragment obtained by restriction enzyme treatment or the like. Meanwhile, the oligonucleotide primer and the oligonucleotide probe of the present invention may be composed only of naturally-occurring nucleotides (deoxyribonucleotides (DNA) or ribonucleotides (RNA)), or part or whole thereof may be composed of artificial nucleotides. Examples of the artificial nucleotides include PNA (polyamide nucleic acids), LNA (registered trademark, locked nucleic acids), ENA (registered trademark, 2'-O,4'-C-Ethylene-bridged nucleic acids), and complexes of these.

In the case where the antibody capable of specifically binding to a BRG1 protein is a polyclonal antibody, the polyclonal antibody can be obtained as follows. Concretely, an animal is immunized with an antigen (a BRG1 protein, a partial peptide thereof, cells expressing any of these, or the like). Then, an antiserum from the animal is purified by conventional means (for example, salting-out, centrifugation, dialysis, column chromatography, or the like) to obtain the polyclonal antibody. Meanwhile, a monoclonal antibody can be prepared by a hybridoma method or a recombinant DNA method.

A typical example of the hybridoma method includes a method by Kohler and Milstein (Kohler & Milstein, Nature, 1975; 256: 495). Antibody-producing cells used in the cell fusion process of this method are spleen cells, lymph node cells, peripheral blood leukocytes, or the like of an animal (for example, mouse, rat, hamster, rabbit, monkey, goat) immunized with an antigen (a BRG1 protein, a partial peptide thereof, cells expressing any of these, or the like). It is also possible to use antibody-producing cells obtained by causing the antigen to act, in a medium, on the above-described types of cells, lymphocytes, or the like, which are isolated from non-immunized animals in advance. As myeloma cells, various known cell lines can be used. The antibody-producing cells and the myeloma cells may be originated from different animal species, as long as they can be fused. However, the antibody-producing cells and the myeloma cells are preferably originated from the same animal species. Hybridomas can be produced, for example, by cell fusion between mouse myeloma cells and spleen cells obtained from a mouse immunized with the antigen. By the subsequent screening, a hybridoma which produces a monoclonal antibody specific to a BRG1 protein can be obtained. The monoclonal antibody against the BRG1 protein can be obtained by culturing the hybridoma, or from the ascitic fluid of a mammal to which the hybridoma is administered.

The recombinant DNA method is a method by which the antibody of the present invention is produced as a recombinant antibody as follows. Concretely, a DNA encoding the aforementioned antibody is cloned from a hybridoma, B cells, or the like. The cloned DNA is incorporated into an appropriate vector, which is then introduced into host cells (for example, a mammalian cell line, *Escherichia coli,* yeast cells, insect cells, plant cells, or the like) for the production (for example, P. J. Delves, Antibody Production: Essential Techniques, 1997 WILEY, P. Shepherd and C. Dean Monoclonal Antibodies, 2000 OXFORD UNIVERSITY PRESS, Vandamme A. M. et al., Eur. J. Biochem. 1990; 192: 767-775). For the expression of the DNA encoding the antibody, DNAs encoding the heavy chain or the light chain may be incorporated separately into expression vectors to transform the host cells. Alternatively, the DNAs encoding the heavy chain and the light chain may be incorporated into a single expression vector to transform the host cells (see WO94/11523). The antibody can be obtained in a substantially pure and homogeneous form by culturing the host cells, followed by separation and purification in the host cells or from the culture liquid. For the separation and purification of the antibody, an ordinary method used for polypeptide purification can be employed. Once a transgenic animal (cattle, goat, sheep, pig, or the like) incorporating the antibody gene is prepared using a transgenic animal production technique, a large amount of monoclonal antibodies derived from the antibody gene can also be obtained from milk of the transgenic animal.

From the antibody obtained in the above described manner or the gene, it is possible to prepare a functional fragment of the antibody such as Fab, Fab', F(ab')2, Fv, scFv, sc(Fv)2, dsFv, and a diabody, and a multimer (for example, dimer, trimer, tetramer, polymer) of the antibody.

In a case where the amount of the antibody bound to the BRG1 protein is directly detected, the obtained anti-BRG1 antibody is directly labeled with an enzyme, a radioisotope, a fluorescence dye, an avidin-biotin system, or the like for use. On the other hand, in a case where an indirect detection method is performed to detect the amount of the antibodybound to the BRG1 protein by utilizing a secondary antibody or the like, the obtained anti-BRG1 antibody (primary antibody) does not necessarily have to be labeled; in the detection, it is only necessary to use a labeled molecule (for example, secondary antibody or Protein A) capable of recognizing the antibody.

The reagent of the present invention may comprise, as necessary, other ingredients acceptable as a reagent such as sterile water, a physiological saline, a buffer, and a preservative, in addition to the above-described molecules as the active ingredients.

### <Screening Method for Compound Used in Cancer Treatment>

Furthermore, the present invention provides a screening method for a compound used in a treatment against a cancer having a suppressed BRG1 function, the method comprising the step of selecting the compound based on whether or not BRM is inhibited.

A test compound used in the screening system of the present invention is not particularly limited. Examples thereof include synthetic low-molecular-weight compound libraries, expression products from gene libraries, peptide libraries, siRNAs, antibodies, substances released from bacteria, liquid extracts and culture supernatants of cells (microorganisms, plant cells, animal cells), purified or partially purified polypeptides, extracts derived from marine organisms, plants, or animals, and random phage peptide display libraries.

In the present invention, "BRM inhibition" and related phrases mean to include both BRM activity inhibition and expression inhibition. As revealed in the present Examples, particularly a loss in the ATPase activity of BRM contributes to the synthetic lethality of BRG1 and BRM. Thus, the BRM inhibition, on which the screening is based, is preferably inhibition of the ATPase activity of BRM.

The screening system based on the ATPase activity inhibition is preferably an *in vitro* system containing a SWI/SNF chromatin-remodeling complex including BRM, and an ATPase reaction solution, from the viewpoint that specific detection is possible with high sensitivity. This *in vitro* system does not necessarily have to have all proteins constituting the SWI/SNF chromatin-remodeling complex, but the complex preferably contains BAF170, BAF155, and INI1 in addition to BRM as described in the present Examples.

In the screening, a test compound is added, and then the ATP consumption is detected. The ATPase consumption can be easily detected using a commercially available product, for example, ADP-glo (manufactured by Promega). As a result of the detection, if the consumption is reduced in comparison with ATPase consumption of a control (for example, a case where no test compound is added), it is possible to evaluate that the ATPase activity is inhibited.

In the case where BRM expression inhibition is detected, for example, a test compound is caused to act on cells expressing BRM, and then the BRM expression should be detected at a transcription level or translation level by the aforementioned method. Alternatively, it is also possible to use a reporter assay system utilizing an expression construct in which a reporter gene is ligated downstream of a BRM promoter. As a result of the detection, if the expression is reduced in comparison with BRM expression (in the reporter system, it is reporter expression instead of the BRM expression) of a control (for example, a case where no test compound is added), it is possible to evaluate that the BRM expression is inhibited.

The compound thus identified by the screening of the present invention may be mixed with a pharmacologically acceptable carrier and formulated into a drug by known pharmaceutical methods. Examples of the pharmacologically acceptable carrier include sterile water, physiological saline, vegetable oils, solvents, bases, emulsifiers, suspensions, surfactants, stabilizers, flavors, aromatic substances, excipients, vehicles, antiseptics, binders, diluents, isotonic agents, soothing agents, bulking agents, disintegrators, buffers, coating agents, lubricants, colorants, sweeteners, viscous agents, flavor modifiers, solubilizers, other additives, and the like, but are not limited to these.

### [Examples]

### (Example 1) Development of Therapeutic Strategy based on Synthetic Lethality of BRG1/SMARCA4 Chromatin-Remodeling Gene Deficient Cancer

### 1. Materials and Methods

### (1) Non-Small-Cell Lung Carcinoma (NSCLC) Cohort

Surgical specimens from 103 patients with NSCLC (51 adenocarcinomas and 53 squamous cell carcinomas), who received surgical resection at the National Cancer Center Hospital (Tokyo, Japan) between 1997 to 2007, were used for the analysis. The subjects were randomly selected from a cohort of 318 cases including similar numbers of lung adenocarcinoma and squamous-cell carcinoma cases of three differentiation grades (well differentiated, moderately differentiated, and poorly differentiated) (Tsuta K, et al. J Thorac Oncol. 2011; 6: 1190-9). The 103 specimens analyzed in this study include similar numbers of specimens representing the three differentiation grades: 18 well, 18 moderately, and 14 poorly differentiated adenocarcinomas, as well as 20 well, 16 moderately, and 17 poorly differentiated squamous-cell carcinomas. Detailed information regarding the 318-case cohort and their pathological characteristics has been reported previously (TsutaK, etal. J Thorac Oncol. 2011; 6: 1190-9). The histological diagnoses were determined according to the World Health Organization (WHO) classification (Pathology and Genetics, Tumours of Lung, Pleura, Thymus and Heart. In Travis, WD, Brambilla, E, Muller-Hermelink, HK and Harris, CC (eds), World Health Organization Classification of Tumors; 2004: 1-344). Table 1 shows the characteristics of the patients. This study was approved by the Institutional Review Board of the National Cancer Center.

**[Table 1]**

| Patient characteristics and their association with lost/reduced BRG1/SMARCA4 expression | | | | | |
|---|---|---|---|---|---|
| Variant | All | BRG1 lost/reduced | BRG1 retained | P value | |
| | No. (%) | No. (%) | No. (%) | Univariate* | Multivariate^{†} |
| Total patients Age | 103 (100.0) | 16 (100.0) | 87 (100.0) | NT | NT |
| Median [range]±SD | 67 [44-82]±8 | 60 [48-78]±8 | 67 [44-82]±8 | | |
| >median | 52 (50.5) | 5 (31.3) | 47 (54.0) | 0.094 | 0.13 |
| Gender | | | | | |
| Male | 73 (70.9) | 16 (100.0) | 57 (65.5) | | |
| | | | | 0.0053 | NT |
| Female | 30 (29.1) | 0 (0.0) | 30 (34.5) | | |
| Histological cell type | | | | | |
| Adenocarcinoma | 50 (48.5) | 8 (50.0) | 42 (48.3) | | |
| | | | | 0.90 | NT |
| Squamous-cell carcinoma | 53 (51.5) | 8 (50.0) | 45 (51.7) | | |
| Differentiation | | | | | |
| Well differentiated | 38 (36.9) | 2 (12.5) | 36 (41.4) | | |
| Moderately differentiated | 34 (33.0) | 3 (18.8) | 31 (35.6) | 0.0011 | 0.022 |
| Poorly differentiated | 31 (30.1) | 11 (68.8) | 20 (23.0) | | |
| Pathological stage | | | | | |
| I | 42 (40.8) | 5 (31.3) | 37 (42.5) | | |
| II | 28 (27.2) | 8 (50.0) | 20 (23.0) | 0.078 | 0.20 |
| III and IV | 33 (32.0) | 3 (18.8) | 30 (34.5) | | |
| Smoking habit | | | | | |
| Never-smoker | 26 (25.2) | 1 (6.3) | 25 (38.7) | | |
| | | | | 0.057 | 0.17 |
| Ever-smoker | 77 (74.8) | 15 (93.8) | 62 (71.3) | | |
| EGFR mutation | | | | | |
| Mutant | 27 (26.2) | 0 (0.0) | 27 (31.0) | | |
| | | | | 0.0095 | NT |
| Wild-type | 76 (73.8) | 16 (100.0) | 60 (69.0) | | |
| KRAS mutation | | | | | |
| Mutant | 10 (9.7) | 0 (0.0) | 10 (11.5) | | |
| | | | | 0.15 | NT |
| Wild-type | 93 (90.3) | 16 (100.0) | 77 (88.5) | | |
| ALK fusion | | | | | |
| Positive | 1 (1.0) | 0 (0.0) | 1 (1.1) | | |
| | | | | 0.67 | NT |
| Negative | 102 (99.0) | 16 (100.0) | 86 (98.9) | | |
| FGFR1 amplification | | | | | |
| Positive | 1 (1.0) | 0 (0.0) | 1 (1.1) | | |
| | | | | 0.67 | NT |
| Negative | 68 (99.0) | 12 (100.0) | 56 (98.9) | | |
| BRM expression | | | | | |
| Lost or reduced | 40 (38.8) | 6 (37.5) | 34 (39.1) | | |
| | | | | 0.91 | NT |
| Retained | 63 (61.2) | 10 (62.5) | 53 (60.9) | | |

| | | | | | |
|---|---|---|---|---|---|
| Abbreviation: NT, not tested *Association was examined by Fisher's exact test. ^{†}Association was examined by logistic regression test. Variables showing associations with P<0.1 in the univariate analysis were subjected to this analysis. Gender and EGFR mutation were not included; they are not suitable for this analysis because no females and EGFR mutants were present in the "BRG lost/reduced" group. | | | | | |

### (2) Immunohistochemical Analysis of BRG1 and BRM

Immunohistochemical analysis was performed on tissue microarray sections obtained from anonymized NSCLC samples. Formalin-fixed paraffin-embedded (FFPE) tissue sections were de-waxed in xylene, rinsed well in ethanol, and then washed in tap water. Antigen retrieval was performed by pressure cooking in 0.01 M citric acid (pH 6.0) (for BRM), or by immersion in TRS (target-retrieval solution) (manufactured by Dako) in a water bath at 95°C (for BRG1). The sections were allowed to cool for 40 minutes at room temperature, and were then blocked in 1% hydrogen peroxide for 10 minutes, rinsed in tap water, placed in a humid staining chamber, and covered with TBS buffer (pH 7.6) for 5 minutes. The TBS buffer was drained off, and the sections were incubated with normal rabbit serum (NRS; Dako) diluted 1:5 in TBS. The sections were then incubated with a rabbit anti-BRG1 monoclonal antibody (1:200; Epitomics, 2822-1) for 1 hour at room temperature, or incubated with a rabbit anti-BRM polyclonal antibody (1:800; manufactured by Sigma, HPA029981) for 30 minutes at room temperature. The sections were then rinsed and incubated with Envision rabbit/HPP (manufactured by Dako) for 20 minutes. Finally, the sections were rinsed in running tap water, counter-stained as required, dehydrated, and mounted.

Salivary-gland tissue specimens exhibiting strong staining for both BRG1 and BRM in the nuclei were used as positive controls in each tissue microarray. The staining intensity of the BRG1 and BRM antibodies in the nuclei of each lung-cancer sample was expressed according to the intensity scores described by Fukuoka et al. (Fukuoka J, et al. Clin Cancer Res. 2004; 10: 4314-24) with some modifications: 0 (no signal), 1 (weak), 2 (moderate), and 3 (strong, i.e., the level found in salivary-gland cells). In addition, the intratumoral fractions of regions with each score (0 to 3) were determined. Six non-cancerous lung tissues (n=6) were also subjected to the same protocol. The expression levels of BRM and BRG1 in each tissue were compared according to the total score (TS), which was calculated as the sum of [intensity scores×fractions] for the scored regions. A tumor sample was judged to be positive when the TS was higher than the average TS calculated for normal lung epithelial tissues (n=6). Specific staining for BRG1 and BRM was confirmed by immunoblot analysis of cancer cell lines.

### (3) Examination of Driver Gene Mutations

Genomic DNA obtained from tumor tissues was analyzed for somatic mutations in the EGFR and KRAS genes using the high-resolution melting method (Takano T, et al. J Clin Oncol. 2005; 23: 6829-37). Mutations in all DDR2 coding regions in squamous cell lung carcinoma samples were examined by targeted genome capture and massively parallel sequencing using an Ion PGM sequencing system and the Ion TargetSeq Custom Enrichment Kit (manufactured by Life Technologies). EML4-ALK and KIF5B-ALK fusions were screened by immunohistochemistry using an anti-ALK antibody (Abcam; 5A4), followed by confirmation via reverse-transcriptase polymerase chain reaction and/or fluorescence/chromogenic *in situ* hybridization (Yoshida A, et al. Am J Surg Pathol. 2011; 35: 1226-34). RET and ROS1 fusions (in cases for which RNA samples were available) were examined by RT-PCR (Kohno T, et al. Nat Med. 2012; 18: 375-7, Takeuchi K, et al. Nat Med. 2012; 18: 378-81). FGFR1 amplification was examined by performing fluorescence *in situ* hybridization (FISH) analysis on 69 NSCLC samples that were available for analysis. This procedure was performed on formalin-fixed, paraffin-embedded, 4-µm-thick tumor tissues that had been sampled in duplicate from representative regions in each tumor. The present inventors used FISH probes for FGFR1 (RP11-513DS and RP11-100B16, which were labeled by Spectrum Orange, Chromosome Science Labo, Inc; Sapporo, Japan) and FISH probes for chromosome 8 centromere (CEN8) (RP11-837P5 and RP11-11P6, which were labeled by Spectrum Green, Chromosome Science Labo, Inc). FISH analysis and signal capture were performed through a fluorescence microscope (AXIO, manufactured by Zeiss) coupled to ISIS FISH Imaging System (manufactured by Metasystems). At least 60 inter-phase nuclei from each tumor were scored, and FGFR1 amplification was defined if one of the following criteria was fulfilled: (I) FGFR1/CEN8 ratio was ≥2.0, (II) average gene copy per nucleus was ≥6.0, and (III) percentage of tumor cells containing ≥15 gene copies or large cluster was ≥10% (Schildhaus HU, et al. Mod Pathol. 2012; 25: 1473-80).

### (4) Cell Lines

A427, A549, NCI-H157, NCI-H460, NCI-H520, NCI-H522, NCI-H661, NCI-H1299, NCI-H1703, NCI-H1819, LK2, HCC515, II-18 (NSCLC), U2OS (osteosarcoma), HT1080 (fibrosarcoma), HeLa (cervical cancer), WiDr, HCT116 (colorectal cancer), HFL-1, and MRC-5 (fibroblast) cells were cultured in RPMI-1640 (Gibco) supplemented with 10% fetal bovine serum (FBS). Then, 293FT cells were cultured in DMEM supplemented with 10% FBS. A549, NCI-H1819, NCI-H661, NCI-H157, NCI-H1299, A427, NCI-H522, and NCI-H1703 were lung cancer cell lines harboring truncating BRG1 mutations, whereas NCI-H520, HeLa, LK2, NCI-H460, U2OS, and HT1080 carried wild-type BRG1 (Medina PP, et al. Hum Mutat. 2008; 29: 617-22, Blanco R, et al. Hum Mutat. 2009; 30: 1199-206). Alterations in BRG1 and other cancer-related genes were verified in the cell lines the present inventors examined. Some of the results were previously reported (Blanco R, et al. Hum Mutat. 2009; 30: 1199-206).

Mutations in BRG1 and BRM coding regions in HCC515 and II-18 cells, which did not express BRG1, and 16 primary tumors negative for BRG staining were examined by targeted genome capture and massively parallel sequencing of genomic DNA, as described above. Mutations detected were verified by Sanger sequencing of genomic PCR products. The effect of single-nucleotide variants resulting in amino-acid changes in the BRG1 ATPase domain, expressed in HCT116 (L1163P) and WiDr (D1284N) colorectal cancer cells (Medina PP, et al. Genes Chromosomes Cancer. 2004; 41: 170-7), was estimated using the Polyphen and Polyphen2 programs. The substitutions in HCT116 cells are thought to abrogate ATPase activity, but those in WiDr cells are benign.

### (5) Short Interfering (si)RNA

ON-TARGET plus SMARTpool siRNA (manufactured by Dharmacon) was used in this study. To knock down the BRM protein, cells were transfected with siBRM (Cat No: L-017253-00: SMARCA2) using Lipofectamine RNAiMAX (manufactured by Invitrogen). Non-targeting siRNA (L-001810-10) was used as a negative control.

### (6) Establishment of Cells Expressing Doxycycline-Inducible Short Hairpin (sh)BRM

To achieve doxycycline (dox)-inducible knockdown of BRM, a lentiviral vector-based shRNA-mediated conditional gene-expression system (Wiznerowicz M, Trono D. J Virol. 2003; 77: 8957-61) was used. In this system, tTRKRAB (a fusion of the tetracycline repressor [tTR] and the Krueppel-associated box [KRAB] proteins) binds to the Tet operator (TetO), which is juxtaposed with the H1 promoter, to create a local heterochromatic state extending over 2 to 3 kb. This leads to suppressed shRNA expression downstream of the H1 promoter. In the presence of doxycycline (dox), tTRKRAB does not bind to TetO, allowing expression of the shRNA, which then down-regulates expression of the target genes. Thus, both up-regulation and down-regulation of the target gene can be reversibly controlled by dox.

shBRM oligos were annealed and cloned into the Mlu1-Cla1 site downstream of the H1 promoter juxtaposed with TetO in the pLV-THM retrovirus plasmid (Addgene) to yield pLV-THM-shBRM. The sequences for the shBRM oligos are:
"5'-cgcgtccccGGAGGTGCTAAGACACTTATGttcaagagaCATAAGTGTCT TAGCACCTCCtttttggaaat-3' (SEQ ID NO: 7)" and "5'-cgatttccaaaaaGGAGGTGCTAAGACACTTATGtctcttgaaCATAAGT GTCTTAGCACCTCCGGGGa-3"' (SEQ ID NO: 8) (Yamamichi N, et al. Cancer Res. 2007; 67: 10727-35). pLV-tTRKRAB, a plasmid vector carrying tTRKRAB, was purchased from Addgene. 293FT cells were co-transfected with pLV-THM-shBRM, pLV-THsi/Control carrying a shRNA targeting green fluorescent protein (GFP) (Bruno T, et al. Cancer Cell. 2010; 18: 122-34), or pLV-tTR-KRAB, together with the packaging plasmids psPAX2 and pMD2.G (Addgene), using Lipofectamine 2000 (Invitrogen), to produce lentiviruses for transduction of LV-THM-shBRM, LV-TH si/Control, or LV-tTRKRAB DNAs, respectively. NCI-H1299 cells were infected with LV-tTRKRAB, and a single clone was isolated. This clone exhibited high tTRKRAB protein expression, as revealed by immunoblot analysis using an antibody specific for tTR (manufacturedbyMobiTec, TET01). This NCI-H1299 clone was then subjected to retroviral infection with either LV-THM-shBRM or LV-TH si/Control (to yield NCI-H1299-shBRM or NCI-H1299-shControl cells, respectively), and then mass-cultured. HeLa-shBRM and HeLa-shControl cells were also established in the same manner. For the *in vitro* knockdown experiments, cells were cultured in growth medium containing 0.1 µg/ml dox. The medium was changed every 3 days.

### (7) Immunoblot Analysis

Immunoblot analysis was performed as previously described (Ogiwara H, et al. Oncogene. 2011; 30: 2135-46), using antibodies specific for the following proteins: BRG1/SMARCA4 (manufactured by Epitomics; 2776-1), BRM/SMARCA2 (manufactured by Abcam; ab15597), SNF5/SMARCB1 (manufactured by Epitomics; 5542-1), ARID1A/SMARCF1 (manufactured by Bethyl; A301-041A-1), ARID2 (manufactured by Bethyl; A302-230A-1), BAF180 (manufactured by Bethyl; A301-591A), SNF2H/SMARCA5 (manufactured by Abcam; ab3749-100), p21/CDKN1A (manufactured by Cell Signaling Technologies; 29475S), α-tubulin (manufactured by Calbiochem; CP06), and β-actin (manufacturedbyCell Signaling Technologies; 3700P). The expression level of BRM protein was quantified using the MultiGauge software (manufactured by FUJIFILM Co., Ltd.).

### (8) Cell Survival Assay

The effect of BRM knockdown on the survival of BRG1-deficient (undetectable by immunoblot analysis) and BRG1-proficient (detectable by immunoblot analysis) cancer cells was evaluated using clonogenic survival assays. For this purpose, cultured cancer cells were trypsinized, counted, seeded in specifiednumbers in 6-well culture dishes, and cultured for a further 10 days to allow colony formation. The cells were then fixed for 5 minutes in a solution containing 50% (v/v) methanol/0.01% (w/v) crystal violet. Colonies containing at least 50 cells were counted.

Since non-cancerous fibroblasts do not form colonies on culture plates, the viability of these cells was determined by examining cellular ATP levels using the CellTiter-Glo Luminescent Cell Viability Assay kit (manufactured by Promega). For this purpose, cultured fibroblasts were trypsinized, counted, seeded in 96-well plates (2000 cells/well), and cultured for an additional 8 hours to allow the cells to attach. This assay was performed every 2 days (up to Day 6).

### (9) Complementation Assay

The complementary role of BRG1 was examined in BRM knockdown cancer cells by transfecting cDNAs encoding wild-type and mutant BRG1 (BRG1^{K785A}, this mutation disrupts the ATP-binding pocket in BRG1; Zhang J, et al. Mol Cell Biol. 2006; 26: 7942-52) into NCI-H1299 cells. The human BRG1 cDNA clone was obtained from the Biological Resource Center (NBRC) (National Institute of Technology and Evaluation: NITE) (Kisarazu, Japan). Complementary DNAs encoding FLAG-tagged wild-type BRG1 and BRG1^{K785A} were subcloned into the pcDNA3.1 expression vector (manufactured by Invitrogen). The BRG1^{K785A} mutation was introduced by changing bases 2353 to 2355 of the human BRG1 cDNA to GCC by site-directed mutagenesis. Control siRNAs (D-001810-02-05) and siRNAs targeting BRM (LQ-017253-00-0002) were obtained from Thermo Scientific.

siRNAs (5 nM) were transfected into NCI-H1299 cells using Lipofectamine RNAiMAX (manufactured by Invitrogen). Twenty-four hours after the transfection, the cells were re-transfected with 2.5 µg of plasmid DNA using Lipofectamine LTX (manufactured by Invitrogen). Another 24 hours later (defined as Day 0), the cells were seeded into a 96-well plate (1000 cells/100 µl/well). On Day 2, the cells were re-transfected with 5 nM of siRNAs. To measure cell viability, ATPlite 1step (manufactured by Perkin Elmer) was added to the cells, and the fluorescence was measured in an Wallac 1420 ARVO SX multilabel counter (manufactured by Perkin Elmer). An anti-BRG1 monoclonal antibody (E1), an anti-β-actin monoclonal antibody (AC-15) (manufacturedby Santa Cruz Biotechnology), and an anti-BRM polyclonal antibody (manufactured by Cell Signaling Technology) were used for immunoblot analysis.

### (10) Cell-Cycle Analysis

Cells were trypsinized, centrifuged, washed in PBS, and fixed in ice-cold 70% ethanol. The cells were then centrifuged again, incubated with PBS containing 200 µg/ml RNase A and 5 µg/ml propidium iodide, and analyzed for cell-cycle distribution by Guava flow cytometry (manufactured by Millipore).

### (11) Senescence-Associated β-Galactosidase (SA-β-Gal) Staining

SA-β-gal staining was performed using the Senescence β-galactosidase Staining Kit (Cell Signaling Technologies). The percentage of SA-β-gal-positive cells was determined from three different fields per sample.

### (12) Fluorescence Microscopy

Cells were grown on glass coverslips, fixed with 2% (w/v) paraformaldehyde in PBS for 15 minutes at room temperature, and permeabilized with 0.3% (v/v) Triton X-100 for 15 minutes at room temperature. After the fixation, the cells were washed three times with PBS and stained with DAPI (0.4 µg/ml diluted in PBS) to visualize the DNA. The coverslips were mounted on glass slides using Prolong Gold mounting agent (manufactured by Invitrogen). Confocal images were taken using an inverted microscope (manufactured by Carl Zeiss) equipped with a 100× oil lens. The images were acquired using the ZEN Software (2008) (manufactured by Carl Zeiss). The percentage of cells containing senescence-associated heterochromatic foci (SAHF) or undergoing mitotic catastrophe was quantified in three different fields per sample. SAHF were judged as previously described (Di Micco R, et al. Nat Cell Biol. 2011; 13: 292-302). Cells having two or more distinct nuclear lobes were scored as positive for mitotic catastrophe (Russo AL, et al. Clin Cancer Res. 2009; 15: 607-12).

### (13) Long-Term In Vitro Cell-Proliferation Assay

NCI-H1299-shBRM cells and HeLa-shBRM cells were seeded in 60-mm culture dishes (1×10⁵ cells/dish) in media with or without 0 . 1 µg/ml dox, and then cultured. The cells were trypsinized and counted every three days, and after each count, 1×10⁵ cells were re-seeded in new dishes with fresh medium. Accumulated cell numbers were calculated at each passage, based on the total number of cells and dilution ratio at the time of passage. The experiment was performed in duplicate.

### (14) Mouse Xenograft Model

The effect of BRM ablation on the growth of NCI-H1299-shBRM tumor cell xenograft and NCI-H1299-shControl tumor cell xenograft was examined in BALB/c-nu/nu mice. NCI-H1299-shBRM cells and H1299-shControl cells (4×10⁶ cells/mouse in 50% Matrigel; manufactured by BD Biosciences) were injected subcutaneously into 40-day-old female BALB/c-nu/nu mice (manufactured by CLEA Japan, Inc.). After 3 weeks, when the tumors reached 100 mm³, the mice were randomly divided into two groups and fed either a diet containing dox (200 ppm) or a control diet. Tumor growth was measured twice per week using calipers. Tumor volume (TV) was calculated as previously described (Bruno T, et al. Cancer Cell. 2010; 18: 122-34) using the formula TV=a×(b²)/2 (in the formula, a and b are the tumor length and width, respectively). At the end of the experiment, the mice were sacrificed in accordance with standard protocols. All the experiments were approved by the Ethical Committee on Animal Experiments at the National Cancer Center.

### (15) Statistical Analysis

Logistic regression analysis was performed using JMP (ver 5.1). Fisher's exact test and Student's t test were performed using Statmate III (ver 3.17). P<0.05 was considered statistically significant. Experiments were performed in triplicate unless otherwise stated.

### 2. Results

### (1) Characteristics of BRG1-Deficient NSCLCs

The present inventors first of all examined the characteristics of BRG1-deficient NSCLCs. Since BRG1 has been reported to play a role in cell differentiation (Romero OA, et al. EMBO Mol Med. 2012; 4: 603-16), the examined cases were selected to include similar numbers of adenocarcinomas and squamous-cell carcinomas of the three different differentiation grades (well differentiated; moderately differentiated, and poorly differentiated) (Table 1). This approach allowed an efficient analysis of the association between BRG1 expression and tumor differentiation. Immunohistochemical analysis revealed that BRG1 protein expression was low or absent in 16/103 (15.5%) NSCLCs (Fig. 1B, C). Such low or absent BRG1 expression was more prevalent in poorly differentiated tumors (Table 1 and Fig. 1D), and was also prevalent in males and smokers. Mutational analysis of BRG1 and BRM in 16 cases negative for BRG1 staining revealed that one case had nonsense BRG1 mutations, whereas the remaining 15 cases did not. This suggests that both genetic alterations and epigenetic silencing of the BRG1 gene can cause BRG1 deficiency, as previously reported (Rodriguez-Nieto S, et al. Hum Mutat. 2011; 32: E1999-2017). On the other hand, deleterious BRM mutations were not detected in these 16 cases including five that were negative for BRM staining.

### (2) Driver Gene Mutations in BRG1-Deficient NSCLCs

Next, the present inventors analyzed representative therapeutically relevant gene mutations (EGFR and KRAS gene mutations and ALK fusions) in the 103 NSCLC cases. The 16 BRG1-deficient cases were negative for mutations in all three driver genes, including two therapeutic targets (EGFR mutation and ALK fusion) (Fig. 1D, Table 1). The present inventors also analyzed other driver gene mutations, RET and ROS1 fusions (Kohno T, et al. Nat Med. 2012; 18: 375-7, Takeuchi K, et al. Nat Med. 2012; 18: 378-81, Lipson D, et al. Nat Med. 2012; 18: 382-4), in the nine BRG1-deficient cases for which tumor tissue RNA was available, but no fusions were detected. Recent whole-genome analyses have also suggested that BRG1 mutations are prevalent in tumors without EGFR mutations or ALK fusions (Seo JS, et al. Genome Res. 2012; 22: 2109-19, Imielinski M, et al. Cell. 2012; 150: 1107-20). In addition, all the BRG1-deficient cases were also negative for two other alterations, FGFR1 amplifications and DDR2 mutations (Hammerman PS, et al. Cancer Discov. 2011; 1: 78-89, Weiss J, et al. Sci Transl Med. 2010; 2: 62ra93) (Fig. 1D, Table 1). These findings suggest that most BRG1-deficient NSCLCs are not suitable for current molecularly targeted therapies based on tyrosine kinase inhibitors (Lovly CM, Carbone DP. Nat Rev Clin Oncol. 2011; 8: 68-70). Of the 16 BRG1-deficient cases, 10 (62.5%, 9.7% of all cases studied) expressed BRM.

### (3) BRM-Dependent Growth of BRG1-Deficient Cancer Cells

The present inventors then compared the effect of siRNA-mediated BRM knockdown on cell growth of BRG1-deficient (n=10) and BRG1-proficient (n=8) cancer cell lines, using a clonogenic survival assay (Clonogenic SURVIVAL assay) (Fig. 2A). Eight out of ten BRG1-deficient cell lines used in the study carry truncating BRG1 mutations/deletions (Medina PP, et al. Hum Mutat. 2008; 29: 617-22, Blanco R, et al. HumMutat. 2009; 30: 1199-206). Of the remaining two BRG1-deficient cell lines, the present inventors detected a homozygous nonsense mutation in one (HCC515) by targeted genome capture sequencing, but no BRG1 mutation in the other (II-18). On the other hand, BRG1-proficient cancer cells did not harbor such deleterious BRG1 mutations (except HCT116 cells, which harbor a heterozygous missense mutation that is likely to disrupt the BRG1 ATPase domain; Wong AK, et al. Cancer Res. 2000; 60: 6171-7).

The present inventors observed suppression of colony formation by BRM knockdown in all BRG1-deficient cells tested, but not in BRG1-proficient cell lines other than HCT116 (Fig. 2B). The cell surviving fractions of cancer cell lines are summarized according to BRG1 and BRM expression status in Fig. 2D. Survival of BRG1-deficient cells upon BRM knockdown was significantly lower than that of BRG1-proficient cell lines (P=6.3×10⁻⁶ by Student's t test). The BRM protein was undetectable, or detectable only at trace levels, in three BRG1-deficient cell lines, A427, NCI-H522, and NCI-H1703 (Fig. 2A). These findings are consistent with a report showing that a fraction of lung-cancer cells lack, or express at low levels, both BRG1 and BRM (Reisman DN, et al. Cancer Res. 2003; 63: 560-6) (Fig. 1B). The effect of siRNA-mediated BRM knockdown on the cell growth and survival in these three cell lines was less evident than in the other seven cell lines. Thus, among BRG1-deficient cell lines, the suppression of colony formation by BRM knockdown was more prominent in cell lines that originally expressed the BRM protein (P=4.5×10⁻⁹ by Student's t test) (Fig. 2D).

BRM knockdown did not affect the growth of the non-cancerous fibroblast cell lines HFL-1 and MRC-5, which expressed both BRG1 and BRM (Fig. 3A, B). Wild-type BRG1 cDNA complemented the sensitivity of NCI-H1299 cells to BRM knockdown-mediated growth inhibition (Fig. 3C, D). On the other hand, cDNA of an ATPase mutant with a disrupted ATP-binding pocket (BRG1^{K785A}) (Zhang J, et al. Mol Cell Biol. 2006; 26: 7942-52) did not complement the sensitivity (BRG1^{K785A} is indicated as "KA" in Fig. 3C, D). These results indicated that BRG1-deficient cancer cells depend on BRM for growth, and that this phenotype is caused by a lack of BRG1 ATPase activity.

### (4) BRM Depletion Induces Senescence in BRG1-Deficient Cancer Cells

To examine the long-term effects of BRM depletion, the present inventors prepared NCI-H1299 (BRG1-deficient) cells and HeLa (BRG1-proficient) cells expressing either a BRM-targeting shRNA inducible by adding doxycycline (NCI-H1299-shBRM and HeLa-shBRM) (Wiznerowicz M, et al. J Virol. 2003; 77: 8957-61, Bruno T, et al. Cancer Cell. 2010; 18: 122-34) or a non-targeting shRNA (NCI-H1299-shControl and HeLa-shControl). To confirm the effects of BRM knockdown, the present inventors used a different BRM target site from the one used in the siRNA experiments. The results of the clonogenic survival assay were confirmed using this system. Next, examined was the mechanism underlying growth inhibition in NCI-H1299-shBRM cells. Seven days after dox treatment, NCI-H1299-shBRM cells, but not HeLa-shBRM cells, exhibited G1 arrest with a flattened morphology, suggesting senescence (Fig. 4A). Biomarkers of senescence (Roninson IB. Cancer Res. 2003; 63: 2705-15, Zhang R, Adams PD. Cell Cycle. 2007; 6: 784-9) (p21/CDKN1A expression, SA-β-gal staining, and SAHF) were observed in dox-treated NCI-H1299-shBRM cells, but not in HeLa-shBRM cells (Fig. 4B, C). Dox treatment did not affect the proportion of cells in sub-G1-phase or the number of cells exhibiting distinct abnormal nuclear lobation. This suggests that ablation of BRM does not induce apoptosis or mitotic catastrophe (Al-Ejeh F, et al. Oncogene. 2010; 29: 6085-98) (Fig. 4A, C). Senescence is an irreversible state of growth arrest that is maintained by p21 (Ohtani N, et al. Nature. 2001; 409: 1067-70); therefore, the present inventors examined p21 expression in NCI-H1299-shBRM cells in which BRM was re-expressed after dox removal (Fig. 4D). In these cells, p21 expression was maintained at the level observed in BRM knockdown cells. Consistent with this, cells re-expressing BRM exhibited suppressed clonogenic growth activity. Meanwhile, the BRM knockdown caused significant increases in SA-β-gal positivity and the number of cells in G1-phase in three BRG1-deficient NSCLC cell lines (NCI-H1299, A549, and NCI-H157), but not in three BRG1-proficient NSCLC cell lines (NCI-H520, LK2, andNCI-H460) (Fig. 4E, supplementary Fig. S7). These results suggest that BRM knockdown specifically suppresses the growth of BRG1-deficient cells by inducing senescence.

### (5) BRM-Dependent Growth of BRG1-Deficient Cancer Cells In Vivo

The number of BRM-knockdown NCI-H1299-shBRM cells was approximately 1,000 times as small as that of non-depleted cells after 4 weeks of culture, whereas the number of HeLa-shBRM cells did not change (Fig. 5A). This observation suggests that inhibition of BRM specifically suppresses the long-term growth of BRG1-deficient cells (i.e., over at least one month). To further confirm these data under more physiologically relevant conditions, the present inventors introduced NCI-H1299-shBRM cells and NCI-H1299-shControl cells into an *in vivo* conditional RNAi model (Fig. 5B). BALB/c-nu/nu mice were injected subcutaneously with NCI-H1299-shBRM cells or NCI-H1299-shControl cells. After 3 weeks, when the injected cells had formed tumor nodules measuring approximately 100 mm³, the animals were randomly divided into two groups and fed a diet containing dox (200 ppm) or a control diet for 1 month. In dox-fed mice, the NCI-H1299-shBRM xenografts were significantly smaller than the NCI-H1299-shControl xenografts.

### (Example 2) Development of Screening Method for BRM Inhibitor

### 1. Preparation of BRM/BRG1 Protein Complex Baculovirus

Recombinant baculovirus were prepared using HighFive cells with a flashBAC ULTRA system, and the human BRM gene (Kazusa Clone pFN21AE0797), the human BRG1 gene (NBRC clone BRHIP3033650), the human BAF170 gene (Kazusa Clone FXC01767), the human BAF155 gene (Kazusa Clone FHC11034), and the human INI1 gene (Kazusa Clone FXC03158).

### 2. Purification of BRM/BRG1 Protein Complex

HighFive cells were infected with the baculoviruses and cultured at 21°C. The culture liquid was centrifuged at 3000 rpm for 15 minutes at 4°C, and the cells were recovered. Then, washing was performed twice with a phosphate buffer containing Complete EDTA free protease inhibitor (manufactured by Roche). After the washing, the cells were suspended in a dissolution buffer (20 mM HEPES, pH 7.5, 200 mM NaCl, 1 mM DTT, 10% glycerol, Complete EDTA free protease inhibitor), and subjected to a ultrasonic disruption treatment (SoniFier (manufactured by BRANSON): 1/2 inch tip, power 5, on/off: 15 seconds/30 seconds, four times on ice). After centrifugation at 35000 rpm for 60 minutes at 4°C, the supernatant was recovered as a cell lysate. To confirm the expression of each protein complex, SDS-PAGE was performed using the cell lysate.

Utilizing the His-tag and FLAG-tag added to the recombinant protein, a protein complex was purified by the following method. To the cell lysate, Ni-NTA agarose (manufactured by Invitrogen) was added and allowed to react at 4 °C overnight. The carrier was washed with a wash buffer (25 mM Tris-HCl, pH 8.0, 600 mM NaCl, 0.05% Tween-20, 10% glycerol), and the protein was eluted using an elution buffer 1 (25 mM Tris-HCl, pH 8.0, 138 mM NaCl, 0.05% Tween-20, 10% glycerol, 500 mM imidazole). Subsequently, ANTI-FLAG M2 Agarose Affinity Gel (manufactured by Sigma-Aldrich) was added to the His-tag purified sample, and allowed to react at 4°C for 6 hours. Thereafter, the carrier was washed with a wash buffer, and the carrier was then suspended in an elution buffer 2 (25 mM Tris-HCl, pH 8.0, 138 mM NaCl, 0.05% Tween-20, 10% glycerol, 300 µg/mL 3xFLAG peptide) and allowed to react at 4 °Covernight. The reacted sample was centrifuged, and the supernatant was recovered as a BRM/BRG1 protein complex. To confirm the recovery of the purified product of each protein complex, SDS-PAGE was performed.

### 3. ATPase Assay

Twenty five nM (calculated through the concentration analysis by SDS-PAGE) of each BRM/BRG1 protein complex purified by the above-described method was incubated in an ATPase reaction solution (final concentration: 32.5 mM Tris-HCl, pH 7.5, 118 mM NaCl, 5 mM MgCl₂, 60 µg/mL BSA, 8% glycerol, 0.025% Tween-20, 56 nM pcDNA3.1, 10 nM ATP) for 3 hours at room temperature. The reaction volume was set to 20 µL. The ATP consumed in this reaction was detected using ADP-glo (manufactured by Promega) and measured with 2104 Envision Multilabel Counter (manufactured by PerkinElmer).

### [Industrial Applicability]

The present invention provides a therapeutic strategy for specifically targeting cancer cells harboring inactivating SWI/SNF mutations. The present invention has revealed that BRG1 and BRM have a synthetic-lethal relationship, and that a treatment for inhibiting BRM is a promising approach for treating a BRG1-deficient cancer having no mutation in known therapeutic target genes. Moreover, it has also been revealed that, in this therapeutic strategy, a BRM inhibitor may be administered to a cancer patient after the selection based on BRG1 function suppression, hence enabling an efficient treatment based on the companion diagnosis. Thus, the present invention can greatly contribute to the medical field, particularly, in the field of cancer treatment.

### [Sequence Listing Free Text]

SEQ ID NOs: 7 and 8
<223> shBRM oligo sequences

## Claims

1. A method for evaluating a cancer, the method comprising detecting whether or not a BRG1 function is suppressed in a biological sample derived from a cancer patient.

2. A method for predicting responsiveness to a cancer treatment with a compound capable of inhibiting BRM, the method comprising:
detecting whether or not a BRG1 function is suppressed in a biological sample derived from a cancer patient; and
determining that the patient, from which the BRG1 function suppression is detected, responds to the cancer treatment with the compound capable of inhibiting BRM.

3. A method for selecting a target of a cancer treatment with a compound capable of inhibiting BRM:
detecting whether or not a BRG1 function is suppressed in a biological sample derived from a cancer patient; and
selecting the patient, from which the BRG1 function suppression is detected, as the target of the cancer treatment with the compound capable of inhibiting BRM.

4. A method for treating a cancer having a suppressed BRG1 function, the method comprising administering a compound capable of inhibiting BRM to a patient from which BRG1 function suppression is detected.

5. A method for treating a cancer having a suppressed BRG1 function, the method comprising:
detecting whether or not a BRG1 function is suppressed in a biological sample derived from a cancer patient; and
administering a compound capable of inhibiting BRM to the patient from which the BRG1 function suppression is detected.

6. A reagent for detecting whether or not a BRG1 function is suppressed in the method according to any one of claims 1 to 3 and 5, the reagent comprising as an active ingredient any one of the following molecules (a) to (c):
(a) an oligonucleotide primer capable of specifically binding to a BRG1 gene;
(b) an oligonucleotide probe capable of specifically binding to a BRG1 gene; and
(c) an antibody capable of specifically binding to a BRG1 protein.

7. A screening method for a compound used in a treatment against a cancer having a suppressed BRG1 function, the method comprising the step of selecting the compound based on whether or not BRM is inhibited.
